(19) Europäisches Patentamt
European Patent Office
Office européen des brevets

(11) Veröffentlichungsnummer: **0 370 332 B1**

(12) **EUROPÄISCHE PATENTSCHRIFT**

(45) Veröffentlichungstag der Patentschrift: **30.03.94**

(21) Anmeldenummer: **89120848.0**

(22) Anmeldetag: **10.11.89**

Die Akte enthält technische Angaben, die nach dem Eingang der Anmeldung eingereicht wurden und die nicht in dieser Patentschrift enthalten sind.

(51) Int. Cl.5: **C07D 231/12**, C07D 231/54, C07D 231/56, C07D 231/16, C07D 271/07, C07D 249/12, C07D 471/04, C07D 405/12, C07D 407/12, C07C 255/66, A01N 43/56

# (54) N-Aryl-Stickstoffheterocyclen, Verfahren sowie neue Zwischenprodukte zu ihrer Herstellung und ihre Verwendung als Herbizide und Pflanzenwuchsregulatoren.

(30) Priorität: **23.11.88 DE 3839480**

(43) Veröffentlichungstag der Anmeldung:
**30.05.90 Patentblatt 90/22**

(45) Bekanntmachung des Hinweises auf die Patenterteilung:
**30.03.94 Patentblatt 94/13**

(84) Benannte Vertragsstaaten:
**BE CH DE FR GB IT LI NL**

(56) Entgegenhaltungen:
**EP-A- 0 138 527**
**EP-A- 0 200 872**
**US-A- 4 124 374**

(73) Patentinhaber: **BAYER AG**

**D-51368 Leverkusen(DE)**

(72) Erfinder: **Fischer, Reiner, Dr.**
**Nelly-Sachs-Strasse 23**
**D-4019 Monheim(DE)**
Erfinder: **Jensen-Korte, Uta, Dr.**
**Geibelstrasse 9**
**D-4000 Düsseldorf 1(DE)**
Erfinder: **Kunisch, Franz, Dr.**
**Zum Hahnenberg 20**
**D-5068 Odenthal-Glöbusch(DE)**
Erfinder: **Marhold, Albrecht, Dr.**
**Carl-Duisberg-Strasse 329**
**D-5090 Leverkusen 1(DE)**
Erfinder: **Ooms, Pieter, Dr.**
**Doerperhofstrasse 16**
**D-4150 Krefeld 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 370 332 B1

**Chemical Abstracts, Band 105, N 25, 22. Dezember 1986, Columbus,Oh io, USA YANAGI MIKIO et al. "Preparation of (nitrophenyl) pyrazoles as herbicides" Seite 791, Spalte 1 , Zusammenfassung- N . 226556j & Jpn. Kokai Tokkyo Koho JP 61165373 (86165373)**

Erfinder: **Schallner, Otto, Dr.**
**Noldeweg 22**
**D-4019 Monheim(DE)**
Erfinder: **Santel, Hans-Joachim, Dr.**
**Grünstrasse 9a**
**D-5090 Leverkusen 1(DE)**
Erfinder: **Schmidt, Robert R., Dr.**
**Im Waldwinkel 110**
**D-5060 Bergisch-Gladbach 2(DE)**
Erfinder: **Krauskopf, Birgit, Dr.**
**Kicke 19**
**D-5060 Bergisch-Gladbach 1(DE)**
Erfinder: **Strang, Harry, Dr.**
**Heiderweg 53**
**D-4000 Düsseldorf 31(DE)**

**Beschreibung**

Die Erfindung betrifft neue N-Aryl-Stickstoffheterocyclen, mehrere Verfahren sowie neue Zwischenprodukte zu ihrer Herstellung und ihre Verwendung als Herbizide und Pflanzenwuchsregulatoren.

Es ist bekannt, daß bestimmte N-Aryl-Stickstoffheterocyclen, wie beispielsweise die Verbindung 1-(2-Chlor-4-trifluormethylphenyl)-5-methyl-4-nitropyrazol herbizide Eigenschaften besitzen (vgl. z.B. EP 200 872).

Desweiteren wurden in der EP 138 527 und in CA 105, 25, 1986, 226556j Phenylpyrazole mit herbiziden Eigenschaften beschrieben.

Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Über eine pflanzenwachstumsregulierende Wirkung der vorbekannten Verbindungen ist bisher nichts bekannt.

Es wurden neue N-Aryl-Stickstoffheterocyclen der allgemeinen Formel (I),

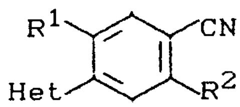

(I)

in welcher

Het für einen Heterocyclus der Formel

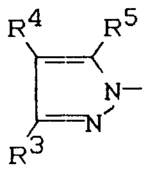 ; 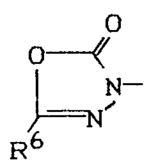 oder 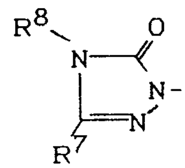

steht,

$R^1$ für Wasserstoff, Fluor, Chlor oder Brom steht und

$R^2$ für Fluor, Chlor oder Brom oder für einen Rest $-X-R^9$ steht,

wobei

$R^3$ für Wasserstoff, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht und

$R^4$ für Wasserstoff, Fluor, Chlor, Brom, Iod, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht oder

$R^3$ und $R^4$ gemeinsam für einen zweifachverknüpften Alkandiylrest mit 2 bis 6 Kohlenstoffatomen stehen,

$R^5$ für Wasserstoff, Fluor, Chlor, Brom, Iod, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$R^6$ für Wasserstoff, für jeweils geradkettiges oder verzweigtes Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Halogenalkenyl mit 3 bis 6 Kohlenstoffatomen und 1 bis 5 gleichen oder verschiedenen Halogenatomen, Halogenalkinyl mit 3 bis 6 Kohlenstoffatomen und 1 bis 5 gleichen oder verschiedenen Halogenatomen oder Alkoxyalkyl mit jeweils 1 bis 4 Kohlenstoffatomen in den einzelnen geradkettigen oder verzweigten Alkylteilen steht oder für gegebenenfalls einfach oder mehrfach, gleich oder verschieden substituiertes Cycloalkyl mit 3 bis 7 Kohlenstoffatomen steht, wobei als Substituenten infrage kommen; Halogen, sowie jeweils geradkettiges oder verzweigtes Alkyl oder Alkoxy mit jeweils 1 bis 4

Kohlenstoffatomen,

$R^7$ für Wasserstoff, für jeweils geradkettiges oder verzweigtes Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Halogenalkenyl mit 3 bis 6 Kohlenstoffatomen und 1 bis 5 gleichen oder verschiedenen Halogenatomen, Halogenalkinyl mit 3 bis 6 Kohlenstoffatomen und 1 bis 5 gleichen oder verschiedenen Halogenatomen oder Alkoxyalkyl mit jeweils 1 bis 4 Kohlenstoffatomen in den einzelnen geradkettigen oder verzweigten Alkylteilen steht oder für gegebenenfalls einfach oder mehrfach, gleich oder verschieden substituiertes Cycloalkyl mit 3 bis 7 Kohlenstoffatomen steht, wobei als Substituenten infrage kommen: Halogen, sowie jeweils geradkettiges oder verzweigtes Alkyl oder Alkoxy mit jeweils 1 bis 4 Kohlenstoffatomen und

$R^8$ für Wasserstoff oder für jeweils geradkettiges oder verzeigtes Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Halogenalkenyl mit 3 bis 6 Kohlenstoffatomen und 1 bis 5 gleichen oder verschiedenen Halogenatomen oder Halogenalkinyl mit 3 bis 6 Kohlenstoffatomen und 1 bis 5 gleichen oder verschiedenen Halogenatomen steht oder

$R^7$ und $R^8$ gemeinsam für einen zweifach verknüpften Alkandiylrest mit 2 bis 6 Kohlenstoffatomen stehen

$R^9$ für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, für Cyanalkyl, Alkoxyalkyl, Alkylthioalkyl, Halogenalkoxyalkyl, Alkoxyalkoxyalkyl, (Bis-Alkoxy)alkyl, (Bis-Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen, für gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten jeweils infrage kommen: Halogen sowie jeweils geradkettiges oder verzweigtes Alkyl oder Alkoxy mit jeweils 1 bis 4 Kohlenstoffatomen, $R^9$ außerdem für jeweils gegebenenfalls durch Alkyl mit 1 bis 4 Kohlenstoffatomen substituiertes Oxetanylalkyl, Tetrahydrofuranylalkyl, Tetrahydrofuranylalkyloxycarbonylalkyl oder Tetrahydropyranylalkyl mit jeweils 1 bis 3 Kohlenstoffatomen in den jeweiligen Alkylteilen steht oder $R^9$ für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Arylsubstituenten infrage kommen: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen und

X für Sauerstoff oder Schwefel steht.

Weiterhin wurde gefunden, daß man die neuen N-Aryl-Stickstoffheterocyclen der allgemeinen Formel (I)

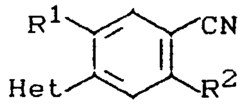

(I)

in welcher

Het, $R^1$ und $R^2$ die oben angegebene Bedeutung haben

nach einem der im Folgenden beschriebenen Verfahren erhält:

(a) Man erhält N-Aryl-Stickstoffheterocyclen der Formel (Ia),

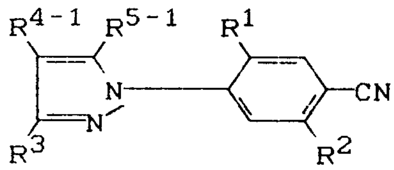

(Ia)

in welcher

$R^{4-1}$ für Wasserstoff, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht oder gemeinsam mit $R^3$ für einen zweifach verknüpften Alkandiylrest steht,

$R^{5-1}$ für Wasserstoff, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht und

$R^1$, $R^2$ und $R^3$ die oben angegebene Bedeutung haben,

wenn man 4-Cyanophenylhydrazine der Formel (II),

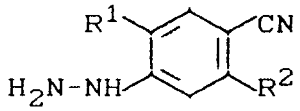

(II)

in welcher

$R^1$ und $R^2$ die oben angegebene Bedeutung haben,

mit 1,3-Diketonen der Formel (III),

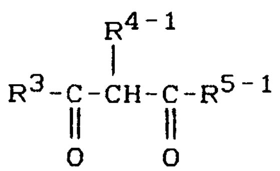

(III)

in welcher

$R^3$, $R^{4-1}$ und $R^{5-1}$ die oben angegebene Bedeutung haben, oder mit Derivaten dieser Diketone, wie beispielsweise Enolethern, Enolestern, Ketalen, Enolether-Ketalen, Enaminen oder $\beta$-Halogenvinylketonen gegebenenfalls in Gegenwart eines Verdünnungsmittels und gegebenenfalls in Gegenwart eines Reaktionshilfsmittels umsetzt;

(b) man erhält N-Aryl-Stickstoffheterocyclen der Formel (Ib),

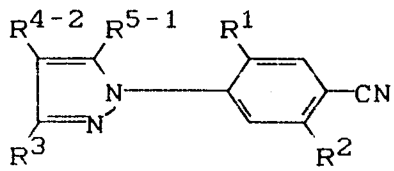

(Ib)

in welcher

$R^{4-2}$ für Halogen steht,

$R^{5-1}$ für geradkettiges oder verzweigtes Alkyl mit 1 bis 6 Kohlenstoffatomen oder für

geradkettiges oder verzweigtes Halogenalkyl mit 1 bis 4 Kohlenstoffatomen und 1 bis 9 gleichen oder verschiedenen Halogenatomen steht und

$R^1$, $R^2$ und $R^3$ die oben angegebene Bedeutung haben,

wenn man N-Aryl-Stickstoffheterocyclen der Formel (Ij),

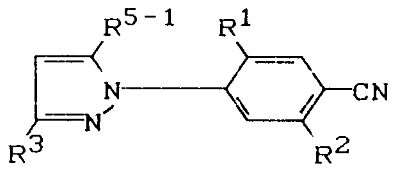 (Ij)

in welcher

$R^1$, $R^2$, $R^3$ und $R^{5-1}$ die oben angegebene Bedeutung haben,

mit einem Halogenierungsmittel gegebenenfalls in Gegenwart eines Verdünnungsmittels umsetzt;

(c) man erhält N-Aryl-Stickstoffheterocyclen der Formel (Ic),

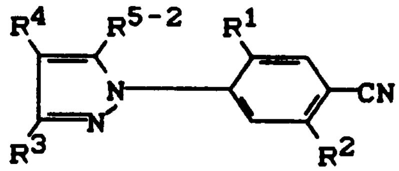 (Ic)

in welcher

$R^{5-2}$ für Halogen steht und

$R^1$, $R^2$, $R^3$ und $R^4$ die oben angegebene Bedeutung haben,

wenn man N-Aryl-pyrazolinone der Formel (IV),

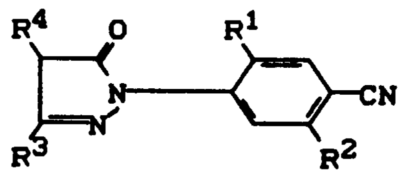 (IV)

in welcher

$R^1$, $R^2$, $R^3$ und $R^4$ die oben angegebene Bedeutung haben,

mit einem Halogenierungsmittel, gegebenenfalls in Gegenwart eines Verdünnungsmittels und gegebenenfalls in Gegenwart eines Reaktionshilfsmittels umsetzt;

(d) man erhält N-Aryl-Stickstoffheterocyclen der Formel (Id),

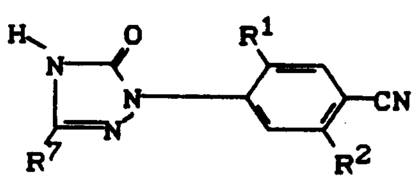 (Id)

in welcher

$R^1$, $R^2$ und $R^7$ die oben angegebene Bedeutung haben,

wenn man 4-Cyanophenylhydrazine der Formel (II),

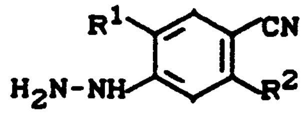

(II)

in welcher
$R^1$ und $R^2$ die oben angegebene Bedeutung haben,
mit Iminocarbonestern der Formel (V),

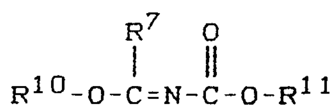

(V)

in welcher
$R^{10}$ und $R^{11}$ unabhängig voneinander jeweils für geradkettiges oder verzweigtes Alkyl mit 1 bis 4 Kohlenstoffatomen stehen und
$R^7$ die oben angegebene Bedeutung hat,
gegebenenfalls in Gegenwart eines Verdünnungsmittels umsetzt;
(e) man erhält N-Aryl-Stickstoffheterocyclen der Formel (Ie),

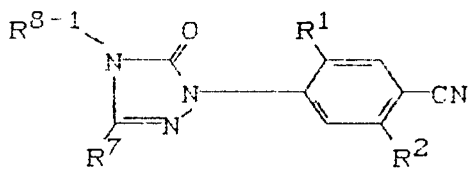

(Ie)

in welcher
$R^{8-1}$ jeweils geradkettiges oder verzweigtes Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Halogenalkenyl mit 3 bis 6 Kohlenstoffatomen und 1 bis 5 gleichen oder verschiedenen Halogenatomen oder Halogenalkinyl mit 3 bis 6 Kohlenstoffatomen und 1 bis 5 gleichen oder verschiedenen Halogenatomen steht und
$R^1$, $R^2$ und $R^7$ die oben angegebene Bedeutung haben,
wenn man N-Aryl-Stickstoffheterocyclen der Formel (Id),

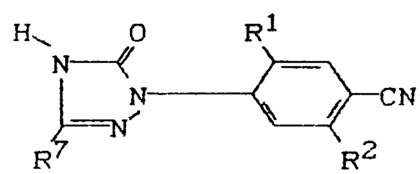

(Id)

in welcher
$R^1$, $R^2$ und $R^7$ die oben angegebene Bedeutung haben,
mit Alkylierungsmitteln der Formel (VI),

$R^{8-1}$-$E^1$ (VI)

in welcher
$R^{8-1}$ die oben angegebene Bedeutung hat und
$E^1$ für Halogen steht,

gegebenenfalls in Gegenwart eines Verdünnungsmittels und gegebenenfalls in Gegenwart eines Reaktionshilfsmittels umsetzt;

(f) man erhält N-Aryl-Stickstoffheterocyclen der Formel (If),

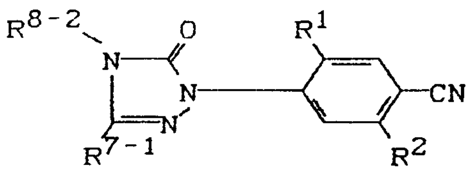

(If)

in welcher

$R^{7-1}$ und $R^{8-2}$ gemeinsam für einen zweifach verknüpften Alkandiylrest mit 2 bis 6 Kohlenstoffatomen stehen,

wenn man Amidrazone der Formel (VII),

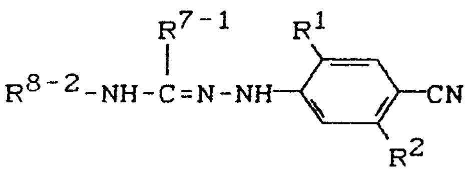

(VII)

in welcher

$R^{7-1}$ und $R^{8-2}$ die oben angegebene Bedeutung haben,

mit Phosgen gegebenenfalls in Gegenwart eines Verdünnungsmittels und gegebenenfalls in Gegenwart eines Reaktionshilfsmittels umsetzt;

(g) man erhält N-Aryl-Stickstoffheterocyclen der Formel (Ig),

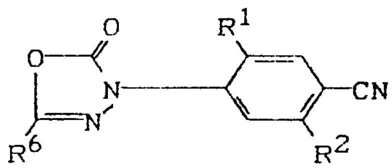

(Ig)

in welcher

$R^1$, $R^2$ und $R^6$ die oben angegebene Bedeutung haben,

wenn man Phenylhydrazide der Formel (VIII),

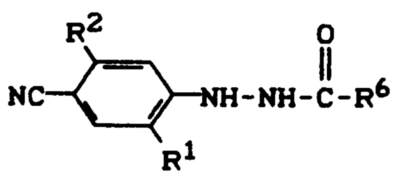

(VIII)

in welcher

$R^1$, $R^2$ und $R^6$ die oben angegebene Bedeutung haben,

mit Phosgen gegebenenfalls in Gegenwart eines Verdünnungsmittels und gegebenfalls in Gegenwart eines Reaktionshilfsmittels umsetzt;

(h) man erhält N-Aryl-Stickstoffheterocyclen der Formel (Ih),

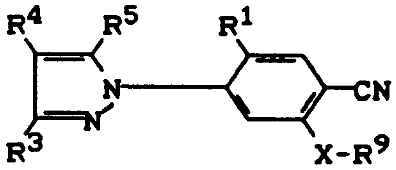

(Ih)

in welcher

$R^1$, $R^3$, $R^4$, $R^5$, $R^9$ und X die oben angegebene Bedeutung haben,

alternativ auch, wenn man

($\alpha$) N-Aryl-Stickstoffheterocyclen der Formel (Ik),

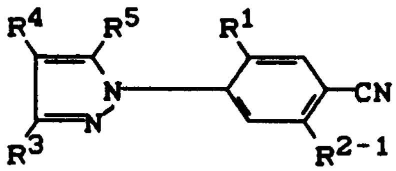

(Ik)

in welcher

$R^{2-1}$ für Halogen steht und

$R^1$, $R^3$, $R^4$ und $R^5$ die oben angegebene Bedeutung haben,

mit Alkoholen oder Thiolen der Formel (IX),

$R^9$-XH (IX)

in welcher

$R^9$ und X die oben angegebene Bedeutung haben,

gegebenenfalls in Gegenwart eines Verdünnungsmittels und gegebenenfalls in Gegenwart eines Reaktionshilfsmittels umsetzt oder wenn man

($\beta$) (Thio)Phenolderivate der Formel (X),

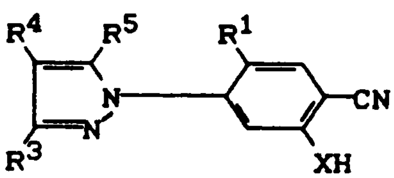

(X)

in welcher

$R^1$, $R^3$, $R^4$, $R^5$ und X die oben angegebene Bedeutung haben,

mit Alkylierungs- bzw. Acylierungsmitteln der Formel (XI),

$R^9$-$E^2$ (XI)

in welcher

$R^9$ die oben angegebene Bedeutung hat und

$E^2$ für eine elektronenanziehende Abgangsgruppe steht,

gegebenenfalls in Gegenwart eines Verdünnungsmittels und gegebenenfalls in Gegenwart eines Reaktionshilfsmittels umsetzt;

(i) man erhält N-Aryl-Stickstoffheterocyclen der Formel (Ii),

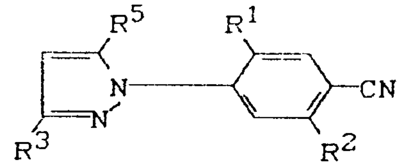
(Ii)

in welcher
$R^1$, $R^2$, $R^3$ und $R^5$ die oben angegebene Bedeutung haben,
alternativ auch, wenn man 1-Arylpyrazolyl-4-carbonsäureester der Formel (XII),

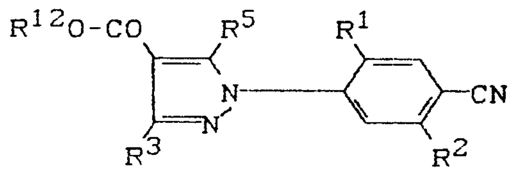
(XII)

in welcher
$R^{12}$ für geradkettiges oder verzweigtes Alkyl mit 1 bis 4 Kohlenstoffatomen steht und
$R^1$, $R^2$, $R^3$ und $R^5$ die oben angegebene Bedeutung haben,
in Gegenwart eines sauren oder basischen Katalysators und gegebenenfalls in Gegenwart eines Verdünnungsmittels verseift und anschließend thermisch decarboxyliert.

Schließlich wurde gefunden, daß die neuen N-Aryl-Stickstoffheterocyclen der allgemeinen Formel (I) herbizide und pflanzenwachstumsregulatorische Eigenschaften besitzen.

Überraschenderweise zeigen die erfindungsgemäßen N-Aryl-Stickstoffheterocyclender allgemeinen Formel (I) eine deutlich höhere herbizide Wirksamkeit gegenüber wichtigen Problemunkräutern bei einer vergleichbar guten oder besseren Kulturpflanzenselektivität im Vergleich zu den aus dem Stand der Technik bekannten N-Aryl-Stickstoffheterocyclen, wie beispielsweise die Verbindung 1-(2-Chlor-4-trifluormethylphenyl)-5-methyl-4-nitropyrazol, welche chemisch und wirkungsmäßig naheliegende Verbindungen sind.

Darüberhinaus zeigen die erfindungsgemäßen Verbindungen der Formel (I) unerwarteterweise zusätzlich eine pflanzenwachstumsregulierende Wirkung.

Die erfindungsgemäßen N-Aryl-Stickstoffheterocyclen sind durch die Formel (I) allgemein definiert. Bevorzugt sind Verbindungen der Formel (I), bei welchen
Het für eine Heterocyclus der Formel

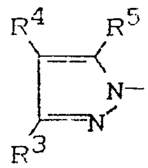 ; 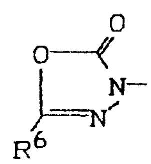 oder 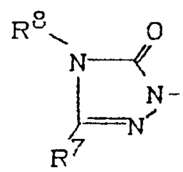

steht,
$R^1$ für Wasserstoff, Fluor oder Chlor steht und
$R^2$ für Fluor, Chlor oder für einen Rest $-X-R^9$ steht,
wobei
$R^3$ für Wasserstoff, Methyl, Ethyl, n- oder i-Propyl, n-, i-, s- oder t-Butyl, für Fluormethyl, Difluormethyl, Trifluormethyl, Chlormethyl, Dichlormethyl, Trichlormethyl, Difluorchlormethyl oder Dichlorfluormethyl steht und
$R^4$ für Wasserstoff, Fluor, Chlor, Brom, für Methyl, Ethyl, n- oder i-Propyl, n-, i-, s- oder t-Butyl, für Fluormethyl, Difluormethyl, Trifluormethyl, Chlormethyl, Dichlormethyl, Trichlormethyl, Difluorchlormethyl oder Dichlorfluormethyl steht oder

$R^3$ und $R^4$ gemeinsam für einen 1,3-Propandiylrest, einen 1,4-Butandiylrest oder einen 1,5-Pentandiylrest stehen,

$R^5$ für Wasserstoff, Fluor, Chlor, Brom, für Methyl, Ethyl, n- oder i-Propyl, n-, i-, s- oder t-Butyl, für Fluormethyl, Difluormethyl, Trifluormethyl, Chlormethyl, Dichlormethyl, Trichlormethyl, Difluorchlormethyl oder Dichlorfluormethyl steht,

$R^6$ für Wasserstoff, für Methyl, Ethyl, n- oder i-Propyl, n-, i-, s- oder t-Butyl, für jeweils einfach, zweifach oder dreifach durch Fluor und/oder Chlor substituiertes Methyl, Ethyl oder t-Butyl, für Allyl, für n- oder i-Butenyl, für Chlorallyl, für Dichlorallyl, für Propargyl, für Chlorpropargyl, für Methoxymethyl oder für jeweils gegebenenfalls ein-bis fünffach, gleich oder verschieden durch Fluor, Chlor, Methyl und/oder Methoxy substituiertes Cyclopropyl, Cyclopentyl oder Cyclohexyl steht,

$R^7$ für Wasserstoff, für Methyl, Ethyl, n- oder i-Propyl, n-, i-, s- oder t-Butyl, für jeweils einfach, zweifach oder dreifach durch Fluor und/oder Chlor substituiertes Methyl, Ethyl oder t-Butyl, für Allyl, für n- oder i-Butenyl, für Chlorallyl, für Dichlorallyl, für Propargyl, für Chlorpropargyl, für Methoxymethyl oder für jeweils gegebenenfalls ein- bis fünffach, gleich oder verschieden durch Fluor, Chlor, Methyl und/oder Methoxy substituiertes Cyclopropyl, Cyclopentyl oder Cyclohexyl steht und

$R^8$ für Wasserstoff, für Methyl, Ethyl, n- oder i-Propyl, n-, i-, s- oder t-Butyl, für jeweils einfach, zweifach oder dreifach durch Fluor und/oder Chlor substituiertes Methyl, Ethyl oder t-Butyl, für Allyl, für n- oder i-Butenyl, für Chlorallyl, für Dichlorallyl, für Propargyl oder für Chlorpropargyl steht oder

$R^7$ und $R^8$ gemeinsam für einen 1,3-Propandiylrest, einen 1,4-Butandiylrest oder einen 1,5-Pentandiylrest stehen,

$R^9$ für Methyl, Ethyl, n- oder i-Propyl, n-, i-, s-oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen, Halogenalkinyl oder Halogenalkenyl mit jeweils 3 bis 5 Kohlenstoffatomen und jeweils 1 bis 8 gleichen oder verschiedenen Halogenatomen, insbesondere Fluor, Chlor oder Brom steht, für jeweils geradkettiges oder verzweigtes Cyanalkyl, Alkoxyalkyl, Alkylthioalkyl, Halogenalkoxyalkyl, Alkoxyalkoxyalkyl, Alkylcarbonylalkyl, Alkoxycarbonylalkyl oder Alkoxyalkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl oder Cyclohexyl steht, für jeweils gegebenenfalls durch Methyl oder Ethyl substituiertes Oxetanylmethyl, Oxetanylethyl, Tetrahydrofuranylmethyl, Tetrahydrofuranylethyl, Tetrahydrofuranylmethyloxycarbonylmethyl, Tetrahydropyranylmethyl oder Tetrahydropyranylethyl steht oder für jeweils gegebenenfalls ein- bis dreifach, gleich oder verschieden substituiertes Benzyl oder Phenylethyl steht, wobei als Substituenten jeweils infrage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio und

X für Sauerstoff oder Schwefel steht.

Ganz besonders bevorzugt sind Verbindungen der Formel (I), bei welchen

Het für einen Heterocyclus der Formel

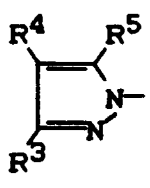 ; 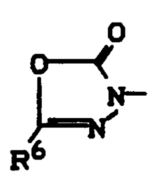 oder 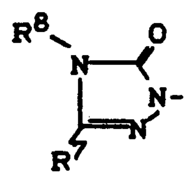

steht,

$R^1$ für Wasserstoff oder Fluor steht und

$R^2$ für Fluor oder für einen Rest -X-$R^9$ steht, wobei
$R^3$ für Wasserstoff, Methyl, Ethyl, n- oder i-Propyl, n-, i-, s- oder t-Butyl, für Fluormethyl, Difluormethyl oder Trifluormethyl steht und
$R^4$ für Wasserstoff, Chlor, Brom, Methyl oder Trifluormethyl steht oder
$R^3$ und $R^4$ gemeinsam für einen 1,3-Propandiylrest oder für einen 1,4-Butandiylrest stehen,
$R^5$ für Wasserstoff, Chlor, Brom, Methyl, Ethyl, t-Butyl oder Trifluormethyl steht,
$R^6$ für Methyl, Ethyl, n- oder i-Propyl, n-, i-, s- oder t-Butyl, für Trifluormethyl, für Fluor-1,1-dimethylethyl oder für gegebenenfalls ein-bis fünffach, gleich oder verschieden durch Fluor, Chlor oder Methyl substituiertes Cyclopropyl steht,
$R^7$ für Methyl steht und
$R^8$ für Wasserstoff, Methyl, Ethyl, Allyl, Propargyl, Fluormethyl, Difluormethyl oder Trifluormethyl steht oder
$R^7$ und $R^8$ gemeinsam für einen 1,3-Propandiylrest oder für einen 1,4-Butandiylrest stehen,
$R^9$ für Methyl, Ethyl, n- oder i-Propyl, n-, i-, s-oder t-Butyl, für Allyl, Propargyl, für jeweils geradkettiges oder verzweigtes Pentyl, Hexyl, Butenyl, Pentenyl, Hexenyl, Butinyl, Pentinyl oder Hexinyl steht, außerdem für jeweils geradkettiges oder verzweigtes Halogenalkyl mit 1 bis 4 Kohlenstoffatomen oder Halogenalkenyl mit 3 bis 5 Kohlenstoffatomen und jeweils 1 bis 5 gleichen oder verschiedenen Halogenatomen, insbesondere Fluor, Chlor oder Brom steht, für jeweils geradkettiges oder verzweigtes Cyanalkyl, Alkoxyalkyl, Alkoxyalkoxyalkyl oder Alkoxycarbonylalkyl mit jeweils 1 bis 5 Kohlenstoffatomen in den einzelnen Alkylteilen steht, außerdem für jeweils gegebenenfalls ein- bis dreifach, gleich oder verschieden durch Methyl, Methoxy, Fluor oder Chlor substituiertes Cyclopropylmethyl, Cyclopentylmethyl, Cyclohexylmethyl, Cyclopropyloxycarbonylmethyl, Cyclopentyloxycarbonylmethyl, Cyclohexyloxycarbonylmethyl, Cyclopropyl, Cyclopentyl, Cyclohexyl steht, für jeweils gegebenenfalls durch Methyl oder Ethyl substituiertes Oxetanylmethyl, Oxetanylethyl, Tetrahydrofuranylmethyl, Tetrahydrofuranylmethyloxycarbonylmethyl oder Tetrahydropyranylmethyl steht oder für jeweils gegebenenfalls ein-bis dreifach gleich oder verschieden substituiertes Benzyl oder Phenylethyl steht, wobei als Substituenten jeweils in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Ethylthio, Methoxycarbonyl, Ethoxycarbonyl, Trifluormethyl, Trifluormethoxy oder Trifluormethylthio und
X für Sauerstoff oder Schwefel steht.

Im einzelnen seien außer den bei den Herstellungsbeispielen genannten Verbindungen die folgenden N-Aryl-Stickstoffheterocyclen der allgemeinen Formel (I) genannt:

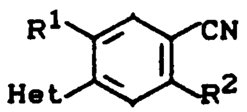

(I)

| Het | $R^1$ | $R^2$ |
|---|---|---|
| 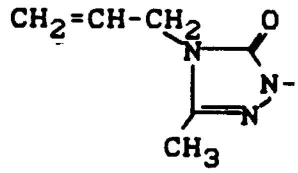 | F | 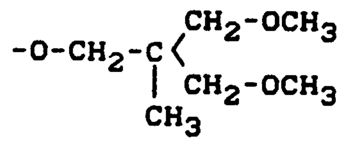 |
| 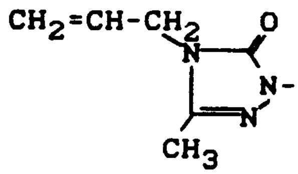 | F | 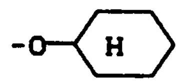 |
| 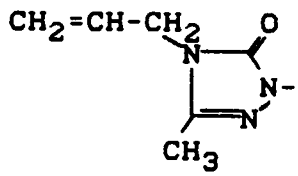 | F | 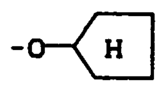 |
| 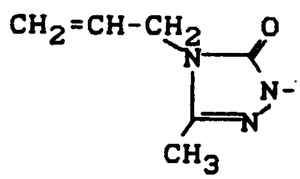 | F | 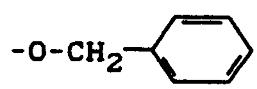 |
| 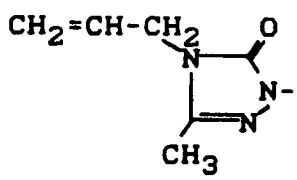 | F | $-O-CH_2-CN$ |
| 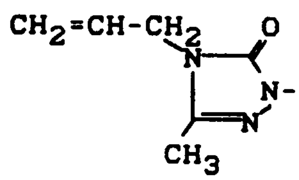 | F | $-O-CH_2-COOC_2H_5$ |
| 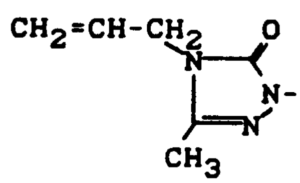 | F | 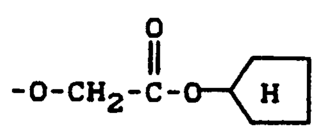 |

| Het | $R^1$ | $R^2$ |
|---|---|---|
| 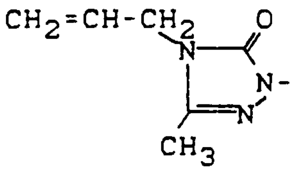 | F | 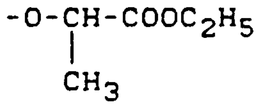 |
| 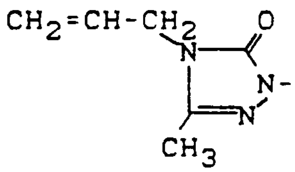 | F | 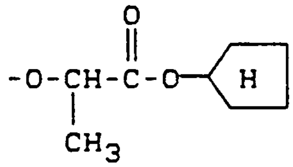 |
| 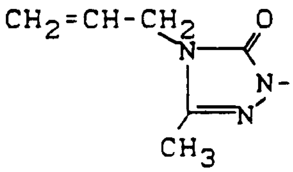 | F | 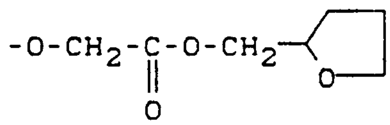 |
| 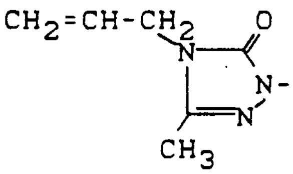 | F | 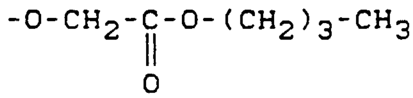 |
| 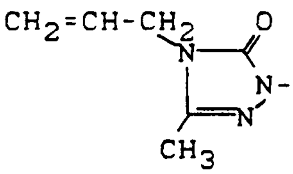 | F | 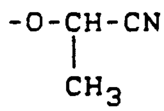 |
| 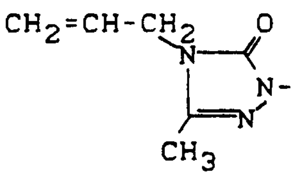 | F | $-O-CH_2-CH_2-O-CH_3$ |
| 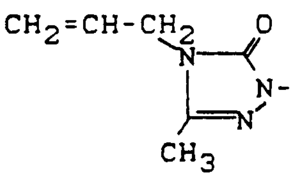 | F | 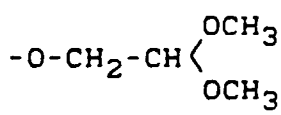 |

| Het | $R^1$ | $R^2$ |
|---|---|---|
| 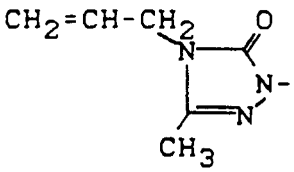 | F | 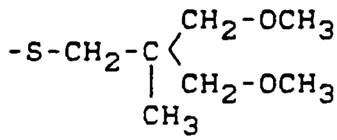 |
| 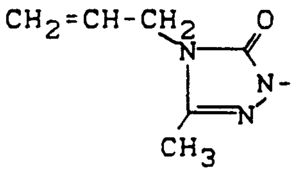 | F | 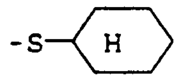 |
| 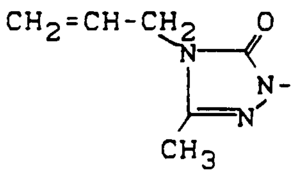 | F | 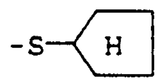 |
| 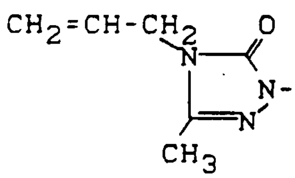 | F | 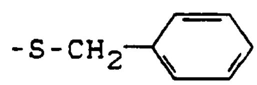 |
| 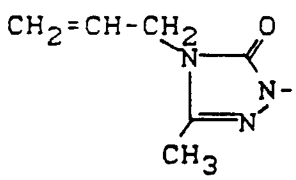 | F | $-S-CH_2-CN$ |
| 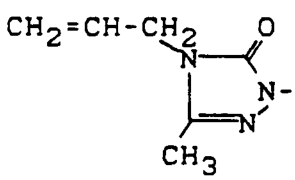 | F | $-S-CH_2-COOC_2H_5$ |
| 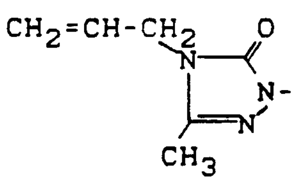 | F | 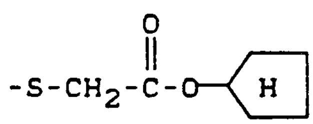 |

| Het | $R^1$ | $R^2$ |
|---|---|---|
| 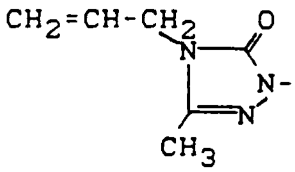 | F | 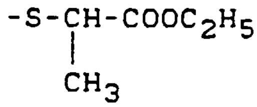 |
| 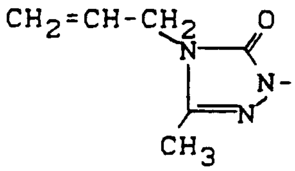 | F | 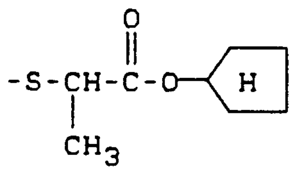 |
| 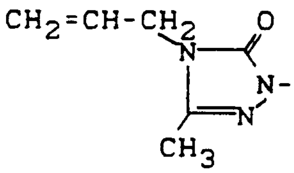 | F | 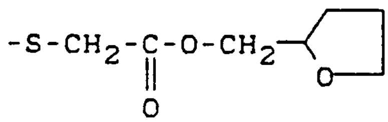 |
| 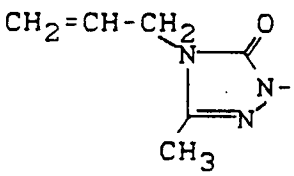 | F | 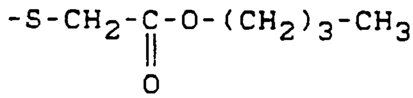 |
| 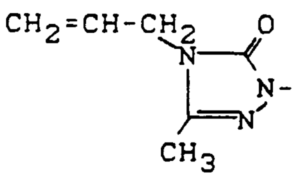 | F | 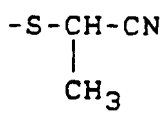 |
| 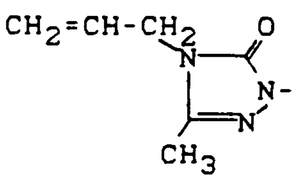 | F | $-S-CH_2-CH_2-O-CH_3$ |
| 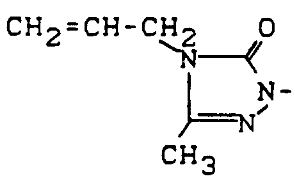 | F | 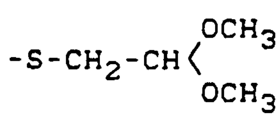 |

| Het | $R^1$ | $R^2$ |
|---|---|---|
| 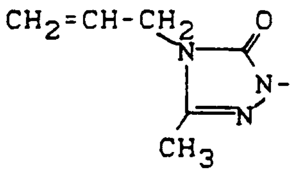 | F | $-OCH_3$ |
| 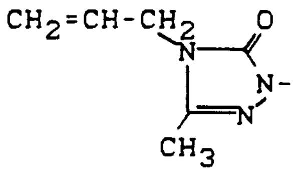 | F | $-OC_2H_5$ |
| 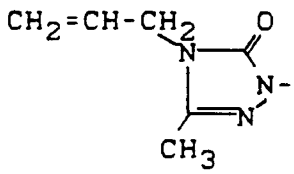 | F | $-O-CH(CH_3)_2$ |
| 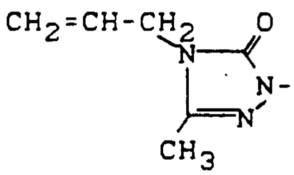 | F | $-O-CH_2-CH=CH_2$ |
| 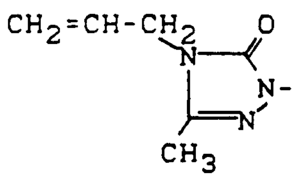 | F | $-O-CH_2-CH=CH-CH_3$ |
| 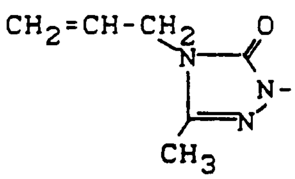 | F | $-O-CH_2-CH=CH-Cl$ |
| 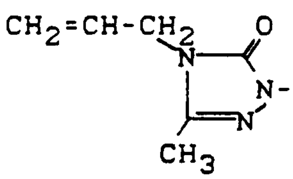 | F | $-O-CH-CH=CH_2$<br>\|<br>$CH_3$ |

| Het | $R^1$ | $R^2$ |
|---|---|---|
| 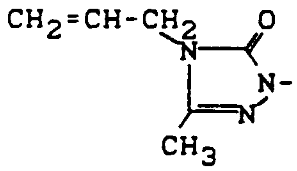 | F | $-O-CH_2-C\equiv CH$ |
| 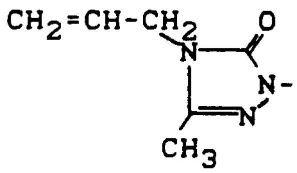 | F | 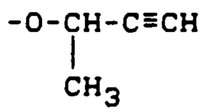 |
| 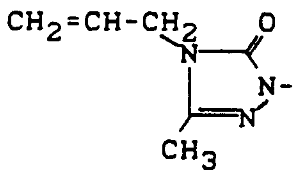 | F | 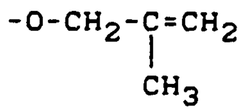 |
| 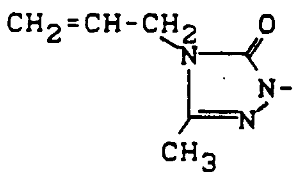 | F | $-O-CH_2-CH_2-OC_2H_5$ |
| 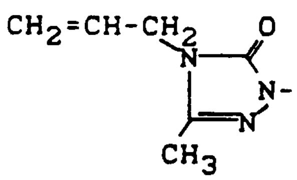 | F | 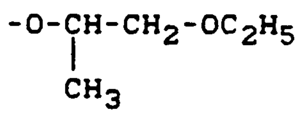 |
| 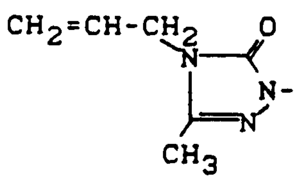 | F | 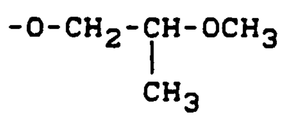 |
| 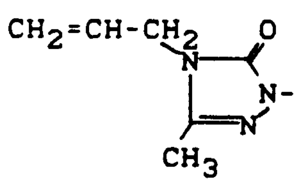 | F | 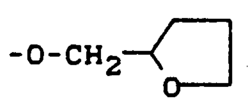 |

| Het | $R^1$ | $R^2$ |
|---|---|---|
| 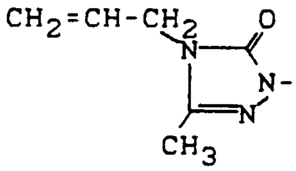 | F | 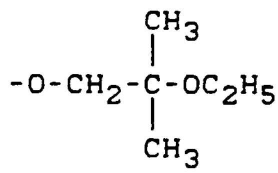 |
| 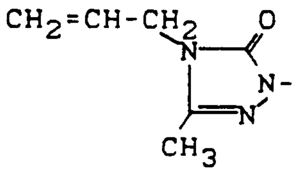 | F | $-O-CH_2-CH_2-O-CH_2-CH_2-OCH_3$ |
| 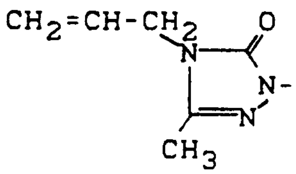 | F | $-O-CH_2-CH_2-O-CH_2-CH_2-OC_2H_5$ |
| 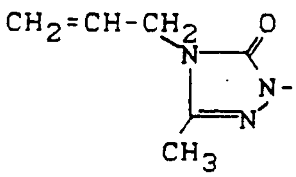 | F | 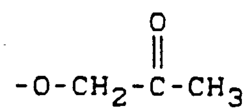 |
| 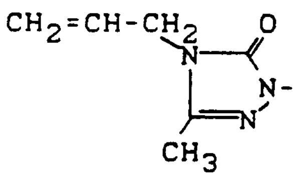 | F | 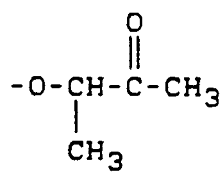 |
| 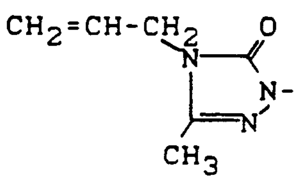 | F | $-SCH_3$ |
| 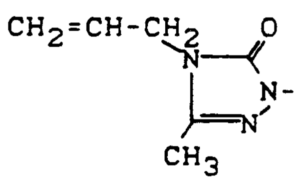 | F | $-SC_2H_5$ |

| Het | $R^1$ | $R^2$ |
|---|---|---|
| 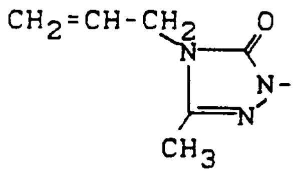 | F | $-S-CH(CH_3)_2$ |
| 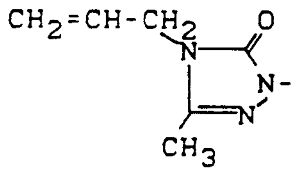 | F | $-S-CH_2-CH=CH_2$ |
| 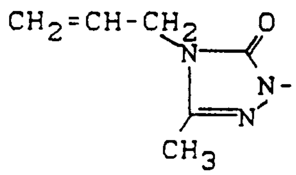 | F | $-S-CH_2-CH=CH-Cl$ |
| 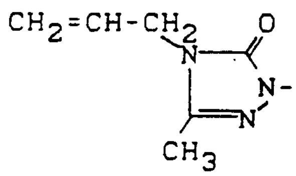 | F | $-S-CH_2-CH=CH-CH_3$ |
| 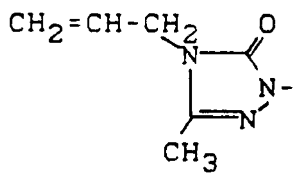 | F | $-S-\underset{CH_3}{CH}-CH=CH_2$ |
| 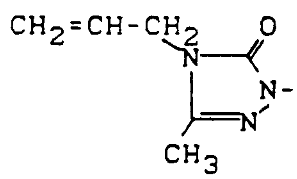 | F | $-S-CH_2-C\equiv CH$ |
| 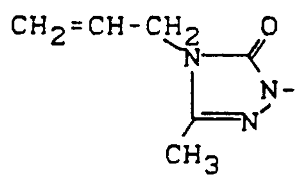 | F | $-S-\underset{CH_3}{CH}-C\equiv CH$ |

| Het | $R^1$ | $R^2$ |
|---|---|---|
| 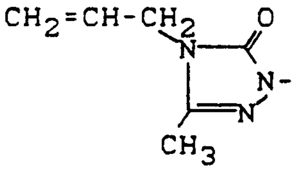 | F | 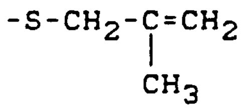 |
| 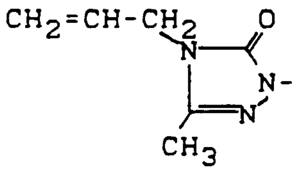 | F | $-S-CH_2-CH_2-OC_2H_5$ |
| 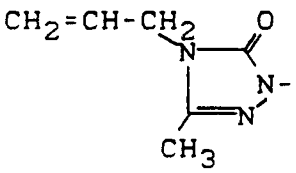 | F | 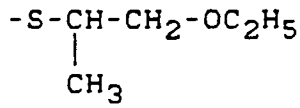 |
| 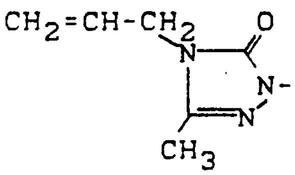 | F | 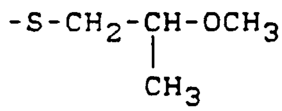 |
| 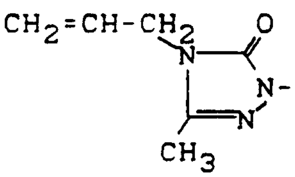 | F | 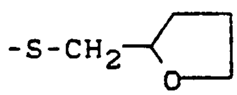 |
| 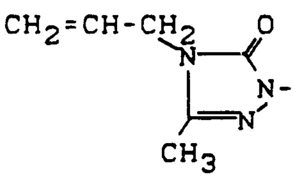 | F | 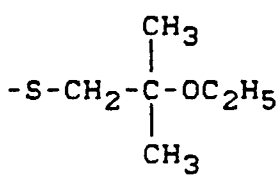 |
| 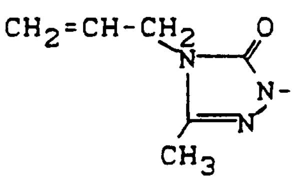 | F | $-S-CH_2-CH_2-O-CH_2-CH_2-OCH_3$ |

| Het | $R^1$ | $R^2$ |
|---|---|---|
| 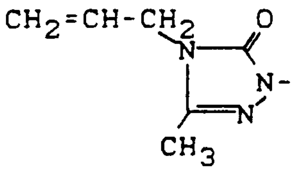 | F | $-S-CH_2-CH_2-O-CH_2-CH_2-OC_2H_5$ |
| 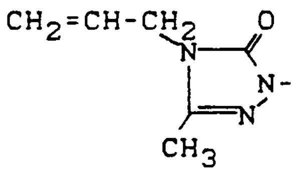 | F | 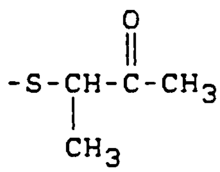 |
| 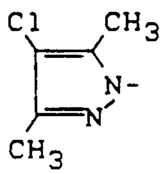 | F | 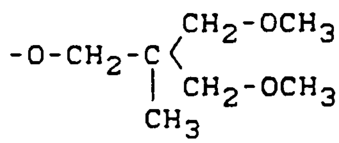 |
| 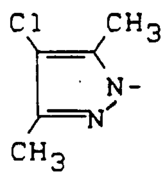 | F | 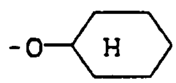 |
| 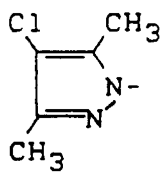 | F | 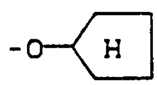 |
| 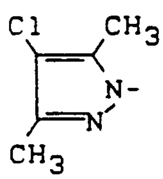 | F | 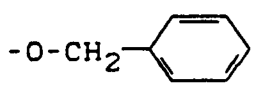 |
| 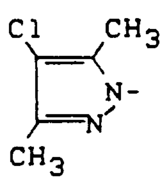 | F | $-O-CH_2-CN$ |

| Het | $R^1$ | $R^2$ |
|---|---|---|
| 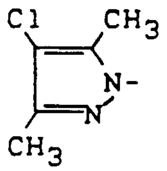 | F | $-O-CH_2-COOC_2H_5$ |
| 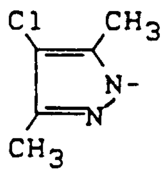 | F | 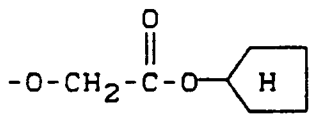 |
| 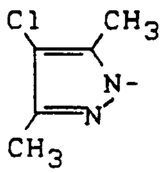 | F | 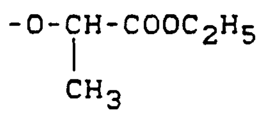 |
| 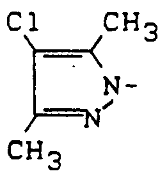 | F | 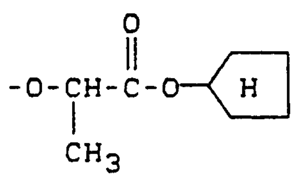 |
| 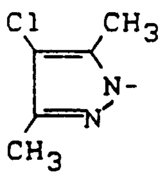 | F | 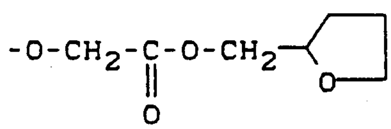 |
| 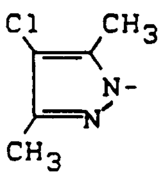 | F | 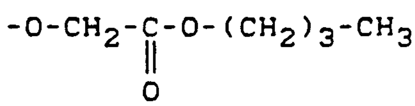 |
| 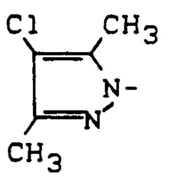 | F | 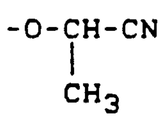 |

| Het | $R^1$ | $R^2$ |
|---|---|---|
| 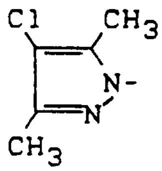 | F | $-O-CH_2-CH_2-O-CH_3$ |
| 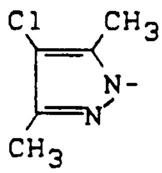 | F | 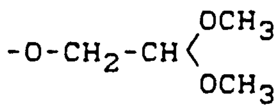 |
| 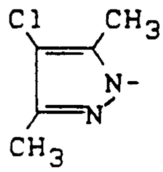 | F | 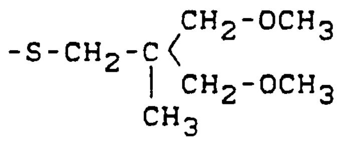 |
| 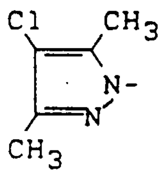 | F | -S 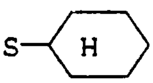 |
| 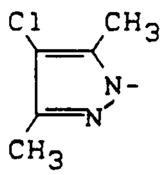 | F | -S 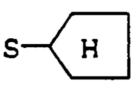 |
| 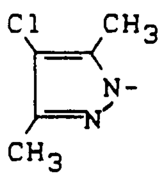 | F | $-S-CH_2$ 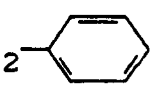 |
| 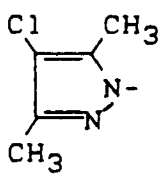 | F | $-S-CH_2-CN$ |

| Het | $R^1$ | $R^2$ |
|---|---|---|
| 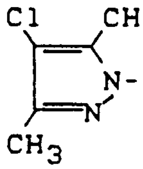 | F | $-S-CH_2-COOC_2H_5$ |
| 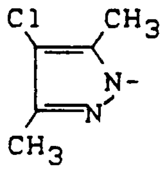 | F | 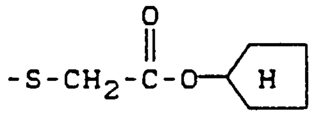 |
| 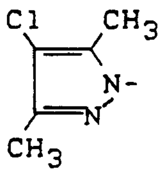 | F | 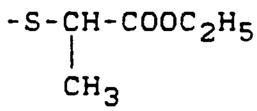 |
| 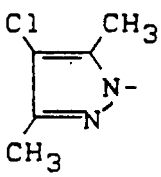 | F | 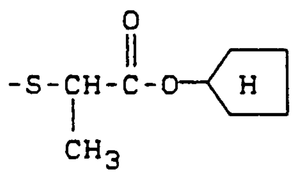 |
| 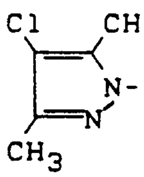 | F | 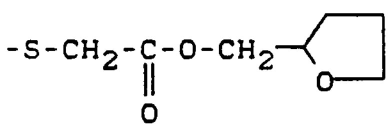 |
| 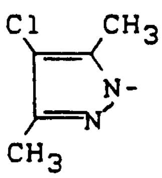 | F | 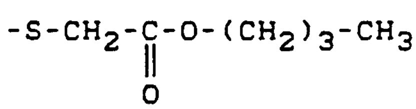 |
| 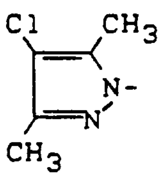 | F | 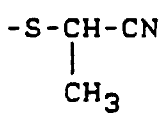 |

| Het | $R^1$ | $R^2$ |
|---|---|---|
| 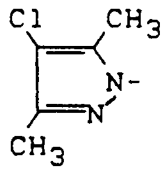 | F | $-S-CH_2-CH_2-O-CH_3$ |
| 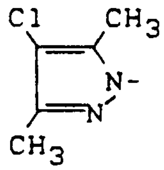 | F | $-S-CH_2-CH\left\langle\begin{matrix}OCH_3\\OCH_3\end{matrix}\right.$ |
| 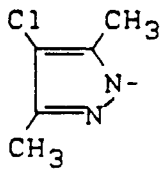 | F | $-OCH_3$ |
| 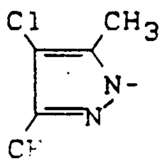 | F | $-OC_2H_5$ |
| 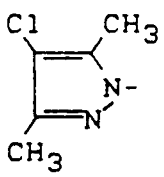 | F | $-O-CH(CH_3)_2$ |
| 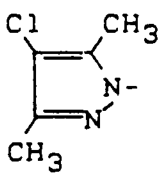 | F | $-O-CH_2-CH=CH_2$ |
| 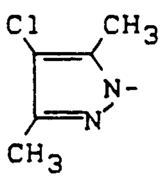 | F | $-O-CH_2-CH=CH-CH_3$ |

| Het | $R^1$ | $R^2$ |
|---|---|---|
| 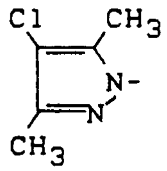 | F | $-O-CH_2-CH=CH-Cl$ |
| 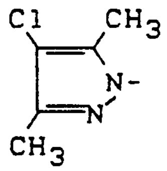 | F | 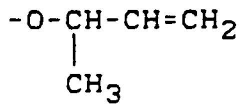 |
| 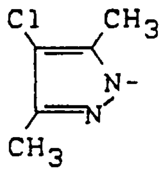 | F | $-O-CH_2-C\equiv CH$ |
| 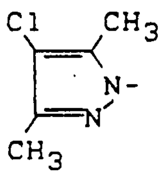 | F | 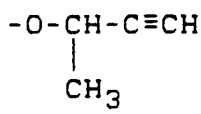 |
| 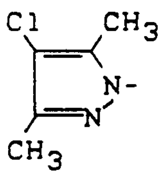 | F | 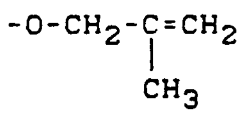 |
| 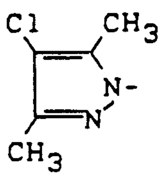 | F | $-O-CH_2-CH_2-OC_2H_5$ |
| 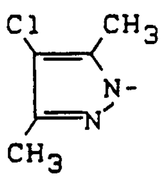 | F | 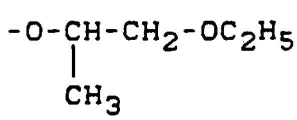 |

| Het | $R^1$ | $R^2$ |
|---|---|---|
| 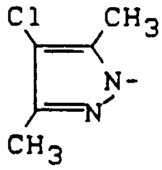 | F | 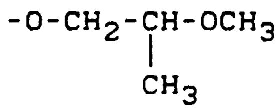 |
| 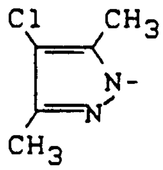 | F | 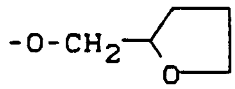 |
| 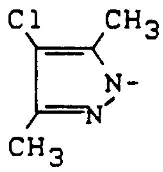 | F | 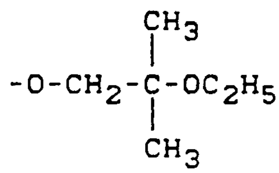 |
| 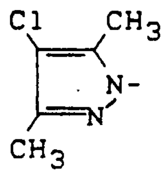 | F | $-O-CH_2-CH_2-O-CH_2-CH_2-OCH_3$ |
| 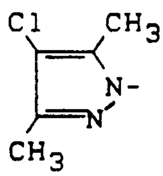 | F | $-O-CH_2-CH_2-O-CH_2-CH_2-OC_2H_5$ |
| 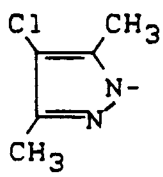 | F | 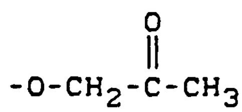 |
| 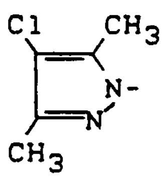 | F | 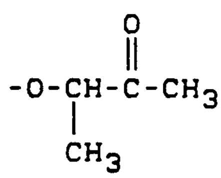 |

| Het | $R^1$ | $R^2$ |
|---|---|---|
| 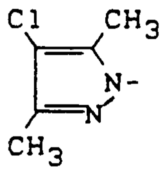 | F | $-SCH_3$ |
| 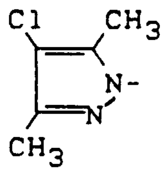 | F | $-SC_2H_5$ |
| 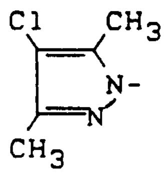 | F | $-S-CH(CH_3)_2$ |
| 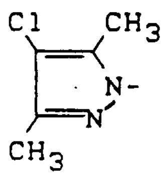 | F | $-S-CH_2-CH=CH_2$ |
| 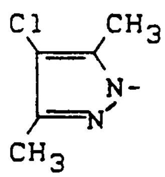 | F | $-S-CH_2-CH=CH-Cl$ |
| 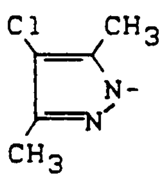 | F | $-S-CH_2-CH=CH-CH_3$ |
| 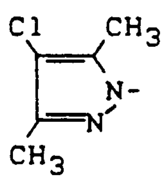 | F | $-S-CH(CH_3)-CH=CH_2$ |

| Het | $R^1$ | $R^2$ |
|---|---|---|
| 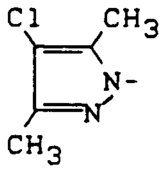 | F | $-S-CH_2-C{\equiv}CH$ |
| 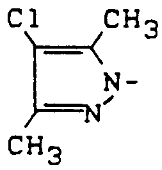 | F | 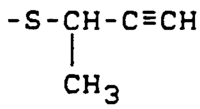 |
| 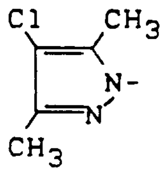 | F | 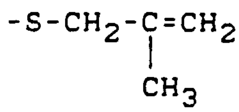 |
| 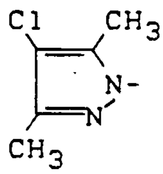 | F | $-S-CH_2-CH_2-OC_2H_5$ |
| 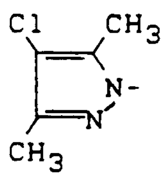 | F | 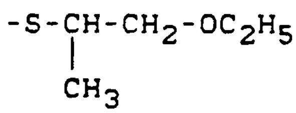 |
| 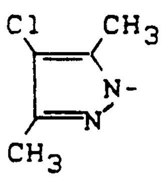 | F | 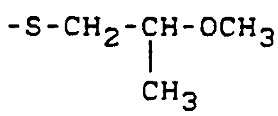 |
| 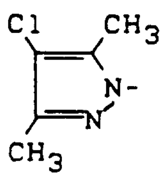 | F | 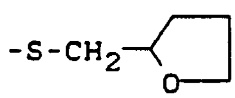 |

| Het | $R^1$ | $R^2$ |
|---|---|---|
| 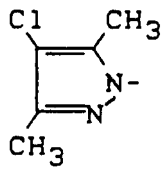 | F | 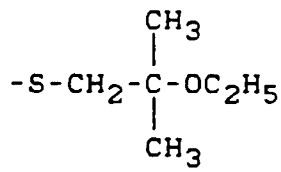 |
| 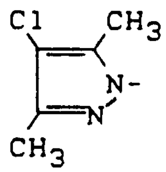 | F | $-S-CH_2-CH_2-O-CH_2-CH_2-OCH_3$ |
| 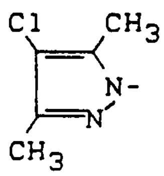 | F | $-S-CH_2-CH_2-O-CH_2-CH_2-OC_2H_5$ |
| 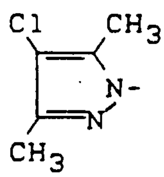 | F | 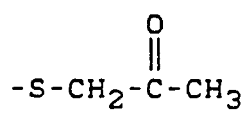 |
| 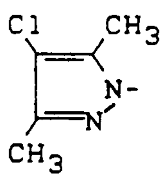 | F | 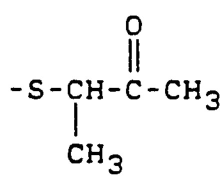 |
| 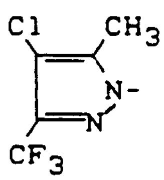 | F | 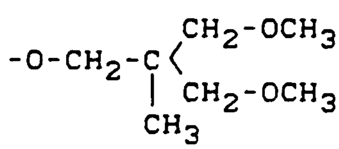 |
| 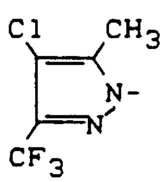 | F | 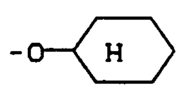 |

| Het | $R^1$ | $R^2$ |
|---|---|---|
| 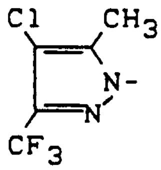 | F | 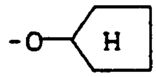 |
| 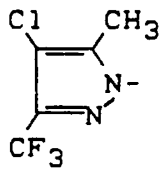 | F | 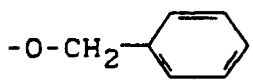 |
| 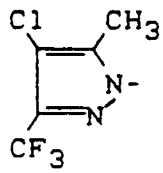 | F | $-O-CH_2-CN$ |
| 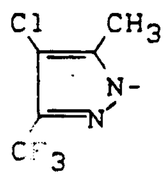 | F | $-O-CH_2-COOC_2H_5$ |
| 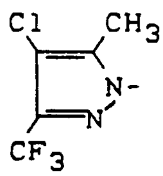 | F | 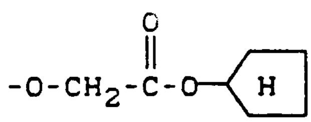 |
| 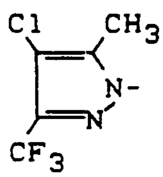 | F | $-O-\underset{CH_3}{\underset{\vert}{CH}}-COOC_2H_5$ |
| 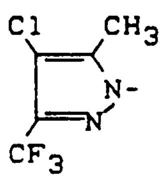 | F | 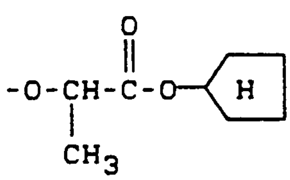 |

| Het | $R^1$ | $R^2$ |
|---|---|---|
| 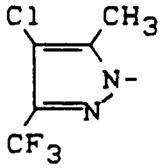 | F | 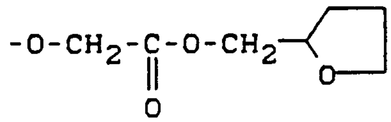 |
| 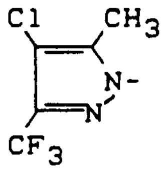 | F | 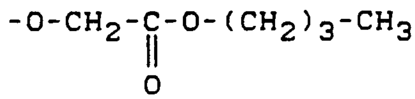 |
| 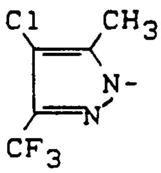 | F | 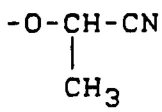 |
| 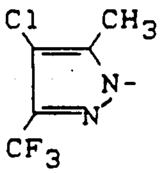 | F | $-O-CH_2-CH_2-O-CH_3$ |
| 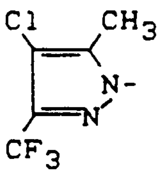 | F | 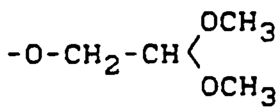 |
| 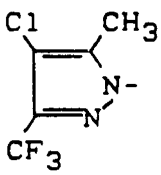 | F | 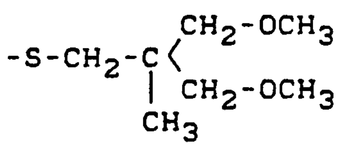 |
| 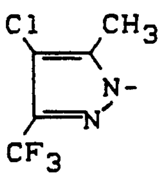 | F | 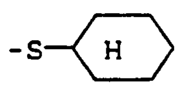 |

| Het | $R^1$ | $R^2$ |
|---|---|---|
| 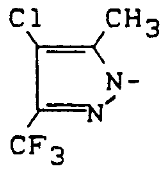 | F | 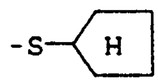 |
| 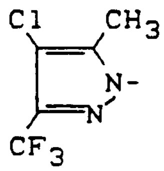 | F | 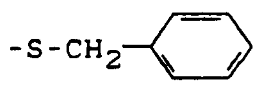 |
| 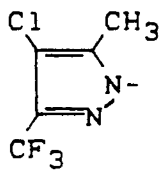 | F | $-S-CH_2-CN$ |
| 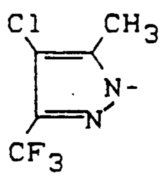 | F | $-S-CH_2-COOC_2H_5$ |
| 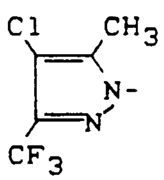 | F | 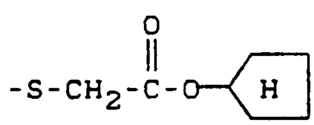 |
| 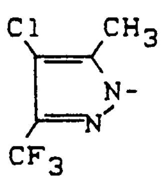 | F | 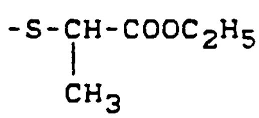 |
| 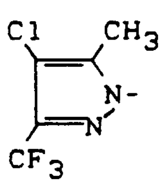 | F | 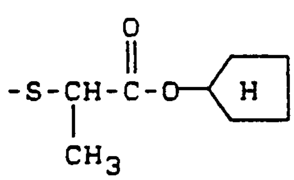 |

| Het | $R^1$ | $R^2$ |
|---|---|---|
| 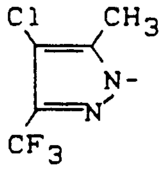 | F | 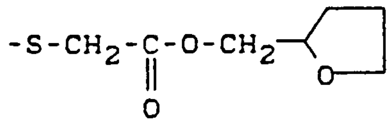 |
| 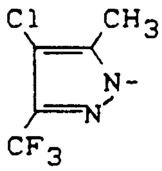 | F | 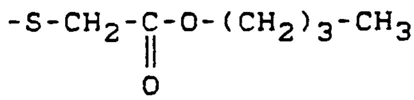 |
| 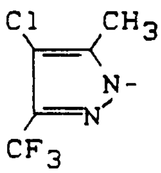 | F | 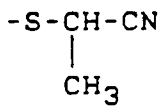 |
| 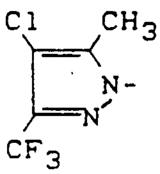 | F | $-S-CH_2-CH_2-O-CH_3$ |
| 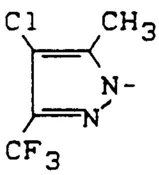 | F | 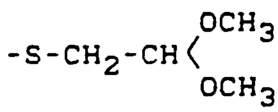 |
| 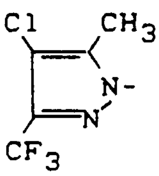 | F | $-OCH_3$ |
| 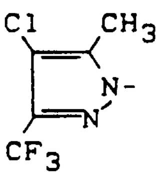 | F | $-OC_2H_5$ |

| Het | $R^1$ | $R^2$ |
|---|---|---|
| 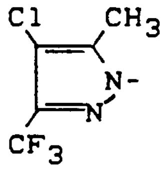 | F | $-O-CH(CH_3)_2$ |
| 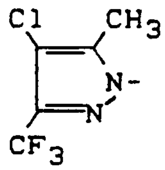 | F | $-O-CH_2-CH=CH_2$ |
| 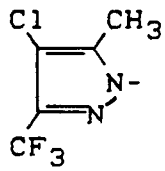 | F | $-O-CH_2-CH=CH-CH_3$ |
| 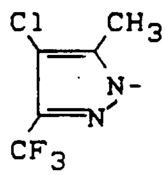 | [illegible] | $-O-CH_2-$[illegible]$-Cl$ |
| 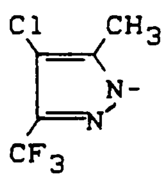 | F | $-O-\underset{CH_3}{CH}-CH=CH_2$ |
| 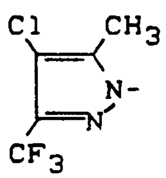 | F | $-O-CH_2-C\equiv CH$ |
| 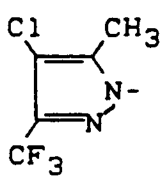 | F | $-O-\underset{CH_3}{CH}-C\equiv CH$ |

| Het | $R^1$ | $R^2$ |
|---|---|---|
| 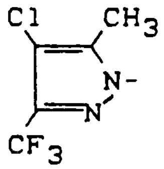 | F | 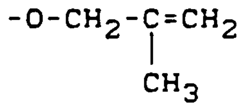 |
| 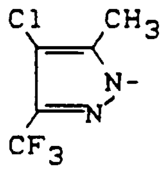 | F | $-O-CH_2-CH_2-OC_2H_5$ |
| 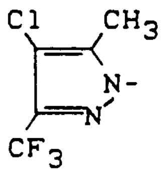 | F | 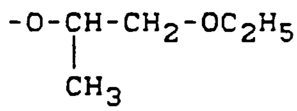 |
| 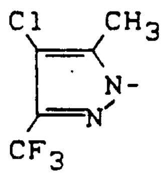 | F | 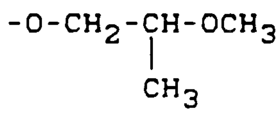 |
| 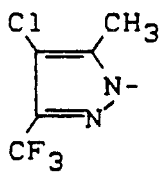 | F | 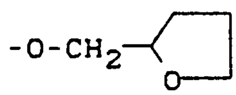 |
| 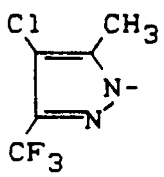 | F | 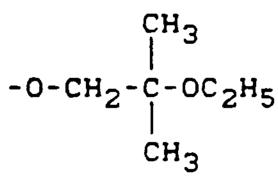 |
| 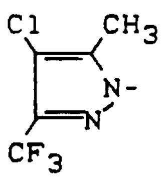 | F | $-O-CH_2-CH_2-O-CH_2-CH_2-OCH_3$ |

| Het | $R^1$ | $R^2$ |
|---|---|---|
| 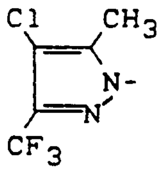 | F | $-O-CH_2-CH_2-O-CH_2-CH_2-OC_2H_5$ |
| 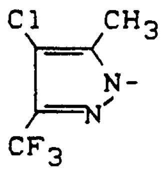 | F | 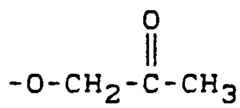 |
| 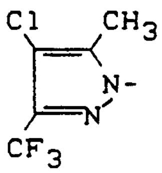 | F | 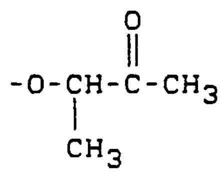 |
| 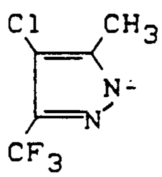 | F | $-SCH_3$ |
| 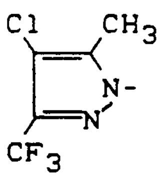 | F | $-SC_2H_5$ |
| 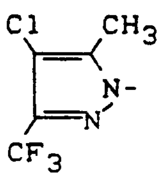 | F | $-S-CH(CH_3)_2$ |
| 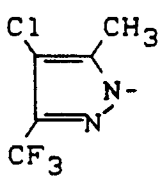 | F | $-S-CH_2-CH=CH_2$ |

| Het | $R^1$ | $R^2$ |
|---|---|---|
| 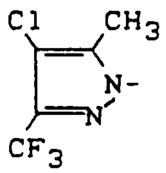 | F | $-S-CH_2-CH=CH-Cl$ |
| 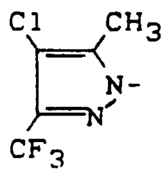 | F | $-S-CH_2-CH=CH-CH_3$ |
| 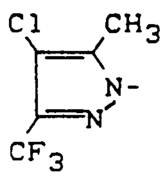 | F | $-S-CH-CH=CH_2$<br>$CH_3$ |
| 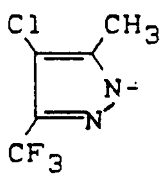 | F | $-S-CH_2-C\equiv CH$ |
| 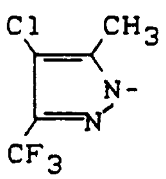 | F | $-S-CH-C\equiv CH$<br>$CH_3$ |
| 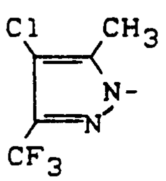 | F | $-S-CH_2-C=CH_2$<br>$CH_3$ |
| 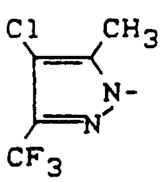 | F | $-S-CH_2-CH_2-OC_2H_5$ |

| Het | $R^1$ | $R^2$ |
|---|---|---|
| 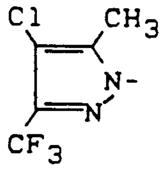 | F | 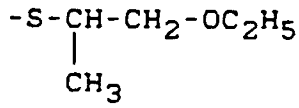 |
| 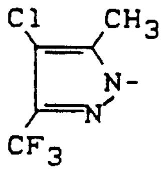 | F | 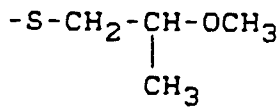 |
| 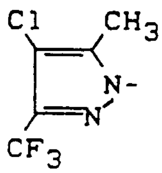 | F | 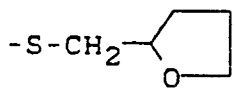 |
| 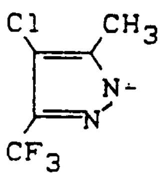 | F | 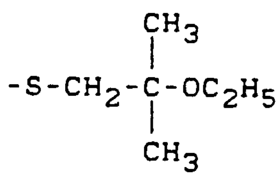 |
| 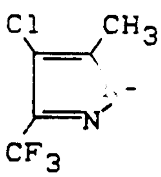 | F | $-S-CH_2-CH_2-O-CH_2-CH_2-OCH_3$ |
| 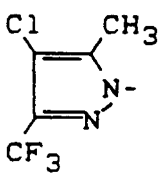 | F | $-S-CH_2-CH_2-O-CH_2-CH_2-OC_2H_5$ |
| 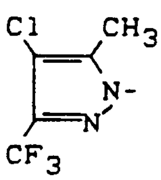 | F | 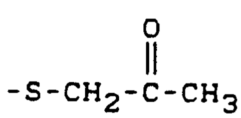 |

| Het | $R^1$ | $R^2$ |
|---|---|---|
| 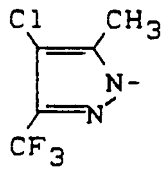 | F | 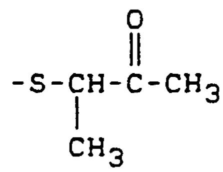 |
| 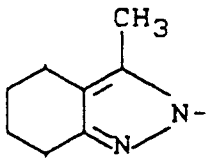 | F | 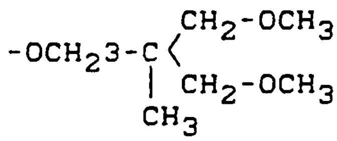 |
| 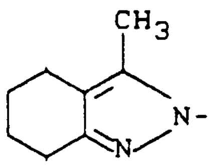 | F | 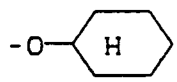 |
| 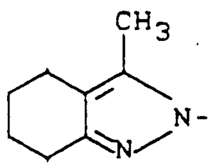 | F | 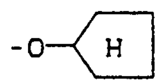 |
| 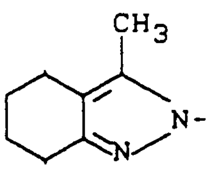 | F | 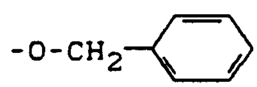 |
| 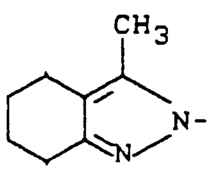 | F | $-O-CH_2-CN$ |
| 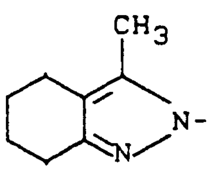 | F | $-O-CH_2-COOC_2H_5$ |

| Het | $R^1$ | $R^2$ |
|---|---|---|
| 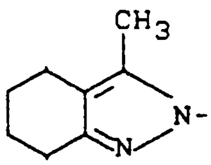 | F | 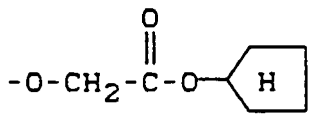 |
| 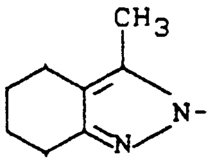 | F | 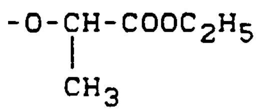 |
| 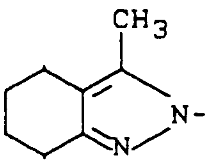 | F | 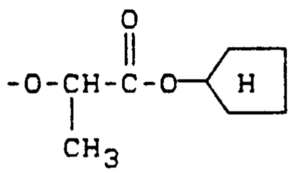 |
| 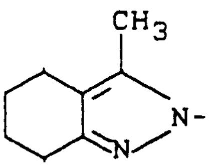 | F | 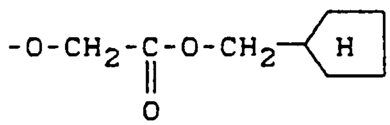 |
| 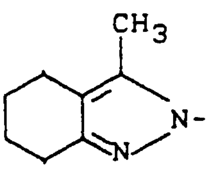 | F | 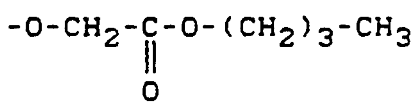 |
| 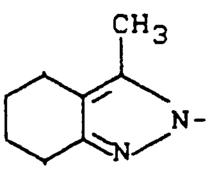 | F | 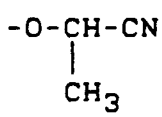 |

| Het | $R^1$ | $R^2$ |
|---|---|---|
| 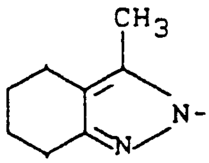 | F | $-O-CH_2-CH_2-O-CH_3$ |
| 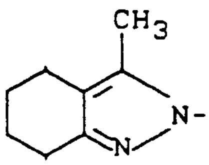 | F | 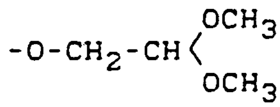 |
| 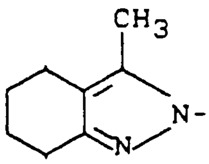 | F | 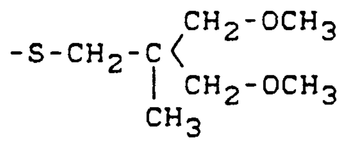 |
| 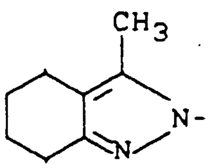 | F | -S 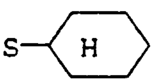 |
| 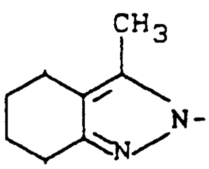 | F | -S 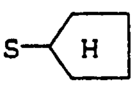 |
| 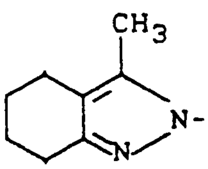 | F | $-S-CH_2$ 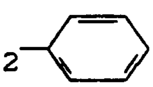 |
| 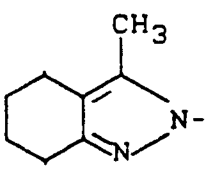 | F | $-S-CH_2-CN$ |

| Het | $R^1$ | $R^2$ |
|---|---|---|
| 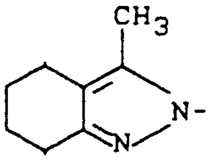 | F | $-S-CH_2-COOC_2H_5$ |
| 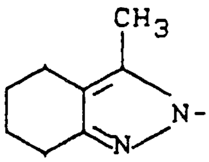 | F | 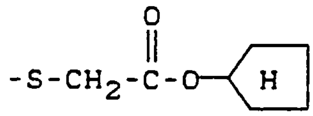 |
| 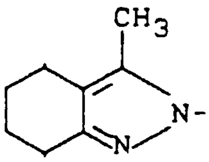 | F | 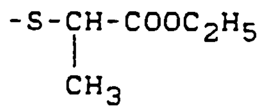 |
| 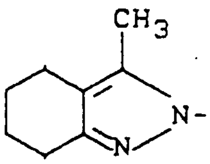 | F | 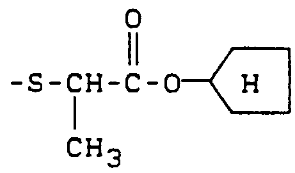 |
| 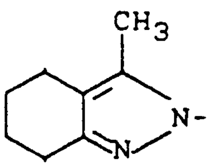 | F | 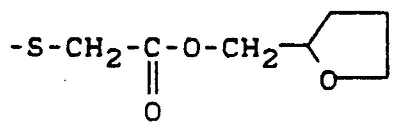 |
| 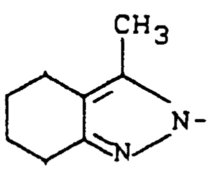 | F | 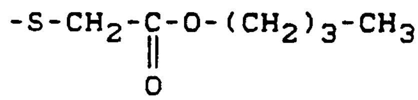 |

| Het | $R^1$ | $R^2$ |
|---|---|---|
| 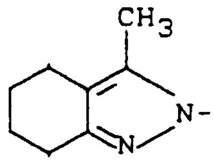 | F | $-S-CH-CN$<br>$CH_3$ |
| 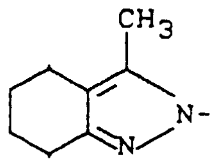 | F | $-S-CH_2-CH_2-O-CH_3$ |
| 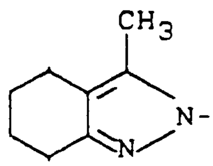 | F | $OCH_3$<br>$-S-CH_2-CH\langle$<br>$OCH_3$ |
| 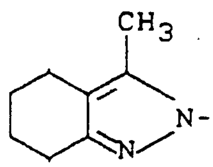 | F | $-OCH_3$ |
| 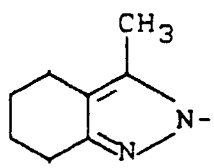 | F | $-OC_2H_5$ |
| 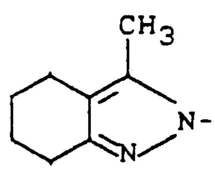 | F | $-O-CH(CH_3)_2$ |
| 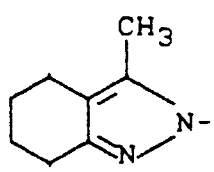 | F | $-O-CH_2-CH{\equiv}CH_2$ |

| Het | $R^1$ | $R^2$ |
|---|---|---|
| 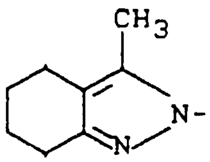 | F | $-O-CH_2-CH=CH-CH_3$ |
| 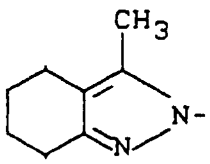 | F | $-O-CH_2-CH=CH-Cl$ |
| 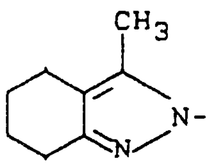 | F | $-O-\underset{CH_3}{\underset{\mid}{CH}}-CH=CH_2$ |
| 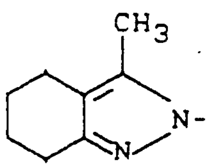 | F | $-O-CH_2-C\equiv CH$ |
| 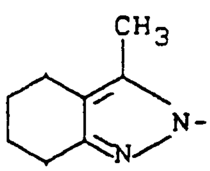 | F | $-O-\underset{CH_3}{\underset{\mid}{CH}}-C\equiv CH$ |
| 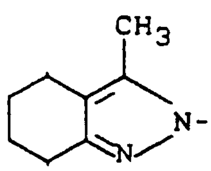 | F | $-O-CH_2-\underset{CH_3}{\underset{\mid}{C}}\equiv CH_2$ |
| 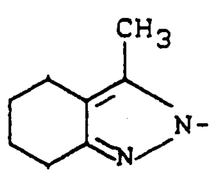 | F | $-O-CH_2-CH_2-OC_2H_5$ |

| Het | $R^1$ | $R^2$ |
|---|---|---|
| 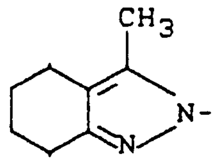 | F | 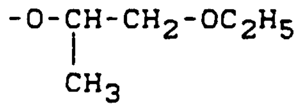 |
| 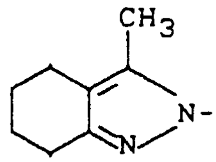 | F | 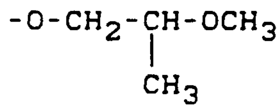 |
| 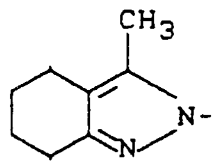 | F | 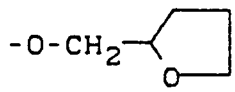 |
| 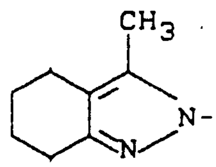 | F | 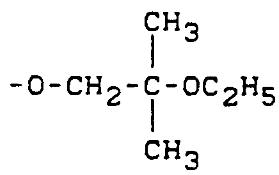 |
| 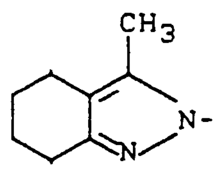 | F | $-O-CH_2-CH_2-O-CH_2-CH_2-OCH_3$ |
| 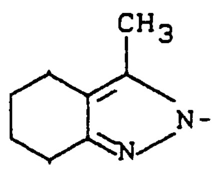 | F | $-OCH_2-CH_2-O-CH_2-CH_2-OC_2H_5$ |
| 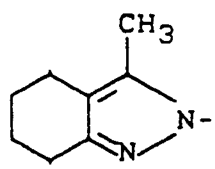 | F | 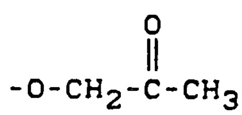 |

| Het | $R^1$ | $R^2$ |
|---|---|---|
| 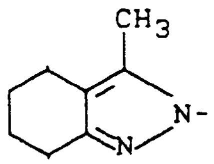 | F | 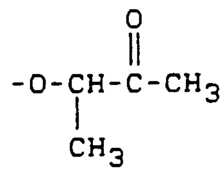 |
| 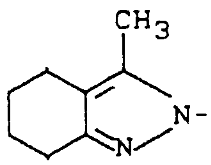 | F | $-SCH_3$ |
| 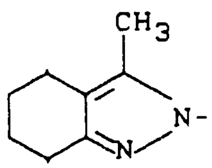 | F | $-SC_2H_5$ |
| 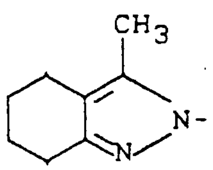 | F | $-S-CH(CH_3)_2$ |
| 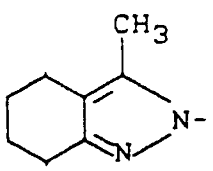 | F | $-S-CH_2-CH=CH_2$ |
| 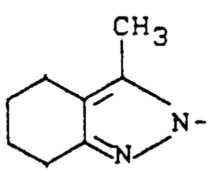 | F | $-S-CH_2-CH=CH-Cl$ |
| 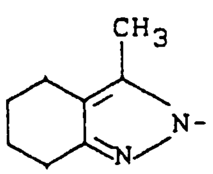 | F | $-S-CH_2-CH=CH-CH_3$ |

| Het | $R^1$ | $R^2$ |
|---|---|---|
| 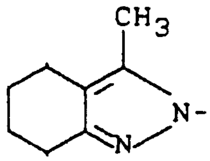 | F | $-S-CH-CH=CH_2$<br>$CH_3$ |
| 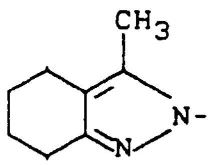 | F | $-S-CH_2-C\equiv CH$ |
| 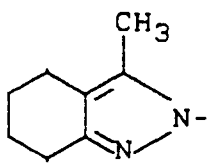 | F | $-S-CH-C\equiv CH$<br>$CH_3$ |
| 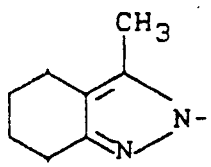 | F | $-S-CH_2-C=CH_2$<br>$CH_3$ |
| 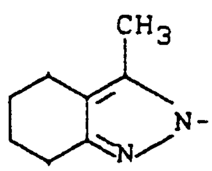 | F | $-S-CH_2-CH_2-OC_2H_5$ |
| 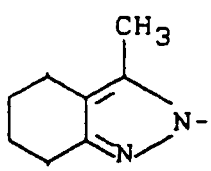 | F | $-S-CH-CH_2-OC_2H_5$<br>$CH_3$ |

| Het | $R^1$ | $R^2$ |
|---|---|---|
| 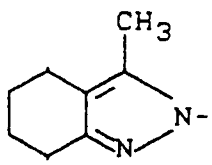 | F | 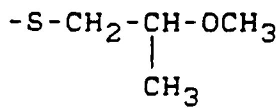 |
| 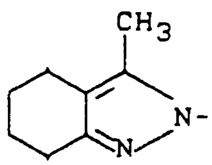 | F | 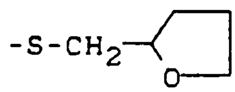 |
| 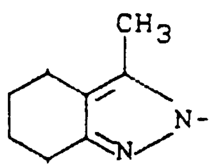 | F | 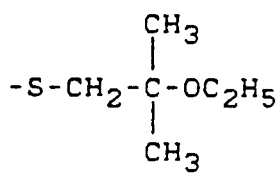 |
| 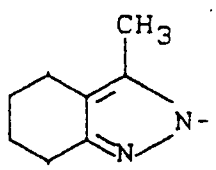 | F | $-S-CH_2-CH_2-O-CH_2-CH_2-OCH_3$ |
| 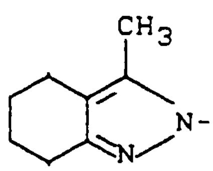 | F | $-S-CH_2-CH_2-O-CH_2-CH_2-OC_2H_5$ |
| 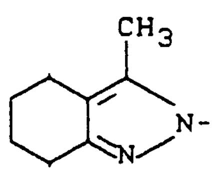 | F | 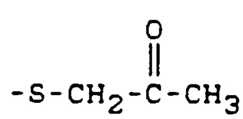 |
| 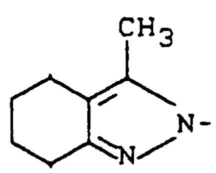 | F | 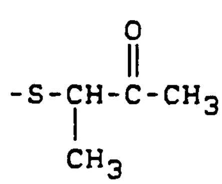 |

| Het | $R^1$ | $R^2$ |
| --- | --- | --- |
| 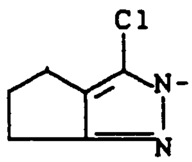 | F | 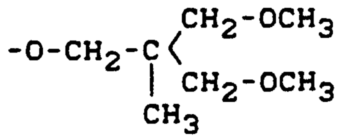 |
| 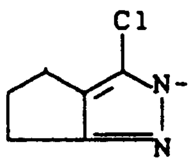 | F | 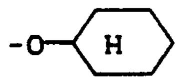 |
| 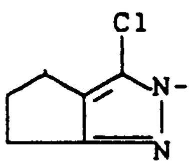 | F | 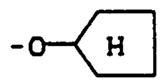 |
| 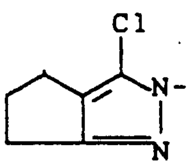 | F | 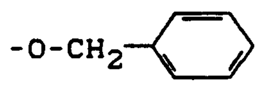 |
| 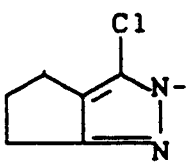 | F | $-O-CH_2-CN$ |
| 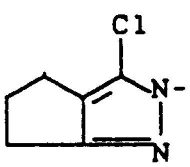 | F | $-O-CH_2-COOCH_3$ |
| 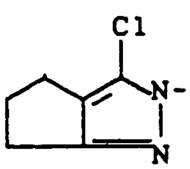 | F | $-O-CH_2-COOC_2H_5$ |

| Het | $R^1$ | $R^2$ |
|---|---|---|
| 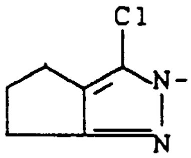 | F | 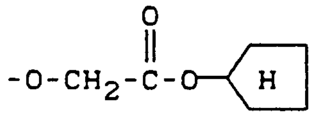 |
| 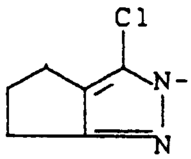 | F | 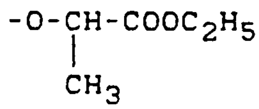 |
| 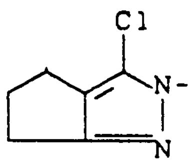 | F | 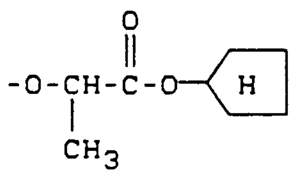 |
| 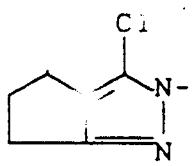 | F | 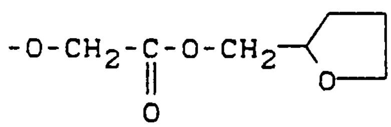 |
| 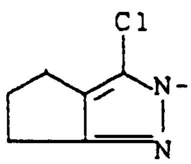 | F | 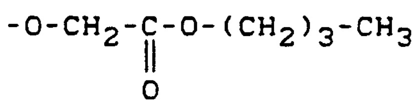 |
| 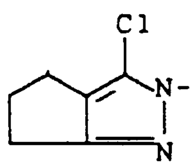 | F | 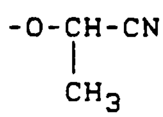 |

| Het | $R^1$ | $R^2$ |
|---|---|---|
| 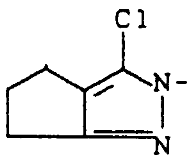 | F | $-O-CH_2-CH_2-O-CH_3$ |
| 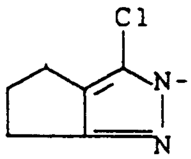 | F | 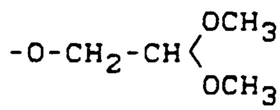 |
| 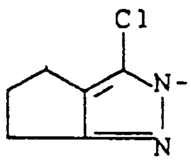 | F | 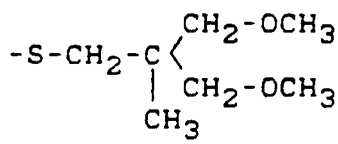 |
| 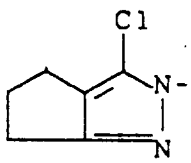 | F | -S- 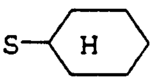 |
| 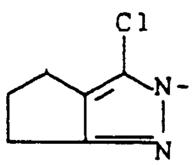 | F | -S- 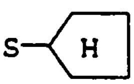 |
| 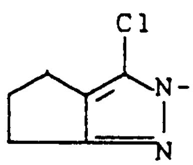 | F | $-S-CH_2-$ 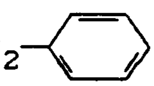 |
| 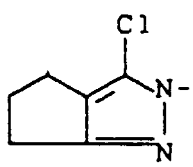 | F | $-S-CH_2-CN$ |

| Het | $R^1$ | $R^2$ |
|---|---|---|
| 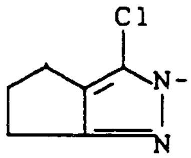 | F | $-S-CH_2-COOC_2H_5$ |
| 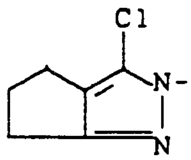 | F | 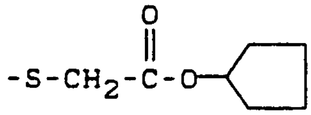 |
| 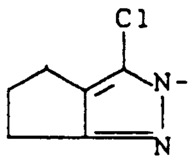 | F | 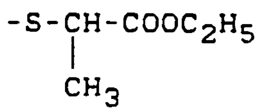 |
| 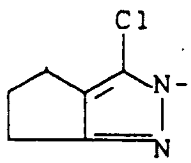 | F | 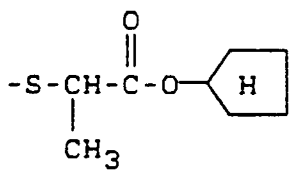 |
| 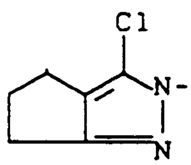 | F | 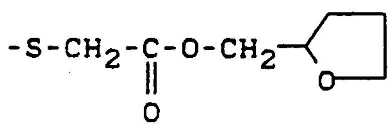 |
| 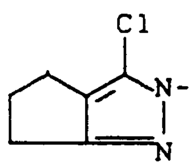 | F | 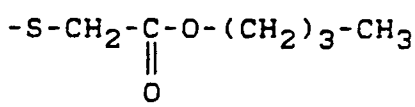 |

| Het | $R^1$ | $R^2$ |
|---|---|---|
| 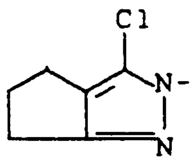 | F | -S-CH-CN<br>\|<br>$CH_3$ |
| 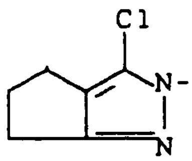 | F | $-S-CH_2-CH_2-O-CH_3$ |
| 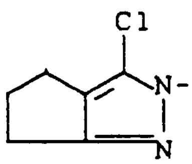 | F | $-S-CH_2-CH\langle^{OCH_3}_{OCH_3}$ |
| 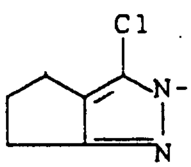 | F | $-OCH_3$ |
| 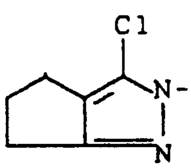 | F | $-OC_2H_5$ |
| 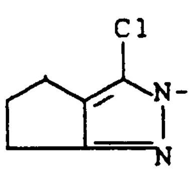 | F | $-OCH(CH_3)_2$ |
| 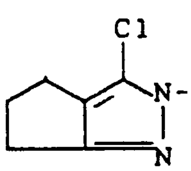 | F | $-O-CH_2-CH=CH_2$ |

| Het | $R^1$ | $R^2$ |
|---|---|---|
| 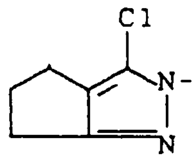 | F | $-O-CH_2-CH=CH-CH_3$ |
| 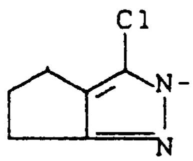 | F | $-O-CH_2-CH=CH-Cl$ |
| 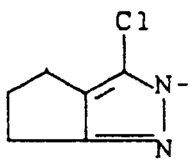 | F | $-O-CH(CH_3)-CH=CH_2$ |
| 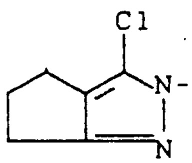 | F | $-O-CH_2-C\equiv CH$ |
| 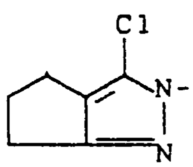 | F | $-O-CH(CH_3)-C\equiv CH$ |
| 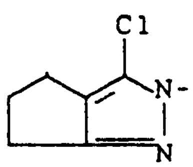 | F | $-O-CH_2-C(CH_3)=CH_2$ |
| 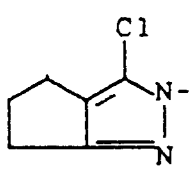 | F | $-OCH_2-CH_2-OC_2H_5$ |

| Het | $R^1$ | $R^2$ |
|---|---|---|
| 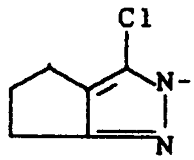 | F | 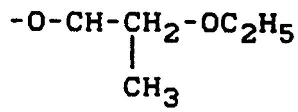 |
| 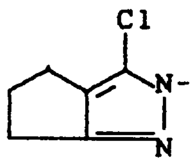 | F | 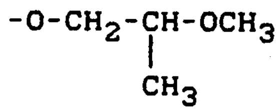 |
| 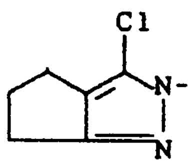 | F | 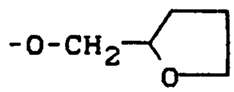 |
| 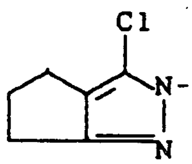 | F | 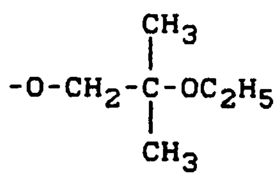 |
| 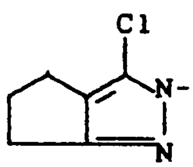 | F | $-O-CH_2-CH_2-O-CH_2-CH_2-OCH_3$ |
| 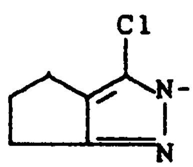 | F | $-O-CH_2-CH_2-O-CH_2-CH_2-OC_2H_5$ |
| 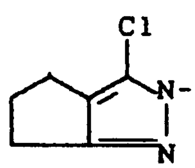 | F | 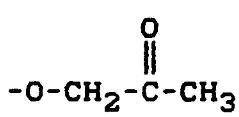 |

| Het | $R^1$ | $R^2$ |
|---|---|---|
| 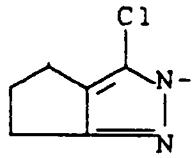 | F | 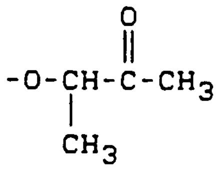 |
| 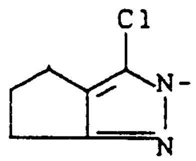 | F | $-SCH_3$ |
| 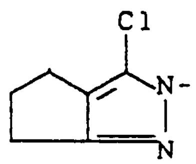 | F | $-SC_2H_5$ |
| 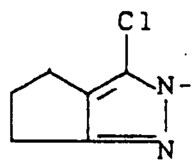 | F | $-S-CH(CH_3)_2$ |
| 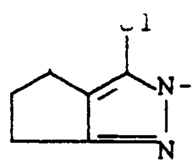 | F | $-S-CH_2-CH=CH_2$ |
| 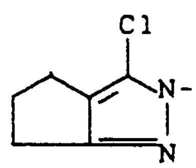 | F | $-S-CH_2-CH=CH-Cl$ |
| 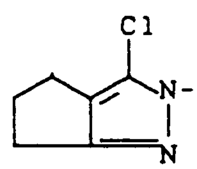 | F | $-S-CH_2-CH=CH-CH_3$ |

| Het | $R^1$ | $R^2$ |
|---|---|---|
| 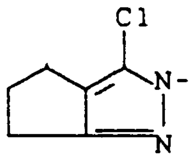 | F | $-S-CH-CH=CH_2$<br>$\vert$<br>$CH_3$ |
| 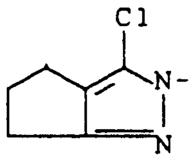 | F | $-S-CH_2-C\equiv CH$ |
| 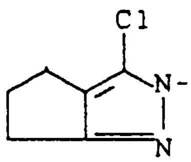 | F | $-S-CH-C\equiv CH$<br>$\vert$<br>$CH_3$ |
| 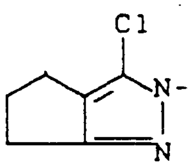 | F | $-S-CH_2-C=CH_2$<br>$\vert$<br>$CH_3$ |
| 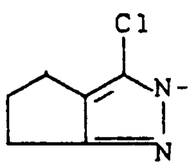 | F | $-S-CH_2-CH_2-OC_2H_5$ |
| 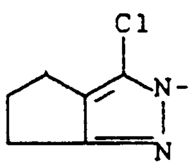 | F | $-S-CH-CH_2-OC_2H_5$<br>$\vert$<br>$CH_3$ |

| Het | $R^1$ | $R^2$ |
|---|---|---|
| 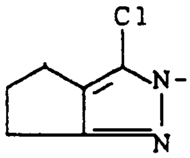 | F | 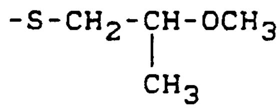 |
| 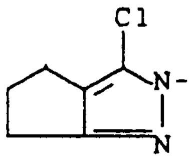 | F | 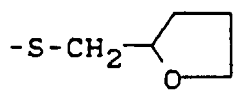 |
| 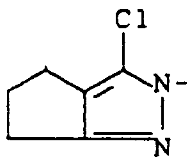 | F | 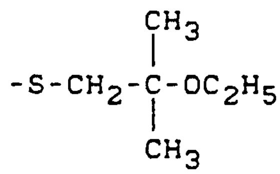 |
| 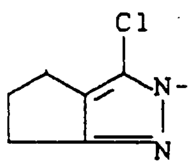 | F | $-S-CH_2-CH_2-O-CH_2-CH_2-OCH_3$ |
| 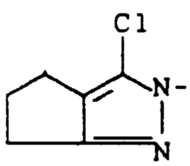 | F | $-S-CH_2-CH_2-O-CH_2-CH_2-OC_2H_5$ |
| 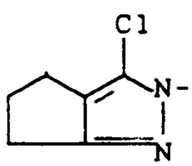 | F | 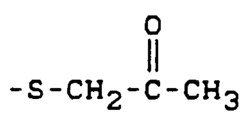 |
| 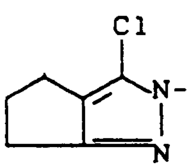 | F | 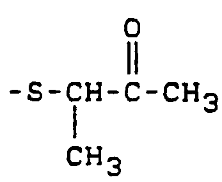 |

| Het | $R^1$ | $R^2$ |
|---|---|---|
| 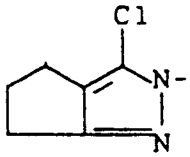 | H | 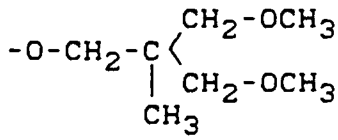 |
| 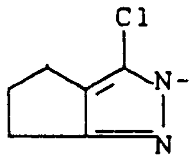 | H | 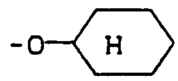 |
| 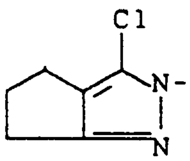 | H | 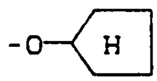 |
| 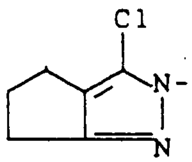 | H | 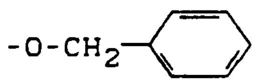 |
| 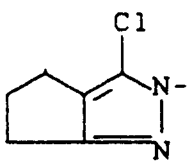 | H | $-O-CH_2-CN$ |
| 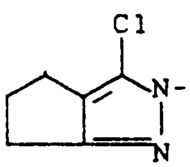 | H | $-O-CH_2-COOC_2H_5$ |
| 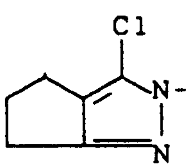 | H | 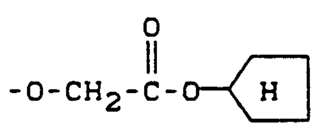 |

| Het | $R^1$ | $R^2$ |
|---|---|---|
| 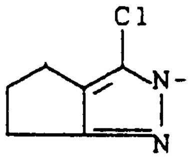 | H | 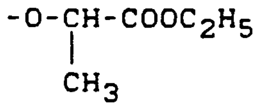 |
| 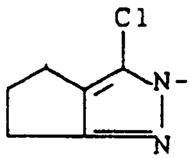 | H | 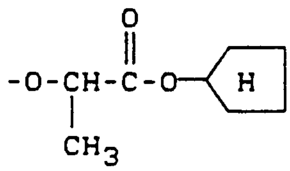 |
| 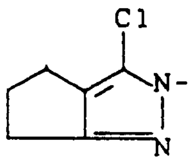 | H | 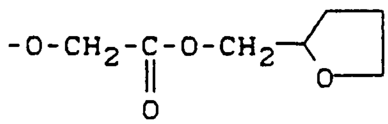 |
| 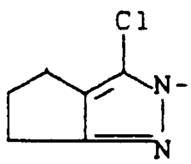 | H | 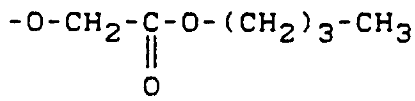 |
| 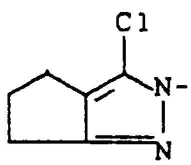 | H | 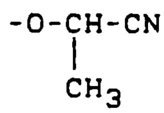 |
| 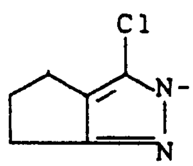 | H | $-O-CH_2-CH_2-O-CH_3$ |

| Het | $R^1$ | $R^2$ |
|---|---|---|
| 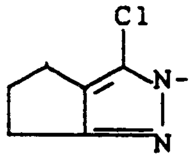 | H | 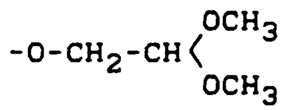 |
| 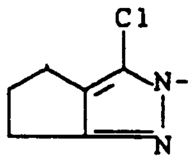 | H | 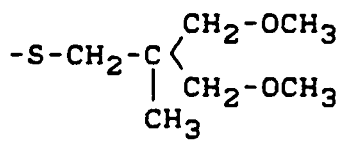 |
| 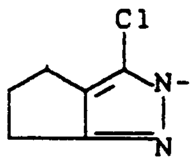 | H | 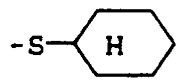 |
| 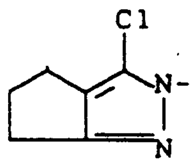 | H | 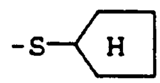 |
| 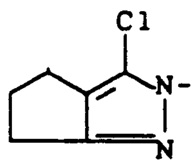 | H | 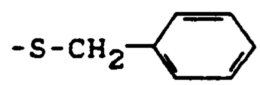 |
| 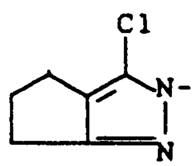 | H | $-S-CH_2-CN$ |
| 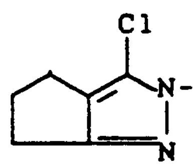 | H | $-S-CH_2-COOC_2H_5$ |

| Het | $R^1$ | $R^2$ |
|---|---|---|
| 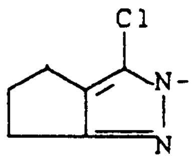 | H | 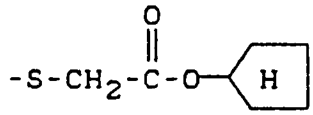 |
| 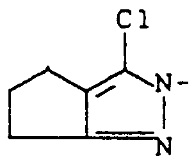 | H | 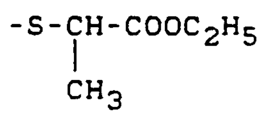 |
| 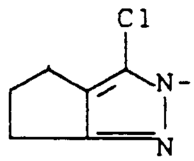 | H | 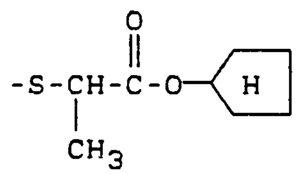 |
| 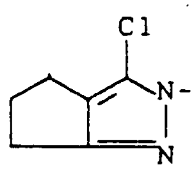 | H | 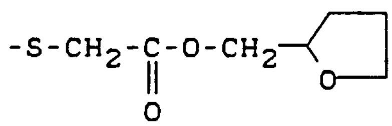 |
| 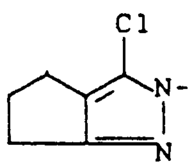 | H | 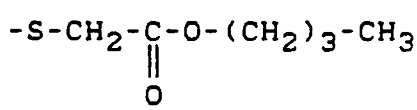 |
| 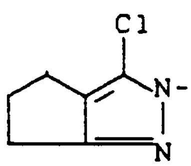 | H | 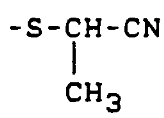 |

| Het | $R^1$ | $R^2$ |
| --- | --- | --- |
| 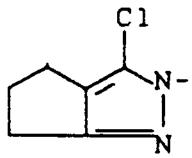 | H | $-S-CH_2-CH_2-O-CH_3$ |
| 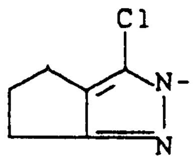 | H | $-S-CH_2-CH\langle\begin{matrix}OCH_3\\OCH_3\end{matrix}$ |
| 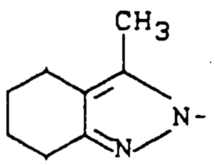 | H | $-OCH_3$ |
| 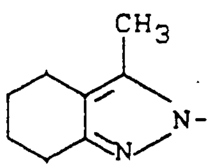 | H | $-OC_2H_5$ |
| 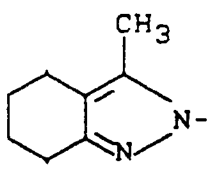 | H | $-O-CH(CH_3)_2$ |
| 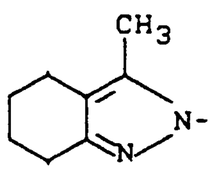 | H | $-O-CH_2-CH=CH_2$ |
| 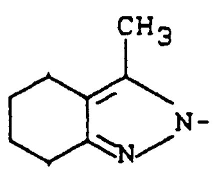 | H | $-O-CH_2-CH=CH-CH_3$ |

| Het | $R^1$ | $R^2$ |
|---|---|---|
| 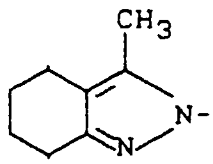 | H | $-O-CH_2-CH=CH-Cl$ |
| 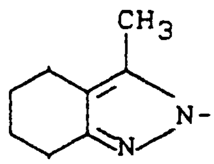 | H | $-O-CH-CH=CH_2$<br>$CH_3$ |
| 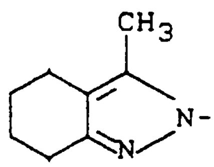 | H | $-O-CH_2-C\equiv CH$ |
| 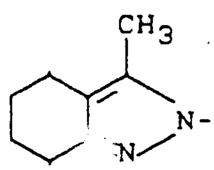 | H | $-O-CH-C\equiv CH$<br>$CH_3$ |
| 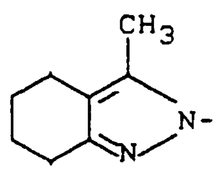 | H | $-O-CH_2-C=CH_2$<br>$CH_3$ |
| 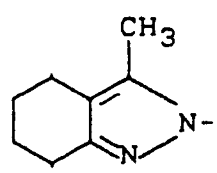 | H | $-O-CH_2-CH_2-OC_2H_5$ |
| 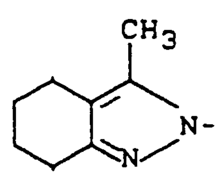 | H | $CH_3$<br>$-O-CH-CH_2-OC_2H_5$ |

| Het | $R^1$ | $R^2$ |
|---|---|---|
| 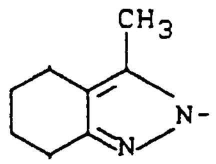 | H | 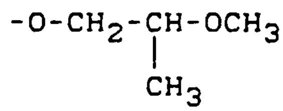 |
| 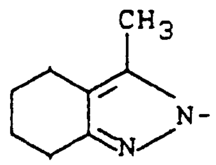 | H | 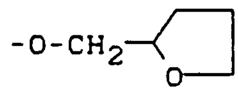 |
| 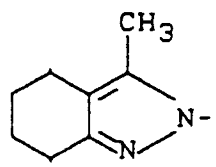 | H | 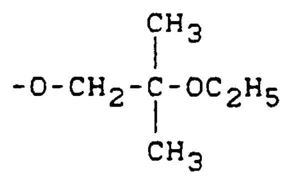 |
| 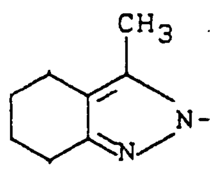 | H | $-O-CH_2-CH_2-O-CH_2-CH_2-OCH_3$ |
| 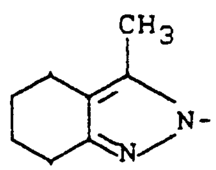 | H | $-O-CH_2-CH_2-O-CH_2-CH_2-OC_2H_5$ |
| 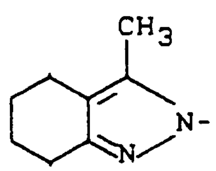 | H | 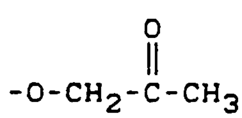 |
| 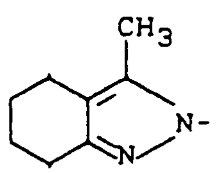 | H | 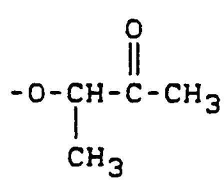 |

| Het | $R^1$ | $R^2$ |
|---|---|---|
| 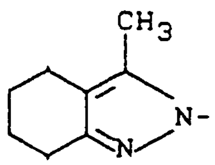 | H | $-SCH_3$ |
| 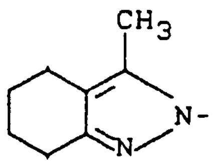 | H | $-SC_2H_5$ |
| 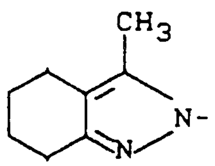 | H | $-S-CH(CH_3)_2$ |
| 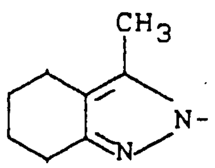 | H | $-S-CH_2-CH=CH_2$ |
| 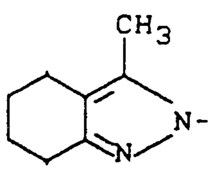 | H | $-S-CH_2-CH=CH-Cl$ |
| 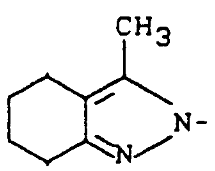 | H | $-S-CH_2-CH=CH-CH_3$ |
| 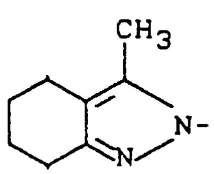 | H | $-S-CH-CH=CH_2$<br>\|<br>$CH_3$ |

| Het | $R^1$ | $R^2$ |
|---|---|---|
| 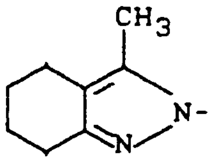 | H | $-S-CH_2-C\equiv CH$ |
| 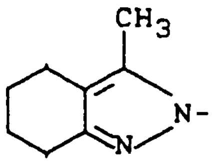 | H | $-S-CH-C\equiv CH$<br>$CH_3$ |
| 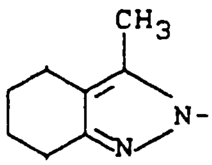 | H | $-S-CH_2-C=CH_2$<br>$CH_3$ |
| 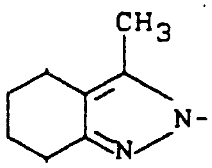 | H | $-S-CH_2-CH_2-OC_2H_5$ |
| 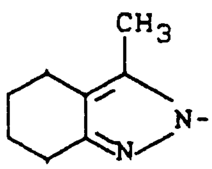 | H | $-S-CH-CH_2-OC_2H_5$<br>$CH_3$ |
| 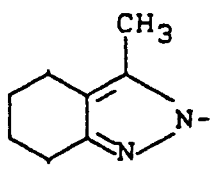 | H | $-S-CH_2-CH-OCH_3$<br>$CH_3$ |

| Het | $R^1$ | $R^2$ |
|---|---|---|
| 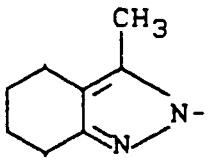 | H | 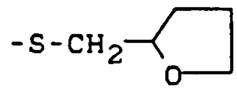 |
| 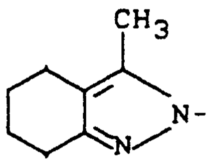 | H | 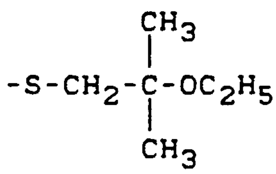 |
| 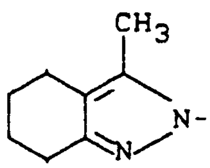 | H | $-S-CH_2-CH_2-O-CH_2-CH_2-OCH_3$ |
| 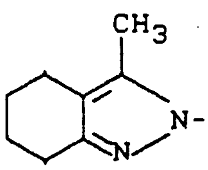 | H | $-S-CH_2-CH_2-O-CH_2-CH_2-OC_2H_5$ |
| 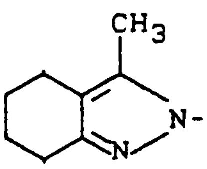 | H | 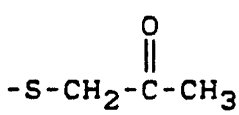 |
| 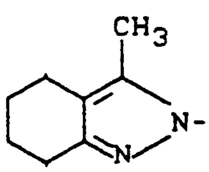 | H | 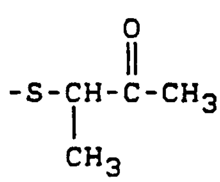 |
| 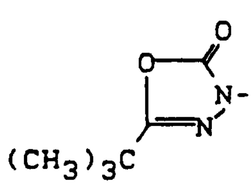 | F | 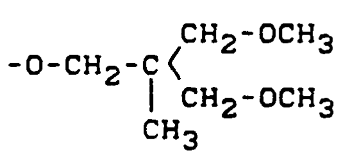 |

| Het | $R^1$ | $R^2$ |
|---|---|---|
| 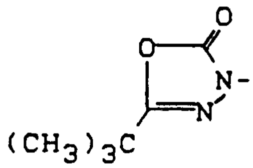 | F | 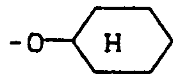 |
| 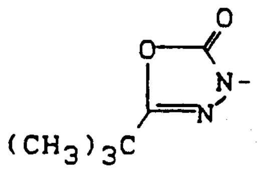 | F | 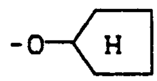 |
| 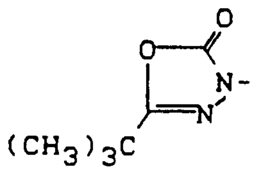 | F | 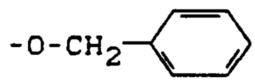 |
| 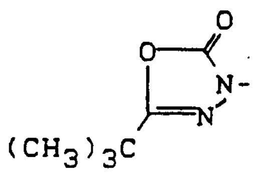 | F | $-O-CH_2-CN$ |
| 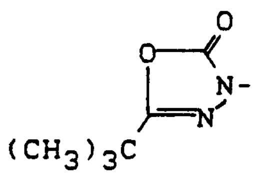 | F | $-O-CH_2-COOC_2H_5$ |
| 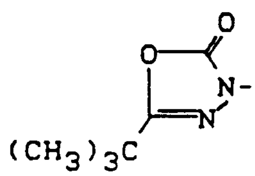 | F | 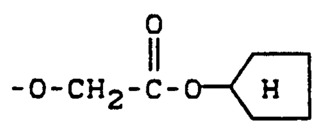 |
| 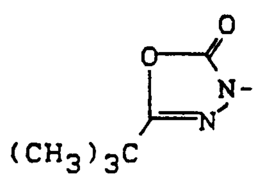 | F | 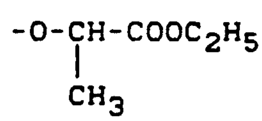 |

| Het | $R^1$ | $R^2$ |
|---|---|---|
| 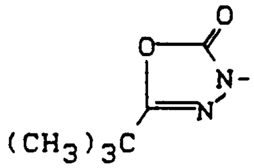 | F | 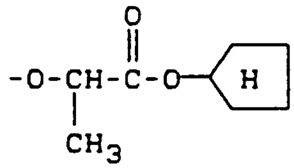 |
| 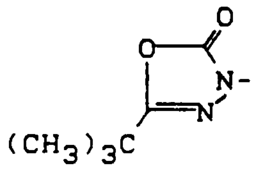 | F | 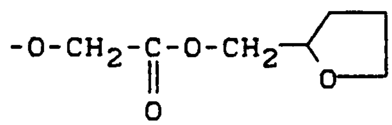 |
| 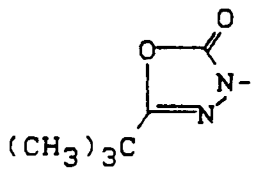 | F | 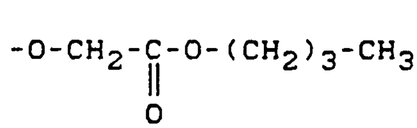 |
| 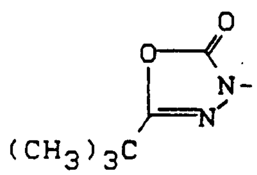 | F | 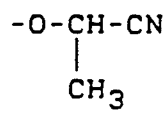 |
| 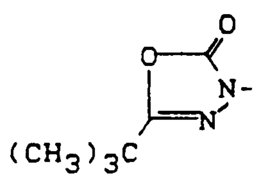 | F | $-O-CH_2-CH_2-O-CH_3$ |
| 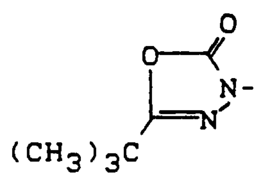 | F | 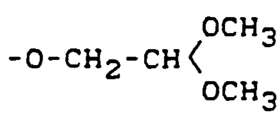 |
| 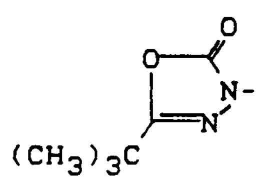 | F | 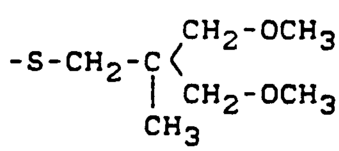 |

| Het | $R^1$ | $R^2$ |
|---|---|---|
| 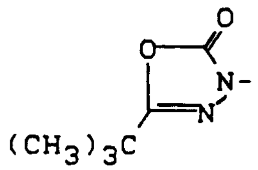 | F | 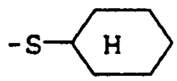 |
| 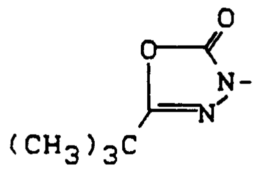 | F | 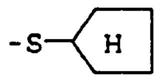 |
| 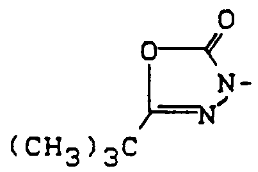 | F | 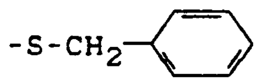 |
| 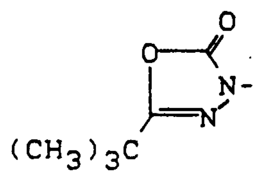 | F | $-S-CH_2-CN$ |
| 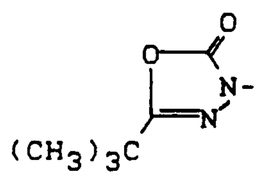 | F | $-S-CH_2-COOC_2H_5$ |
| 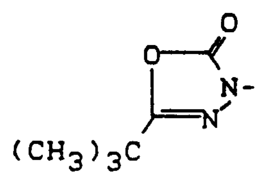 | F | 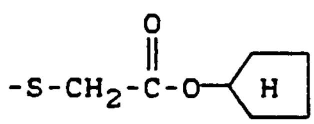 |
| 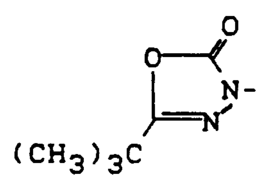 | F | 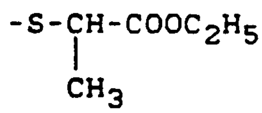 |

| Het | $R^1$ | $R^2$ |
|---|---|---|
| 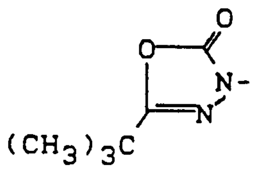 | F | 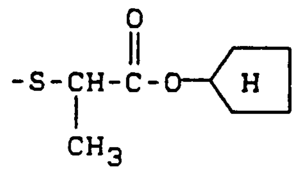 |
| 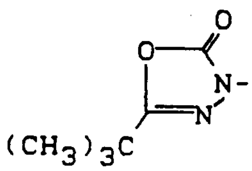 | F | 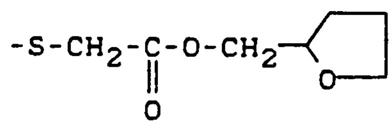 |
| 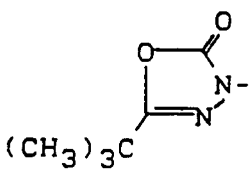 | F | 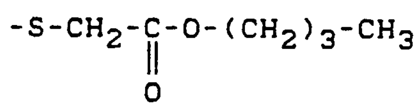 |
| 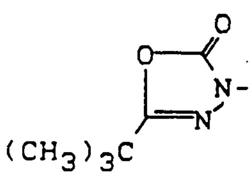 | F | 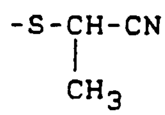 |
| 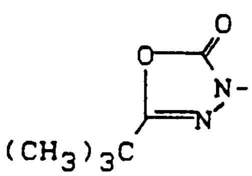 | F | $-S-CH_2-CH_2-O-CH_3$ |
| 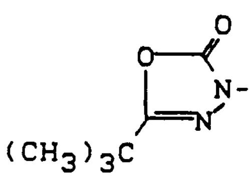 | F | 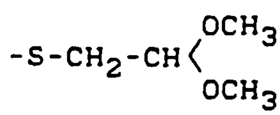 |
| 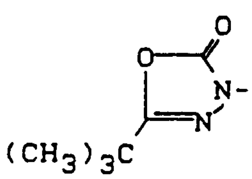 | F | $-OCH_3$ |

| Het | $R^1$ | $R^2$ |
| --- | --- | --- |
| 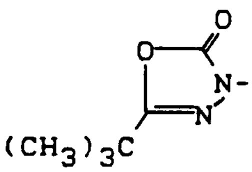 | F | $-OC_2H_5$ |
| 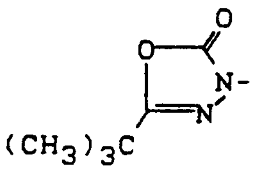 | F | $-O-CH(CH_3)_2$ |
| 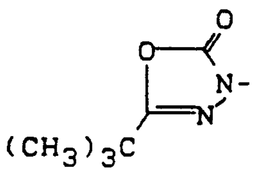 | F | $-O-CH_2-CH=CH_2$ |
| 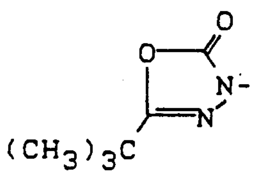 | F | $-O-CH_2-CH=CH-CH_3$ |
| 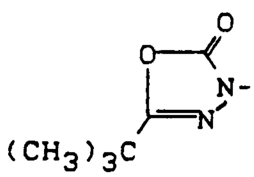 | F | $-O-CH_2-CH=CH-Cl$ |
| 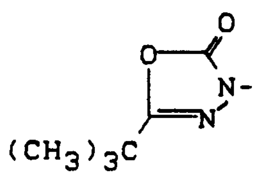 | F | $-O-\underset{CH_3}{\underset{\vert}{CH}}-CH=CH_2$ |
| 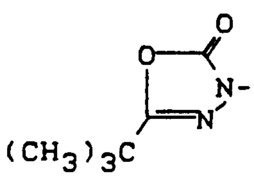 | F | $-O-CH_2-C\equiv CH$ |

| Het | $R^1$ | $R^2$ |
|---|---|---|
| 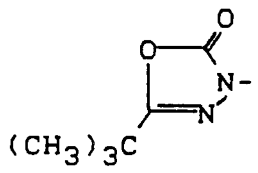 | F | $-O-CH-C\equiv CH$<br>$CH_3$ |
| 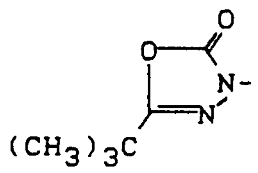 | F | $-O-CH_2-C=CH_2$<br>$CH_3$ |
| 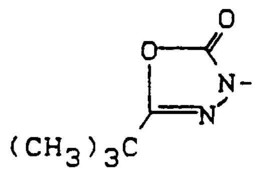 | F | $-O-CH_2-CH_2-OC_2H_5$ |
| 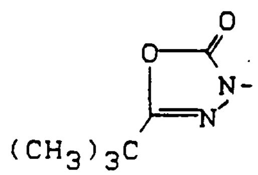 | F | $-O-CH-CH_2-OC_2H_5$<br>$CH_3$ |
| 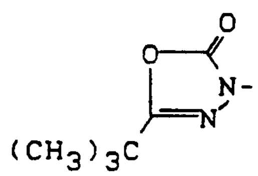 | F | $-O-CH_2-CH-OCH_3$<br>$CH_3$ |
| 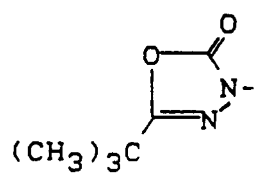 | F | $-O-CH_2$ 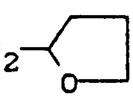 |
| 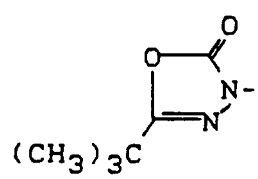 | F | $CH_3$<br>$-O-CH_2-C-OC_2H_5$<br>$CH_3$ |

| Het | $R^1$ | $R^2$ |
|---|---|---|
| 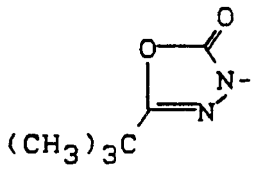 | F | $-O-CH_2-CH_2-O-CH_2-CH_2-OCH_3$ |
| 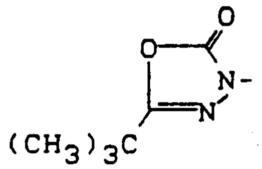 | F | $-O-CH_2-CH_2-O-CH_2-CH_2-OC_2H_5$ |
| 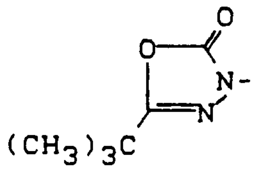 | F | 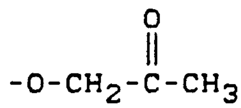 |
| 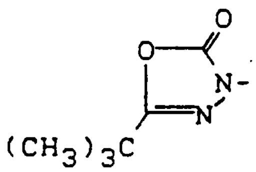 | F | 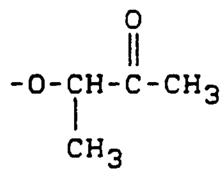 |
| 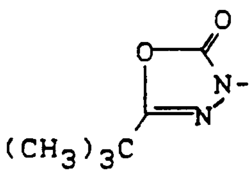 | F | $-SCH_3$ |
| 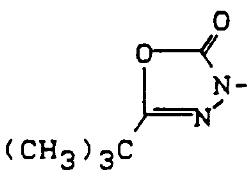 | F | $-SC_2H_5$ |
| 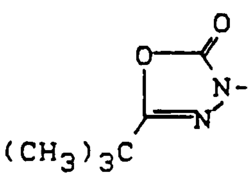 | F | $-S-CH(CH_3)_2$ |

| Het | $R^1$ | $R^2$ |
|---|---|---|
| 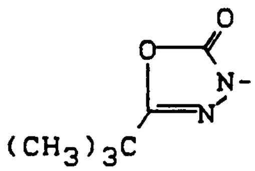 | F | $-S-CH_2-CH=CH_2$ |
| 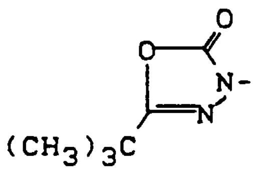 | F | $-S-CH_2-CH=CH-Cl$ |
| 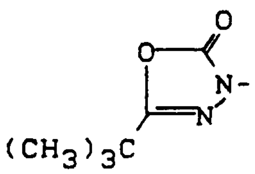 | F | $-S-CH_2-CH=CH-CH_3$ |
| 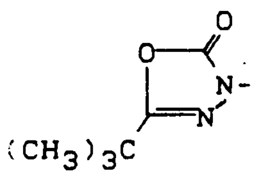 | F | $-S-\underset{\substack{\vert\\CH_3}}{CH}-CH=CH_2$ |
| 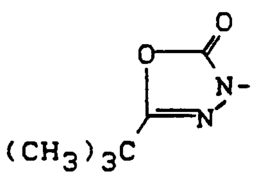 | F | $-S-CH_2-C\equiv CH$ |
| 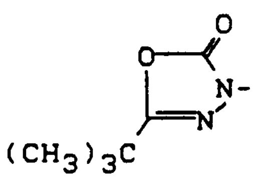 | F | $-S-\underset{\substack{\vert\\CH_3}}{CH}-C\equiv CH$ |
| 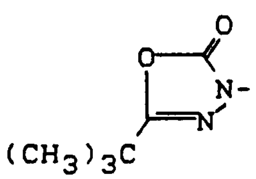 | F | $-S-CH_2-\underset{\substack{\vert\\CH_3}}{C}=CH_2$ |

| Het | $R^1$ | $R^2$ |
|---|---|---|
| 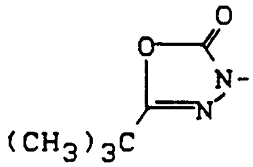 | F | $-S-CH_2-CH_2-OC_2H_5$ |
| 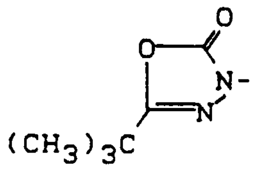 | F | 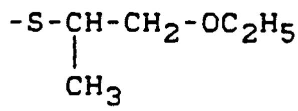 |
| 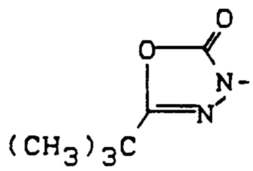 | F | 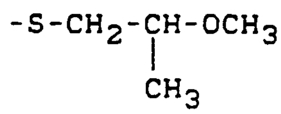 |
| 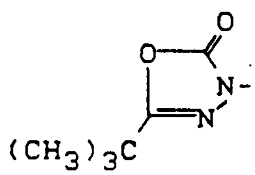 | F | 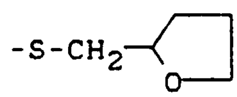 |
| 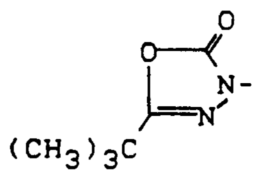 | F | 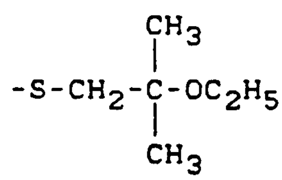 |
| 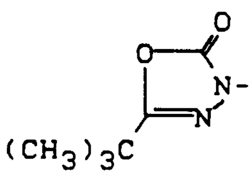 | F | $-S-CH_2-CH_2-O-CH_2-CH_2-OCH_3$ |
| 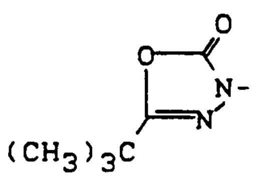 | F | $-S-CH_2-CH_2-O-CH_2-CH_2-OC_2H_5$ |

| Het | $R^1$ | $R^2$ |
|---|---|---|
| 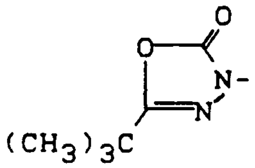 | F | 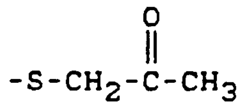 |
| 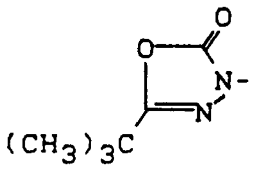 | F | 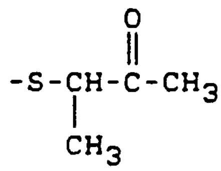 |
| 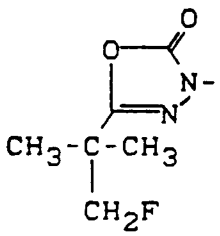 | F | 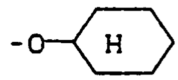 |
| 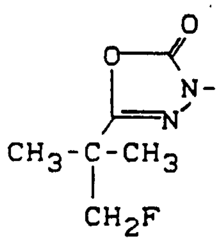 | F | 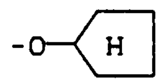 |
| 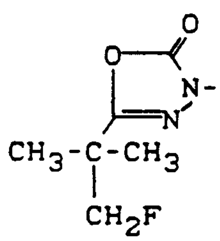 | F | 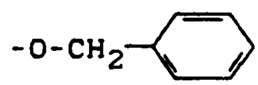 |
| 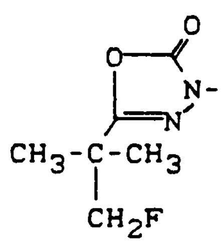 | F | $-O-CH_2-CN$ |

| Het | $R^1$ | $R^2$ |
|---|---|---|
| 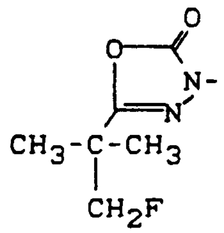 | F | $-O-CH_2-COOC_2H_5$ |
| 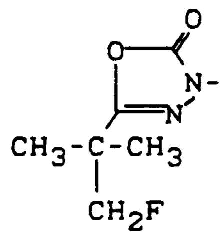 | F | 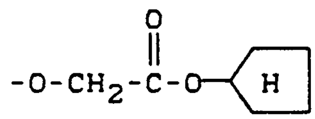 |
| 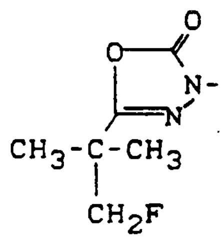 | F | 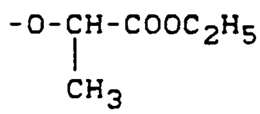 |
| 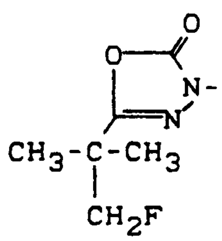 | F | 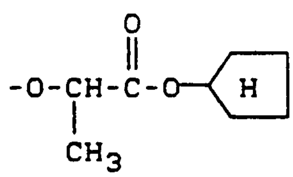 |
| 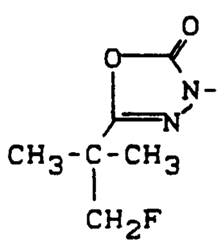 | F | 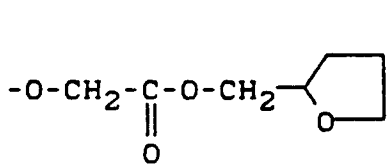 |

| Het | $R^1$ | $R^2$ |
|---|---|---|
| 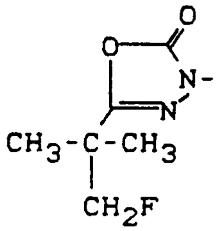 | F | 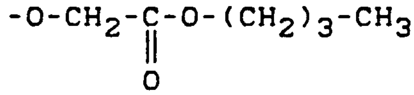 |
| 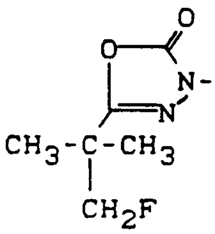 | F | 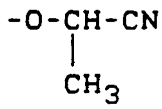 |
| 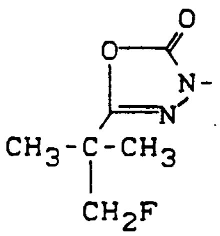 | F | $-O-CH_2-CH_2-O-CH_3$ |
| 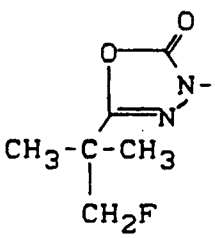 | F | 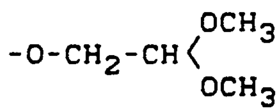 |
| 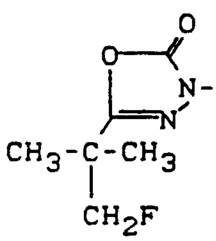 | F | 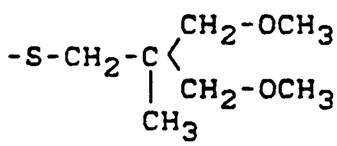 |

| Het | $R^1$ | $R^2$ |
|---|---|---|
| 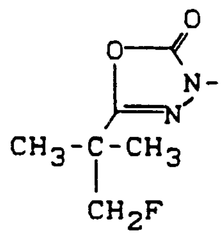 | F | 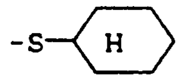 |
| 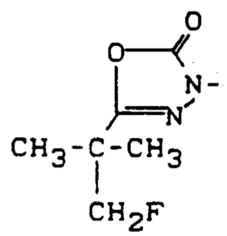 | F | 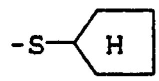 |
| 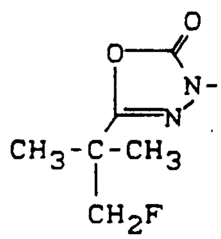 | F | 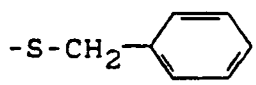 |
| 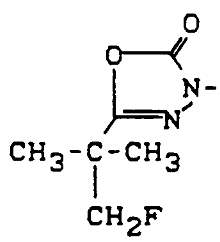 | F | $-S-CH_2-CN$ |
| 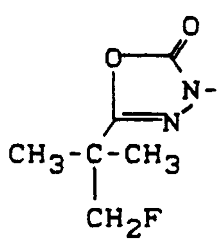 | F | $-S-CH_2-COOC_2H_5$ |

| Het | $R^1$ | $R^2$ |
|---|---|---|
| 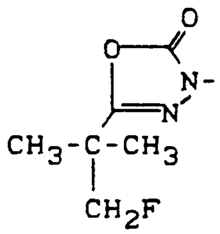 | F | 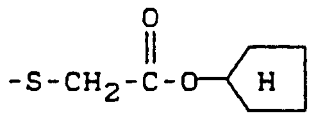 |
| 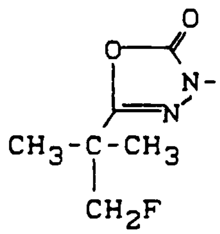 | F | 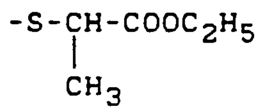 |
| 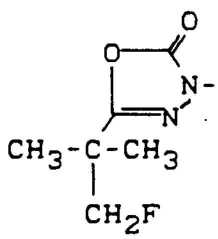 | F | 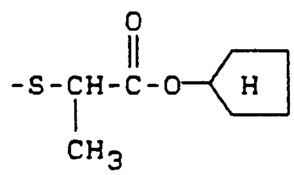 |
| 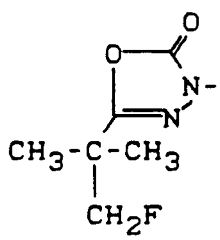 | F | 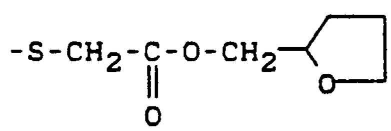 |
| 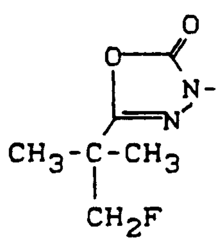 | F | 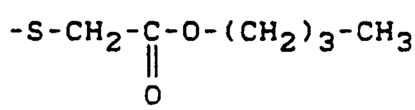 |

| Het | $R^1$ | $R^2$ |
|---|---|---|
| 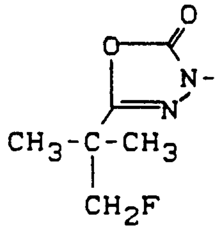 | F | 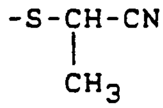 |
| 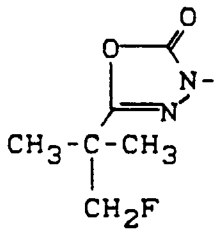 | F | $-S-CH_2-CH_2-OCH_3$ |
| 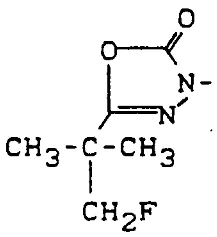 | F | 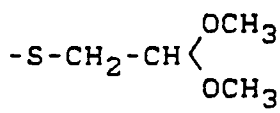 |
| 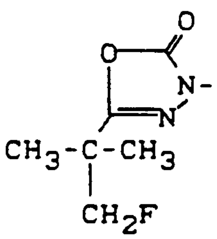 | H | $-O-CH_2-CH_2-O-CH_2-CH_2-OCH_3$ |
| 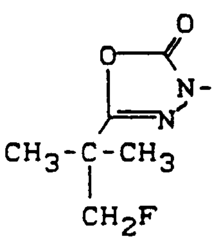 | H | $-O-CH_2-CH_2-O-CH_2-CH_2-OC_2H_5$ |

| Het | $R^1$ | $R^2$ |
|---|---|---|
| 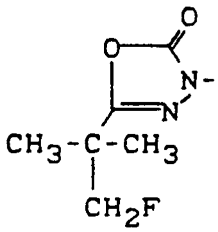 | H | 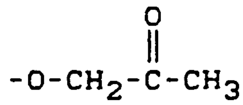 |
| 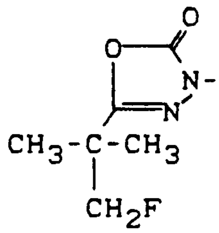 | H | 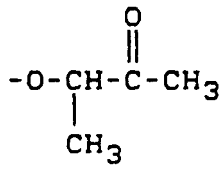 |
| 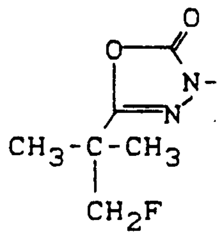 | H | $-SCH_3$ |
| 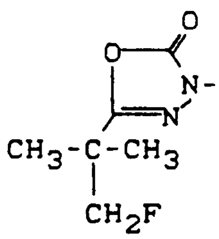 | H | $-SC_2H_5$ |
| 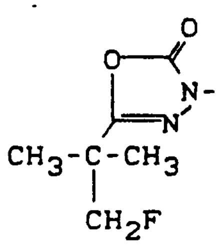 | H | $-S-CH(CH_3)_2$ |

| Het | $R^1$ | $R^2$ |
|---|---|---|
| 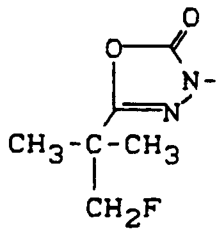 | H | $-S-CH_2-CH=CH_2$ |
| 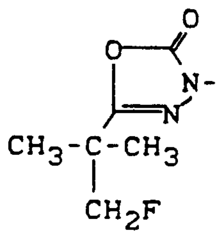 | H | $-S-CH_2-CH=CH-Cl$ |
| 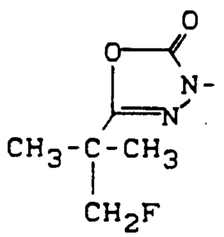 | H | $-S-CH_2-CH=CH-CH_3$ |
| 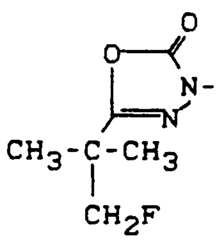 | H | $-S-\underset{\displaystyle CH_3}{\underset{\vert}{CH}}-CH=CH_2$ |
| 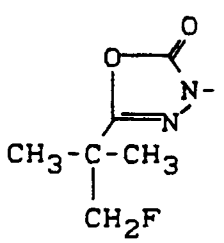 | H | $-S-CH_2-C\equiv CH$ |

| Het | $R^1$ | $R^2$ |
|---|---|---|
| 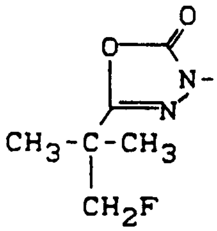 | H | 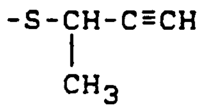 |
| 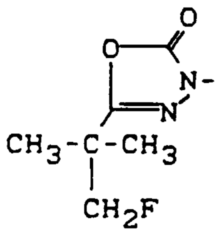 | H | 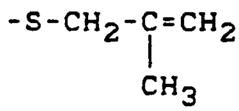 |
| 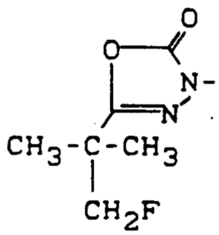 | H | $-S-CH_2-CH_2-OC_2H_5$ |
| 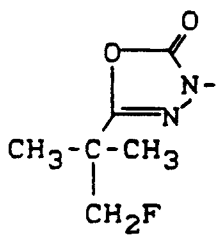 | H | 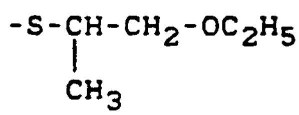 |
| 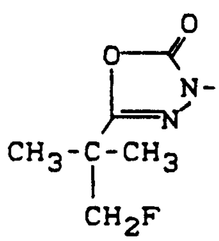 | H | 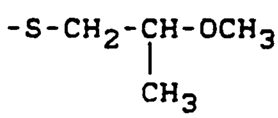 |

| Het | $R^1$ | $R^2$ |
|---|---|---|
| 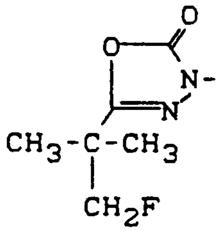 | H | 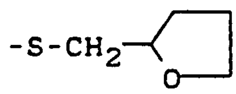 |
| 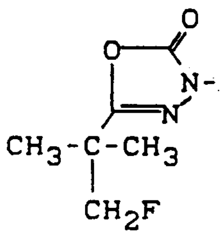 | H | 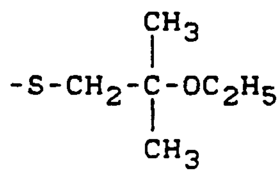 |
| 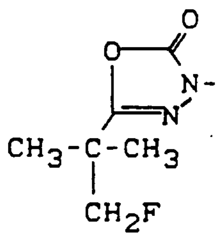 | H | $-S-CH_2-CH_2-O-CH_2-CH_2-OCH_3$ |
| 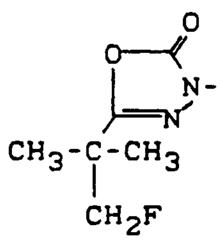 | H | $-S-CH_2-CH_2-O-CH_2-CH_2-OC_2H_5$ |
| 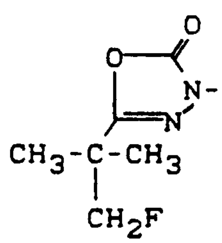 | H | 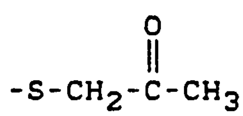 |

| Het | $R^1$ | $R^2$ |
|---|---|---|
| 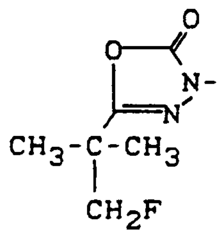 | H | 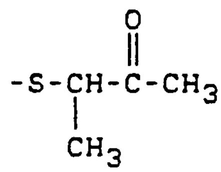 |
| 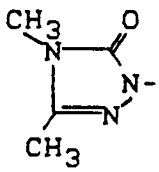 | F | 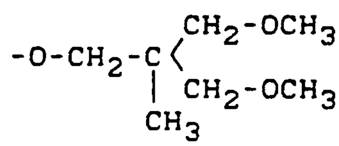 |
| 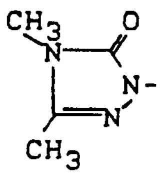 | F | 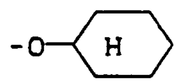 |
| 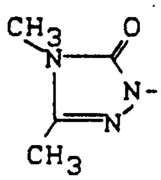 | F | 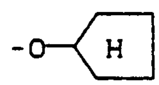 |
| 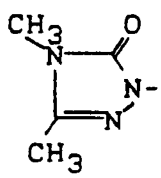 | F | 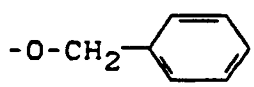 |
| 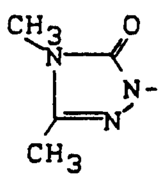 | F | $-O-CH_2-CN$ |
| 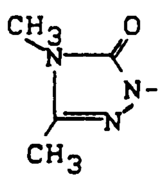 | F | $-O-CH_2-COOC_2H_5$ |

| Het | $R^1$ | $R^2$ |
|---|---|---|
| 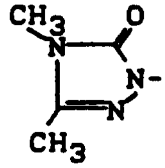 | F | 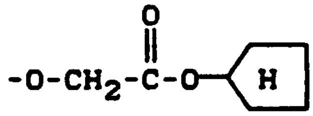 |
| 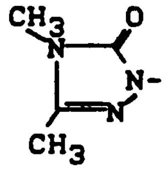 | F | 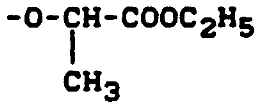 |
| 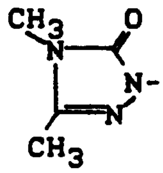 | F | 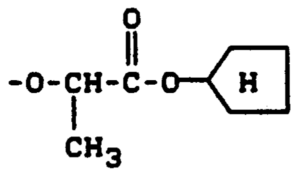 |
| 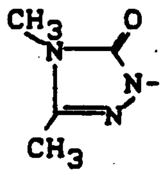 | F | 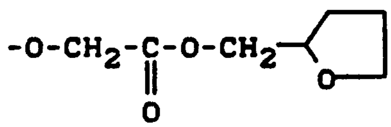 |
| 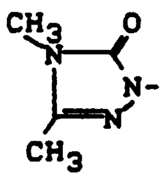 | F | 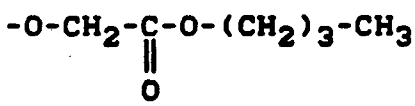 |
| 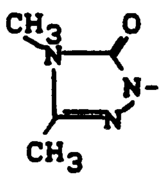 | F | 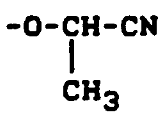 |
| 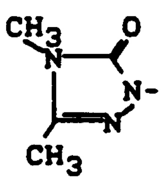 | F | $-O-CH_2-CH_2-O-CH_3$ |

| Het | $R^1$ | $R^2$ |
|---|---|---|
| 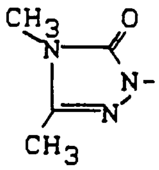 | F | 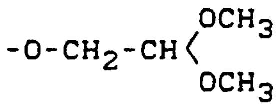 |
| 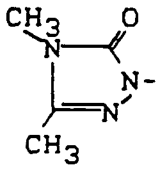 | F | 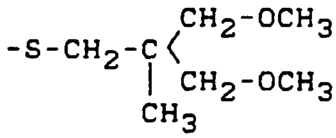 |
| 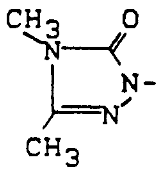 | F | 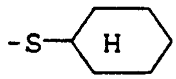 |
| 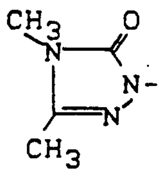 | F | 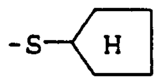 |
| 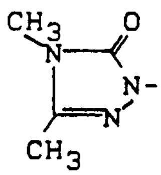 | F | 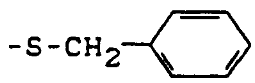 |
| 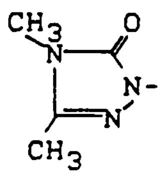 | F | $-S-CH_2-CN$ |
| 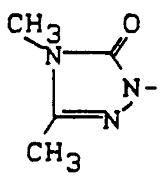 | F | $-S-CH_2-COOC_2H_5$ |

| Het | $R^1$ | $R^2$ |
|---|---|---|
| 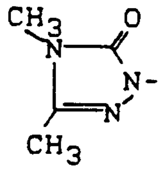 | F | 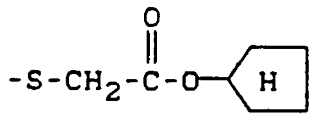 |
| 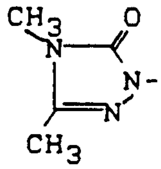 | F | 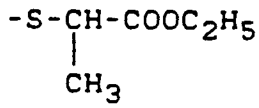 |
| 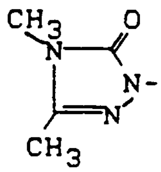 | F | 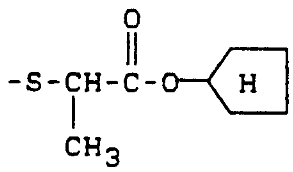 |
| 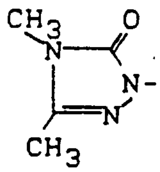 | F | 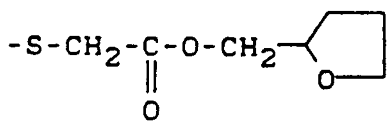 |
| 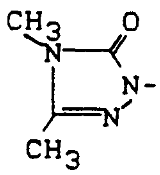 | F | 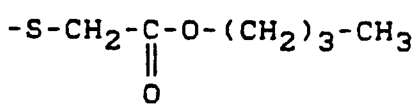 |
| 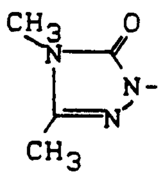 | F | 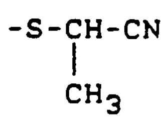 |
| 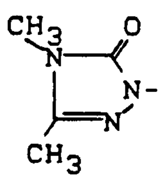 | F | $-S-CH_2-CH_2-O-CH_3$ |

| Het | $R^1$ | $R^2$ |
|---|---|---|
| 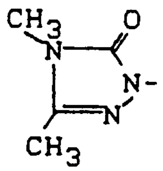 | F | $-S-CH_2-CH\left\langle \begin{matrix} OCH_3 \\ OCH_3 \end{matrix}\right.$ |
| 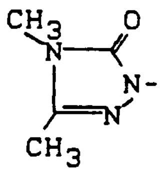 | F | $-OCH_3$ |
| 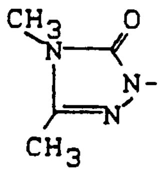 | F | $-OC_2H_5$ |
| 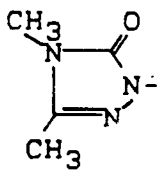 | F | $-O-CH(CH_3)_2$ |
| 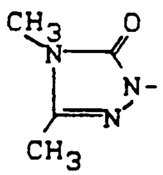 | F | $-O-CH_2-CH=CH_2$ |
| 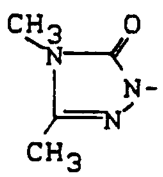 | F | $-O-CH_2-CH=CH-CH_3$ |
| 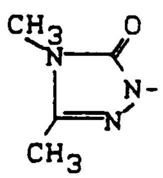 | F | $-O-CH_2-CH=CH-Cl$ |

| Het | $R^1$ | $R^2$ |
|---|---|---|
| 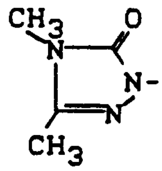 | F | 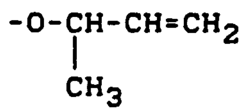 |
| 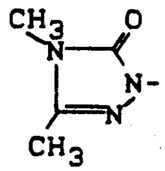 | F | $-O-CH_2-C\equiv CH$ |
| 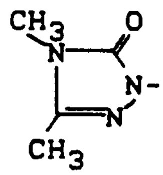 | F | 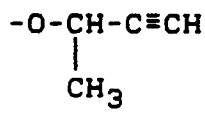 |
| 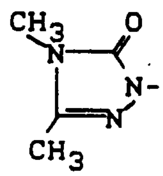 | F | 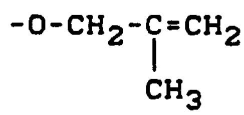 |
| 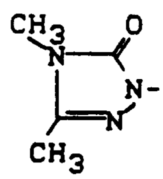 | F | $-O-CH_2-CH_2-OC_2H_5$ |
| 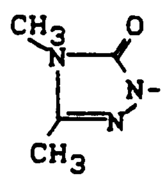 | F | 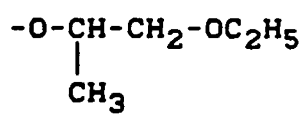 |
| 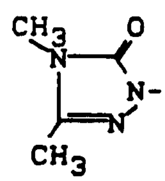 | F | 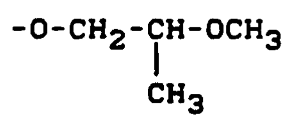 |

| Het | $R^1$ | $R^2$ |
|---|---|---|
| 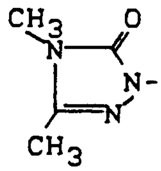 | F | 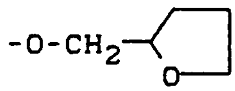 |
| 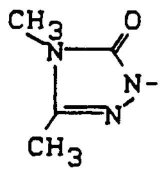 | F | 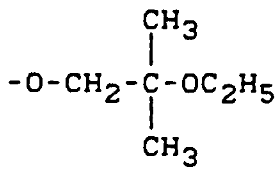 |
| 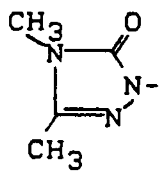 | F | $-O-CH_2-CH_2-O-CH_2-CH_2-OCH_3$ |
| 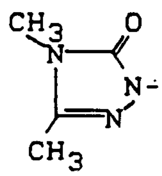 | F | $-O-CH_2-CH_2-O-CH_2-CH_2-OC_2H_5$ |
| 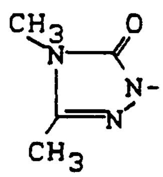 | F | 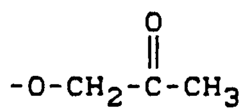 |
| 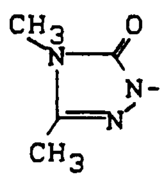 | F | 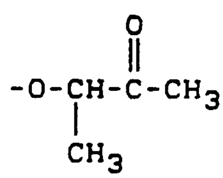 |
| 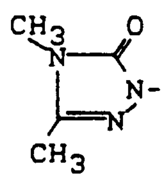 | F | $-SCH_3$ |

| Het | $R^1$ | $R^2$ |
|---|---|---|
| 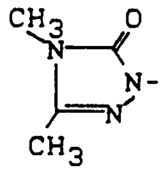 | F | $-SC_2H_5$ |
| 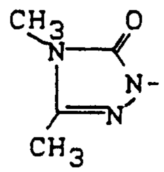 | F | $-S-CH(CH_3)_2$ |
| 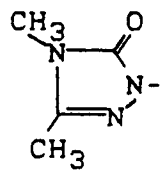 | F | $-S-CH_2-CH=CH_2$ |
| 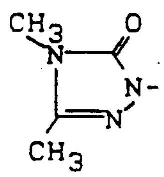 | F | $-S-CH_2-CH=CH-Cl$ |
| 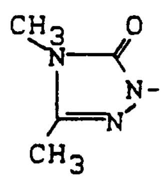 | F | $-S-CH_2-CH=CH-CH_3$ |
| 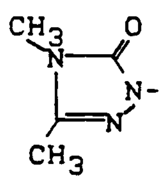 | F | $-S-CH-CH=CH_2$<br>$CH_3$ |
| 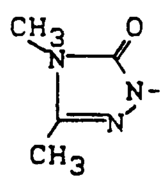 | F | $-S-CH_2-C\equiv CH$ |

| Het | $R^1$ | $R^2$ |
|---|---|---|
| 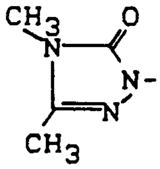 | F | 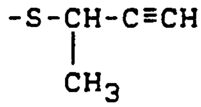 |
| 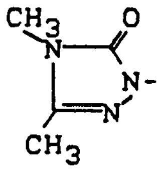 | F | 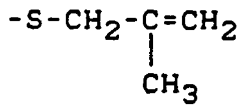 |
| 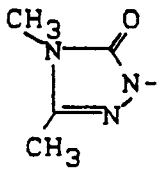 | F | $-S-CH_2-CH_2-OC_2H_5$ |
| 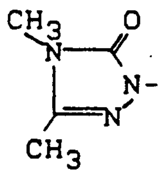 | F | 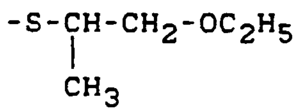 |
| 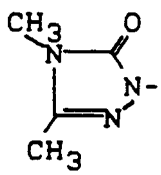 | F | 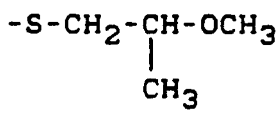 |
| 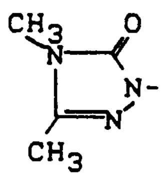 | F | 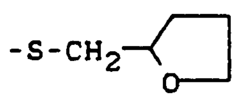 |
| 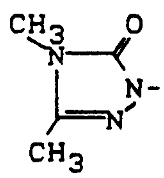 | F | 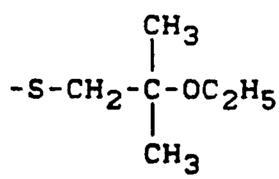 |

| Het | $R^1$ | $R^2$ |
|---|---|---|
| 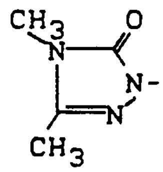 | F | $-S-CH_2-CH_2-O-CH_2-CH_2-OCH_3$ |
| 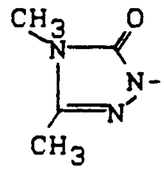 | F | $-S-CH_2-CH_2-O-CH_2-CH_2-OC_2H_5$ |
| 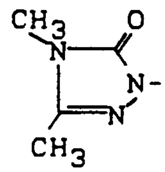 | F | 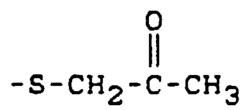 |
| 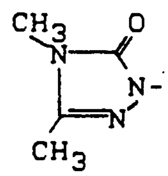 | F | 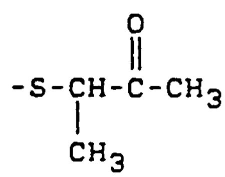 |
| 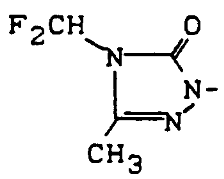 | F | 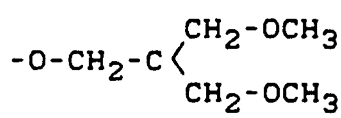 |
| 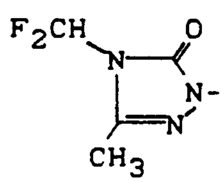 | F | 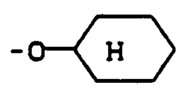 |
| 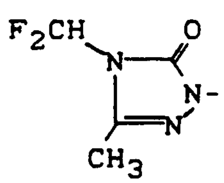 | F | 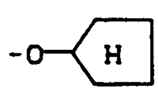 |

| Het | $R^1$ | $R^2$ |
|---|---|---|
| 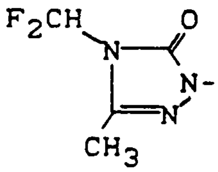 | F | 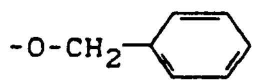 |
| 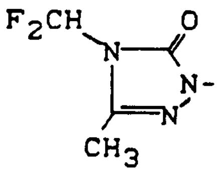 | F | $-O-CH_2-CN$ |
| 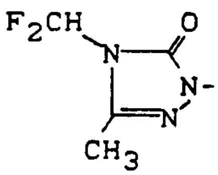 | F | $-O-CH_2-COOC_2H_5$ |
| 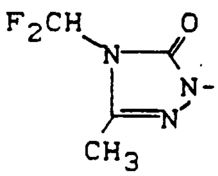 | F | 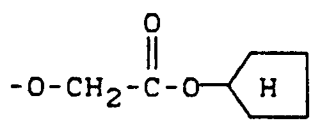 |
| 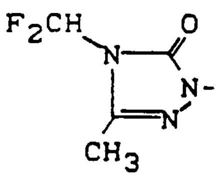 | F | 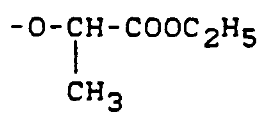 |
| 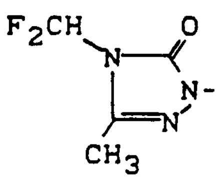 | F | 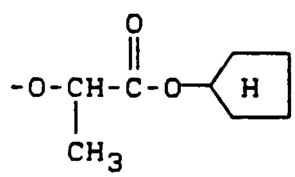 |
| 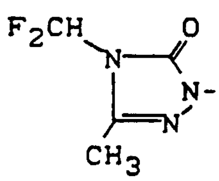 | F | 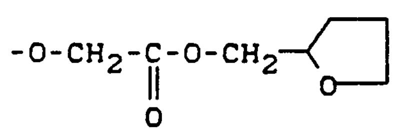 |

| Het | $R^1$ | $R^2$ |
|---|---|---|
| 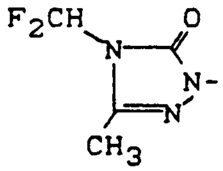 | F | 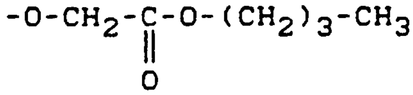 |
| 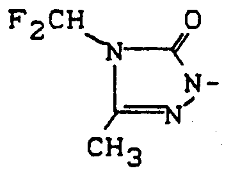 | F | 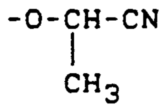 |
| 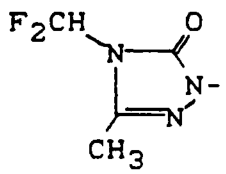 | F | $-O-CH_2-CH_2-O-CH_3$ |
| 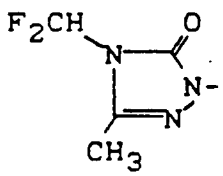 | F | 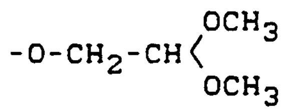 |
| 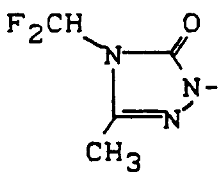 | H | 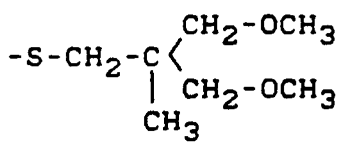 |
| 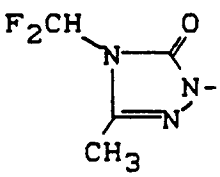 | H | 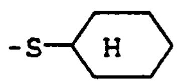 |
| 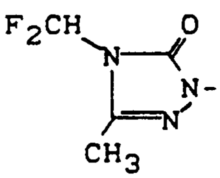 | H | 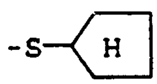 |

| Het | $R^1$ | $R^2$ |
|---|---|---|
| 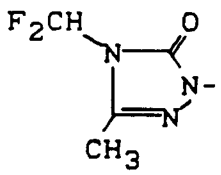 | H | 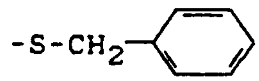 |
| 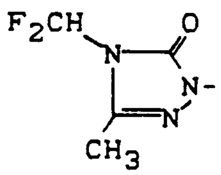 | H | $-S-CH_2-CN$ |
| 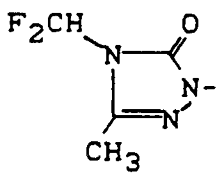 | H | $-S-CH_2-COOC_2H_5$ |
| 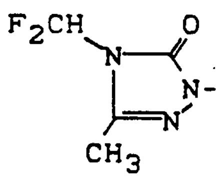 | H | 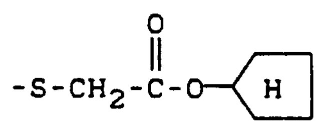 |
| 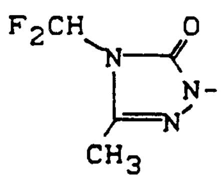 | H | 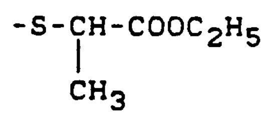 |
| 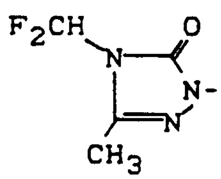 | H | 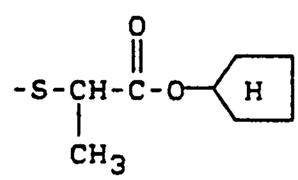 |
| 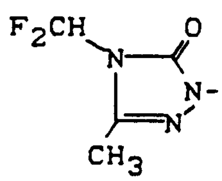 | H | 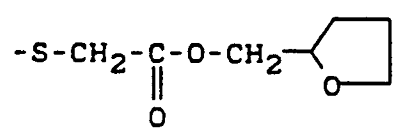 |

| Het | $R^1$ | $R^2$ |
|---|---|---|
| 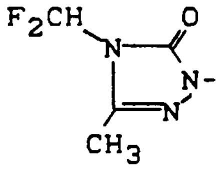 | H | 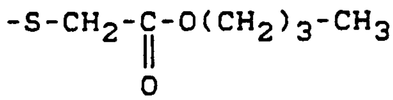 |
| 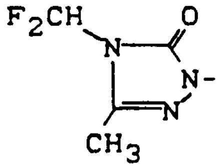 | H | 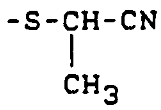 |
| 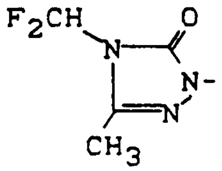 | H | $-S-CH_2-CH_2-O-CH_3$ |
| 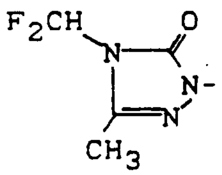 | H | 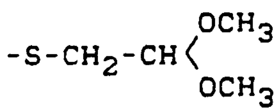 |
| 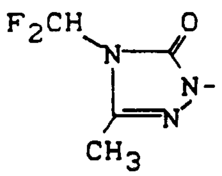 | F | $-OCH_3$ |
| 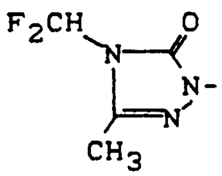 | F | $-OC_2H_5$ |
| 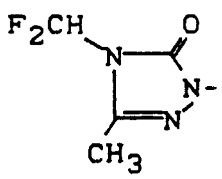 | F | $-O-CH(CH_3)_2$ |

| Het | $R^1$ | $R^2$ |
|---|---|---|
| 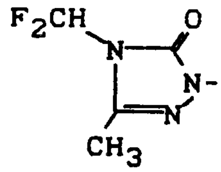 | F | $-O-CH_2-CH{\equiv}CH_2$ |
| 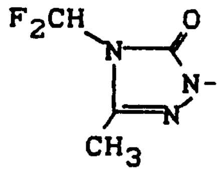 | F | $-O-CH_2-CH=CH-CH_3$ |
| 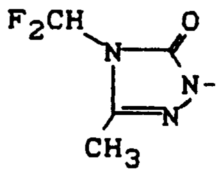 | F | $-O-CH_2-CH=CH-Cl$ |
| 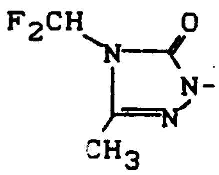 | F | $-O-\underset{CH_3}{\underset{\vert}{C}}H-CH=CH_2$ |
| 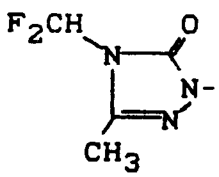 | F | $-O-CH_2-C{\equiv}CH$ |
| 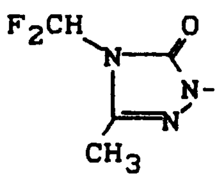 | F | $-O-\underset{CH_3}{\underset{\vert}{C}}H-C{\equiv}CH$ |
| 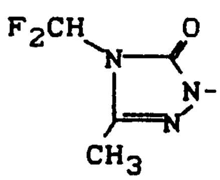 | F | $-O-CH_2-\underset{CH_3}{\underset{\vert}{C}}=CH_2$ |

| Het | $R^1$ | $R^2$ |
|---|---|---|
| 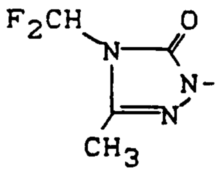 | F | $-O-CH_2-CH_2-OC_2H_5$ |
| 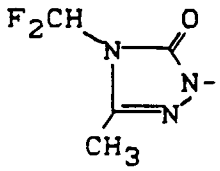 | F | 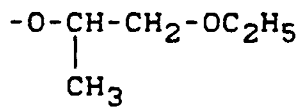 |
| 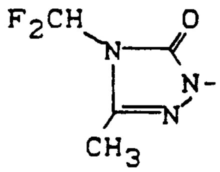 | F | 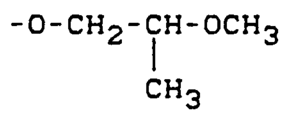 |
| 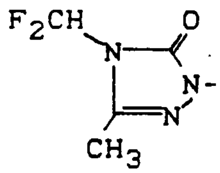 | F | 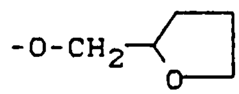 |
| 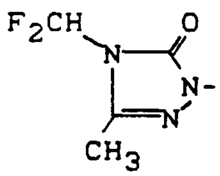 | F | 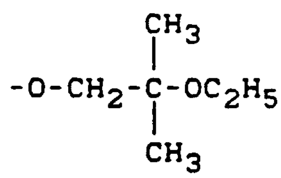 |
| 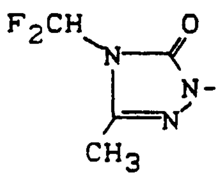 | F | $-O-CH_2-CH_2-O-CH_2-CH_2-OCH_3$ |
| 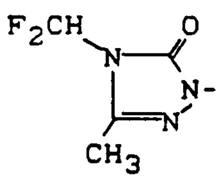 | F | $-O-CH_2-CH_2-O-CH_2-CH_2-OC_2H_5$ |

| Het | $R^1$ | $R^2$ |
|---|---|---|
| 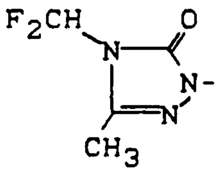 | F | 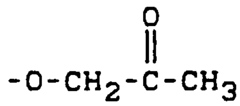 |
| 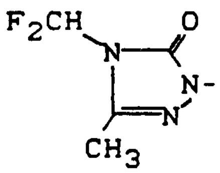 | F | 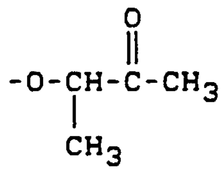 |
| 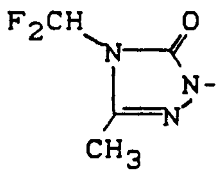 | H | $-SCH_3$ |
| 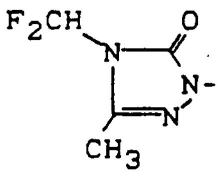 | H | $-SC_2H_5$ |
| 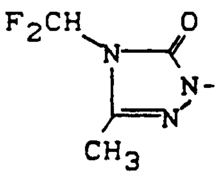 | H | $-S-CH(CH_3)_2$ |
| 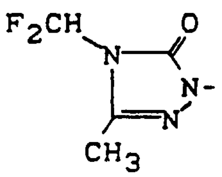 | H | $-S-CH_2-CH=CH_2$ |
| 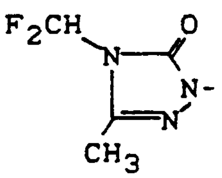 | H | $-S-CH_2-CH=CH-Cl$ |

| Het | $R^1$ | $R^2$ |
|---|---|---|
| 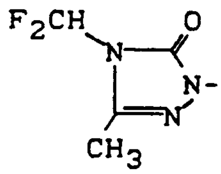 | H | $-S-CH_2-CH=CH-CH_3$ |
| 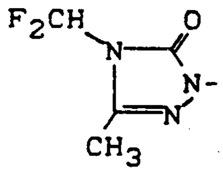 | H | $-S-CH-CH=CH_2$<br>\|<br>$CH_3$ |
| 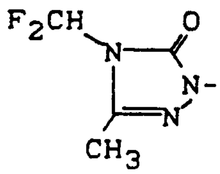 | H | $-S-CH_2-C\equiv CH$ |
| 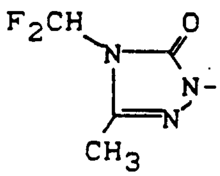 | H | $-S-CH-C\equiv CH$<br>\|<br>$CH_3$ |
| 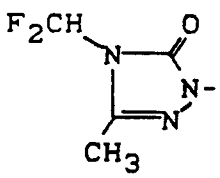 | H | $-S-CH_2-C=CH_2$<br>\|<br>$CH_3$ |
| 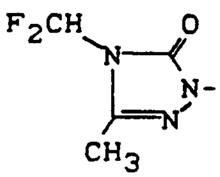 | H | $-S-CH_2-CH_2-OC_2H_5$ |
| 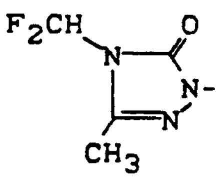 | H | $-S-CH-CH_2-OC_2H_5$ |

| Het | $R^1$ | $R^2$ |
|---|---|---|
| 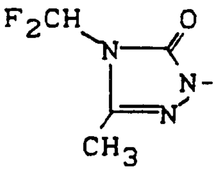 | H | 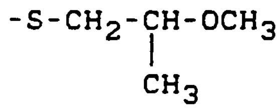 |
| 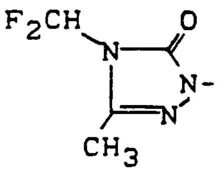 | H | 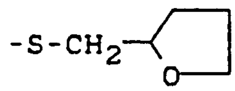 |
| 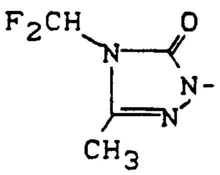 | H | 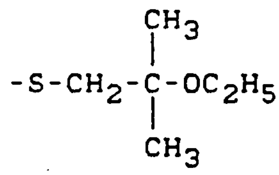 |
| 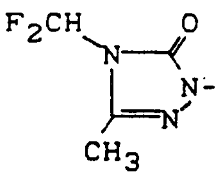 | H | $-S-CH_2-CH_2-O-CH_2-CH_2-OCH_3$ |
| 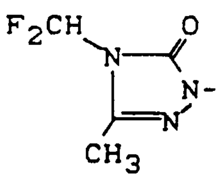 | H | $-S-CH_2-CH_2-O-CH_2-CH_2-OC_2H_5$ |
| 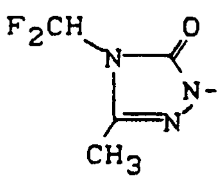 | H | 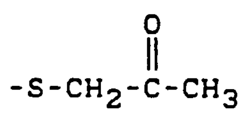 |
| 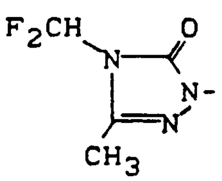 | H | 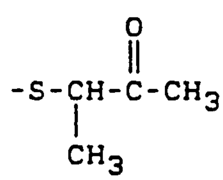 |

| Het | $R^1$ | $R^2$ |
|---|---|---|
| 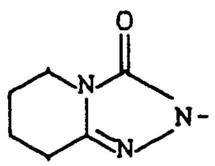 | F | 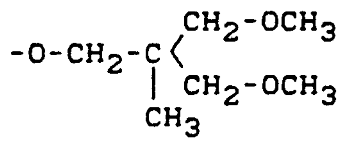 |
| 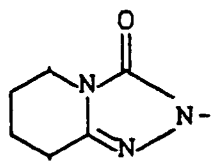 | F | 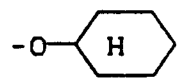 |
| 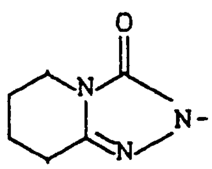 | F | 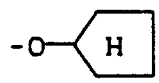 |
| 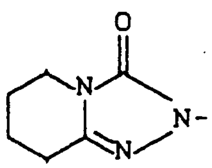 | F | 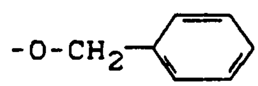 |
| 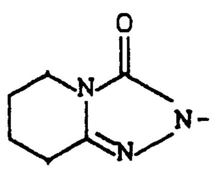 | F | $-O-CH_2-CN$ |
| 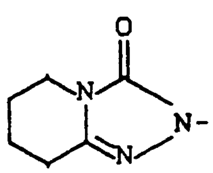 | F | $-O-CH_2-COOC_2H_5$ |
| 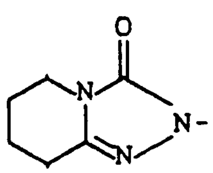 | F | 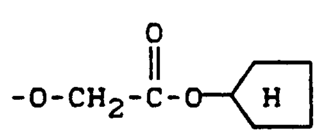 |

| Het | $R^1$ | $R^2$ |
|---|---|---|
| 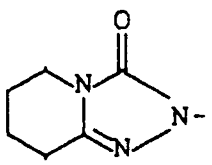 | F | 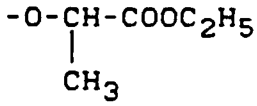 |
| 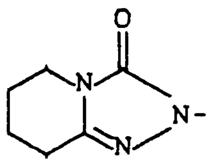 | F | 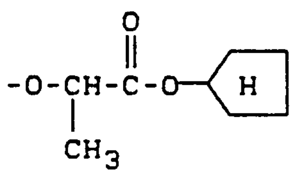 |
| 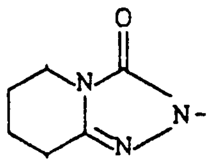 | F | 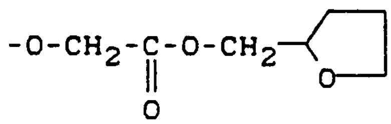 |
| 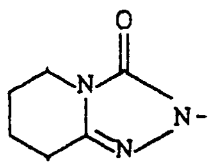 | F | 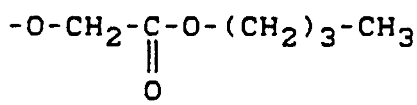 |
| 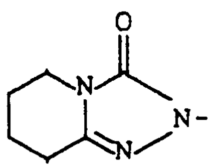 | F | 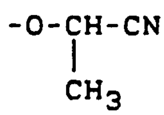 |
| 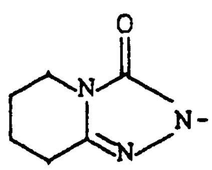 | F | $-O-CH_2-CH_2-O-CH_3$ |

| Het | $R^1$ | $R^2$ |
|---|---|---|
| 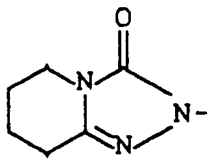 | F | 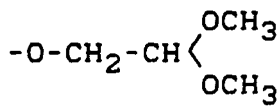 |
| 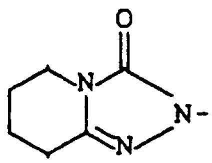 | F | 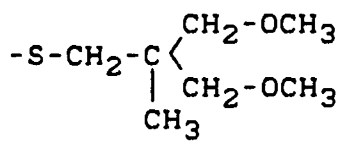 |
| 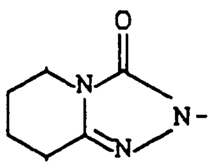 | F | 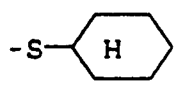 |
| 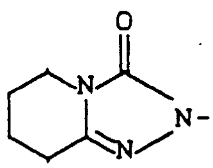 | F | 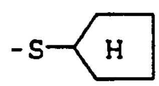 |
| 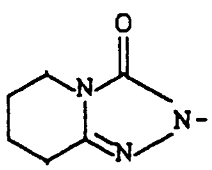 | F | 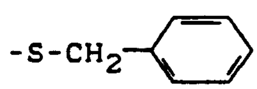 |
| 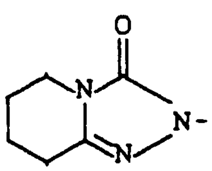 | F | $-S-CH_2-CN$ |
| 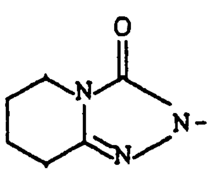 | F | $-S-CH_2-COOC_2H_5$ |

| Het | $R^1$ | $R^2$ |
|---|---|---|
| 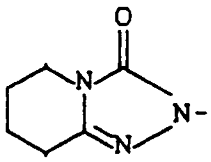 | F | 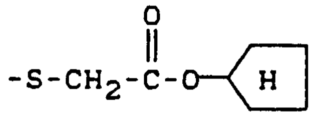 |
| 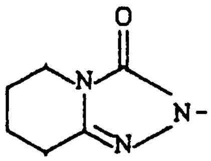 | F | 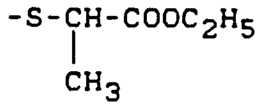 |
| 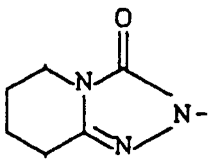 | F | 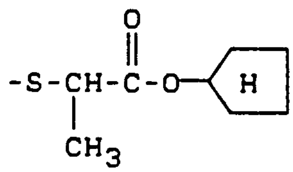 |
| 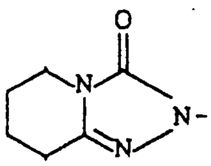 | F | 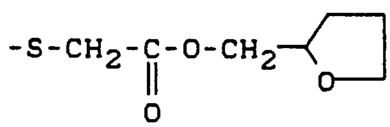 |
| 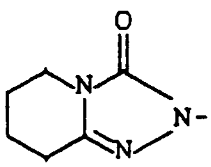 | F | 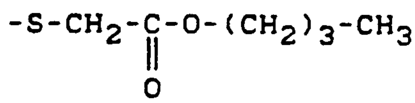 |
| 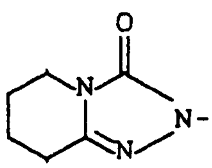 | F | 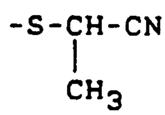 |

| Het | $R^1$ | $R^2$ |
|---|---|---|
| 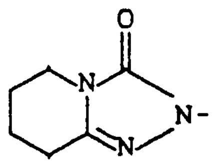 | F | $-S-CH_2-CH_2-O-CH_3$ |
| 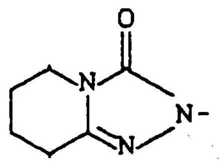 | F | $-S-CH_2-CH\langle{}^{OCH_3}_{OCH_3}$ |
| 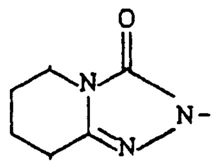 | F | $-OCH_3$ |
| 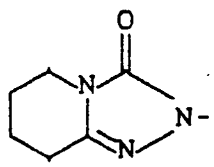 | F | $-OC_2H_5$ |
| 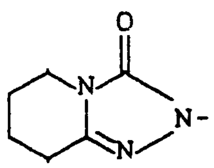 | F | $-O-CH(CH_3)_2$ |
| 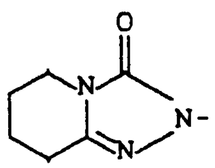 | F | $-O-CH_2-CH=CH_2$ |
| 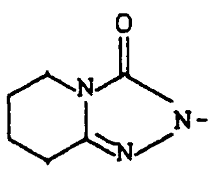 | F | $-O-CH_2-CH=CH-CH_3$ |

| Het | $R^1$ | $R^2$ |
|---|---|---|
| 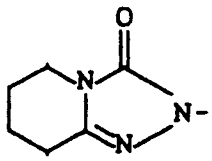 | F | $-O-CH_2-CH=CH-Cl$ |
| 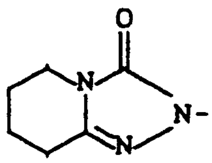 | F | $-O-CH-CH=CH_2$<br>$CH_3$ |
| 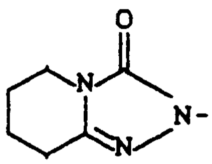 | F | $-O-CH_2-C\equiv CH$ |
| 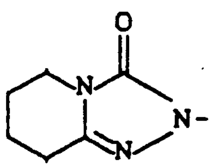 | F | $-O-CH-C\equiv CH$<br>$CH_3$ |
| 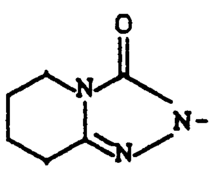 | F | $-O-CH_2-C=CH_2$<br>$CH_3$ |
| 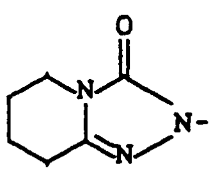 | F | $-O-CH_2-CH_2-OC_2H_5$ |
| 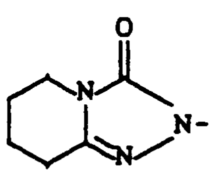 | F | $-O-CH-CH_2-OC_2H_5$<br>$CH_3$ |

| Het | $R^1$ | $R^2$ |
|---|---|---|
| 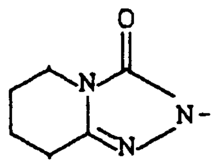 | F | 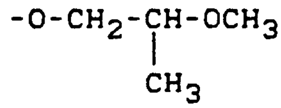 |
| 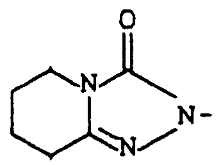 | F | 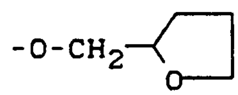 |
| 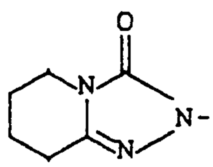 | F | 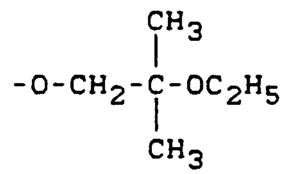 |
| 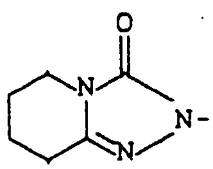 | F | $-O-CH_2-CH_2-O-CH_2-CH_2-OCH_3$ |
| 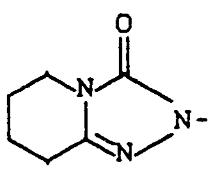 | F | $-O-CH_2-CH_2-O-CH_2-CH_2-OC_2H_5$ |
| 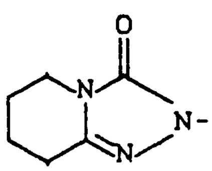 | F | 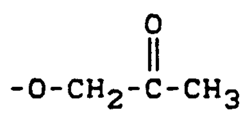 |
| 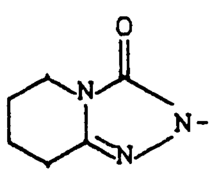 | F | 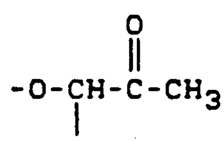 |

| Het | $R^1$ | $R^2$ |
|---|---|---|
| 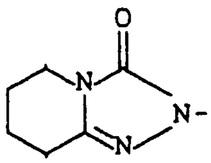 | F | $-SCH_3$ |
| 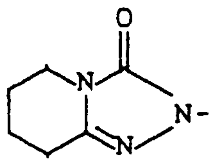 | F | $-SC_2H_5$ |
| 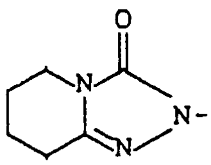 | F | $-S-CH(CH_3)_2$ |
| 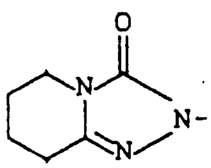 | F | $-S-CH_2-CH=CH_2$ |
| 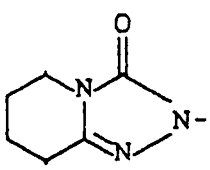 | F | $-S-CH_2-CH=CH-Cl$ |
| 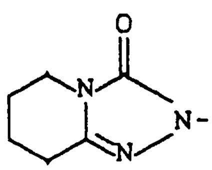 | F | $-S-CH_2-CH=CH-CH_3$ |
| 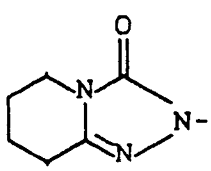 | F | $-S-\underset{CH_3}{\underset{\vert}{CH}}-CH=CH_2$ |

| Het | $R^1$ | $R^2$ |
|---|---|---|
| 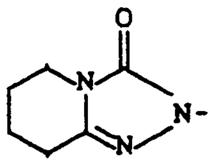 | F | $-S-CH_2-C\equiv CH$ |
| 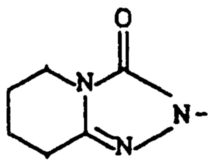 | F | $-S-CH(CH_3)-C\equiv CH$ |
| 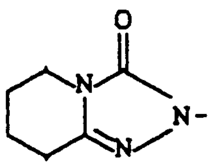 | F | $-S-CH_2-C(CH_3)=CH_2$ |
| 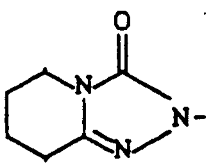 | F | $-S-CH_2-CH_2-OC_2H_5$ |
| 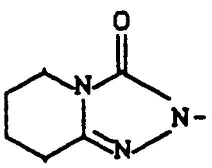 | F | $-S-CH(CH_3)-CH_2-OC_2H_5$ |
| 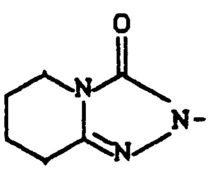 | F | $-S-CH_2-CH(CH_3)-OCH_3$ |

| Het | $R^1$ | $R^2$ |
|---|---|---|
| 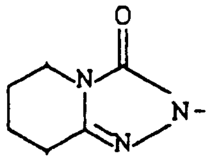 | F | 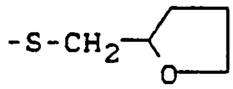 |
| 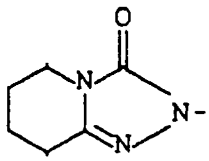 | F | 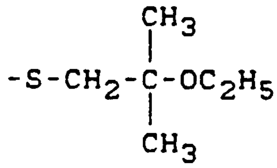 |
| 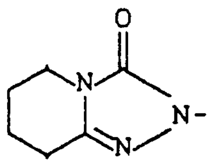 | F | $-S-CH_2-CH_2-O-CH_2-CH_2-OCH_3$ |
| 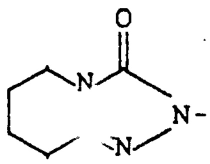 | F | $-S-CH_2-CH_2-O-CH_2-CH_2-OC_2H_5$ |
| 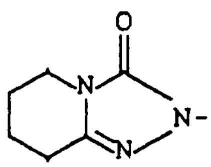 | F | 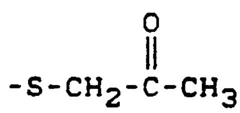 |
| 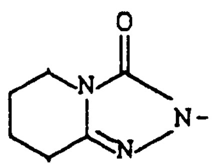 | F | 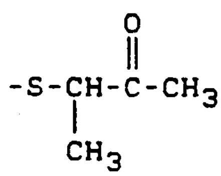 |
| 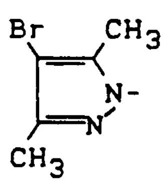 | H | 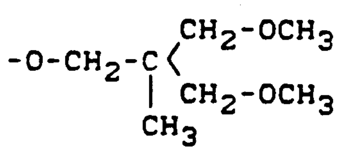 |

| Het | $R^1$ | $R^2$ |
|---|---|---|
| 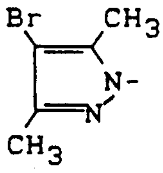 | H | 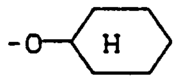 |
| 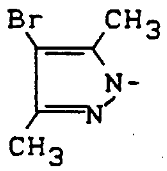 | H | 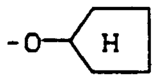 |
| 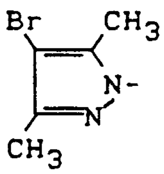 | H | 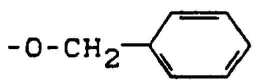 |
| 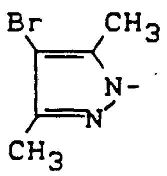 | H | $-O-CH_2-CN$ |
| 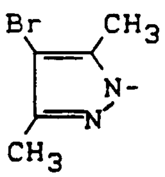 | H | $-O-CH_2-COOC_2H_5$ |
| 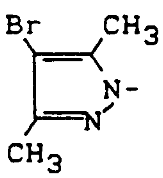 | H | 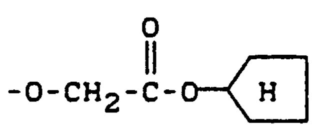 |
| 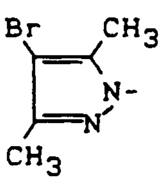 | H | 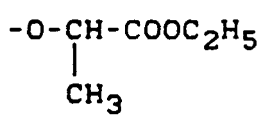 |

| Het | $R^1$ | $R^2$ |
|---|---|---|
| 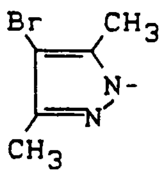 | H | 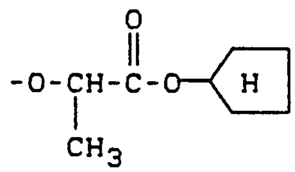 |
| 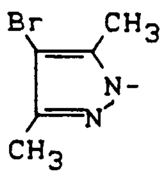 | H | 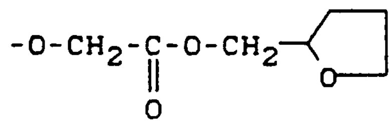 |
| 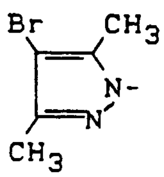 | H | 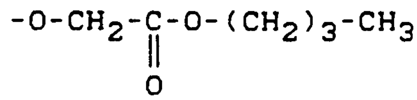 |
| 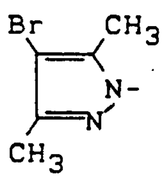 | H | 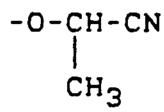 |
| 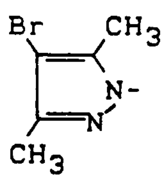 | H | $-O-CH_2-CH_2-O-CH_3$ |
| 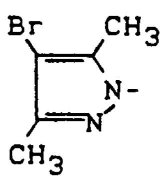 | H | 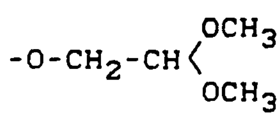 |
| 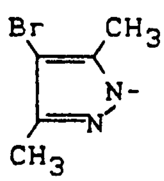 | H | 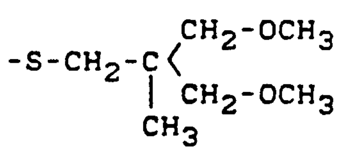 |

| Het | $R^1$ | $R^2$ |
|---|---|---|
| 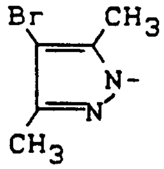 | H | 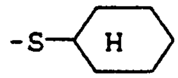 |
| 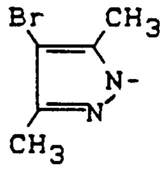 | H | 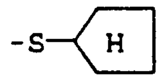 |
| 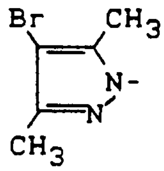 | H | 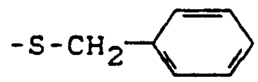 |
| 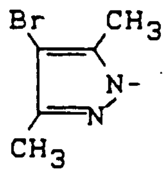 | H | $-S-CH_2-CN$ |
| 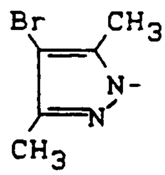 | H | $-S-CH_2-COOC_2H_5$ |
| 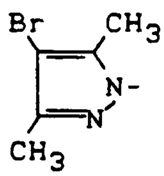 | H | 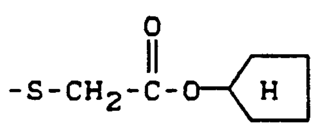 |
| 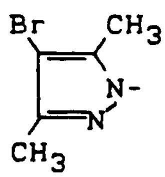 | H | 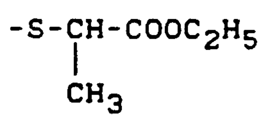 |

| Het | $R^1$ | $R^2$ |
|---|---|---|
| 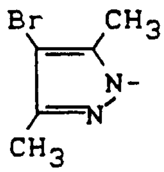 | H | 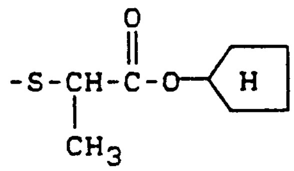 |
| 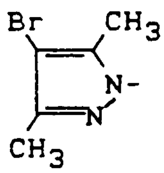 | H | 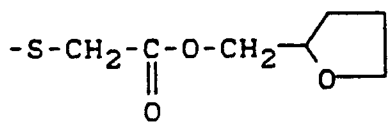 |
| 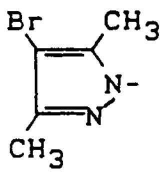 | H | 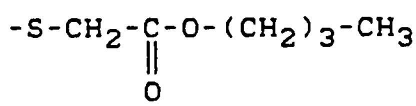 |
| 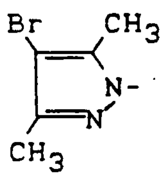 | H | 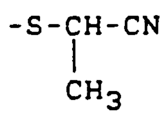 |
| 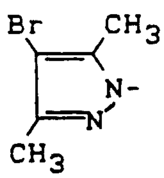 | H | $-S-CH_2-CH_2-O-CH_3$ |
| 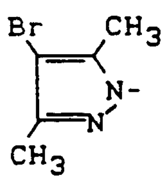 | H | 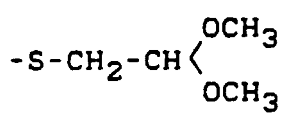 |
| 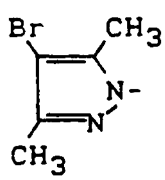 | F | $-OCH_3$ |

| Het | $R^1$ | $R^2$ |
|---|---|---|
| 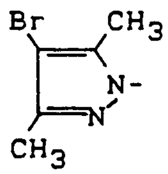 | F | $-OC_2H_5$ |
| 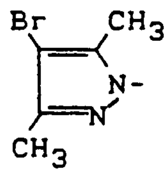 | F | $-O-CH(CH_3)_2$ |
| 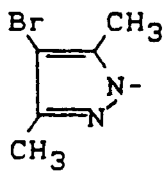 | F | $-O-CH_2-CH=CH_2$ |
| 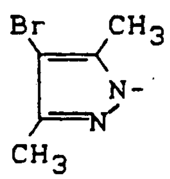 | F | $-O-CH_2-CH=CH-CH_3$ |
| 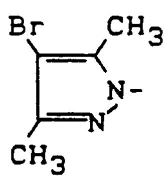 | F | $-O-CH_2-CH=CH-Cl$ |
| 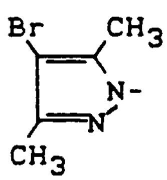 | F | $-O-\underset{CH_3}{\underset{\mid}{CH}}-CH=CH_2$ |
| 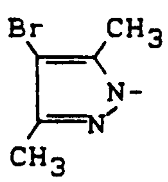 | F | $-O-CH_2-C\equiv CH$ |

| Het | $R^1$ | $R^2$ |
|---|---|---|
| 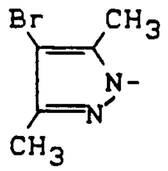 | F | 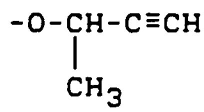 |
| 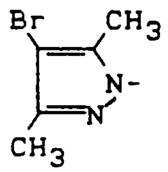 | F | 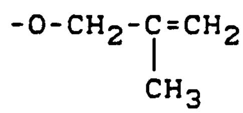 |
| 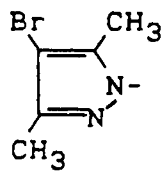 | F | $-O-CH_2-CH_2-OC_2H_5$ |
| 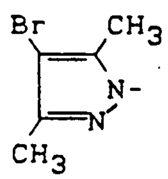 | F | 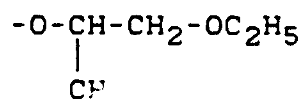 |
| 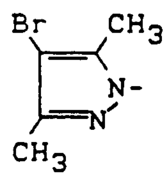 | F | 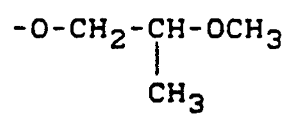 |
| 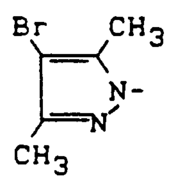 | F | 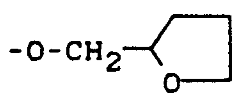 |
| 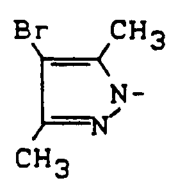 | F | 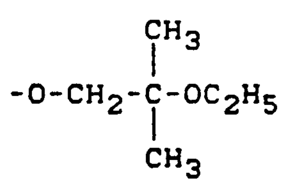 |

| Het | $R^1$ | $R^2$ |
|---|---|---|
| 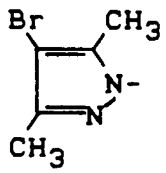 | F | $-O-CH_2-CH_2-O-CH_2-CH_2-OCH_3$ |
| 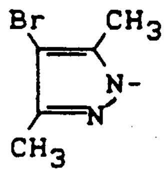 | F | $-O-CH_2-CH_2-O-CH_2-CH_2-OC_2H_5$ |
| 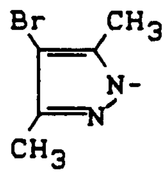 | F | 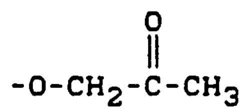 |
| 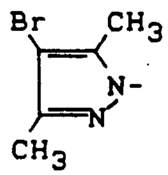 | F | 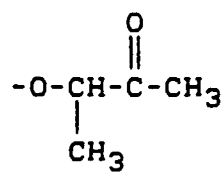 |
| 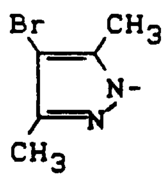 | F | $-SCH_3$ |
| 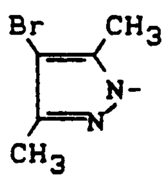 | F | $-SC_2H_5$ |
| 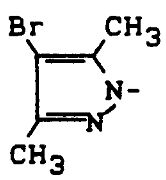 | F | $-S-CH(CH_3)_2$ |

| Het | $R^1$ | $R^2$ |
|---|---|---|
| 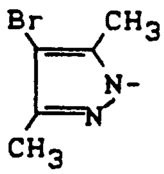 | F | $-S-CH_2-CH=CH_2$ |
| 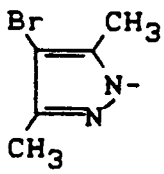 | F | $-S-CH_2-CH=CH-Cl$ |
| 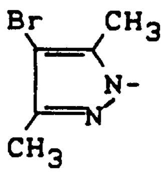 | F | $-S-CH_2-CH=CH-CH_3$ |
| 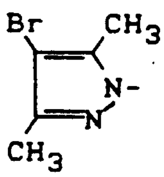 | F | $-S-\underset{CH_3}{\underset{\vert}{CH}}-CH=CH_2$ |
| 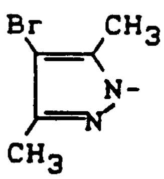 | F | $-S-CH_2-C\equiv CH$ |
| 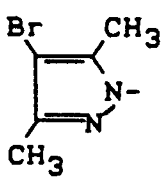 | F | $-S-\underset{CH_3}{\underset{\vert}{CH}}-C\equiv CH$ |
| 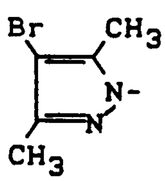 | F | $-S-CH_2-\underset{CH_3}{\underset{\vert}{C}}=CH_2$ |

| Het | $R^1$ | $R^2$ |
|---|---|---|
| 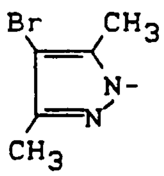 | F | $-S-CH_2-CH_2-OC_2H_5$ |
| 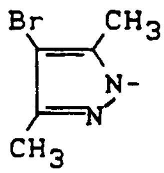 | F | $-S-CH-CH_2-OC_2H_5$ |
| 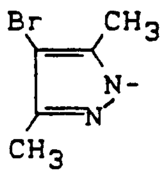 | F | 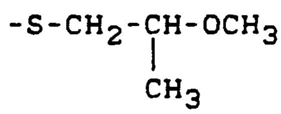 |
| 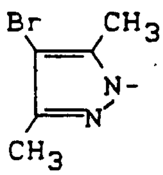 | F | 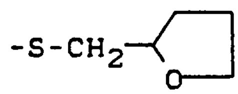 |
| 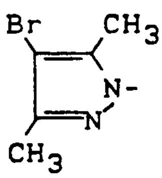 | F | 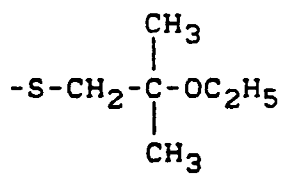 |
| 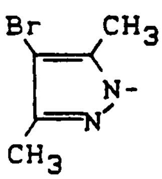 | F | $-S-CH_2-CH_2-O-CH_2-CH_2-OCH_3$ |
| 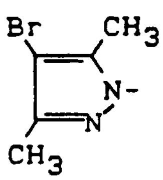 | F | $-S-CH_2-CH_2-O-CH_2-CH_2-OC_2H_5$ |

| Het | $R^1$ | $R^2$ |
|---|---|---|
| 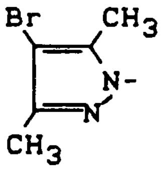 | F | 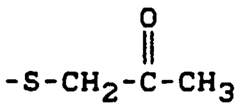 |
| 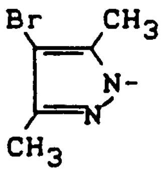 | F | 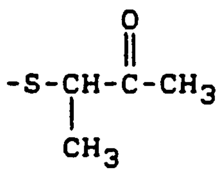 |
| 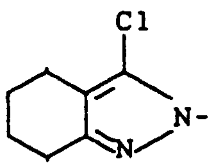 | H | 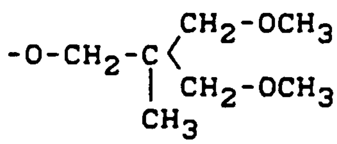 |
| 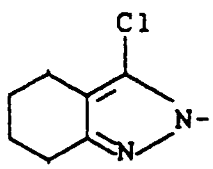 | H | 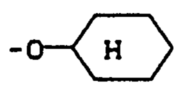 |
| 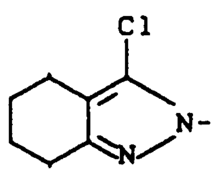 | H | 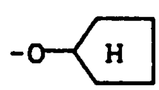 |
| 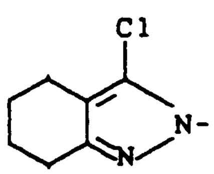 | H | 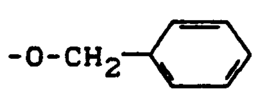 |
| 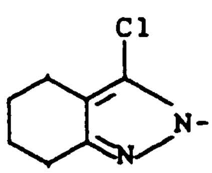 | H | $-O-CH_2-CN$ |

| Het | $R^1$ | $R^2$ |
|---|---|---|
| 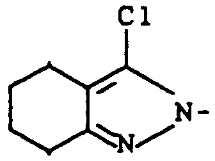 | H | $-O-CH_2-COOC_2H_5$ |
| 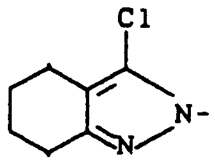 | H | 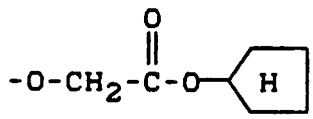 |
| 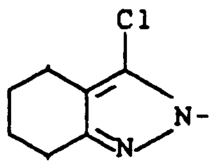 | H | 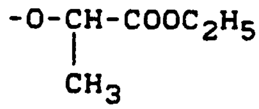 |
| 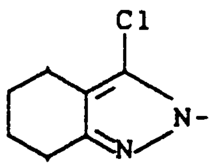 | H | 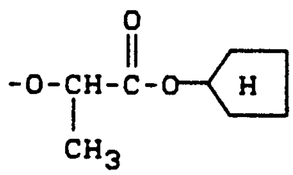 |
| 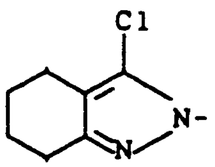 | H | 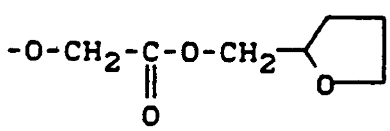 |
| 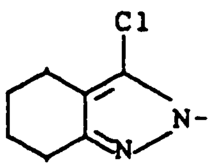 | H | 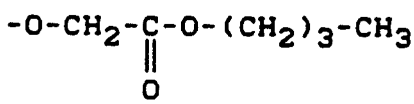 |

| Het | $R^1$ | $R^2$ |
|---|---|---|
| 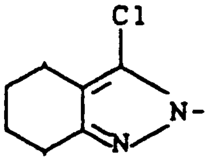 | H | 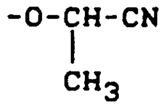 |
| 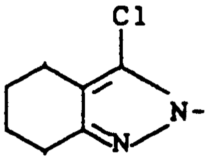 | H | $-O-CH_2-CH_2-O-CH_3$ |
| 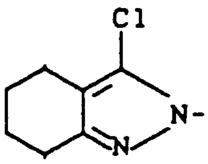 | H | 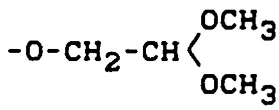 |
| 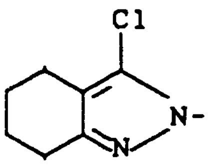 | H | 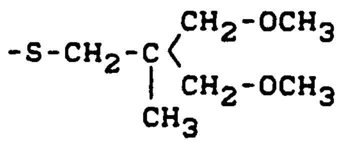 |
| 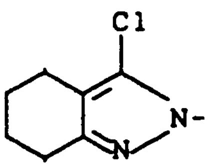 | H | 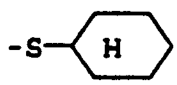 |
| 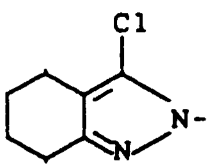 | H | 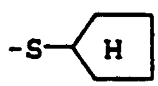 |
| 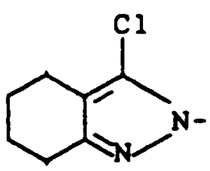 | H | 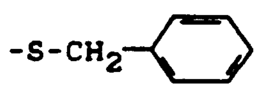 |

| Het | $R^1$ | $R^2$ |
|---|---|---|
| 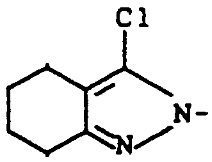 | H | $-S-CH_2-CN$ |
| 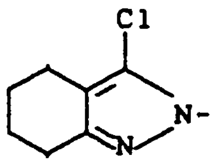 | H | $-S-CH_2-COOC_2H_5$ |
| 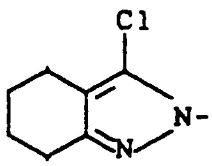 | H | 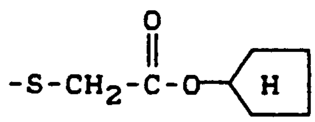 |
| 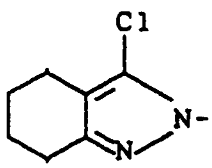 | H | 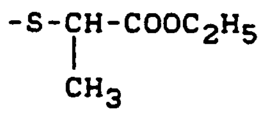 |
| 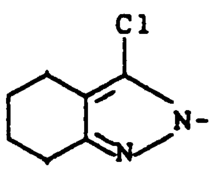 | H | 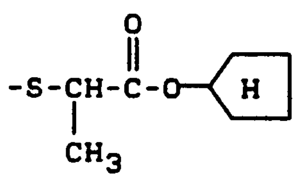 |
| 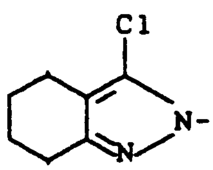 | H | 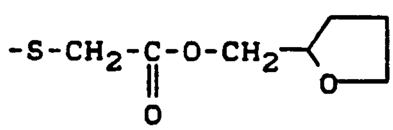 |
| 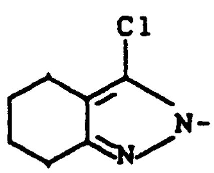 | H | 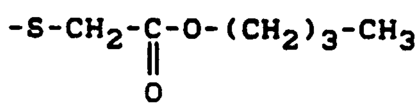 |

| Het | $R^1$ | $R^2$ |
|---|---|---|
| 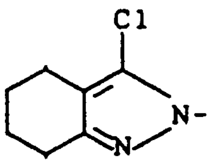 | H | $-S-CH-CN$<br>$CH_3$ |
| 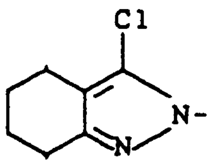 | H | $-S-CH_2-CH_2-O-CH_3$ |
| 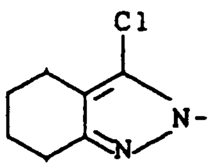 | H | $OCH_3$<br>$-S-CH_2-CH\langle$<br>$OCH_3$ |
| 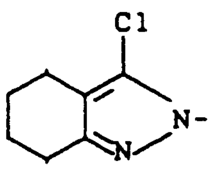 | H | $-OCH_3$ |
| 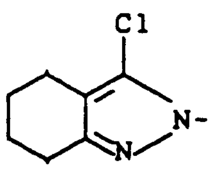 | H | $-OC_2H_5$ |
| 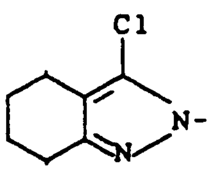 | H | $-O-CH(CH_3)_2$ |
| 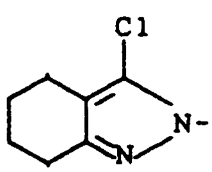 | H | $-O-CH_2-CH=CH_2$ |

| Het | $R^1$ | $R^2$ |
|---|---|---|
| 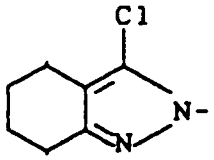 | H | $-O-CH_2-CH=CH-CH_3$ |
| 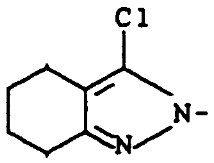 | H | $-O-CH_2-CH=CH-Cl$ |
| 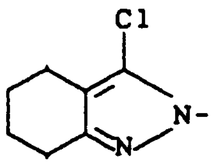 | H | $-O-CH(CH_3)-CH=CH_2$ |
| 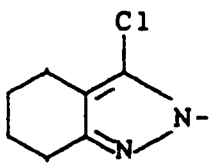 | H | $-O-CH_2-C\equiv CH$ |
| 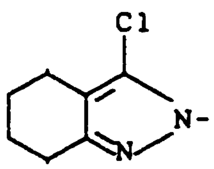 | H | $-O-CH(CH_3)-C\equiv CH$ |
| 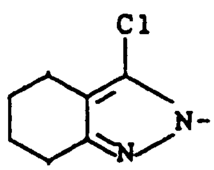 | H | $-O-CH_2-C(CH_3)=CH_2$ |
| 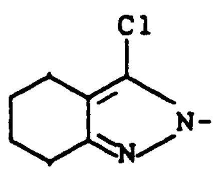 | H | $-O-CH_2-CH_2-OC_2H_5$ |

| Het | $R^1$ | $R^2$ |
|---|---|---|
| 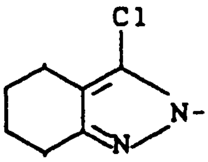 | H | 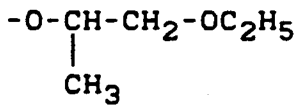 |
| 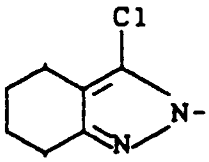 | H | 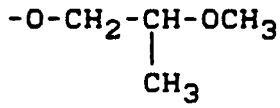 |
| 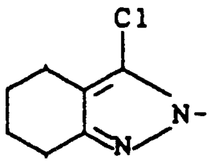 | H | 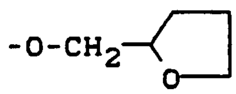 |
| 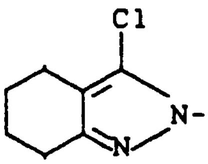 | H | 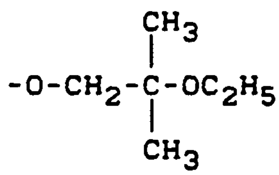 |
| 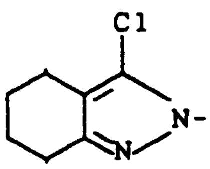 | H | $-O-CH_2-CH_2-O-CH_2-CH_2-OCH_3$ |
| 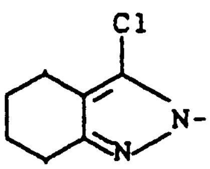 | H | $-O-CH_2-CH_2-O-CH_2-CH_2-OC_2H_5$ |
| 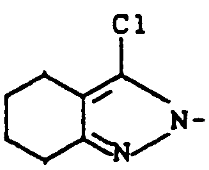 | H | 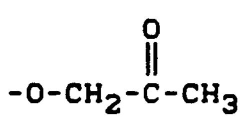 |

| Het | $R^1$ | $R^2$ |
|---|---|---|
| 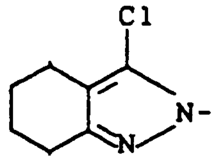 | H | 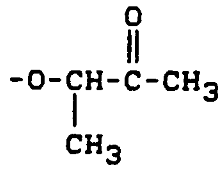 |
| 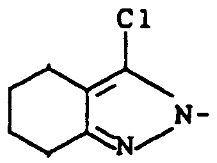 | H | $-SCH_3$ |
| 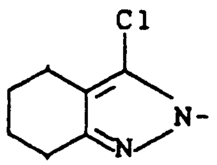 | H | $-SC_2H_5$ |
| 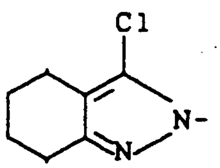 | H | $-S-CH(CH_3)_2$ |
| 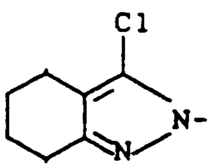 | H | $-S-CH_2-CH=CH_2$ |
| 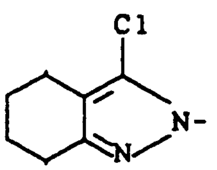 | H | $-S-CH_2-CH=CH-Cl$ |
| 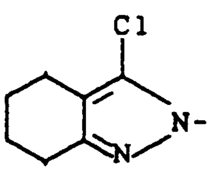 | H | $-S-CH_2-CH=CH-CH_3$ |

| Het | $R^1$ | $R^2$ |
|---|---|---|
| 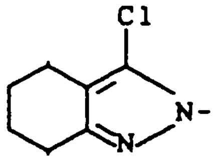 | H | $-S-CH-CH=CH_2$<br>$CH_3$ |
| 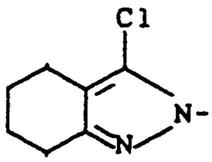 | H | $-S-CH_2-C\equiv CH$ |
| 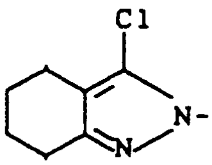 | H | $-S-CH-C\equiv CH$<br>$CH_3$ |
| 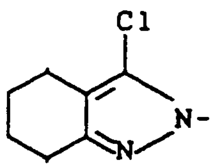 | H | $-S-CH_2-C=CH_2$<br>$CH_3$ |
| 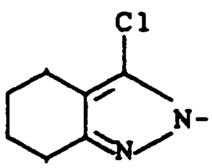 | H | $-S-CH_2-CH_2-OC_2H_5$ |
| 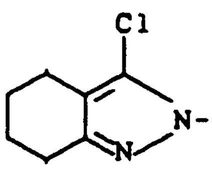 | H | $-S-CH-CH_2-OC_2H_5$<br>$CH_3$ |

| Het | $R^1$ | $R^2$ |
|---|---|---|
| 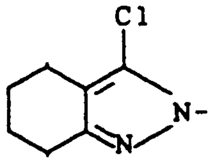 | H | 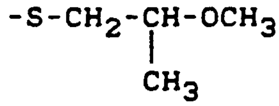 |
| 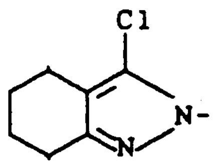 | H | 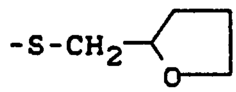 |
| 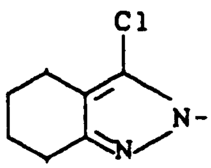 | H | 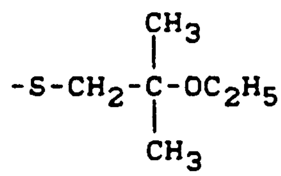 |
| 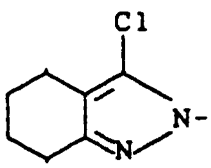 | H | $-S-CH_2-CH_2-O-CH_2-CH_2-OCH_3$ |
| 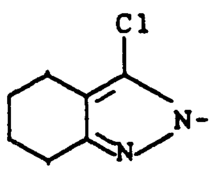 | H | $-S-CH_2-CH_2-O-CH_2-CH_2-OC_2H_5$ |
| 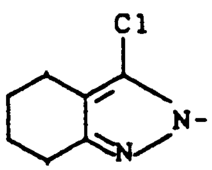 | H | 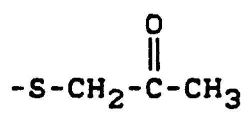 |
| 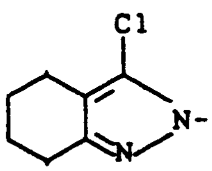 | H | 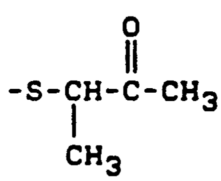 |

| Het | $R^1$ | $R^2$ |
|---|---|---|
| 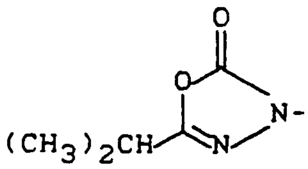 | F | 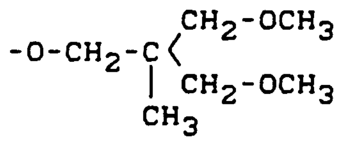 |
| 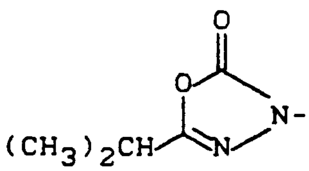 | F | 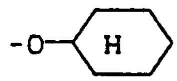 |
| 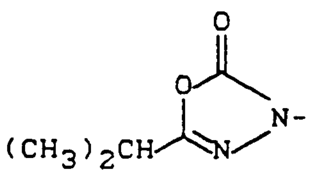 | F | 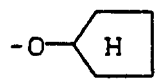 |
| 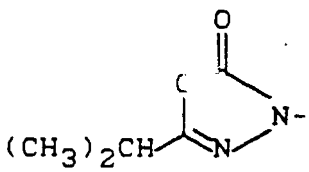 | F | 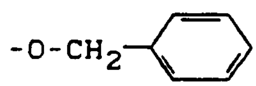 |
| 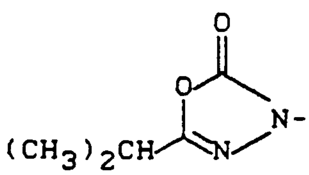 | F | $-O-CH_2-CN$ |
| 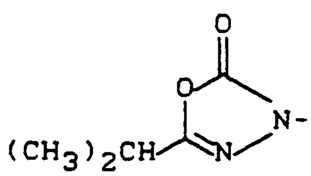 | F | $-O-CH_2-COOC_2H_5$ |
| 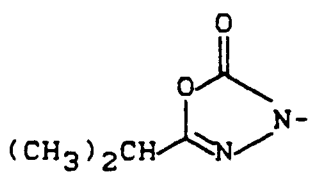 | F | 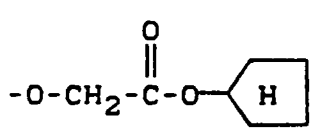 |

| Het | $R^1$ | $R^2$ |
|---|---|---|
| 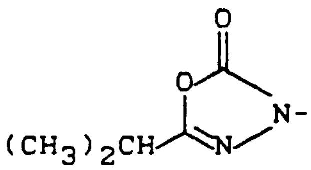 | F | 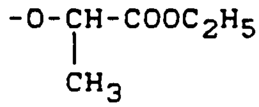 |
| 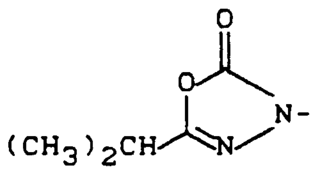 | F | 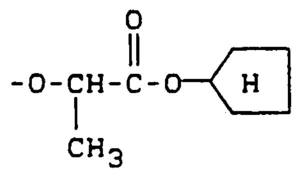 |
| 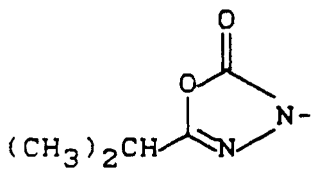 | F | 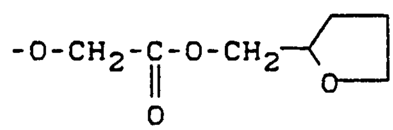 |
| 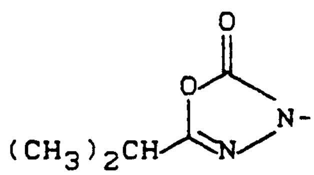 | F | 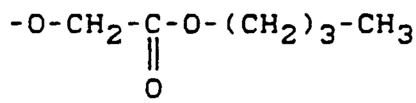 |
| 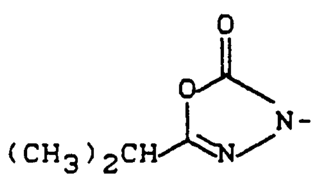 | F | 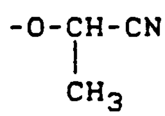 |
| 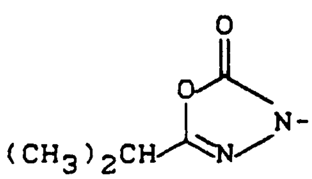 | F | $-O-CH_2-CH_2-O-CH_3$ |

| Het | $R^1$ | $R^2$ |
|---|---|---|
| 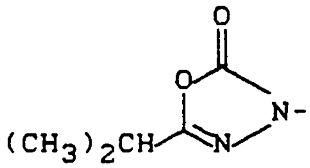 | F | 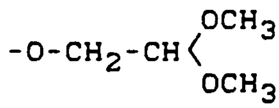 |
| 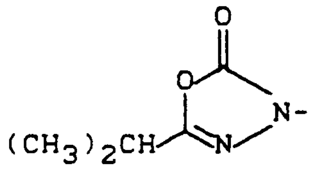 | F | 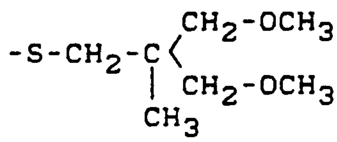 |
| 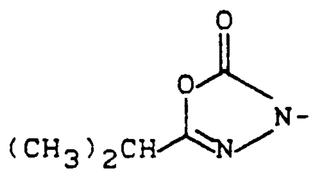 | F | 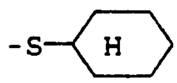 |
| 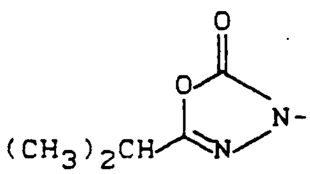 | F | 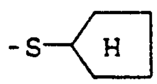 |
| 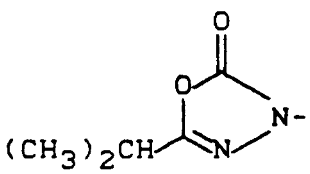 | F | 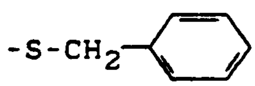 |
| 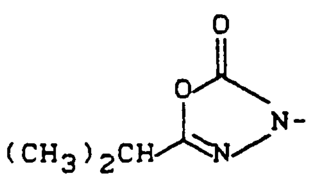 | F | $-S-CH_2-CN$ |
| 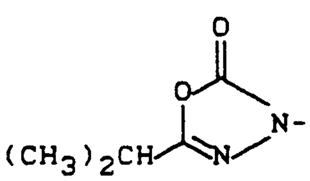 | F | $-S-CH_2-COOC_2H_5$ |

| Het | $R^1$ | $R^2$ |
|---|---|---|
| 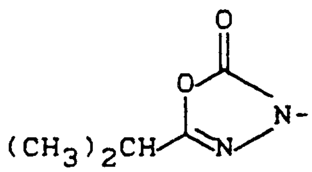 | F | 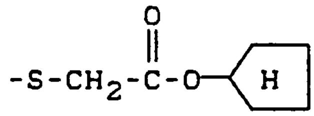 |
| 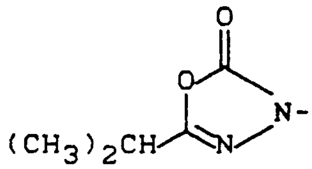 | F | 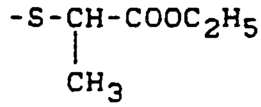 |
| 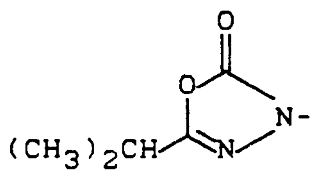 | F | 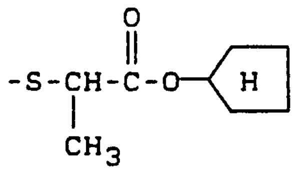 |
| 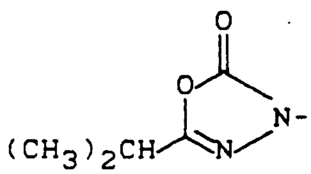 | F | 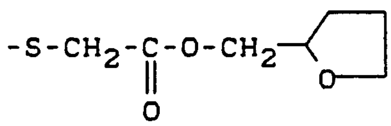 |
| 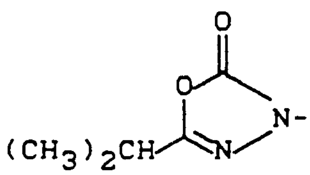 | F | 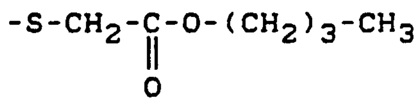 |
| 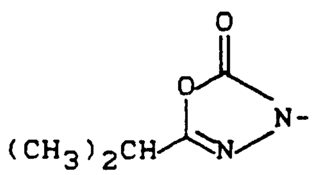 | F | 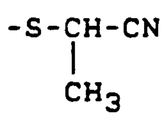 |

| Het | $R^1$ | $R^2$ |
|---|---|---|
| 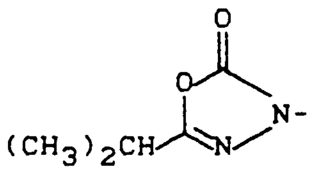 | F | $-S-CH_2-CH_2-O-CH_3$ |
| 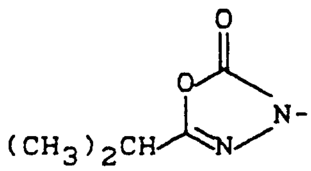 | F | $-S-CH_2-CH\langle^{OCH_3}_{OCH_3}$ |
| 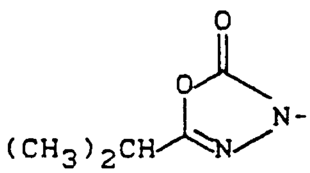 | F | $-OCH_3$ |
| 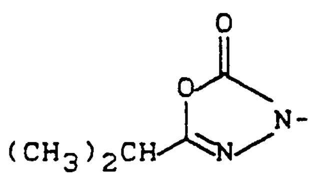 | F | $-OC_2H_5$ |
| 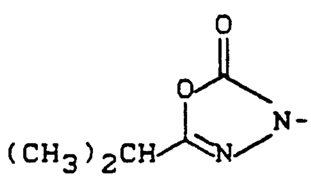 | F | $-O-CH(CH_3)_2$ |
| 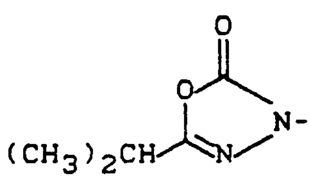 | F | $-O-CH_2-CH=CH_2$ |
| 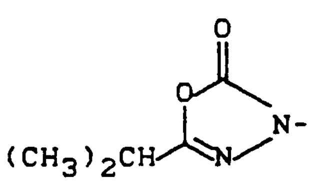 | F | $-O-CH_2-CH=CH-CH_3$ |

| Het | $R^1$ | $R^2$ |
|---|---|---|
| 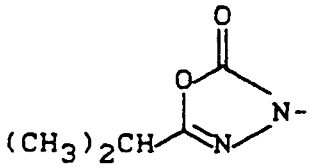 | F | $-O-CH_2-CH=CH-Cl$ |
| 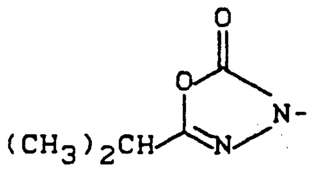 | F | $-O-CH(CH_3)-CH=CH_2$ |
| 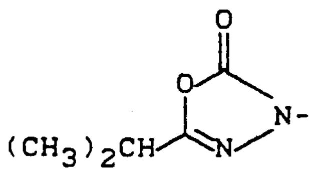 | F | $-O-CH_2-C\equiv CH$ |
| 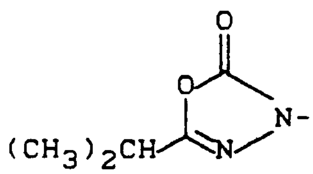 | F | $-O-CH(CH_3)-C\equiv CH$ |
| 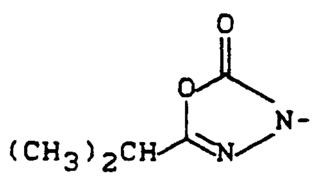 | F | $-O-CH_2-C(CH_3)=CH_2$ |
| 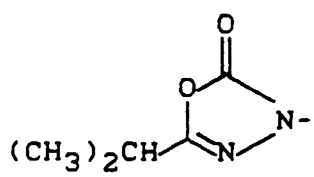 | F | $-O-CH_2-CH_2-OC_2H_5$ |
| 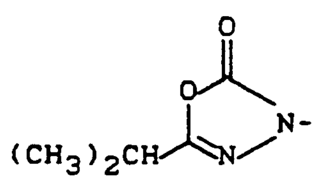 | F | $-O-CH(CH_3)-CH_2-OC_2H_5$ |

| Het | $R^1$ | $R^2$ |
|---|---|---|
| 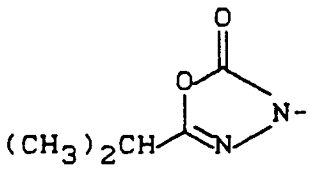 | F | 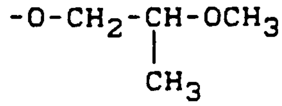 |
| 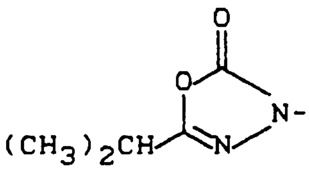 | F | 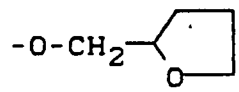 |
| 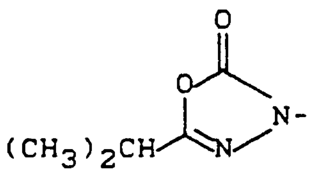 | F | 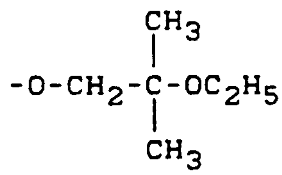 |
| 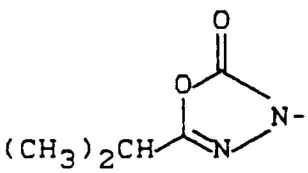 | F | $-O-CH_2-CH_2-O-CH_2-CH_2-OCH_3$ |
| 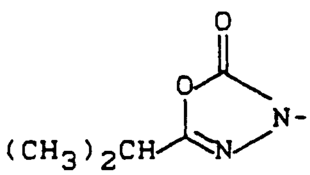 | F | $-O-CH_2-CH_2-O-CH_2-CH_2-OC_2H_5$ |
| 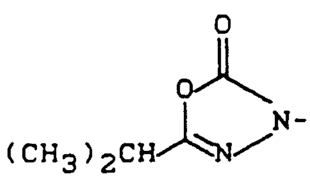 | F | 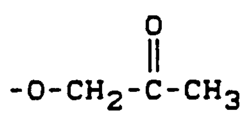 |
| 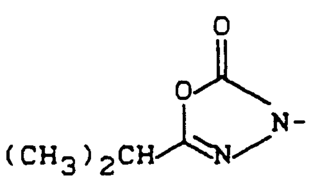 | F | 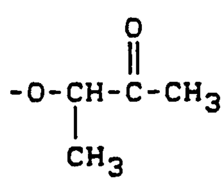 |

| Het | $R^1$ | $R^2$ |
|---|---|---|
| 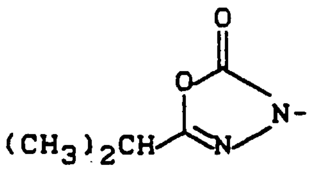 | H | $-SCH_3$ |
| 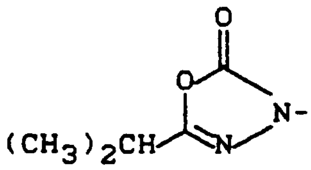 | H | $-SC_2H_5$ |
| 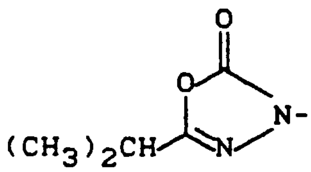 | H | $-S-CH(CH_3)_2$ |
| 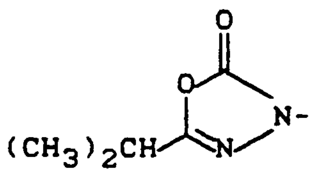 | H | $-S-CH_2-CH=CH_2$ |
| 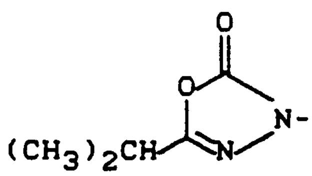 | H | $-S-CH_2-CH=CH-Cl$ |
| 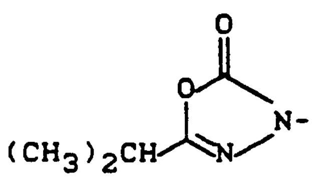 | H | $-S-CH_2-CH=CH-CH_3$ |
| 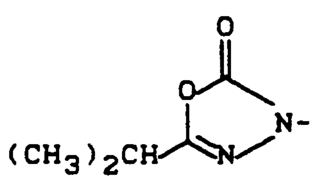 | H | $-S-\underset{\displaystyle CH_3}{\underset{\vert}{CH}}-CH=CH_2$ |

| Het | $R^1$ | $R^2$ |
|---|---|---|
| 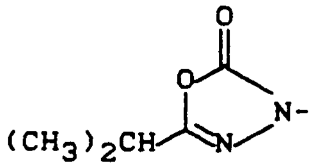 | H | $-S-CH_2-C\equiv CH$ |
| 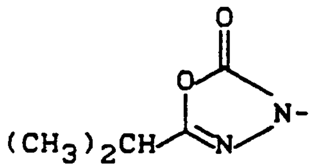 | H | $-S-CH-C\equiv CH$<br>$CH_3$ |
| 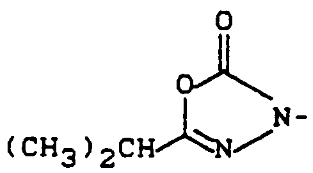 | H | $-S-CH_2-C=CH_2$<br>$CH_3$ |
| 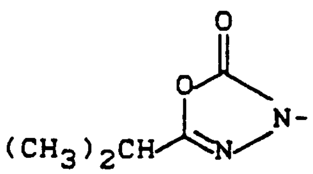 | H | $-S-CH_2-CH_2-OC_2H_5$ |
| 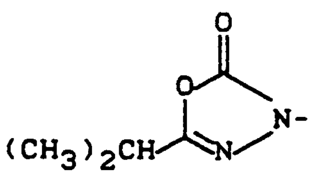 | H | $-S-CH-CH_2-OC_2H_5$<br>$CH_3$ |
| 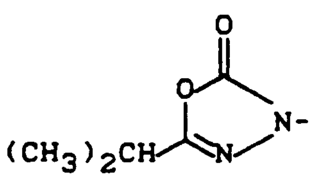 | H | $-S-CH_2-CH-OCH_3$<br>$CH_3$ |

| Het | $R^1$ | $R^2$ |
|---|---|---|
| 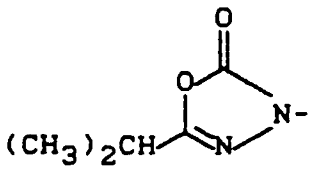 | H | 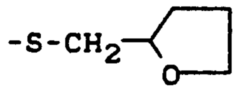 |
| 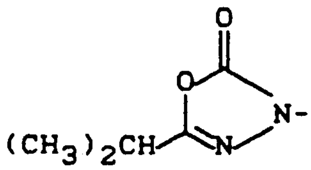 | H | 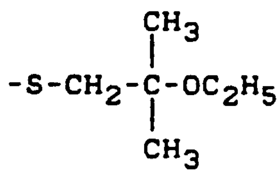 |
| 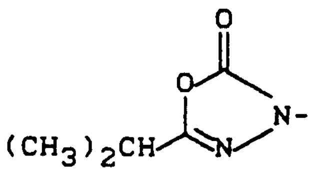 | H | $-S-CH_2-CH_2-O-CH_2-CH_2-OCH_3$ |
| 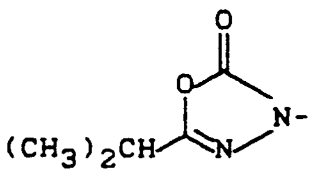 | H | $-S-CH_2-CH_2-O-CH_2-CH_2-OC_2H_5$ |
| 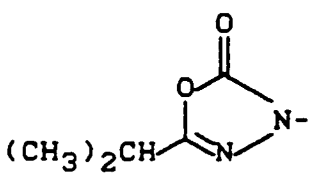 | H | 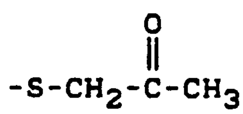 |
| 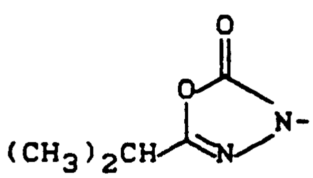 | H | 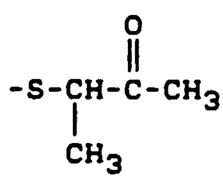 |
| 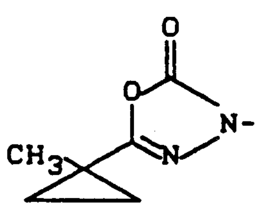 | F | 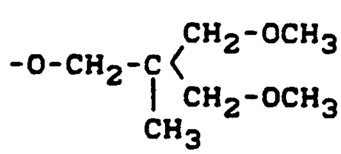 |

| Het | $R^1$ | $R^2$ |
|---|---|---|
| 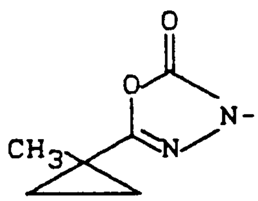 | F | 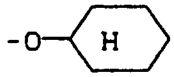 |
| 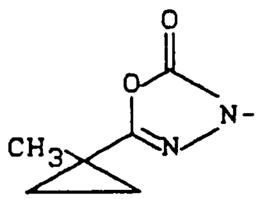 | F | 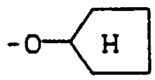 |
| 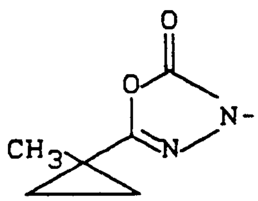 | F | 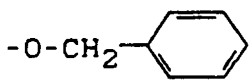 |
| 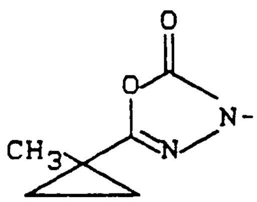 | F | $-O-CH_2-CN$ |
| 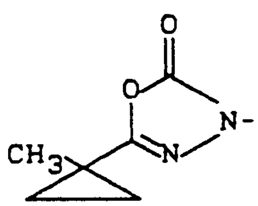 | F | $-O-CH_2-COOC_2H_5$ |
| 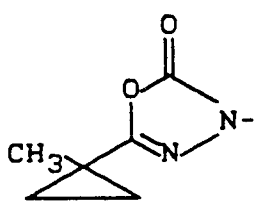 | F | 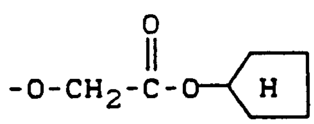 |

| Het | $R^1$ | $R^2$ |
|---|---|---|
| 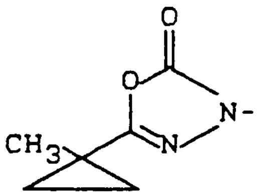 | F | 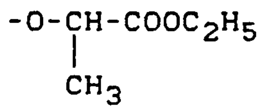 |
| 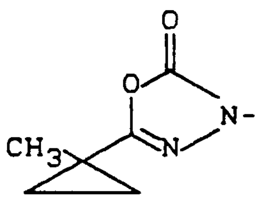 | F | 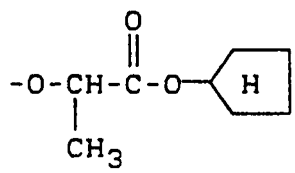 |
| 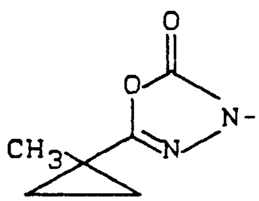 | F | 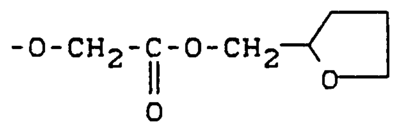 |
| 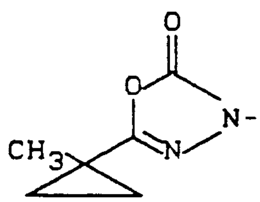 | F | 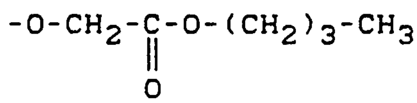 |
| 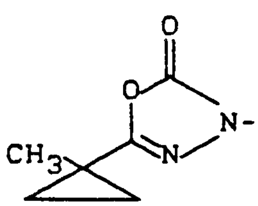 | F | 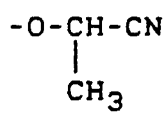 |
| 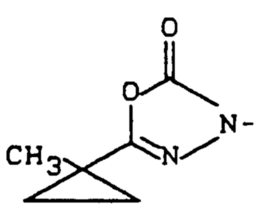 | F | $-O-CH_2-CH_2-O-CH_3$ |

| Het | $R^1$ | $R^2$ |
|---|---|---|
| 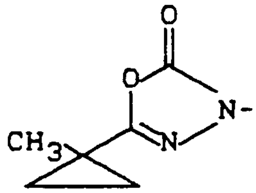 | F | 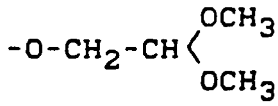 |
| 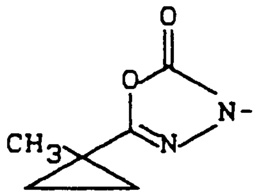 | H | 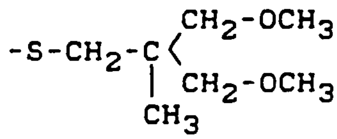 |
| 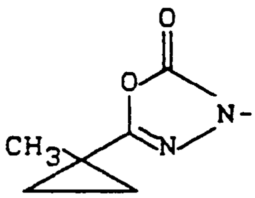 | H | 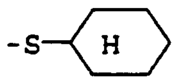 |
| 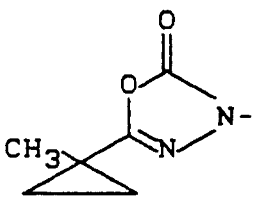 | H | 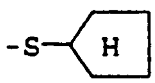 |
| 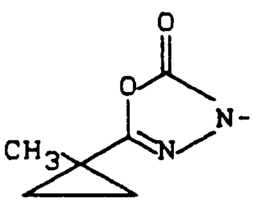 | H | 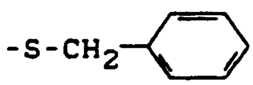 |
| 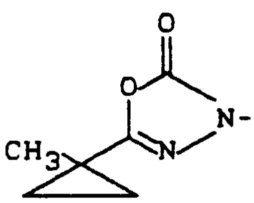 | H | $-S-CH_2-CN$ |

| Het | $R^1$ | $R^2$ |
|---|---|---|
| 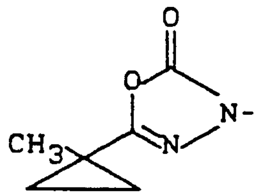 | H | $-S-CH_2-COOC_2H_5$ |
| 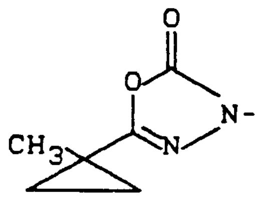 | H | 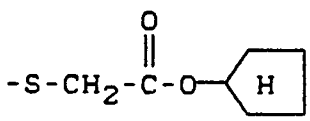 |
| 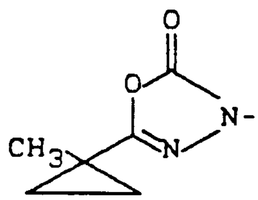 | H | 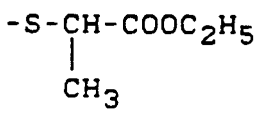 |
| 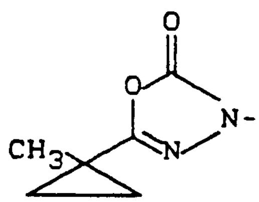 | H | 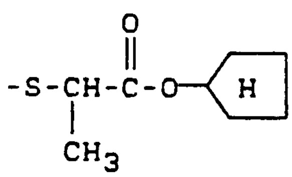 |
| 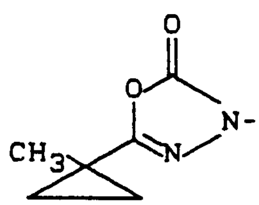 | H | 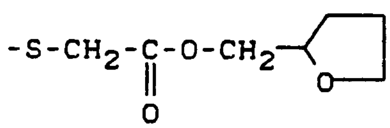 |
| 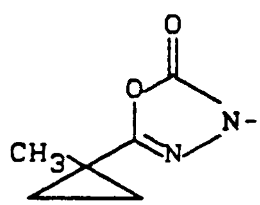 | H | 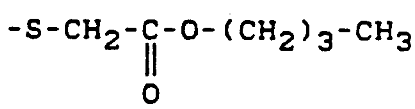 |

| Het | $R^1$ | $R^2$ |
|---|---|---|
| 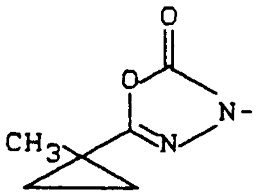 | H | 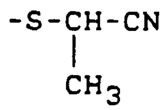 |
| 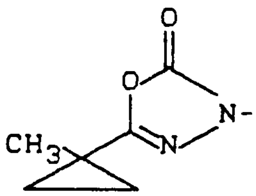 | H | $-S-CH_2-CH_2-O-CH_3$ |
| 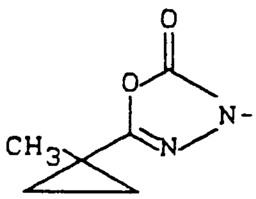 | H | 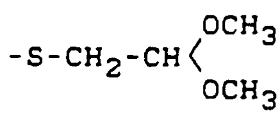 |
| 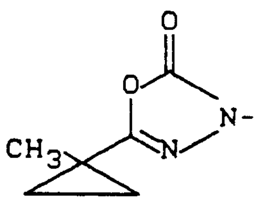 | F | $-OCH_3$ |
| 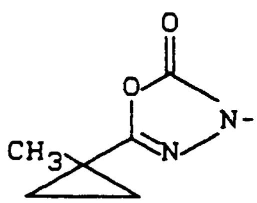 | F | $-OC_2H_5$ |
| 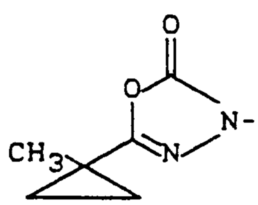 | F | $-O-CH(CH_3)_2$ |

| Het | $R^1$ | $R^2$ |
|---|---|---|
| 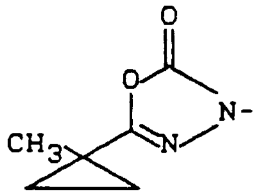 | F | $-O-CH_2-CH=CH_2$ |
| 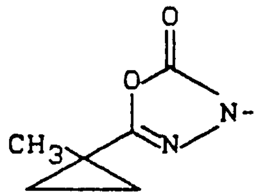 | F | $-O-CH_2-CH=CH-CH_3$ |
| 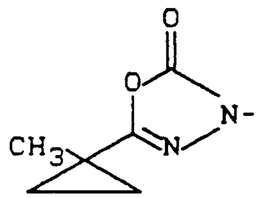 | F | $-O-CH_2-CH=CH-Cl$ |
| 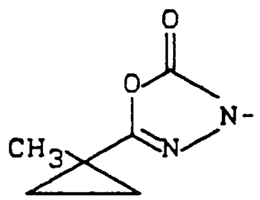 | F | $-O-CH(CH_3)-CH=CH_2$ |
| 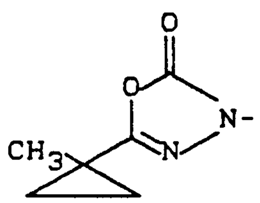 | F | $-O-CH_2-C\equiv CH$ |
| 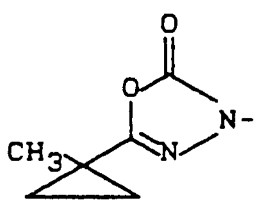 | F | $-O-CH(CH_3)-C\equiv CH$ |

| Het | $R^1$ | $R^2$ |
|---|---|---|
| 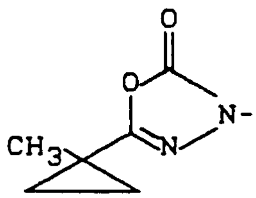 | F | 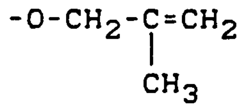 |
| 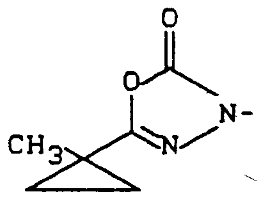 | F | $-O-CH_2-CH_2-OC_2H_5$ |
| 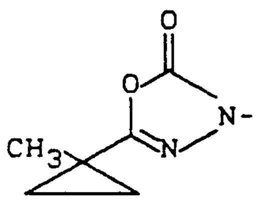 | F | 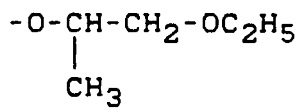 |
| 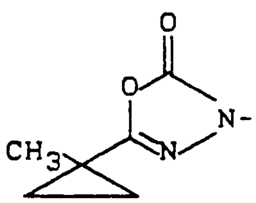 | F | 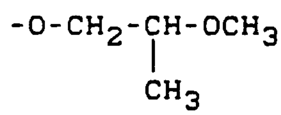 |
| 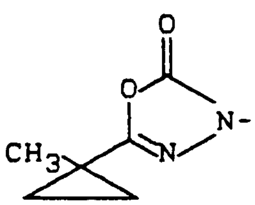 | F | 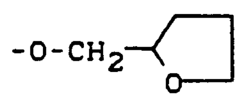 |
| 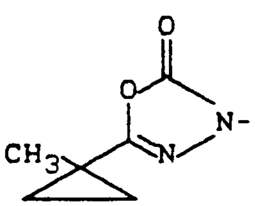 | F | 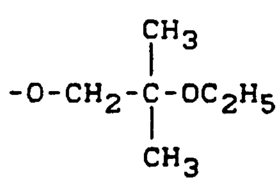 |

| Het | $R^1$ | $R^2$ |
|---|---|---|
| 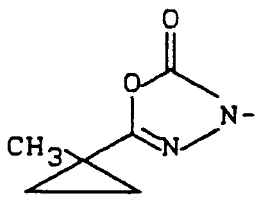 | F | $-O-CH_2-CH_2-O-CH_2-CH_2-OCH_3$ |
| 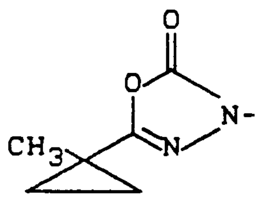 | F | $-O-CH_2-CH_2-O-CH_2-CH_2-OC_2H_5$ |
| 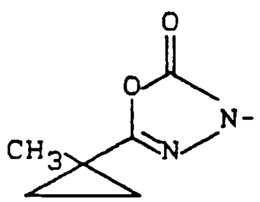 | F | $-O-CH_2-\overset{\overset{O}{\|}}{C}-CH_3$ |
| 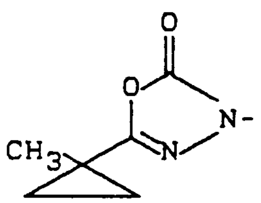 | F | $-O-\underset{\underset{CH_3}{\|}}{CH}-\overset{\overset{O}{\|}}{C}-CH_3$ |
| 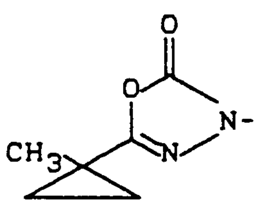 | F | $-SCH_3$ |
| 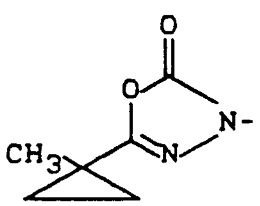 | F | $-SC_2H_5$ |

| Het | $R^1$ | $R^2$ |
|---|---|---|
| 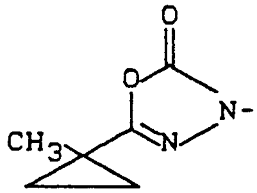 | F | $-S-CH(CH_3)_2$ |
| 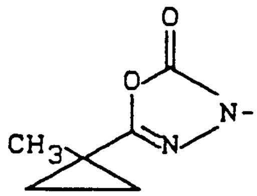 | F | $-S-CH_2-CH=CH_2$ |
| 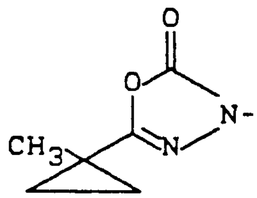 | F | $-S-CH_2-CH=CH-Cl$ |
| 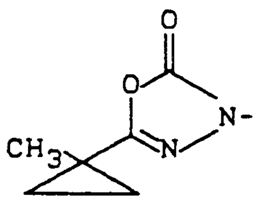 | F | $-S-CH_2-CH=CH-CH_3$ |
| 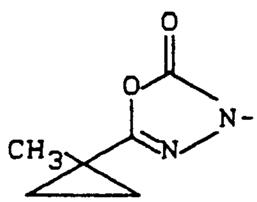 | F | $-S-CH(CH_3)-CH=CH_2$ |
| 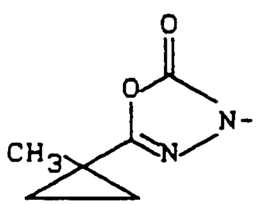 | F | $-S-CH_2-C{\equiv}CH$ |

| Het | $R^1$ | $R^2$ |
|---|---|---|
| 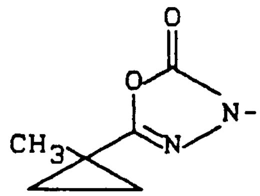 | F | 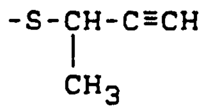 |
| 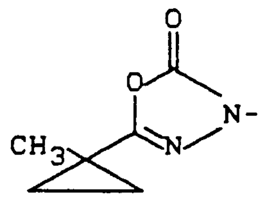 | F | 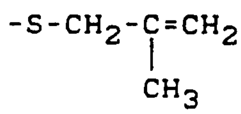 |
| 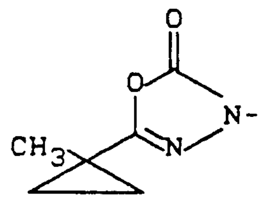 | F | $-S-CH_2-CH_2-OC_2H_5$ |
| 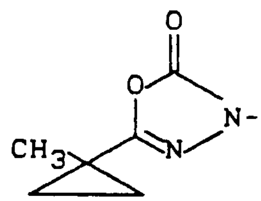 | F | 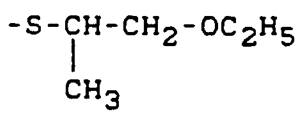 |
| 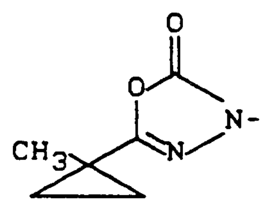 | F | 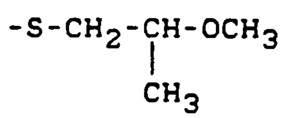 |
| 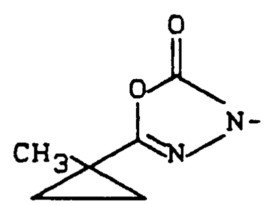 | F | 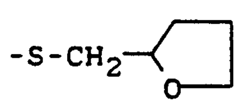 |

| Het | $R^1$ | $R^2$ |
|---|---|---|
| 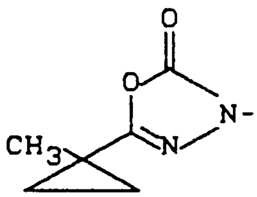 | F | 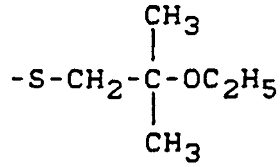 |
| 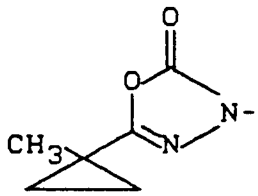 | F | $-S-CH_2-CH_2-O-CH_2-CH_2-OCH_3$ |
| 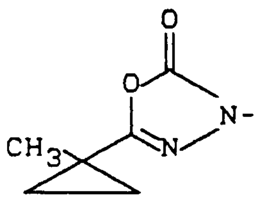 | F | $-S-CH_2-CH_2-O-CH_2-CH_2-OC_2H_5$ |
| 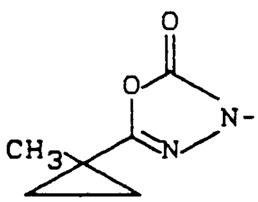 | F | 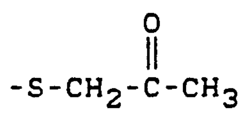 |
| 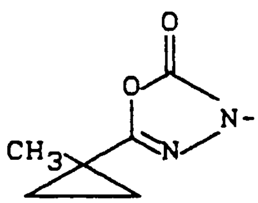 | F | 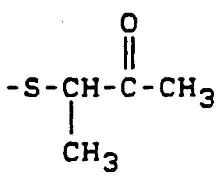 |
| 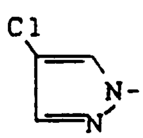 | F | 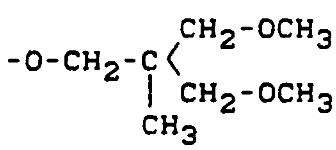 |

| Het | $R^1$ | $R^2$ |
|---|---|---|
| 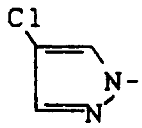 | F | 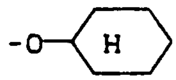 |
| 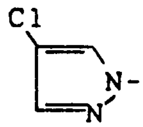 | F | 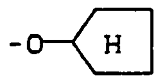 |
| 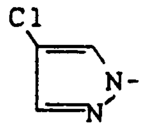 | F | 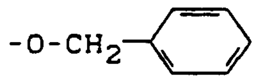 |
| 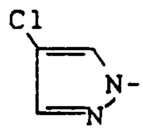 | F | $-O-CH_2-CN$ |
| 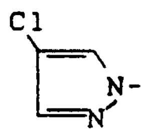 | F | $-O-CH_2-COOC_2H_5$ |
| 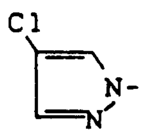 | F | 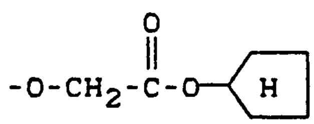 |
| 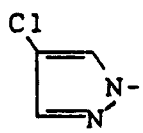 | F | 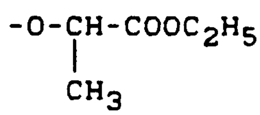 |
| 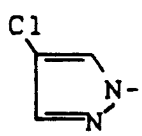 | F | 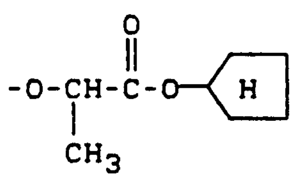 |

| Het | $R^1$ | $R^2$ |
|---|---|---|
| 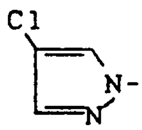 | F | 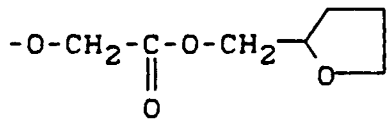 |
| 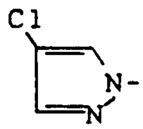 | F | 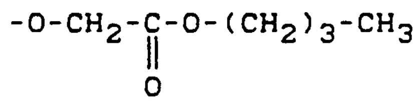 |
| 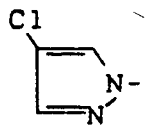 | F | 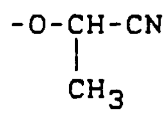 |
| 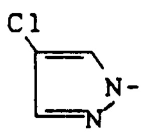 | F | $-O-CH_2-CH_2-O-CH_3$ |
| 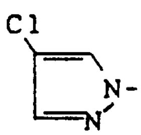 | F | 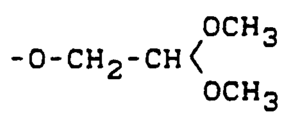 |
| 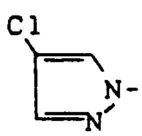 | F | 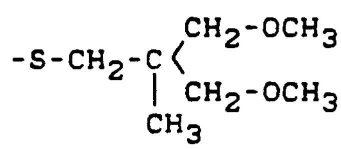 |
| 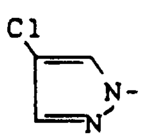 | F | 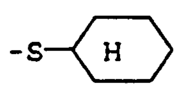 |
| 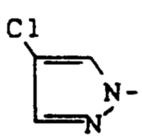 | F | 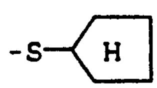 |

| Het | $R^1$ | $R^2$ |
|---|---|---|
| 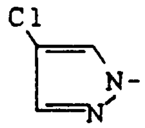 | F | 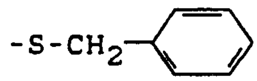 |
| 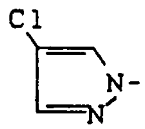 | F | $-S-CH_2-CN$ |
| 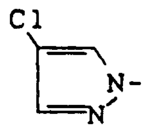 | F | $-S-CH_2-COOC_2H_5$ |
| 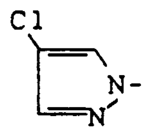 | F | 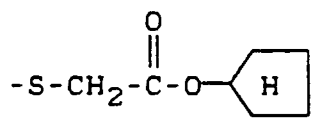 |
| 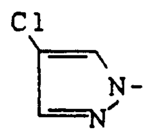 | F | 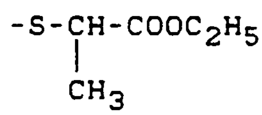 |
| 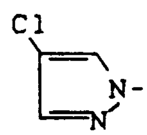 | F | 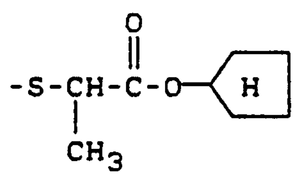 |
| 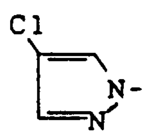 | F | 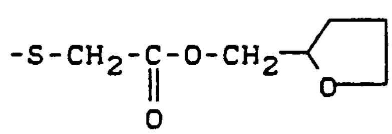 |
| 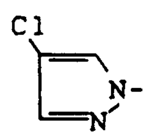 | F | 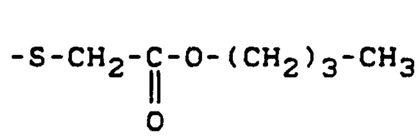 |

| Het | $R^1$ | $R^2$ |
| --- | --- | --- |
| 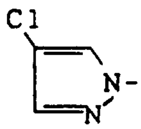 | F | $-S-CH-CN$<br>$CH_3$ |
| 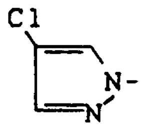 | F | $-S-CH_2-CH_2-O-CH_3$ |
| 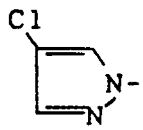 | F | $OCH_3$<br>$-S-CH_2-CH\langle$<br>$OCH_3$ |
| 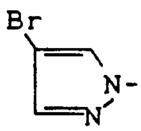 | F | $-OCH_3$ |
| 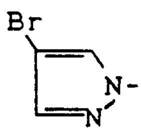 | F | $-OC_2H_5$ |
| 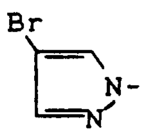 | F | $-O-CH(CH_3)_2$ |
| 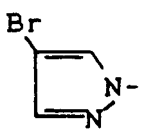 | F | $-O-CH_2-CH=CH_2$ |
| 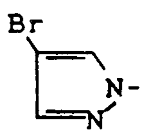 | F | $-O-CH_2-CH=CH-CH_3$ |

| Het | $R^1$ | $R^2$ |
| --- | --- | --- |
| 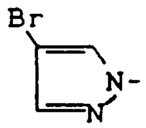 | F | $-O-CH_2-CH=CH-Cl$ |
| 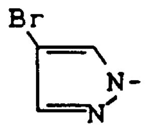 | F | $-O-CH-CH=CH_2$<br>$CH_3$ |
| 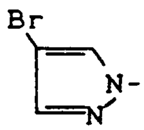 | F | $-O-CH_2-C\equiv CH$ |
| 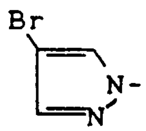 | F | $-O-CH-C\equiv CH$<br>$CH_3$ |
| 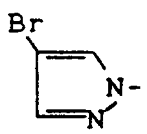 | F | $-O-CH_2-C=CH_2$<br>$CH_3$ |
| 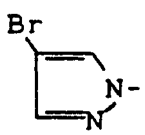 | F | $-O-CH_2-CH_2-OC_2H_5$ |
| 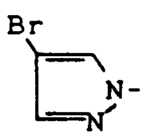 | F | $-O-CH-CH_2-OC_2H_5$<br>$CH_3$ |
| 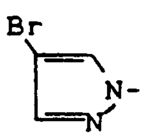 | F | $-O-CH_2-CH-OCH_3$<br>$CH_3$ |

| Het | $R^1$ | $R^2$ |
| --- | --- | --- |
| 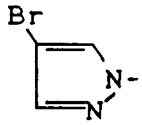 | F | 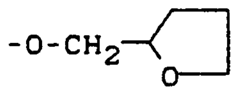 |
| 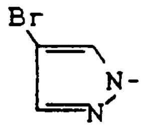 | F | 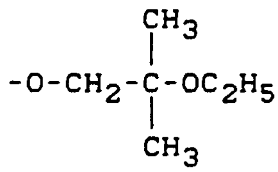 |
| 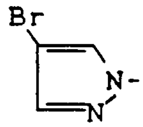 | F | $-O-CH_2-CH_2-O-CH_2-CH_2-OCH_3$ |
| 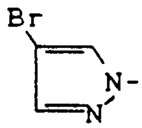 | F | $-O-CH_2-CH_2-O-CH_2-CH_2-OC_2H_5$ |
| 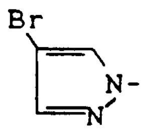 | F | 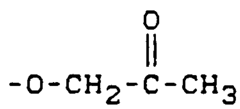 |
| 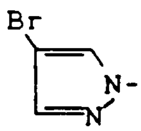 | F | 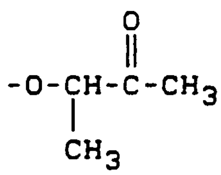 |
| 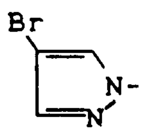 | F | $-SCH_3$ |
| 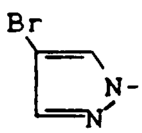 | F | $-SC_2H_5$ |

| Het | $R^1$ | $R^2$ |
|---|---|---|
| 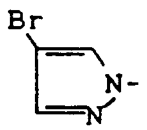 | F | $-S-CH(CH_3)_2$ |
| 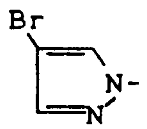 | F | $-S-CH_2-CH=CH_2$ |
| 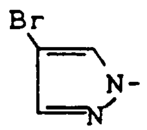 | F | $-S-CH_2-CH=CH-Cl$ |
| 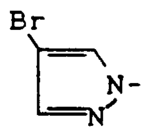 | F | $-S-CH_2-CH=CH-CH_3$ |
| 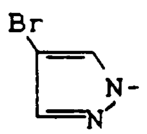 | F | $-S-CH(CH_3)-CH=CH_2$ |
| 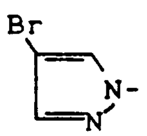 | F | $-S-CH_2-C\equiv CH$ |
| 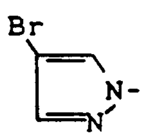 | F | $-S-CH(CH_3)-C\equiv CH$ |
| 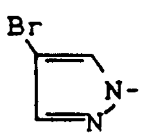 | F | $-S-CH_2-C(CH_3)\equiv CH_2$ |

| Het | $R^1$ | $R^2$ |
|---|---|---|
| 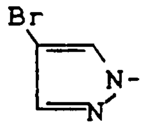 | F | $-S-CH_2-CH_2-OC_2H_5$ |
| 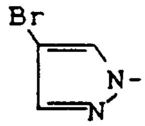 | F | 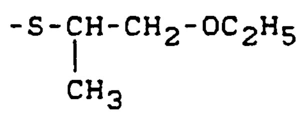 |
| 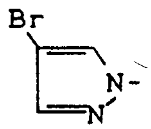 | F | 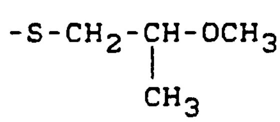 |
| 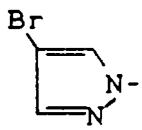 | F | 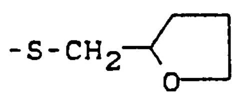 |
| 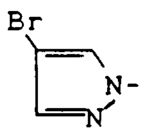 | F | 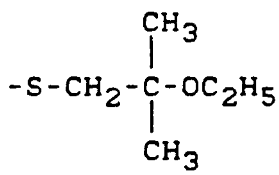 |
| 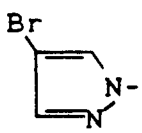 | F | $-S-CH_2-CH_2-O-CH_2-CH_2-OCH_3$ |
| 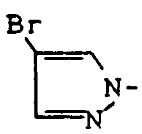 | F | $-S-CH_2-CH_2-O-CH_2-CH_2-OC_2H_5$ |
| 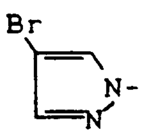 | F | 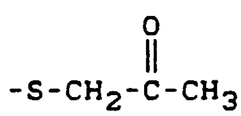 |

| Het | $R^1$ | $R^2$ |
|---|---|---|
| 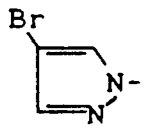 | F | 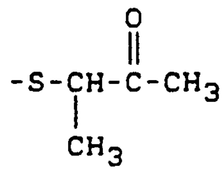 |
| 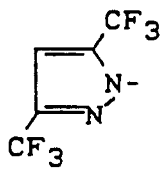 | F | 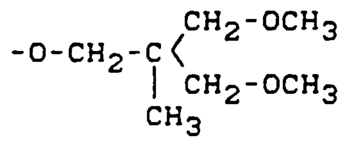 |
| 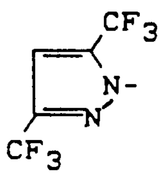 | F | 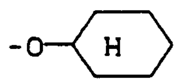 |
| 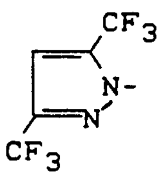 | F | 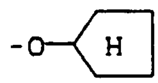 |
| 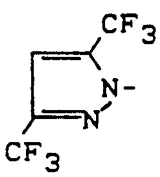 | F | 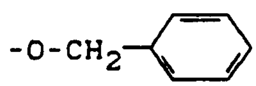 |
| 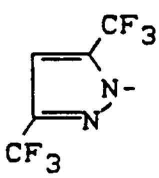 | F | $-O-CH_2-CN$ |
| 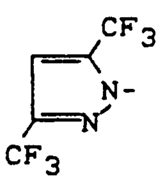 | F | $-O-CH_2-COOC_2H_5$ |

| Het | $R^1$ | $R^2$ |
|---|---|---|
| 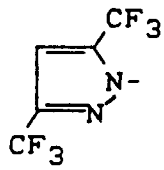 | F | 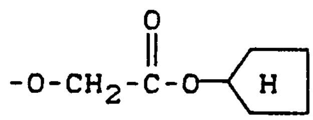 |
| 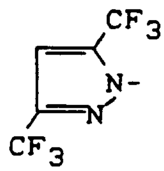 | F | 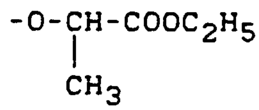 |
| 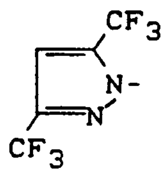 | F | 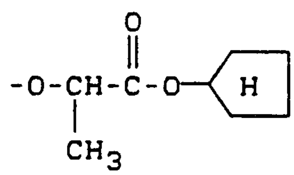 |
| 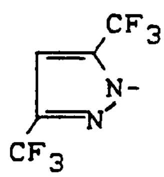 | F | 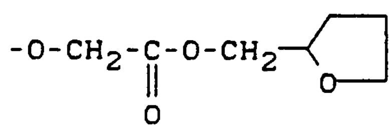 |
| 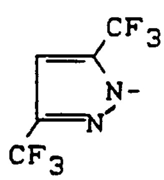 | F | 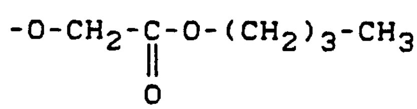 |
| 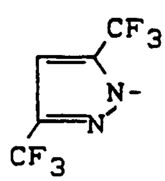 | F | 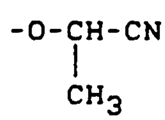 |
| 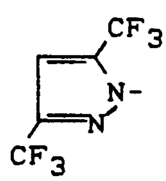 | F | $-O-CH_2-CH_2-O-CH_3$ |

| Het | $R^1$ | $R^2$ |
|---|---|---|
| 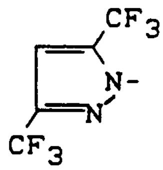 | F | 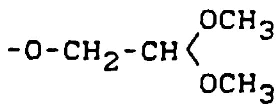 |
| 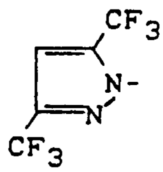 | F | 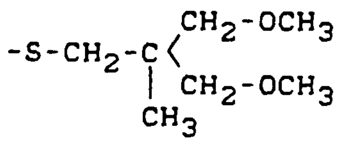 |
| 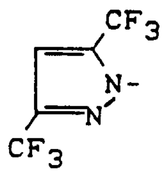 | F | 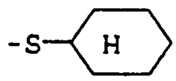 |
| 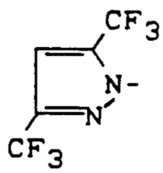 | F | 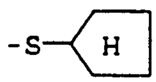 |
| 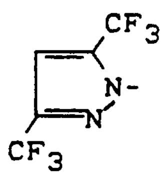 | F | 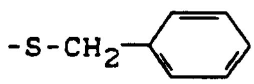 |
| 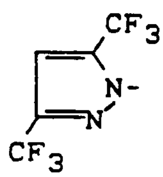 | F | $-S-CH_2-CN$ |
| 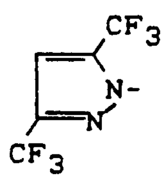 | F | $-S-CH_2-COOC_2H_5$ |

| Het | $R^1$ | $R^2$ |
|---|---|---|
| 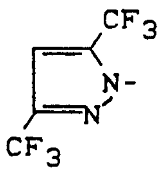 | F | 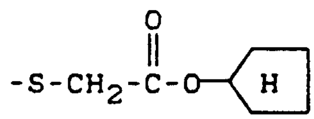 |
| 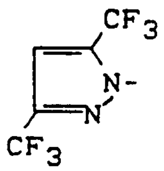 | F | 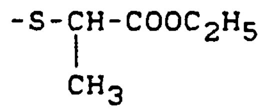 |
| 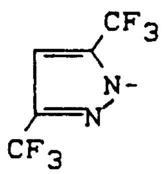 | F | 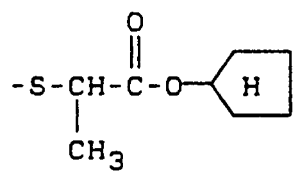 |
| 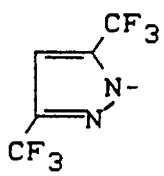 | F | 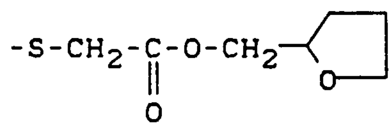 |
| 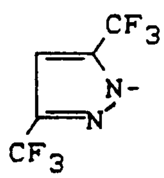 | F | 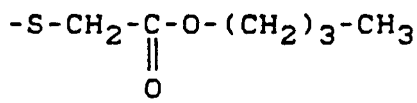 |
| 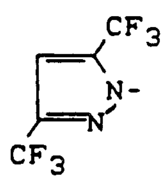 | F | 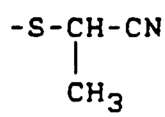 |
| 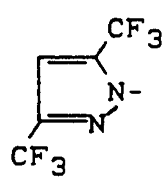 | F | $-S-CH_2-CH_2-O-CH_3$ |

| Het | $R^1$ | $R^2$ |
|---|---|---|
| 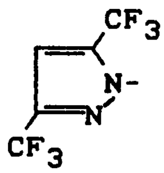 | F | 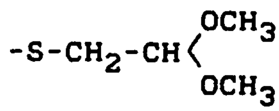 |
| 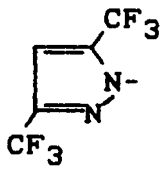 | H | $-OCH_3$ |
| 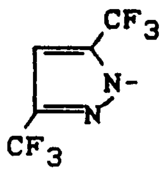 | H | $-OC_2H_5$ |
| 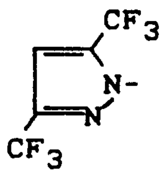 | H | $-O-CH(CH_3)_2$ |
| 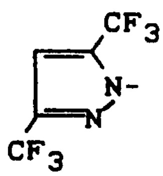 | H | $-O-CH_2-CH=CH_2$ |
| 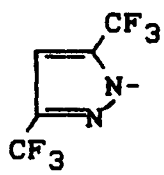 | H | $-O-CH_2-CH=CH-CH_3$ |
| 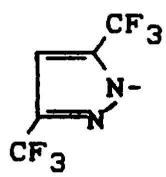 | H | $-O-CH_2-CH_2-Cl$ |

| Het | $R^1$ | $R^2$ |
|---|---|---|
| 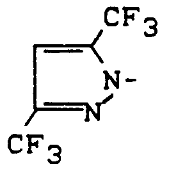 | H | $-O-CH-CH=CH_2$<br>$CH_3$ |
| 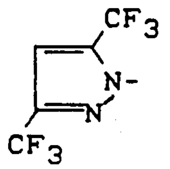 | H | $-O-CH_2-C\equiv CH$ |
| 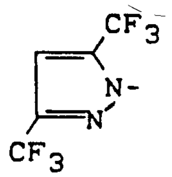 | H | $-O-CH-C\equiv CH$<br>$CH_3$ |
| 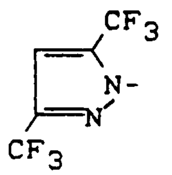 | H | $-O-CH_2-C=CH_2$<br>$CH_3$ |
| 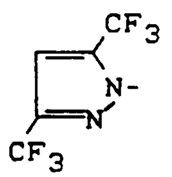 | H | $-O-CH_2-CH_2-OC_2H_5$ |
| 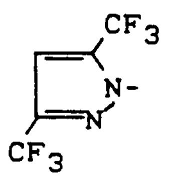 | H | $-O-CH-CH_2-OC_2H_5$<br>$CH_3$ |
| 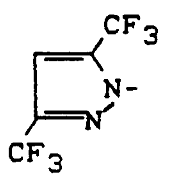 | H | $-O-CH_2-CH-OCH_3$<br>$CH_3$ |

| Het | $R^1$ | $R^2$ |
|---|---|---|
| 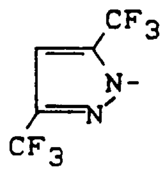 | H | 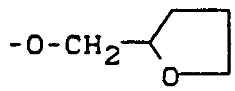 |
| 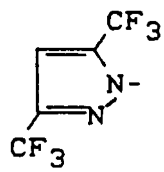 | H | 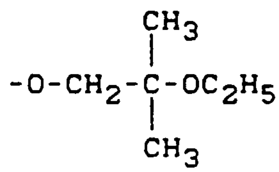 |
| 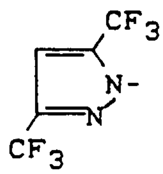 | H | $-O-CH_2-CH_2-O-CH_2-CH_2-OCH_3$ |
| 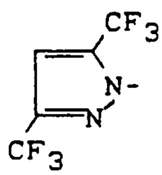 | H | $-O-CH_2-CH_2-O-CH_2-CH_2-OC_2H_5$ |
| 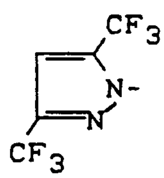 | H | 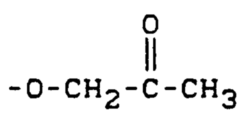 |
| 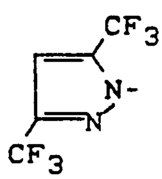 | H | 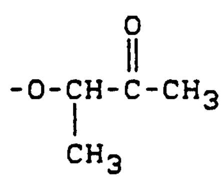 |
| 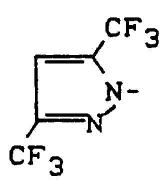 | H | $-SCH_3$ |

| Het | $R^1$ | $R^2$ |
|---|---|---|
| 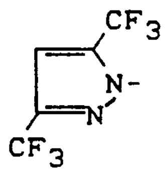 | H | $-SC_2H_5$ |
| 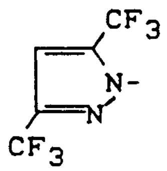 | H | $-S-CH(CH_3)_2$ |
| 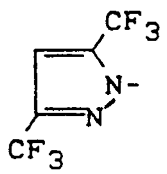 | H | $-S-CH_2-CH=CH_2$ |
| 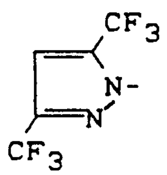 | H | $-S-CH_2-CH=CH-Cl$ |
| 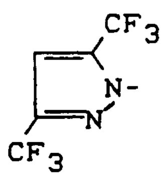 | H | $-S-CH_2-CH=CH-CH_3$ |
| 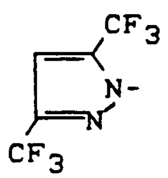 | H | $-S-\underset{CH_3}{\underset{\mid}{C}}H-CH=CH_2$ |
| 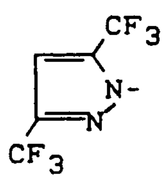 | H | $-S-CH_2-C\equiv CH$ |

| Het | $R^1$ | $R^2$ |
|---|---|---|
| 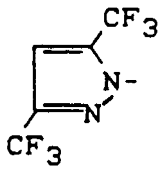 | H | 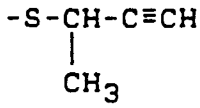 |
| 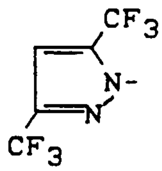 | H | 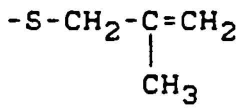 |
| 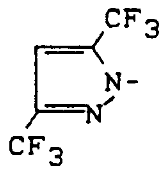 | H | $-S-CH_2-CH_2-OC_2H_5$ |
| 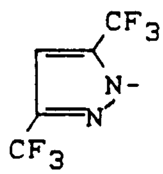 | H | 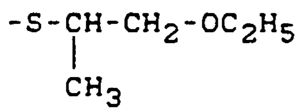 |
| 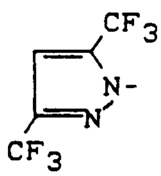 | H | 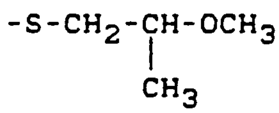 |
| 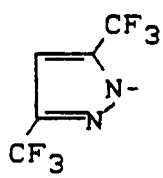 | H | 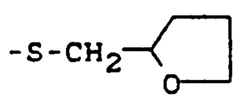 |
| 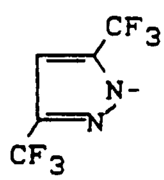 | H | 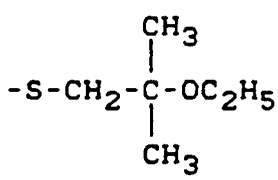 |

| Het | $R^1$ | $R^2$ |
|---|---|---|
| 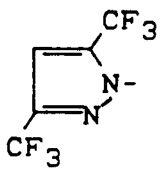 | H | $-S-CH_2-CH_2-O-CH_2-CH_2-OCH_3$ |
| 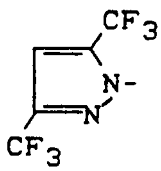 | H | $-S-CH_2-O-CH_2-CH_2-OC_2H_5$ |
| 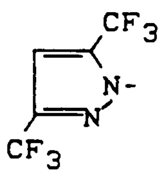 | H | 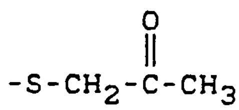 |
| 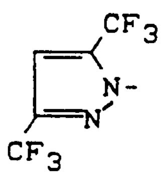 | H | 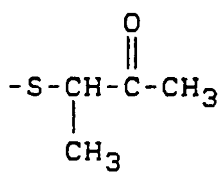 |
| 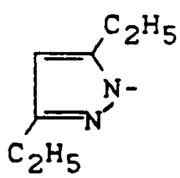 | F | 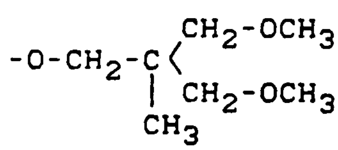 |
| 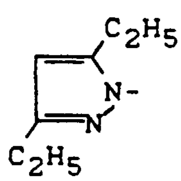 | F | 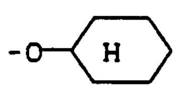 |
| 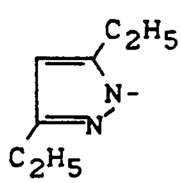 | F | 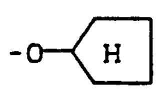 |

| Het | $R^1$ | $R^2$ |
| --- | --- | --- |
| 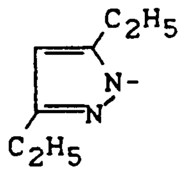 | F | 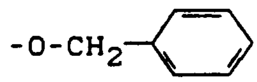 |
| 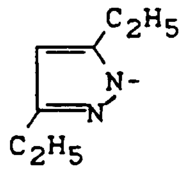 | F | $-O-CH_2-CN$ |
| 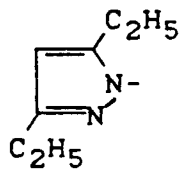 | F | $-O-CH_2-COOC_2H_5$ |
| 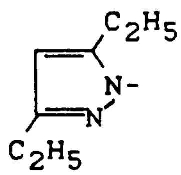 | F | 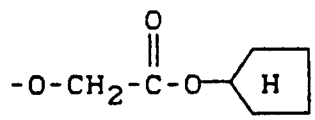 |
| 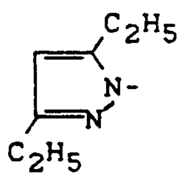 | F | 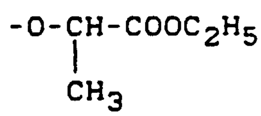 |
| 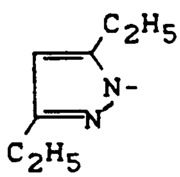 | F | 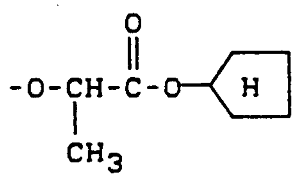 |
| 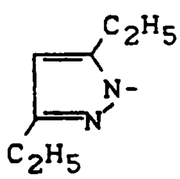 | F | 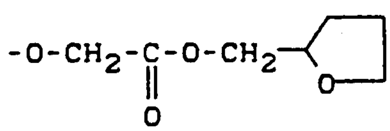 |

| Het | $R^1$ | $R^2$ |
|---|---|---|
| 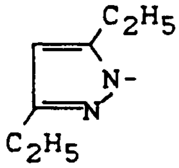 | F | 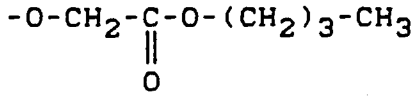 |
| 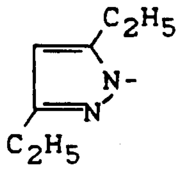 | F | 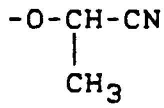 |
| 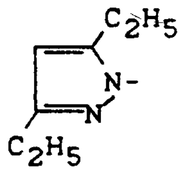 | F | $-O-CH_2-CH_2-O-CH_3$ |
| 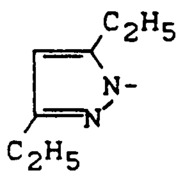 | F | 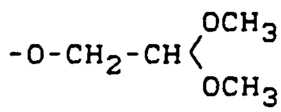 |
| 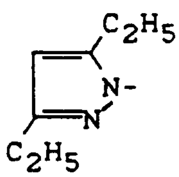 | H | 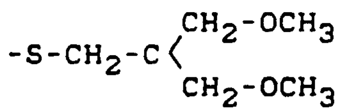 |
| 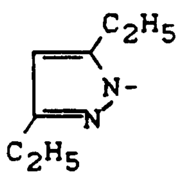 | H | 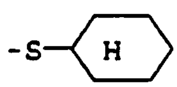 |
| 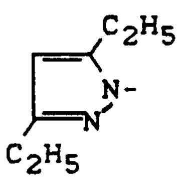 | H | 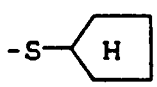 |

| Het | $R^1$ | $R^2$ |
|---|---|---|
| 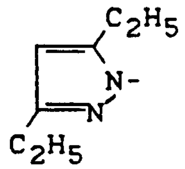 | H | 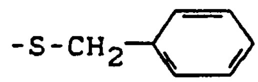 |
| 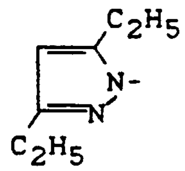 | H | $-S-CH_2-CN$ |
| 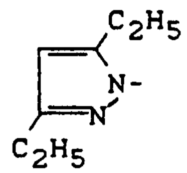 | H | $-S-CH_2-COOC_2H_5$ |
| 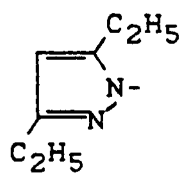 | H | 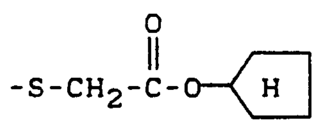 |
| 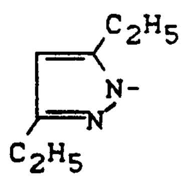 | H | 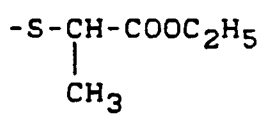 |
| 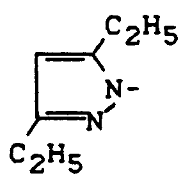 | H | 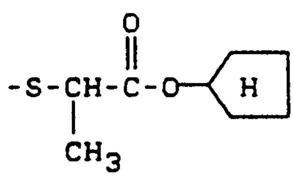 |
| 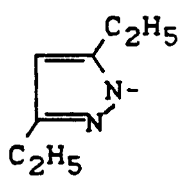 | H | 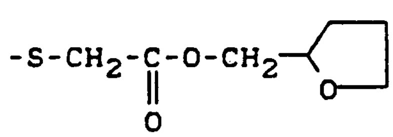 |

| Het | $R^1$ | $R^2$ |
|---|---|---|
| 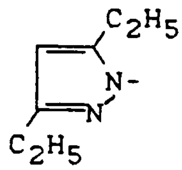 | H | 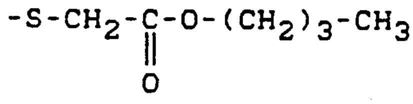 |
| 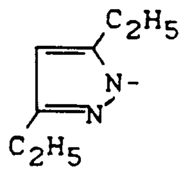 | H | 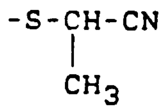 |
| 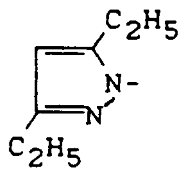 | H | $-S-CH_2-CH_2-O-CH_3$ |
| 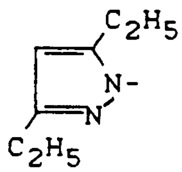 | H | 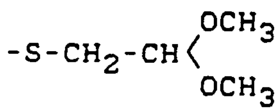 |
| 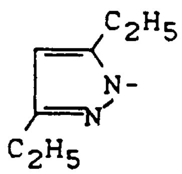 | F | $-OCH_3$ |
| 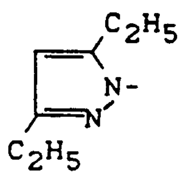 | F | $-OC_2H_5$ |
| 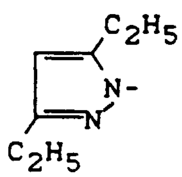 | F | $-O-CH(CH_3)_2$ |

| Het | $R^1$ | $R^2$ |
|---|---|---|
| 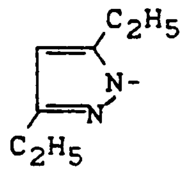 | F | $-O-CH_2-CH=CH_2$ |
| 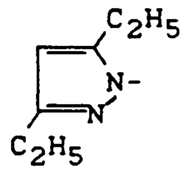 | F | $-O-CH_2-CH=CH-CH_3$ |
| 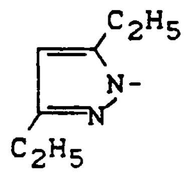 | F | $-O-CH_2-CH=CH-Cl$ |
| 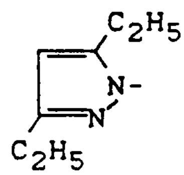 | F | $-O-CH-CH=CH_2$<br>$CH_3$ |
| 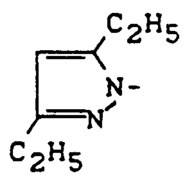 | F | $-O-CH_2-C\equiv CH$ |
| 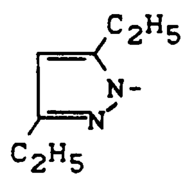 | F | $-O-CH-C\equiv CH$<br>$CH_3$ |
| 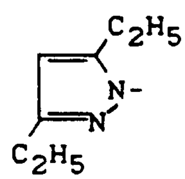 | F | $-O-CH_2-C=CH_2$<br>$CH_3$ |

| Het | $R^1$ | $R^2$ |
|---|---|---|
| 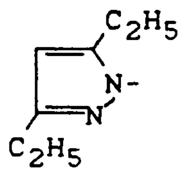 | F | $-O-CH_2-CH_2-OC_2H_5$ |
| 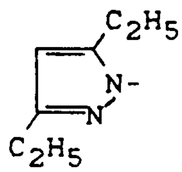 | F | 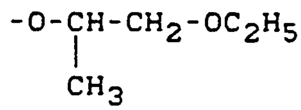 |
| 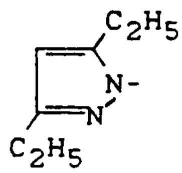 | F | 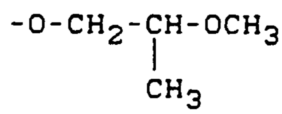 |
| 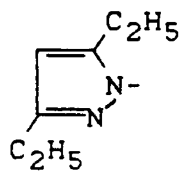 | F | 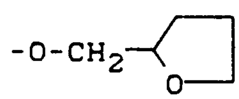 |
| 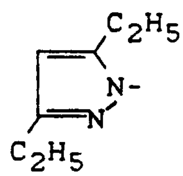 | F | 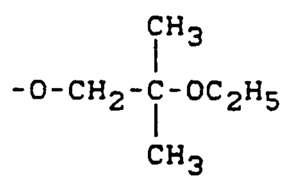 |
| 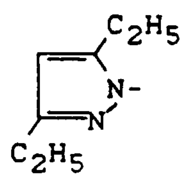 | F | $-O-CH_2-CH_2-OCH_2-CH_2-OCH_3$ |
| 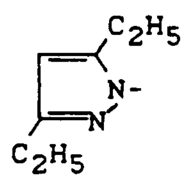 | F | $-O-CH_2-CH_2-O-CH_2-CH_2-OC_2H_5$ |

| Het | $R^1$ | $R^2$ |
|---|---|---|
| 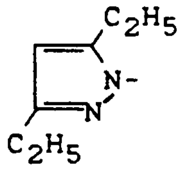 | F | 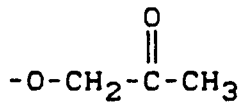 |
| 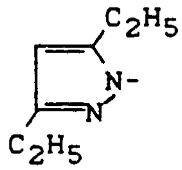 | F | 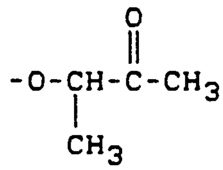 |
| 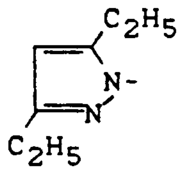 | H | $-SCH_3$ |
| 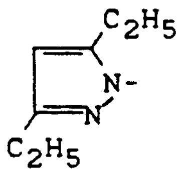 | H | $-SC_2H_5$ |
| 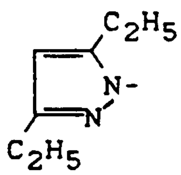 | H | $-S-CH(CH_3)_2$ |
| 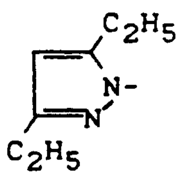 | H | $-S-CH_2-CH=CH_2$ |
| 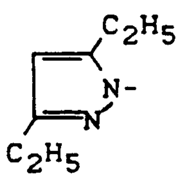 | H | $-S-CH_2-CH=CH-Cl$ |

| Het | $R^1$ | $R^2$ |
|---|---|---|
| 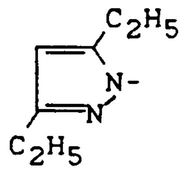 | H | $-S-CH_2-CH=CH-CH_3$ |
| 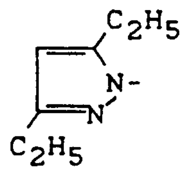 | H | $-S-\underset{CH_3}{\underset{\vert}{CH}}-CH=CH_2$ |
| 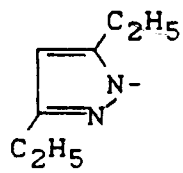 | H | $-S-CH_2-C\equiv CH$ |
| 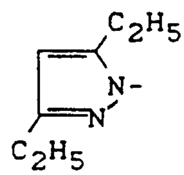 | H | $-S-\underset{CH_3}{\underset{\vert}{CH}}-C\equiv CH$ |
| 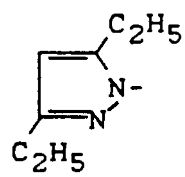 | H | $-S-CH_2-\underset{CH_3}{\underset{\vert}{C}}=CH_2$ |
| 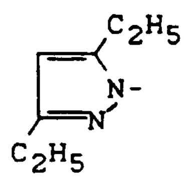 | H | $-S-CH_2-CH_2-OC_2H_5$ |
| 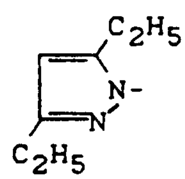 | H | $-S-\underset{CH_3}{\underset{\vert}{CH}}-CH_2-OC_2H_5$ |

| Het | $R^1$ | $R^2$ |
|---|---|---|
| 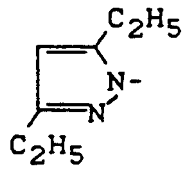 | H | 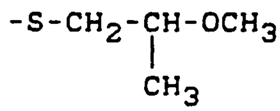 |
| 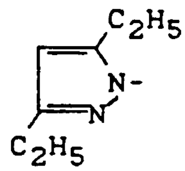 | H | 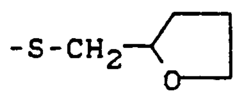 |
| 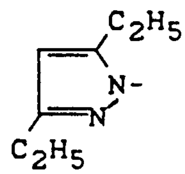 | H | 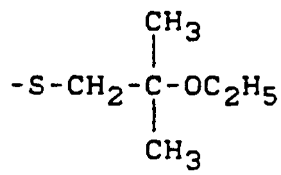 |
| 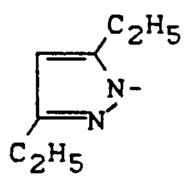 | H | $-S-CH_2-CH_2-O-CH_2-CH_2-OCH_3$ |
| 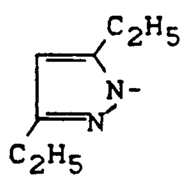 | H | $-S-CH_2-CH_2-O-CH_2-CH_2-OC_2H_5$ |
| 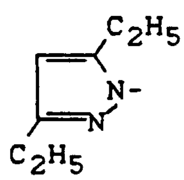 | H | 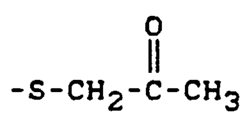 |
| 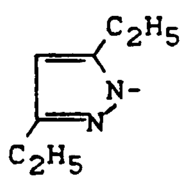 | H | 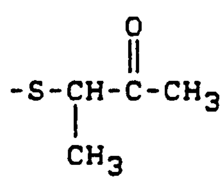 |

| Het | $R^1$ | $R^2$ |
|---|---|---|
| 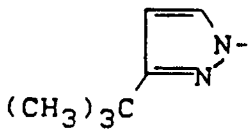 | F | 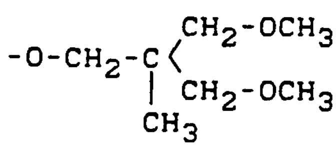 |
| 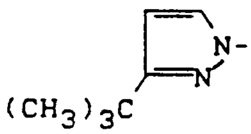 | F | 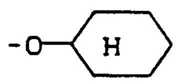 |
| 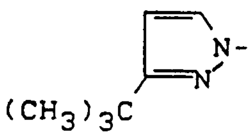 | F | 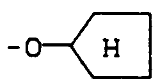 |
| 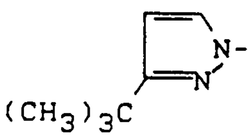 | F | 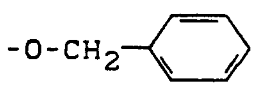 |
| 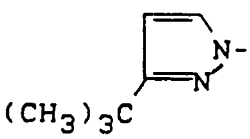 | F | $-O-CH_2-CN$ |
| 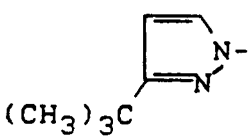 | F | $-O-CH_2-COOC_2H_5$ |
| 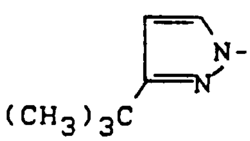 | F | 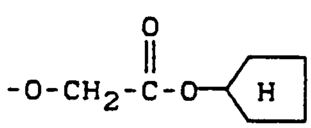 |

| Het | $R^1$ | $R^2$ |
|---|---|---|
| 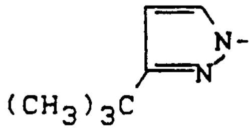 | F | 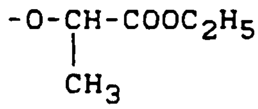 |
| 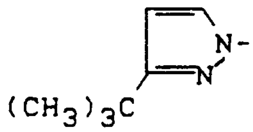 | F | 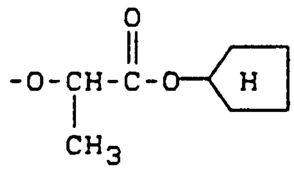 |
| 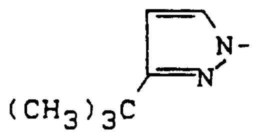 | F | 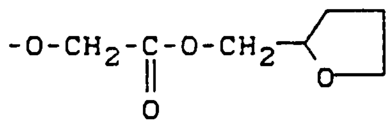 |
| 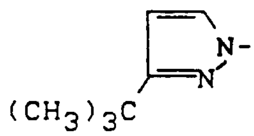 | F | 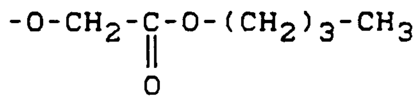 |
| 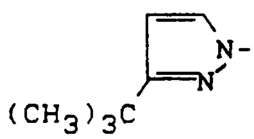 | F | 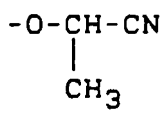 |
| 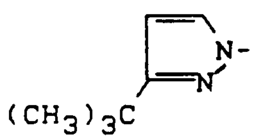 | F | $-O-CH_2-CH_2-O-CH_3$ |
| 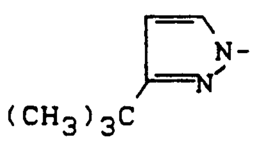 | F | 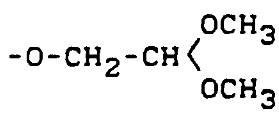 |

| Het | $R^1$ | $R^2$ |
|---|---|---|
| 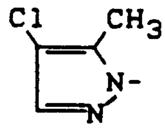 | F | $-OCH_3$ |
| 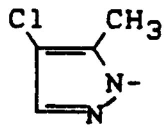 | F | $-OC_2H_5$ |
| 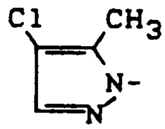 | F | $-O-CH(CH_3)_2$ |
| 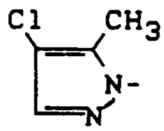 | F | $-O-CH_2-CH=CH_2$ |
| 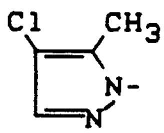 | F | $-O-CH_2-CH=CH-CH_3$ |
| 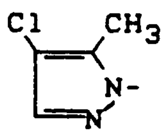 | F | $-O-CH_2-CH=CH-Cl$ |
| 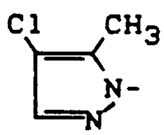 | F | $-O-CH-CH=CH_2$<br>$CH_3$ |
| 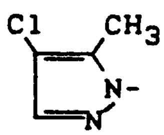 | F | $-O-CH_2-C\equiv CH$ |

| Het | $R^1$ | $R^2$ |
|---|---|---|
| 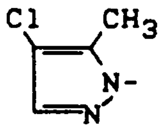 | F | 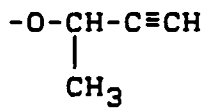 |
| 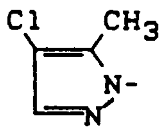 | F | 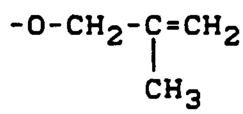 |
| 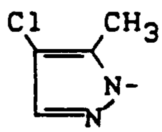 | F | $-O-CH_2-CH_2-OC_2H_5$ |
| 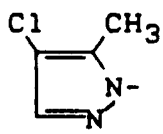 | F | 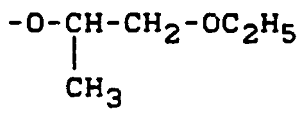 |
| 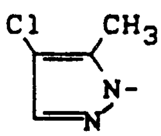 | F | 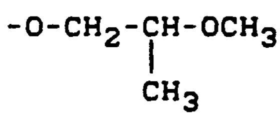 |
| 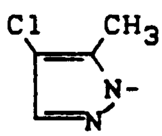 | F | 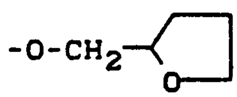 |
| 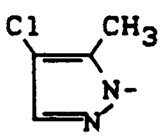 | F | 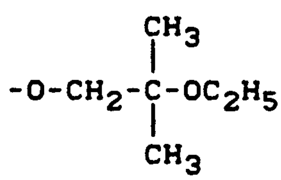 |
| 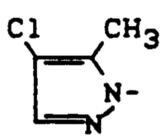 | F | $-O-CH_2-CH_2-O-CH_2-CH_2-OCH_3$ |

| Het | $R^1$ | $R^2$ |
|---|---|---|
| 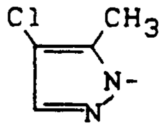 | F | $-O-CH_2-CH_2-O-CH_2-CH_2-OC_2H_5$ |
| 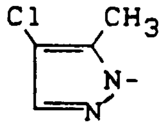 | F | 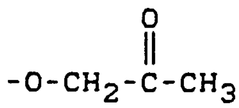 |
| 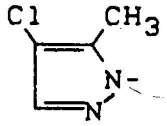 | F | 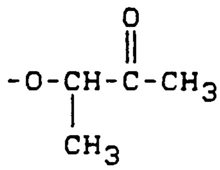 |
| 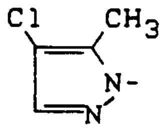 | F | $-SCH_3$ |
| 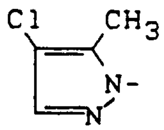 | F | $-SC_2H_5$ |
| 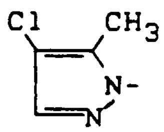 | F | $-S-CH(CH_3)_2$ |
| 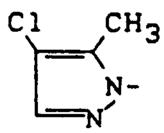 | F | $-S-CH_2-CH=CH_2$ |
| 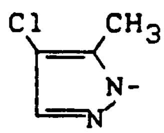 | F | $-S-CH_2-CH=CH-Cl$ |

| Het | $R^1$ | $R^2$ |
|---|---|---|
| 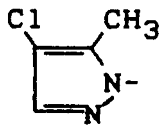 | F | $-S-CH_2-CH=CH-CH_3$ |
| 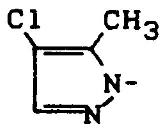 | F | $-S-CH-CH=CH_2$<br>$CH_3$ |
| 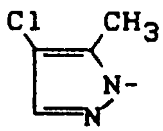 | F | $-S-CH_2-C\equiv CH$ |
| 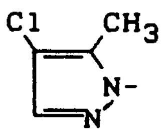 | F | $-S-CH-C\equiv CH$<br>$CH_3$ |
| 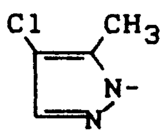 | F | $-S-CH_2-C=CH_2$<br>$CH_3$ |
| 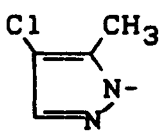 | F | $-S-CH_2-CH_2-OC_2H_5$ |
| 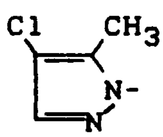 | F | $-S-CH-CH_2-OC_2H_5$<br>$CH_3$ |
| 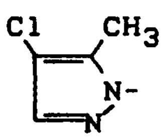 | F | $-S-CH_2-CH-OCH_3$<br>$CH_3$ |

| Het | $R^1$ | $R^2$ |
|---|---|---|
| 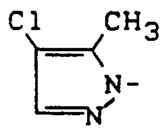 | F | 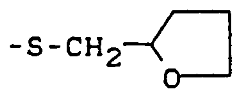 |
| 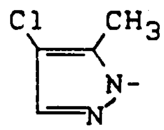 | F | 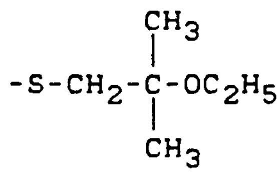 |
| 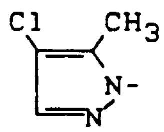 | F | $-S-CH_2-CH_2-O-CH_2-CH_2-OCH_3$ |
| 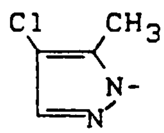 | F | $-S-CH_2-CH_2-O-CH_2-CH_2-OC_2H_5$ |
| 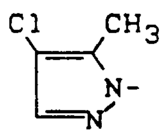 | F | 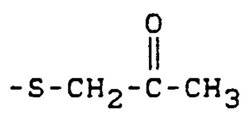 |
| 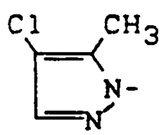 | F | 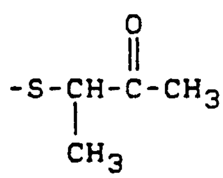 |
| 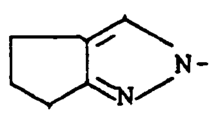 | F | 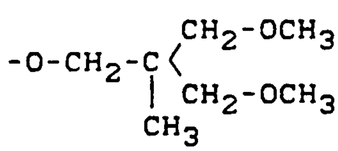 |
| 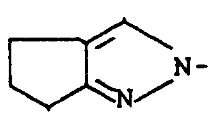 | F | 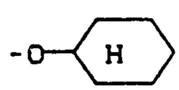 |

| Het | $R^1$ | $R^2$ |
|---|---|---|
| 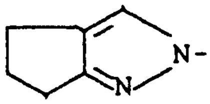 | F | 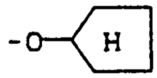 |
| 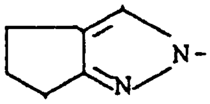 | F | 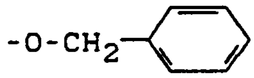 |
| 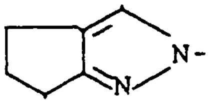 | F | $-O-CH_2-CN$ |
| 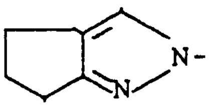 | F | $-O-CH_2-COOC_2H_5$ |
| 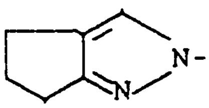 | F | 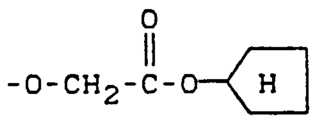 |
| 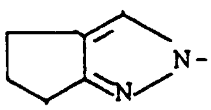 | F | 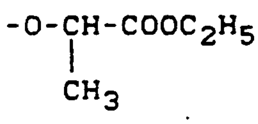 |
| 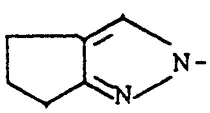 | F | 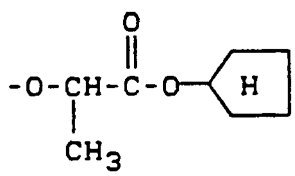 |
| 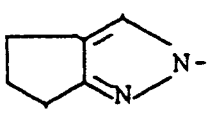 | F | 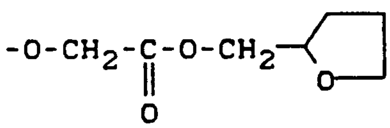 |

| Het | $R^1$ | $R^2$ |
|---|---|---|
| 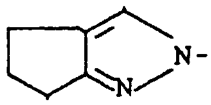 | F | 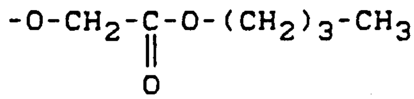 |
| 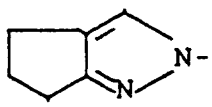 | F | 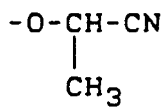 |
| 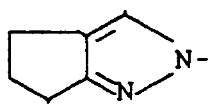 | F | $-O-CH_2-CH_2-O-CH_3$ |
| 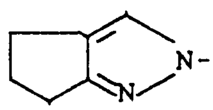 | F | 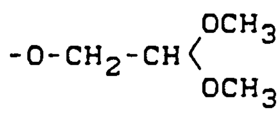 |
| 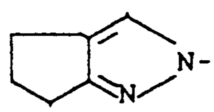 | F | 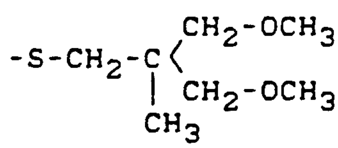 |
| 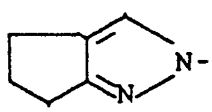 | F | -S- 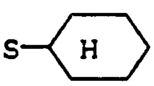 |
| 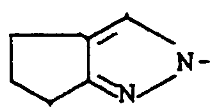 | F | -S- 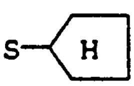 |
| 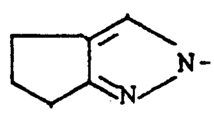 | F | $-S-CH_2-$ 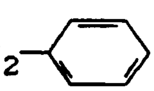 |

| Het | $R^1$ | $R^2$ |
|---|---|---|
| 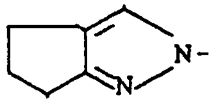 | F | $-S-CH_2-CN$ |
| 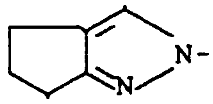 | F | $-S-CH_2-COOC_2H_5$ |
| 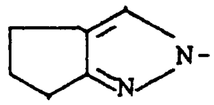 | F | 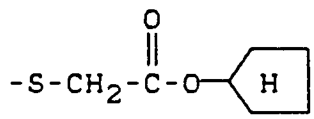 |
| 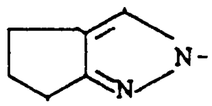 | F | 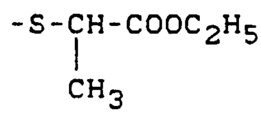 |
| 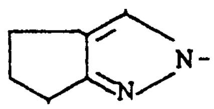 | F | 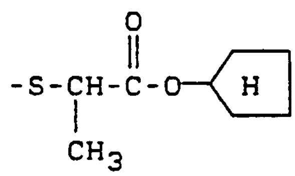 |
| 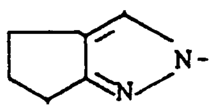 | F | 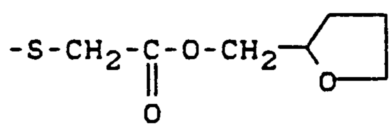 |
| 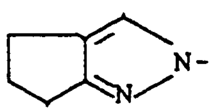 | F | 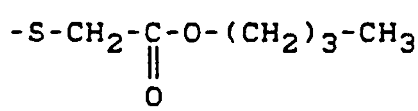 |
| 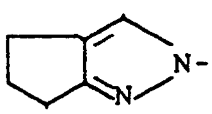 | F | 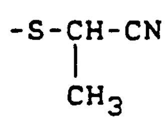 |

| Het | $R^1$ | $R^2$ |
|---|---|---|
| 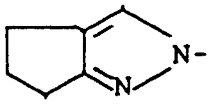 | F | $-S-CH_2-CH_2-O-CH_3$ |
| 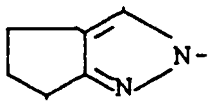 | F | $-S-CH_2-CH(OCH_3)_2$ |
| 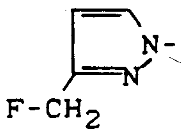 | F | $-OCH_3$ |
| 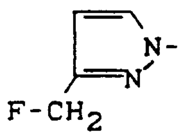 | F | $-OC_2H_5$ |
| 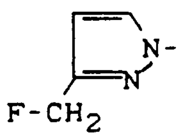 | F | $-O-CH(CH_3)_2$ |
| 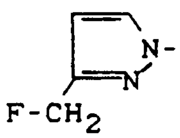 | F | $-O-CH_2-CH=CH_2$ |
| 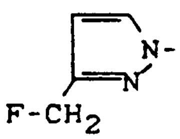 | F | $-O-CH_2-CH=CH-CH_3$ |

| Het | $R^1$ | $R^2$ |
|---|---|---|
| 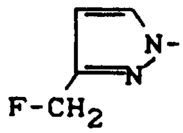 | F | $-O-CH_2-CH=CH-Cl$ |
| 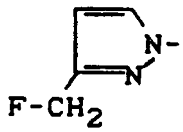 | F | $-O-CH-CH=CH_2$<br>$CH_3$ |
| 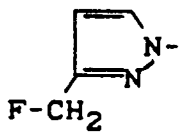 | F | $-O-CH_2-C \equiv CH$ |
| 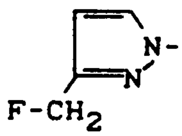 | F | $-O-CH-C \equiv CH$<br>$CH_3$ |
| 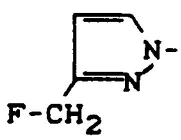 | F | $-O-CH_2-C=CH_2$<br>$CH_3$ |
| 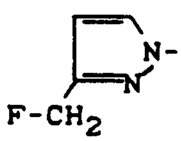 | F | $-O-CH_2-CH_2-OC_2H_5$ |
| 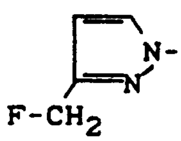 | F | $-O-CH-CH_2-OC_2H_5$<br>$CH_3$ |

| Het | $R^1$ | $R^2$ |
|---|---|---|
| 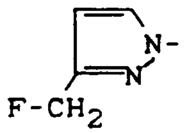 | F | 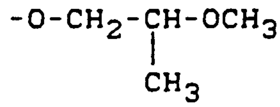 |
| 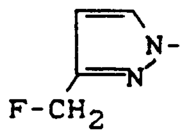 | F | 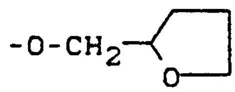 |
| 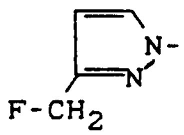 | F | 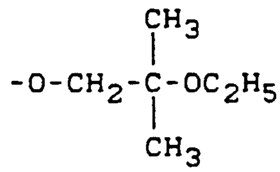 |
| 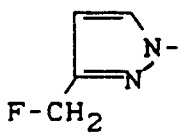 | F | $-O-CH_2-CH_2-O-CH_2-CH_2-OCH_3$ |
| 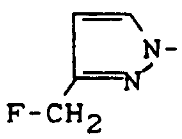 | F | $-O-CH_2-CH_2-O-CH_2-CH_2-OC_2H_5$ |
| 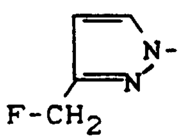 | F | 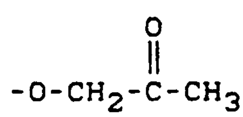 |
| 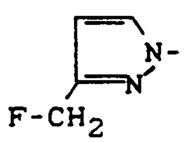 | F | 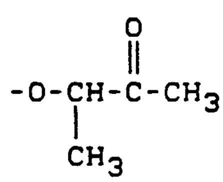 |

| Het | $R^1$ | $R^2$ |
|---|---|---|
| 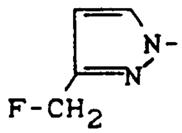 | F | $-SCH_3$ |
| 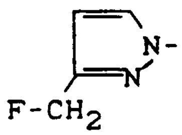 | F | $-SC_2H_5$ |
| 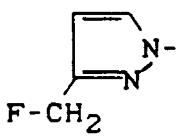 | F | $-S-CH(CH_3)_2$ |
| 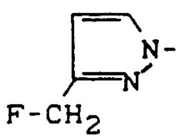 | F | $-S-CH_2-CH=CH_2$ |
| 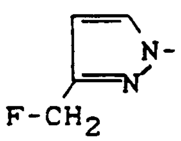 | F | $-S-CH_2-CH=CH-Cl$ |
| 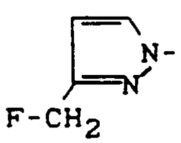 | F | $-S-CH_2-CH=CH-CH_3$ |
| 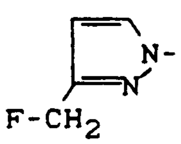 | F | $-S-\underset{CH_3}{CH}-CH=CH_2$ |

| Het | $R^1$ | $R^2$ |
|---|---|---|
| 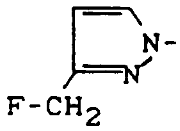 | F | $-S-CH_2-C{\equiv}CH$ |
| 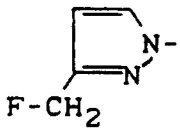 | F | 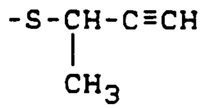 |
| 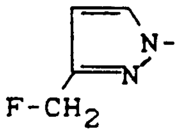 | F | 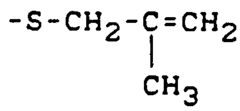 |
| 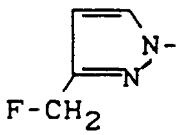 | F | $-S-CH_2-CH_2-OC_2H_5$ |
| 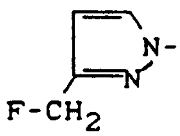 | F | 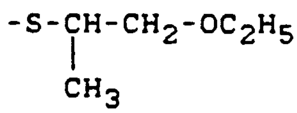 |
| 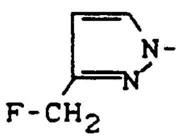 | F | 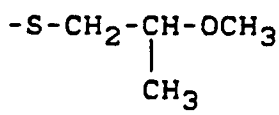 |
| 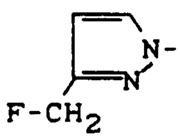 | F | 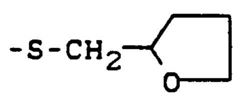 |

| Het | $R^1$ | $R^2$ |
|---|---|---|
| 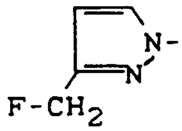 | F | 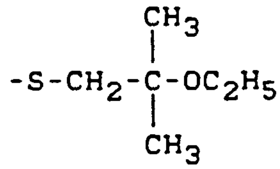 |
| 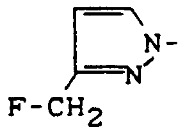 | F | $-S-CH_2-CH_2-O-CH_2-CH_2-OCH_3$ |
| 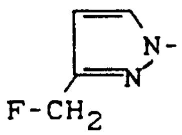 | F | $-S-CH_2-CH_2-O-CH_2-CH_2-OC_2H_5$ |
| 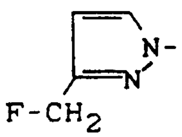 | F | 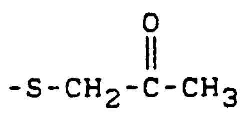 |
| 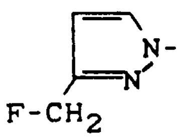 | F | 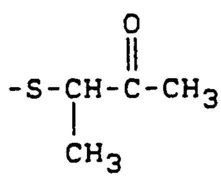 |
| 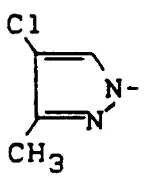 | F | 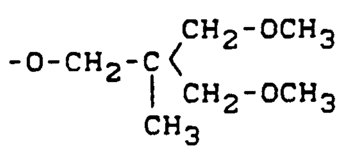 |
| 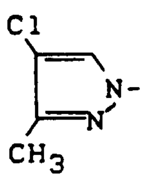 | F | 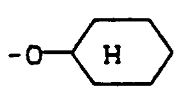 |

| Het | $R^1$ | $R^2$ |
|---|---|---|
| 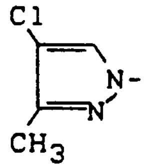 | F | 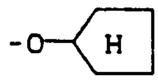 |
| 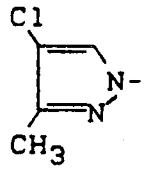 | F | 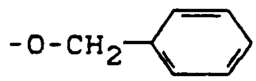 |
| 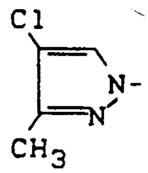 | F | $-O-CH_2-CN$ |
| 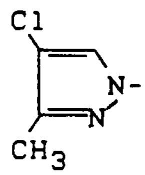 | F | $-O-CH_2-COOC_2H_5$ |
| 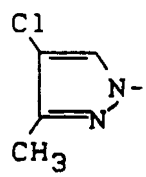 | F | 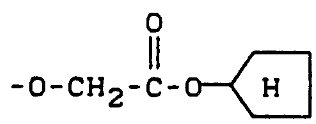 |
| 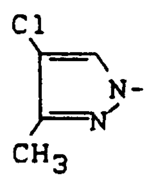 | F | 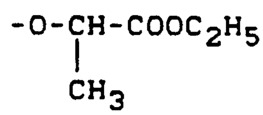 |
| 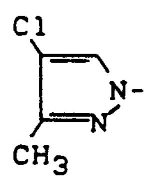 | F | 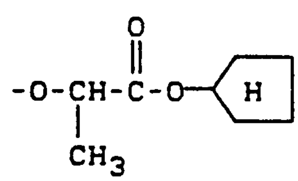 |

| Het | $R^1$ | $R^2$ |
|---|---|---|
| 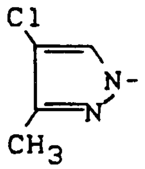 | F | 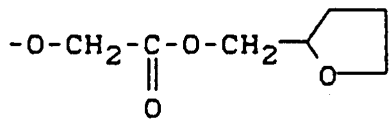 |
| 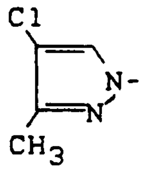 | F | 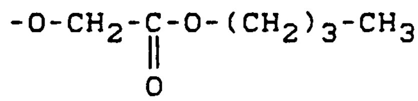 |
| 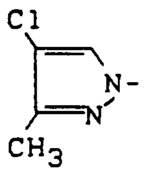 | F | 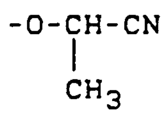 |
| 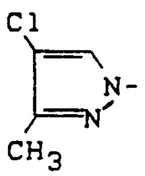 | F | $-O-CH_2-CH_2-O-CH_3$ |
| 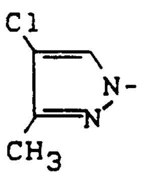 | F | 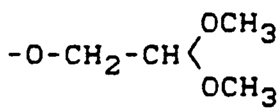 |
| 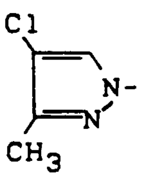 | F | 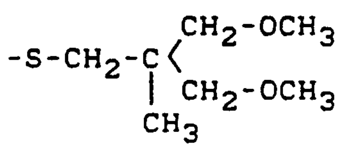 |
| 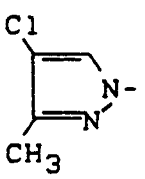 | F | 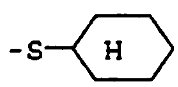 |

| Het | $R^1$ | $R^2$ |
|---|---|---|
| 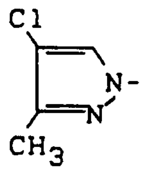 | F | 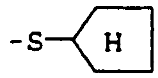 |
| 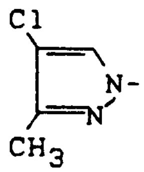 | F | 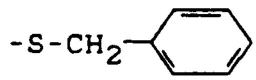 |
| 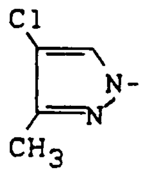 | F | $-S-CH_2-CN$ |
| 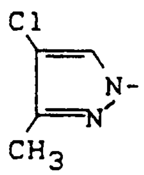 | F | $-S-CH_2-COOC_2H_5$ |
| 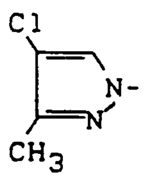 | F | 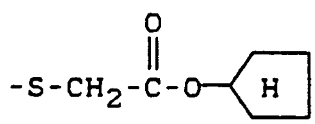 |
| 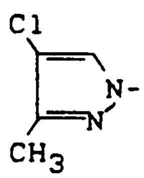 | F | 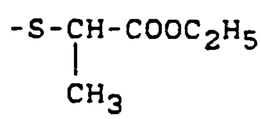 |
| 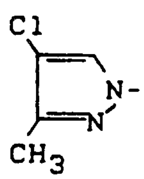 | F | 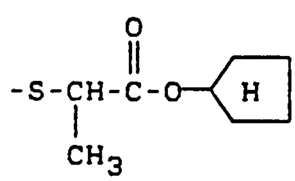 |

| Het | $R^1$ | $R^2$ |
|---|---|---|
| 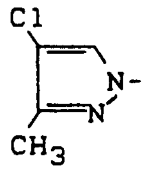 | F | 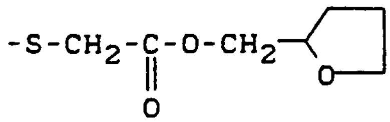 |
| 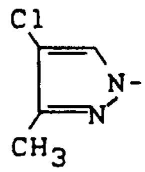 | F | 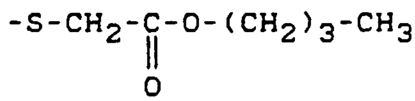 |
| 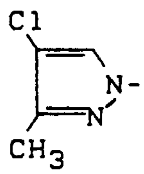 | F | 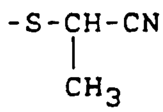 |
| 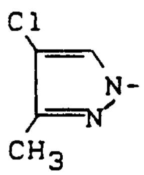 | F | $-S-CH_2-CH_2-O-CH_3$ |
| 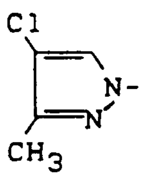 | F | 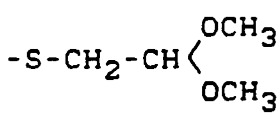 |
| 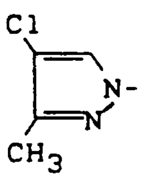 | F | $-OCH_3$ |
| 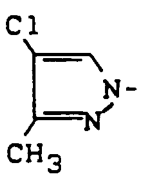 | F | $-OC_2H_5$ |

| Het | $R^1$ | $R^2$ |
|---|---|---|
| 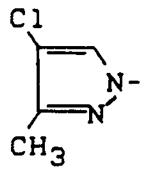 | F | $-O-CH(CH_3)_2$ |
| 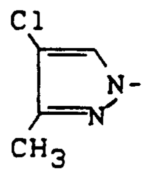 | F | $-O-CH_2-CH=CH_2$ |
| 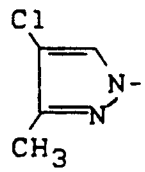 | F | $-O-CH_2-CH=CH-CH_3$ |
| 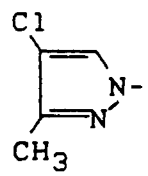 | F | $-O-CH_2-CH=CH-Cl$ |
| 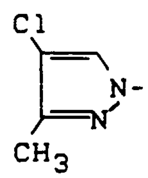 | F | 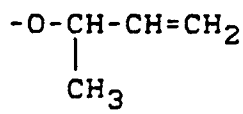 |
| 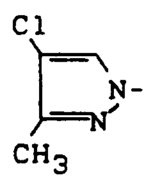 | F | $-O-CH_2-C\equiv CH$ |
| 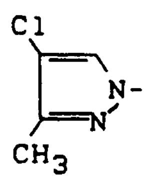 | F | 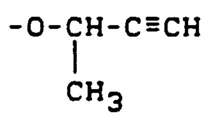 |

| Het | $R^1$ | $R^2$ |
|---|---|---|
| 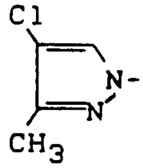 | F | 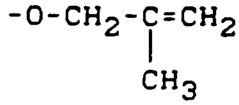 |
| 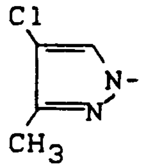 | F | $-O-CH_2-CH_2-OC_2H_5$ |
| 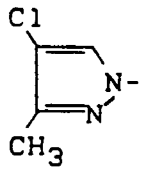 | F | 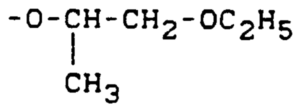 |
| 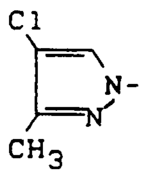 | F | 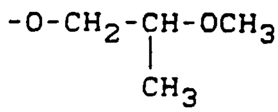 |
| 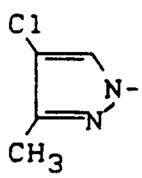 | F | 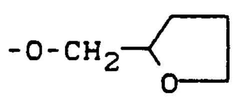 |
| 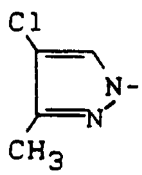 | F | 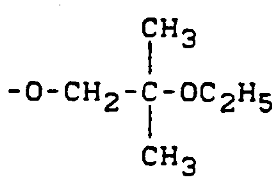 |
| 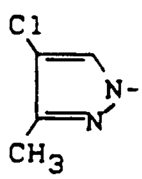 | F | $-O-CH_2-CH_2-O-CH_2-CH_2-OCH_3$ |

| Het | $R^1$ | $R^2$ |
|---|---|---|
| 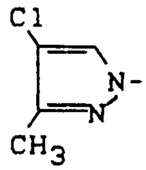 | F | $-O-CH_2-CH_2-O-CH_2-CH_2-OC_2H_5$ |
| 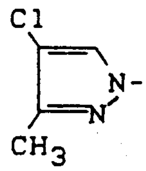 | F | 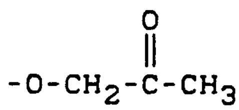 |
| 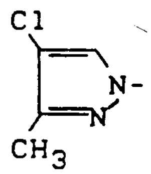 | F | 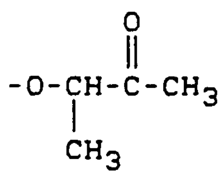 |
| 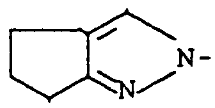 | F | $-SCH_3$ |
| 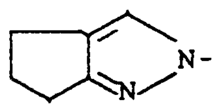 | F | $-SC_2H_5$ |
| 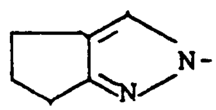 | F | $-S-CH(CH_3)_2$ |
| 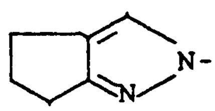 | F | $-S-CH_2-CH=CH_2$ |
| 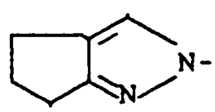 | F | $-S-CH_2-CH=CH-Cl$ |

| Het | $R^1$ | $R^2$ |
|---|---|---|
| 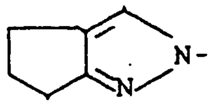 | F | $-S-CH_2-CH=CH-CH_3$ |
| 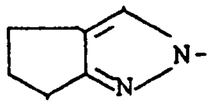 | F | $-S-CH-CH=CH_2$<br>$CH_3$ |
| 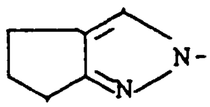 | F | $-S-CH_2-C\equiv CH$ |
| 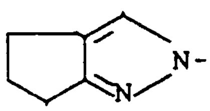 | F | $-S-CH-C\equiv CH$<br>$CH_3$ |
| 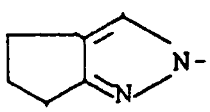 | F | $-S-CH_2-C=CH_2$<br>$CH_3$ |
| 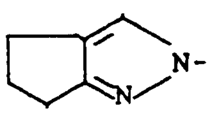 | F | $-S-CH_2-CH_2-OC_2H_5$ |
| 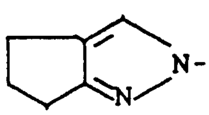 | F | $-S-CH-CH_2-OC_2H_5$<br>$CH_3$ |
| 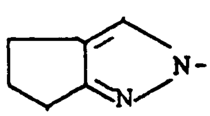 | F | $-S-CH_2-CH-OCH_3$<br>$CH_3$ |

| Het | $R^1$ | $R^2$ |
|---|---|---|
| 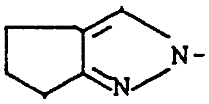 | F | 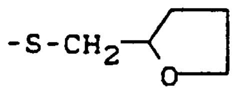 |
| 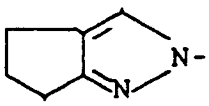 | F | 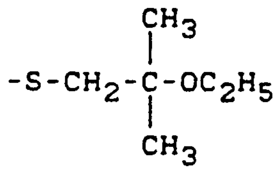 |
| 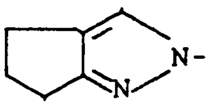 | F | $-S-CH_2-CH_2-O-CH_2-CH_2-OCH_3$ |
| 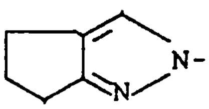 | F | $-S-CH_2-CH_2-O-CH_2-CH_2-OC_2H_5$ |
| 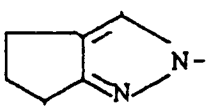 | F | 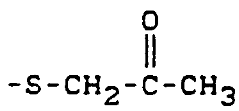 |
| 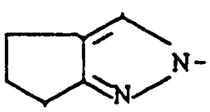 | F | 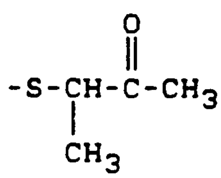 |

Verwendet man beispielsweise 4-Cyano-2,5-difluorphenylhydrazin und Acetylaceton als Ausgangsstoffe, so läßt sich der Reaktionsablauf des erfindungsgemäßen Verfahrens (a) durch das folgende Formelschema darstellen:

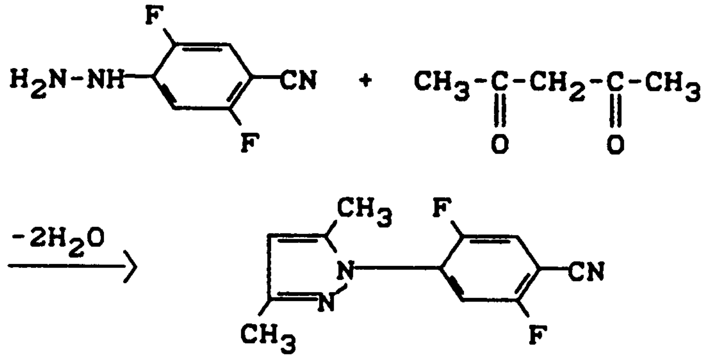

Verwendet man beispielsweise 4-(3,5-Dimethylpyrazol-1-yl)-2,5-difluorbenzonitril als Ausgangsverbindung und Sulfurylchlorid als Halogenierungsmittel, so läßt sich der Reaktionsablauf des erfindungsgemäßen Verfahrens (b) durch das folgende Formelschema darstellen:

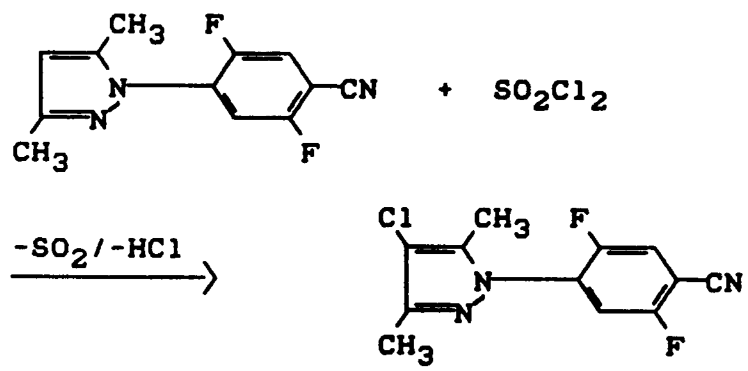


Verwendet man beispielsweise 1-(2,5-Difluor-4-cyanophenyl)-4H-pyrazolin-5-on als Ausgangsverbindung und Phosphoroxychlorid in Gegenwart von Triphenylphosphin als Halogenierungsmittel, so läßt sich der Reaktionsablauf des erfindungsgemäßen Verfahrens (c) durch das folgende Formelschema darstellen:

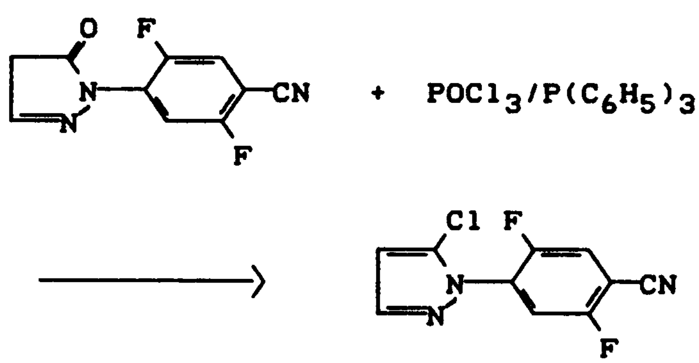


Verwendet man beispielsweise 4-Cyano-2-fluor-5-isopropoxyphenylhydrazin und N-Ethoxycarbonyl-2,2-dimethylpropanimidsäureethylester als Ausgangsstoffe, so läßt sich der Reaktionsablauf des erfindungsgemäßen Verfahrens (d) durch das folgende Formelschema darstellen:

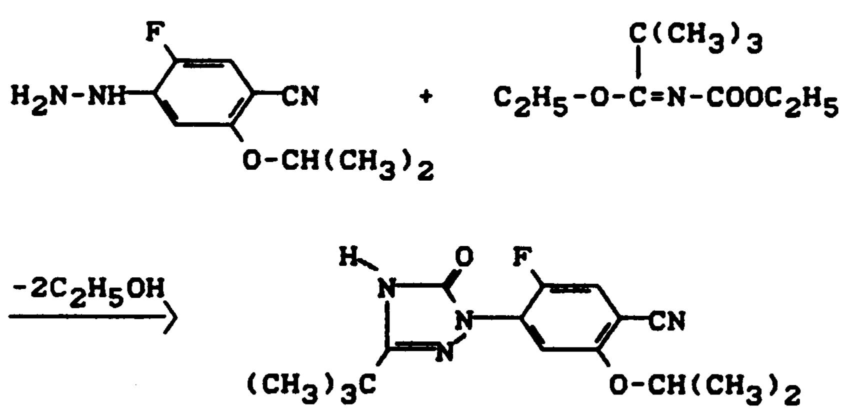


Verwendet man beispielsweise 1-(4-Cyano-2-fluor-5-allyloxyphenyl)-3-t-butyl-4H-1,2,4-triazolin-5-on und Propargylbromid als Ausgangsstoffe, so läßt sich der Reaktionsablauf des erfindungsgemäßen Verfahrens (e) durch das folgende Formelschema darstellen:

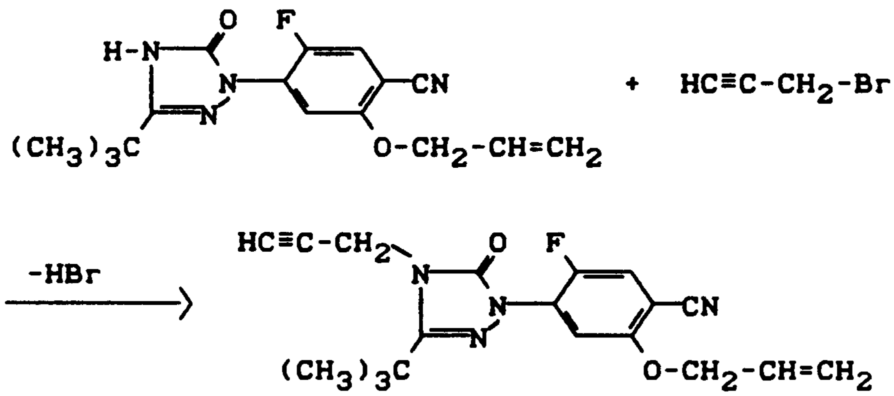


Verwendet man beispielsweise Piperidin-2-on-(2,5-difluor-4-cyanophenylhydrazon als Ausgangsverbindung, so läßt sich der Reaktionsablauf des erfindungsgemäßen Verfahrens (f) durch das folgende Formelschema darstellen:

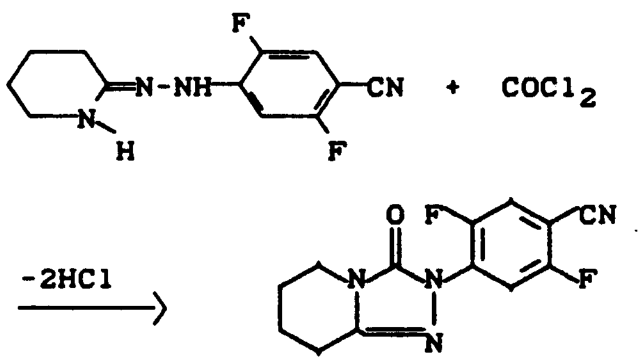


Verwendet man beispielsweise N-Pivaloyl-N'-(4-cyano-2-fluor-5-methoxyphenyl)-hydrazin als Ausgangsverbindung, so läßt sich der Reaktionsablauf des erfindungsgemäßen Verfahrens (g) durch das folgende Formelschema darstellen:

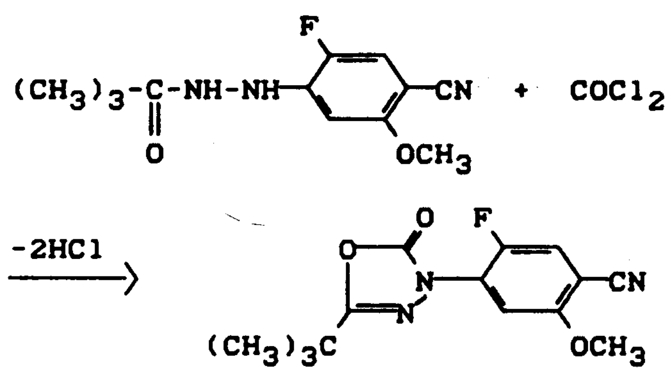


Verwendet man beispielsweise 4-(3,4,5-Trimethylpyrazol-1-yl)-2-fluorbenzonitril und t-Butylmercaptan als Ausgangsstoffe, so läßt sich der Reaktionsablauf des erfindungsgemäßen Verfahrens (h-α) durch das folgende Formelschema darstellen:

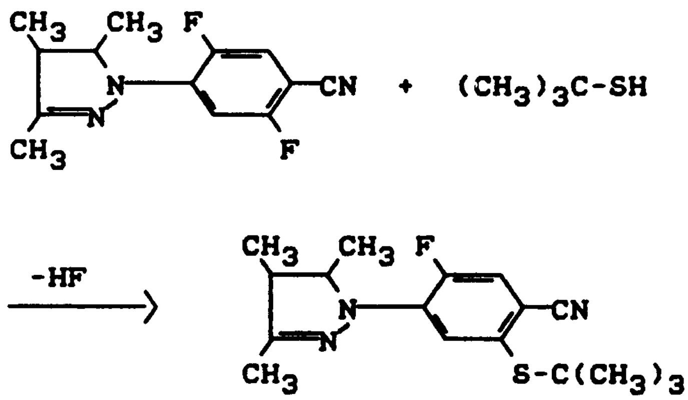


Verwendet man beispielsweise 4-(1-Pyrazolyl)-2-hydroxybenzonitril und Allylbromid als Ausgangsstoffe, so läßt sich der Reaktionsablauf des erfindungsgemäßen Verfahrens (h-$\beta$) durch das folgende Formelschema darstellen:

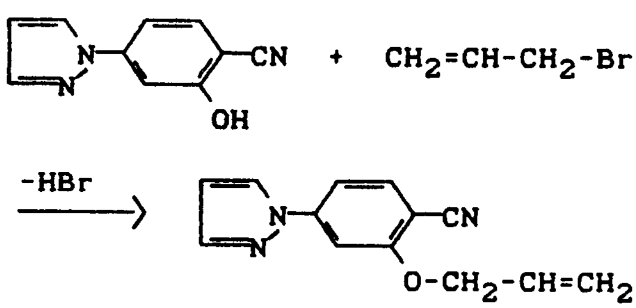


Verwendet man beispielsweise 4-(3,5-dimethyl-4-ethoxycarbonylpyrazol-1-yl)-2,5-difluorbenzonitril als Ausgangsverbindung, so läßt sich der Reaktionsablauf des erfindungsgemäßen Verfahrens (i) durch das folgende Formelschema darstellen:

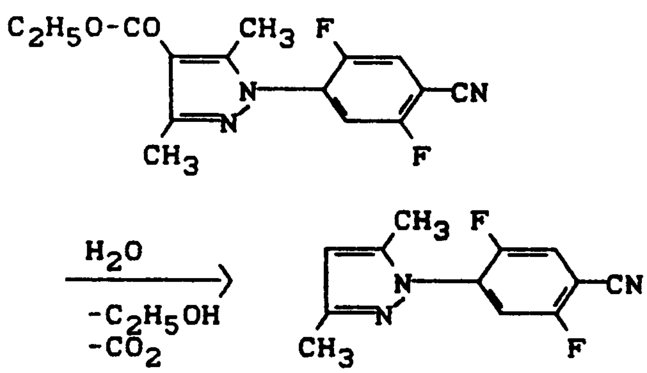


Die zur Durchführung der erfindungsgemäßen Verfahren (a) und (d) als Ausgangsstoffe benötigten 4-Cyanophenylhydrazine sind durch die Formel (II) allgemein definiert. In dieser Formel (II) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

Die 4-Cyanophenylhydrazine der Formel (II) sind noch nicht bekannt und ebenfalls Gegenstand der Erfindung.

Man erhält sie, wenn man 4-Fluorbenzonitrile der Formel (XIII),

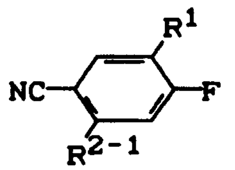

(XIII)

in welcher

$R^{2-1}$ für Halogen steht und

$R^1$ die oben angegebene Bedeutung hat,

mit Hydrazinhydrat gegebenenfalls in Gegenwart eines Verdünnungsmittels wie beispielsweise Ethanol bei Temperaturen zwischen 20 °C und 130 °C umsetzt und die so erhältlichen 4-Cyano-phenylhydrazine der Formel (IIa),

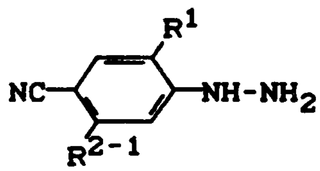

(IIa)

in welcher

$R^1$ und $R^{2-1}$ die oben angegebene Bedeutung haben,

gegebenenfalls in einer nachfolgenden 2. Stufe mit Alkoholen oder Thiolen der Formel (IX),

$R^9$-XH (IX)

in welcher

$R^9$ für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, für Cyanalkyl, Alkoxyalkyl, Alkylthioalkyl, Halogenalkoxyalkyl, Alkoxyalkoxyalkyl, (Bis-Alkoxy)alkyl, (Bis-Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen, für gegebenenfalls einfach oder mehrfach, gleich oder verschieden substituiertes Cycloalkyl, Cycloalkyloxycarbonylalkyl oder Cycloalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten jeweils infrage kommen: Halogen sowie jeweils geradkettiges oder verzweigtes Alkyl oder Alkoxy mit jeweils 1 bis 4 Kohlenstoffatomen, $R^9$ außerdem fü jeweils gegebenenfalls durch Alkyl mit 1 bis 4 Kohlenstoffatomen substituiertes Oxetanylalkyl, Tetrahydrofuranylalkyl, Tetrahydrofuranylalkyloxycarbonylalkyl oder Tetrahydropyranylalkyl mit jeweils 1 bis 3 Kohlenstoffatomen in den jeweiligen Alkylteilen steht oder $R^9$ für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Arylsubstituenten infrage kommen: Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen und

X für Sauerstoff oder Schwefel steht,

gegebenenfalls in Gegenwart eines Verdünnungsmittels wie beispielsweise Acetonitril und gegebenenfalls in Gegenwart eines Reaktionshilfsmittels wie beispielsweise Natriumhydrid oder Kaliumhydroxid bei Temperaturen zwischen 0 °C und 80 °C umsetzt.

4-Cyanophenylhydrazine der Formel (IIb),

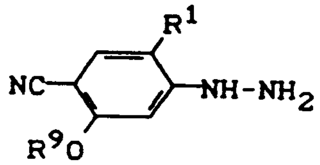

(IIb)

in welcher
$R^1$ und $R^9$ die oben angegebenen Bedeutungen haben,
erhält man alternativ auch, wenn man 4-Aminobenzonitrile der Formel (XIV),

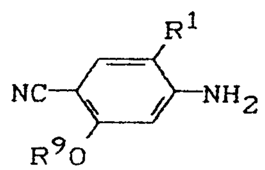

(XIV)

in welcher
$R^1$ und $R^9$ die oben angegebene Bedeutung haben,
zunächst in üblicher Art und Weise mit Natriumnitrit in Gegenwart einer Säure wie beispielsweise Salzsäure diazotiert und anschließend ebenfalls in allgemein üblicher Art und Weise mit einem Reduktionsmittel wie beispielsweise Zinn(II)chlorid reduziert.

4-Fluorbenzonitrile der Formel (XIII) sind bekannt oder erhältlich in Analogie zu bekannten Verfahren (vgl. z.B. EP 191 185; US 3 978 127; DE 2 104 312; J. Heterocycl. Chem. 15, 1373-1378 [1978]; DE 2 711 332; PCT Int. Appl. WO87/7602; JP 56/79 660; Zh. org. Khim. 3, 1257-1259 [1967]; J. Chem. Res., Synop. 1984, 382-383; Collect. Czech. Chem. Commun. 49, 992-1000 [1984]; Collect. Czech. Chem. Commun. 42, 2001-2017 [1977]).

4-Aminobenzonitrile der Formel (XIV) sind ebenfalls bekannt oder erhältlich in Analogie zu bekannten Verfahren (vgl. z.b. JP 46/3368; EP 100 172 oder EP 224 001).

Die zur Durchführung des erfindungsgemäßen Verfahrens (a) weiterhin als Ausgangsstoffe benötigten 1,3-Diketone sind durch die Formel (III) allgemein definiert. In dieser Formel (III) steht $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

$R^{4-1}$ steht vorzugsweise für Wasserstoff, Methyl, Ethyl, n- oder i-Propyl, n-, i-, s- oder t-Butyl, für Fluormethyl, Difluormethyl, Trifluormethyl, Chlormethyl, Dichlormethyl, Trichlormethyl, Difluorchlormethyl oder Dichlorfluormethyl.

$R^{5-1}$ steht vorzugsweise für Wasserstoff, Methyl, Ethyl, n- oder i-Propyl, n-, i-, s- oder t-Butyl, für Fluormethyl, Difluormethyl, Trifluormethyl, Chlormethyl, Dichlormethyl, Trichlormethyl, Difluorchlormethyl oder Dichlorfluormethyl.

1,3-Diketone der Formel (III) und Derivate dieser Diketone wie beispielsweise Enolether, Enolester, Ketale, Enoletherketale, Enamine oder $\beta$-Halogenvinylketone sind allgemein bekannte Verbindungen der organischen Chemie.

Die zur Durchführung des erfindungsgemäßen Verfahrens (b) als Ausgangsstoffe benötigten N-Aryl-Stickstoffheterocyclen sind durch die Formel (Ij) allgemein definiert. In dieser Formel (Ij) stehen $R^1$, $R^2$ und $R^3$ vorzugsweise für diejenigen Reste, die bereits im Zusammenhang mit der Beschreibung der erfindungsgemäßen Stoffe der Formel (I) als bevorzugt für diese Substituenten genannt wurden.

$R^{5-1}$ steht vorzugsweise für Wasserstoff, Methyl, Ethyl, n- oder i-Propyl, n-, i-, s- oder t-Butyl, für Fluormethyl, Difluormethyl, Trifluormethyl, Chlormethyl, Dichlormethyl, Trichlormethyl, Difluorchlormethyl oder Dichlorfluormethyl.

Die N-Aryl-Stickstoffheterocyclen der Formel (Ij) sind erfindungsgemäße Verbindungen und erhältlich mit Hilfe der erfindungsgemäßen Verfahren (a), (h-$\alpha$), (h-$\beta$) und (i).

Die zur Durchführung des erfindungsgemäßen Verfahrens (c) als Ausgangsstoffe benötigten N-Arylpyrazolinone sind durch die Formel (IV) allgemein definiert. In dieser Formel (IV) stehen $R^1$, $R^2$, $R^3$ und $R^4$ vorzugsweise für diejenigen Reste, die bereits im Zusammenhang mit der Beschreibung der erfindungsgemäßen Stoffe der Formel (I) als bevorzugt für diese Substituenten genannt wurden.

Die N-Arylpyrazolinone der Formel (IV) sind noch nicht bekannt und ebenfalls Gegenstand der Erfindung. Man erhält sie, wenn man $\beta$-Ketoester der Formel (XV),

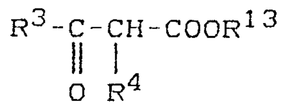 (XV)

in welcher
$R^{13}$ für Methyl oder Ethyl steht und
$R^3$ und $R^4$ die oben angegebene Bedeutung haben,
mit 4-Cyanophenylhydrazinen der Formel (II),

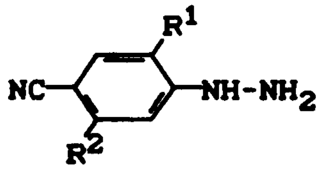 (II)

in welcher
$R^1$ und $R^2$ die oben angegebene Bedeutung haben,
gegebenenfalls in Gegenwart eines Verdünnungsmittels wie beispielsweise Ethanol und gegebenenfalls in Gegenwart eines Reaktionshilfmittels wie beispielsweise Schwefelsäure bei Temperaturen zwischen 20 °C und 120 °C umsetzt.

$\beta$-Ketoester der Formel (XV) sind allgemein bekannte Verbindungen der organischen Chemie.

Die zur Durchführung des erfindungsgemäßen Verfahrens (d) als Ausgangsstoffe benötigten Iminocarbonester sind durch die Formel (V) allgemein definiert. In dieser Formel (V) steht $R^7$ vorzugsweise für diejenigen Reste, die bereits im Zusammenhang mit der Beschreibung der erfindungsgemäßen Stoffe der Formel (I) als bevorzugt für diesen Substituenten genannt wurden.

$R^{10}$ und $R^{11}$ stehen vorzugsweise unabhängig voneinander jeweils für Methyl oder Ethyl.

Die Iminocarbonester der Formel (V) sind bekannt oder erhältlich in Analogie zu bekannten Verfahren (vgl. z.B. Chem. Ber. 119, 2444-2457 [1986]; Bull. chem. Soc. Jpn. 55, 3943-3944 [1982]; Chem. Lett. 1982, 1015-1016; Chem. Lett. 1978, 1403-1404; J. Amer. chem. Soc. 95, 3957-3963 [1973]; J. org. Chem. 36, 3251-3252 [1971]).

Die zur Durchführung des erfindungsgemäßen Verfahrens (e) als Ausgangsstoffe benötigten N-Aryl-Stickstoffheterocyclen sind durch die Formel (Id) allgemein definiert. In dieser Formel (Id) stehen $R^1$, $R^2$ und $R^7$ vorzugsweise für diejenigen Reste, die bereits im Zusammenhang mit der Beschreibung der erfindungsgemäßen Stoffe der Formel (I) als bevorugt für diese Substituenten genannt wurden.

Die N-Aryl-Stickstoffheterocyclen der Formel (Id) sind erfindungsgemäße Verbindungen und erhältlich mit Hilfe des erfindungsgemäßen Verfahrens (d).

Die Zur Durchführung des erfindungsgemäßen Verfahrens (e) weiterhin als Ausgangsstoffe benötigten Alkylierungsmittel sind durch die Formel (VI) allgemein definiert. In dieser Formel (VI) steht $R^{8-1}$ vorzugsweise für Methyl, Ethyl, n- oder i- Propyl, n-, i-, s- oder t-Butyl, für jeweils einfach, zweifach oder dreifach durch Fluor und/oder Chlor substituiertes Methyl, Ethyl oder t-Butyl, für Allyl, für n- oder i-Butenyl, für Chlorallyl, für Dichlorallyl, für Propargyl oder für Chlorpropargyl.

$E^1$ steht vorzugsweise für Chlor, Brom oder Iod, oder für jeweils gegebenenfalls substituiertes Alkylsulfonyloxy, Alkoxysulfonyloxy oder Arylsulfonyloxy, wie beispielsweise Methansulfonyloxy, Trifluormethansulfonyloxy, Methoxysulfonyloxy, Ethoxysulfonyloxy oder p-Toluolsulfonyloxy.

Die Alkylierungsmittel der Formel (VI) sind allgemein bekannte Verbindungen der organischen Chemie.

Die zur Durchführung des erfindungsgemäßen Verfahrens (f) als Ausgangsstoffe benötigten Amidrazone sind durch die Formel (VII) allgemein definiert. In dieser Formel (VII) stehen $R^1$ und $R^2$ vorzugsweise für diejenigen Reste, die bereits im Zusammenhang mit der Beschreibung der erfindungsgemäßen Stoffe der Formel (I) als bevorzugt für diese Substituenten genannt wurden.

$R^{7-1}$ und $R^{8-2}$ stehen vorzugsweise gemeinsam für einen zweifach verknüpften 1,3-Propandiylrest,

einen 1,4-Butandiylrest oder einen 1,5-Pentandiylrest.

Die Amidrazone der Formel (VII) sind noch nicht bekannt. Man erhält sie in Analogie zu bekannten Verfahren (vgl. z.B. US 4 080 192 oder DE-OS 1 957 783), wenn man Lactame der Formel (XVI),

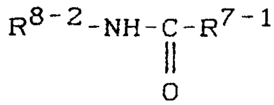 (XVI)

in welcher

$R^{7-1}$ und $R^{8-2}$ die oben angegebene Bedeutung haben,

zunächst in einer 1. Stufe mit einem Halogenierungsmittel wie beispielsweise Phosphoroxychlorid, Thionylchlorid oder Phosgen gegebenenfalls in Gegenwart eines Verdünnungsmittels wie beispielsweise Ethanol oder Dioxan und gegebenenfalls in Gegenwart eines Reaktionshilfsmittels wie beispielsweise Triethylamin oder Pyridin bei Temperaturen zwischen 0 °C und 50 °C umsetzt und anschließend die so erhältlichen Imidchloride der Formel (XVII),

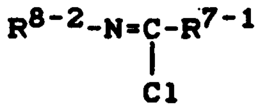 (XVII)

in welcher

$R^{7-1}$ und $R^{8-2}$ die oben angegebene Bedeutung haben,

mit 4-Cyanophenylhydrazinen der Formel (II),

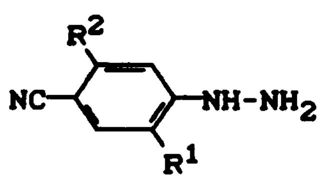 (II)

in welcher

$R^1$ und $R^2$ die oben angegebene Bedeutung haben,

gegebenenfalls in Gegenwart eines Verdünnungsmittels wie beispielsweise Ethanol und gegebenenfalls in Gegenwart eines Reaktionshilfsmittels wie beispielsweise Triethylamin oder Pyridin bei Temperaturen zwischen 0 °C und 80 °C umsetzt.

Lactame der Formel (XVI) sind allgemein bekannte Verbindungen der organischen Chemie.

Die zur Durchführung des erfindungsgemäßen Verfahrens (g) als Ausgangsstoffe benötigten Phenylhydrazide sind durch die Formel (VIII) allgemein definiert. In dieser Formel (VIII) stehen $R^1$, $R^2$ und $R^6$ vorzugsweise für diejenigen Reste, die bereits im Zusammenhang mit der Beschreibung der erfindungsgemäßen Stoffe der Formel (I) als bevorzugt für diese Substituenten genannt wurden.

Die Phenylhydrazide der Formel (VIII) sind noch nicht bekannt.

Man erhält sie, wenn man 4-Cyanophenylhydrazine der Formel (II),

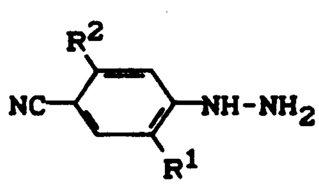 (II)

in welcher
$R^1$ und $R^2$ die oben angegebene Bedeutung haben,
mit Acylierungsmitteln der Formel (XVIII),

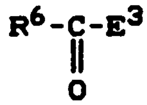

(XVIII)

in welcher
$R^6$ die oben angegebene Bedeutung hat und
$E^3$ für eine Elektronenanziehende Abgangsgruppe, vorzugsweise für Halogen oder für einen Rest

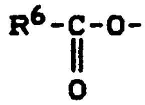

steht, wobei $R^6$ die oben angegebene Bedeutung hat und insbesondere für Chlor steht,
gegebenenfalls in Gegenwart eines Verdünnungsmittels wie beispielsweise Dichlormethan und gegebenenfalls in Gegenwart eines Reaktionshilfsmittels wie beispielsweise Triethylamin bei Temperaturen zwischen - 20 °C und + 60 °C umsetzt.

Acylierungsmittel der Formel (XVIII) sind allgemein bekannte Verbindungen der organischen Chemie.

Die zur Durchführung des erfindungsgemäßen Verfahrens (h-$\alpha$) als Ausgangsstoffe benötigten N-Aryl-Stickstoffheterocyclen sind durch die Formel (Ik) allgemein definiert. In dieser Formel (Ik) stehen $R^1$, $R^3$, $R^4$ und $R^5$ vorzugsweise für diejenigen Reste, die bereits im Zusammenhang mit der Beschreibung der erfindungsgemäßen Stoffe der Formel (I) als bevorzugt für diese Substituenten genannt wurden.

$R^{2-1}$ steht vorzugsweise für Fluor, Chlor oder Brom, insbesondere für Fluor.

Die N-Aryl-Stickstoffheterocyclen der Formel (Ik) sind erfindungsgemäße Verbindungen und erhältlich mit Hilfe der erfindungsgemäßen Verfahren (a), (b), (c) oder (i).

Die zur Durchführung des erfindungsgemäßen Verfahrens (h-$\alpha$) weiterhin als Ausgangsstoffe benötigten Alkohole oder Thiole sind durch die Formel (IX)) allgemein definiert. In dieser Formel (IX) stehen $R^9$ und X vorzugsweise für diejenigen Reste, die bereits im Zusammenhang mit der Beschreibung der erfindungsgemäßen Stoffe der Formel (I) als bevorzugt für diese Substituenten genannt wurden.

Die Alkohole und Thiole der Formel (IX) sind allgemein bekannte Verbindungen der organischen Chemie.

Die zur Durchführung des erfindungsgemäßen Verfahrens (h-$\beta$) als Ausgangsstoffe benötigten (Thio)-Phenolderivate sind durch die Formel (X) allgemein definiert. In dieser Formel (X) stehen $R^1$, $R^3$, $R^4$, $R^5$ und X vorzugsweise für diejenigen Reste, die bereits im Zusammenhang mit der Beschreibung der erfindungsgemäßen Stoffe der Formel (I) als bevorzugt für diese Substituenten genannt wurden.

Die (Thio)Phenolderivate der Formel (X) sind noch nicht bekannt und ebenfalls Gegenstand der Erfindung.

Man erhält sie entweder wenn man N-Aryl-Stickstoffheterocyclen der Formel (Ik),

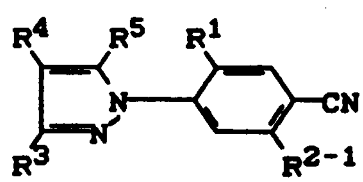

(Ik)

in welcher
$R^{2-1}$ für Halogen, insbesondere für Fluor steht und
$R^1$, $R^3$, $R^4$ und $R^5$ die oben angegebene Bedeutung haben,
mit Natriumhydrogensulfid gegebenenfalls in Gegenwart eines Verdünnungsmittels wie beispielsweise Methanol, Ethanol oder deren Gemische mit Wasser und gegebenenfalls in Gegenwart eines Reaktionshilfsmittels wie beispielsweise Natriumhydroxid oder Kaliumcarbonat, gegebenenfalls in Gegenwart einer Stickstoff- oder Argonschutzgasatomosphäre bei Temperaturen zwischen 0 °C und 50 °C umsetzt oder wenn man N-Aryl-Stickstoffheterocyclen der Formel (II),

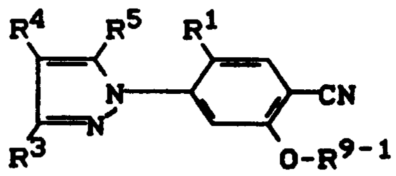

(I1)

in welcher

$R^1$, $R^3$, $R^4$ und $R^5$ die oben angegebene Bedeutung haben und

$R^{9-1}$ für Allyl oder für Benzyl steht,

mit üblichen Reduktionsmitteln, wie beispielsweise molekularem Wasserstoff in Gegenwart eines üblichen Hydrierkatalysators, wie Trimethylsilyliodid oder wie Tris-Triphenylphosphin-Rhodiumchlorid gegebenenfalls in Gegenwart eines Verdünnungsmittels wie beispielsweise Ethanol oder Dichlormethan und gegebenenfalls in Gegenwart eines Reaktionshilfsmittels wie beispielsweise Diazabicyclooctan (DABCO) bei Temperaturen zwischen 20 °C und 120 °C umsetzt.

N-Aryl-Stickstoffheterocyclen der Formel (Ik) sind erfindungsgemäße Verbindungen und erhältlich mit Hilfe der erfindungsgemäßen Verfahren (a), (b), (c) oder (i).

N-Aryl-Stickstoffheterocyclen der Formel (Il) sind ebenfalls erfindungsgemäße Verbindungen und erhältlich mit Hilfe der erfindungsgemäßen Verfahren (a), (b), (c) und (i).

Die zur Durchführung des erfindungsgemäßen Verfahrens (h-$\beta$) weiterhin als Ausgangsstoffe benötigten Alkylierungsmittel sind durch die Formel (XI) allgemein definiert. In dieser Formel (XI) steht $R^9$ vorzugsweise für diejenigen Feste, die bereits im Zusammenhang mit der Beschreibung der erfindungsgemäßen Stoffe der Formel (I) als bevorzugt für diesen Substituenten genannt wurden.

$E^2$ steht vorzugsweise für Chlor, Brom oder Iod, oder für jeweils gegebenenfalls substituiertes Alkylsulfonyloxy, Alkoxysulfonyloxy oder Arylsulfonyloxy, wie beispielsweise Methansulfonyloxy, Trifluormethansulfonyloxy, Methoxysulfonyloxy, Ethoxysulfonyloxy oder p-Toluolsulfonyloxy.

Die Alkylierungsmittel der Formel (XI) sind allgemein bekannte Verbindungen der organischen Chemie.

Die zur Durchführung des erfindungsgemäßen Verfahrens (i) als Ausgangsstoffe benötigten N-Arylpyrazolyl-4-carbonsäureester sind durch die Formel (XII) allgemein definiert. In dieser Formel (XII) stehen $R^1$, $R^2$, $R^3$ und $R^5$ vorzugsweise für diejenigen Reste, die bereits im Zusammenhang mit der Beschreibung der erfindungsgemäßen Stoffe der Formel (I) als bevorzugt für diese Substituenten genannt wurden.

$R^{12}$ steht vorzugsweise für Methyl oder Ethyl.

Die N-Arylpyrazolyl-4-carbonsäureester der Formel (XII) sind noch nicht bekannt.

Man erhält sie, wenn man Acrylester-Derivate der Formel (XIX),

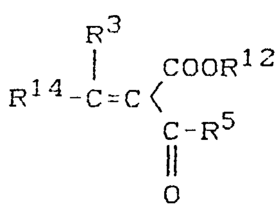

(XIX)

in welcher

$R^3$, $R^5$ und $R^{12}$ die oben angegebene Bedeutung haben und

$R^{14}$ für einen Alkoxyrest oder für einen Dialkylaminorest mit jeweils 1 bis 4 Kohlenstoffatomen in den einzelnen geradkettigen oder verzweigten Alkylteilen, insbesondere mit jeweils 1 bis 2 Kohlenstoffatomen in den einzelnen Alkylteilen steht,

mit 4-Cyanophenylhydrazinen der Formel (II),

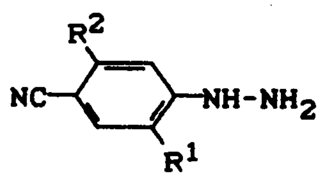

(II)

in welcher

$R^1$ und $R^2$ die oben angegebene Bedeutung haben,

gegebenenfalls in Gegenwart eines Verdünnungsmittels wie beispielsweise Ethanol oder Ethylenglykolmonoethylether und gegebenenfalls in Gegenwart eines Reaktionshilfsmittels wie beispielsweise Salzsäure oder Schwefelsäure bei Temperaturen zwischen 20 °C und 150 °C umsetzt.

Acrylester-Derivate der Formel (XIX) sind bekannt oder erhältlich in Analogie zu bekannten Verfahren (vgl. z.B. EP 257 882; JP 62/148 482; PCT Int. Appl. WO 86/1202; EP 188 094; US 4 555 517; EP 104 432; J. org. Chem. 49, 140-152 [1984]; Austral. J. Chem. 34, 2401-2421 [1981]; BE 888 389; US 4 277 418; Farmaco. Ed. Sci. 34, 898-906 [1979]; J. chem. Soc. Perkin Trans. 1, 1979; 464-471; J. chem Soc. Perkin Trans. 1, 1978, 1041-1046).

Als Verdünnungsmittel zur Durchführung des erfindungsgemäßen Verfahren (a) kommen inerte organische Lösungsmittel infrage. Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid, Alkohole wie Methanol, Ethanol oder Propanol oder Säuren, wie Essigsäure.

Das erfindungsgemäße Verfahren (a) wird gegebenenfalls in Gegenwart eines geeigneten Reaktionshilfsmittels durchgeführt. Als solche kommen insbesondere anorganische Mineralsäuren wie beispielsweise Salzsäure oder Schwefelsäure infrage. Es ist auch möglich, die als Ausgangsstoffe infrage kommenden 4-Cyanophenylhydrazine der Formel (II) in Form von entsprechenden Säureadditionssalzen, wie beispielsweise Hydrochloriden einzusetzen.

Die Reaktionstemperaturen können bei der Durchführung des erfindungsgemäßen Verfahrens (a) in einem größeren Bereich variiert werden. Im allgemeinen arbeitet man bei Temperaturen zwischen -20 °C und 180 °C, vorzugsweise bei Temperaturen zwischen 20 °C und 120 °C.

Zur Durchführung des erfindungsgemäßen Verfahrens (a) setzt man pro Mol an 4-Cyanophenylhydrazin der Formel (II) bzw. an einem entsprechenden Säureadditionssalz im allgemeinen 0.5 bis 10.0 Mol an 1,3-Diketon der Formel (III) oder an einem entsprechenden Derivat und gegebenenfalls 0.01 bis 1.0 Mol an Reaktionshilfsmittel ein. Die Reaktionsdurchführung, Aufarbeitung und Isolierung der N-Aryl-Stickstoffheterocyclen der Formel (Ia) erfolgt nach allgemein üblichen Methoden (vgl. auch die Herstellungsbeispiele).

Bei Verwendung von 1,3-Diketonen der Formel (III), bei welchen der Substituent $R^{5-1}$ verschieden von dem Substituenten $R^3$ ist, erhält man in der Regel Isomerengemische aus Verbindungen der Formel (Ia1),

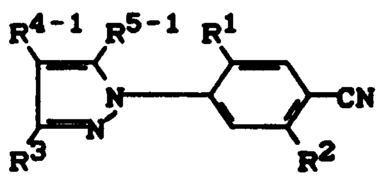

(Ia1)

und Verbindungen der Formel (Ia2),

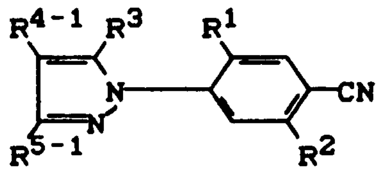

(Ia2)

wobei

$R^1$, $R^2$, $R^3$, $R^{4-1}$ und $R^{5-1}$ jeweils die oben angegebene Bedeutung haben.

Aus diesen Isomerengemischen lassen sich mit üblichen Trennverfahren (Destillation, Kristallisation, Chromatographie) die gewünschten Reaktionsprodukte der Formel (Ia) isolieren.

Als Halogenierungsmittel zur Durchführung des erfindungsgemäßen Verfahrens (b) kommen übliche Halogenierungsmittel infrage. Mit besonderem Vorzug verwendet man Sulfurylchlorid, elementares Chlor oder elementares Brom als Halogenierungsmittel.

Als Verdünnungsmittel zur Durchführung des erfindungsgemäßen Verfahrens (b) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, O-Dichlorbenzol, Petrolether, Hexan, Cyclohexan, Dichlormethan, Chloroform oder Tetrachlorkohlenstoff.

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 150 °C, vorzugsweise bei Temperaturen zwischen 35 °C und 70 °C.

Zur Durchführung des erfindungsgemäßen Verfahrens (b) setzt man pro Mol an N-Aryl-Stickstoffheterocyclus der Formel (Ij) im allgemeinen 1.0 bis 5.0 Mol, vorzugsweise 1.0 bis 2.0 Mol an Halogenierungsmittel ein.

Die Reaktionsdurchführung, Aufarbeitung und Isolierung der Reaktionsprodukte erfolgt nach allgemein üblichen Methoden (vgl. auch die Herstellungsbeispiele).

Als Halogenierungsmittel zur Durchführung des erfindungsgemäßen Verfahrens (c) kommen ebenfalls übliche Halogenierungsmittel infrage. Mit besonderem Vorzug verwendet man anorganische Säurehalogenide wie beispielsweise Phosphoroxychlorid, Thionylchlorid, Phosgen, Phosphortribromid oder Diphosgen ($Cl_3C$-O-CO-Cl).

Als Verdünnungsmittel zur Durchführung des erfindungsgemäßen Verfahrens (c) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, O-Dichlorbenzol, Petrolether, Hexan, Cyclohexan, Dichlormethan, Chloroform oder Tetrachlorkohlenstoff oder basische Verdünnungsmittel wie beispielsweise Pyridin. Es ist auch möglich, einen entsprechenden Überschuß an Halogenierungsmittel gleichzeitig als Verdünnungsmittel einzusetzen.

Das erfindungsgemäße Verfahren (c) wird vorzugsweise in Gegenwart eines geeigneten Reaktionshilfsmittels durchgeführt. Als solche kommen insbesondere übliche Hilfsnukleophile wie beispielsweise Triphenylphosphin, Dimethylanilin oder Dimethylformamid infrage.

Die Reaktionstemperaturen können bei der Durchführung des erfindungsgemäßen Verfahrens (c) in einem größeren Bereich variiert werden. Im allgemeinen arbeitet man bei Temperaturen zwischen 0 °C und 200 °C, vorzugsweise bei Temperaturen zwischen 30 °C und 150 °C.

Zur Durchführung des erfindungsgemäßen Verfahrens (c) setzt man pro Mol an N-Arylpyrazolinon der Formel (IV) im allgemeinen 1.0 bis 10.0 Mol, vorzugsweise 1.0 bis 5.0 Mol an Halogenierungsmittel und gegebenenfalls 0.01 bis 1.0 Mol, vorzugsweise 0.05 bis 0.1 Mol an Reaktionshilfsmittel ein.

Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (d) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide, wie Dimethylsulfoxid oder Alkohole, wie Methanol, Ethanol oder Propanol.

Die Reaktionstemperaturen können bei der Durchführung des erfindungsgemäßen Verfahrens (d) in einem größeren Bereich variiert werden. Im allgemeinen arbeitet man bei Temperaturen zwischen 0 °C und 180 °C, vorzugsweise bei Temperaturen zwischen 50 °C und 150 °C.

Zur Durchführung des erfindungsgemäßen Verfahrens (d) setzt man pro Mol an 4-Cyanophenylhydrazin der Formel (II) im allgemeinen 1.0 bis 3.0 Mol, vorzugsweise 1.0 bis 2.0 Mol an Iminocarbonester der Formel (V) ein.
Die Reaktionsdurchführung, Aufarbeitung und Isolierung der Reaktionsprodukte erfolgt nach allgemein üblichen Methoden (vgl. auch die Herstellungsbeispiele).

Zur Durchführung des erfindungsgemäßen Verfahrens (e) kommen als Verdünnungsmittel inerte organische Lösungsmittel infrage. Vorzugsweise verwendet man aliphatische, cyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Nitrile, wie Acetonitril oder Propionitril oder Amide, wie Dimethylformamid, Dimethylacetamid, N-Methylpyrrolidon oder Hexamethylphosphorsäuretriamid. Verwendet man Alkylierungsmittel der Formel (VI) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (e) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat, Kaliumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des Herstellungsverfahrens (e) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (e) setzt man pro Mol an N-Aryl-Stickstoffheterocyclus der Formel (Id) im allgemeinen 1.0 bis 15.0 Mol, vorzugsweise 1.0 bis 5.0 Mol an Alkylierungsmittel der Formel (VI) und gegebenenfalls 1.0 bis 3.0 Mol, vorzugsweise 1.0 bis 2.0 Mol an Reaktionshilfsmittel ein.
Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung der erfindungsgemäßen Verfahren (f) und (g) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, O-Dichlorbenzol, Petrolether, Hexan, Cyclohexan, Dichlormethan, Chloroform oder Tetrachlorkohlenstoff.

Die erfindungsgemäßen Verfahren (f) und (g) werden vorzugsweise in Gegenwart eines geeigneten Reaktionshilfsmittels durchgeführt. Als solche kommen alle üblichen Basen in Frage. Mit besonderem Vorzug verwendet man tertiäre Amine, wieTriethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (f) und (g) in einem größeren Bereich variiert werden. Im allgemeinen arbeitet man bei Temperaturen zwischen -20 °C und 180 °C, vorzugsweise bei Temperaturen zwischen 0 °C und 150 °C.

Zur Durchführung des erfindungsgemäßen Verfahrens (f) setzt man pro Mol an Amidrazon der Formel (VII) im allgemeinen 1.0 bis 5.0 Mol, vorzugsweise 1.0 bis 1.5 Mol an Phosgen und gegebenenfalls 1.0 bis 5.0 Mol, vorzugsweise 1.0 bis 3.0 Mol an Reaktionshilfsmittel ein.
Die Reaktionsdurchführung, Aufarbeitung und Isolierung der Reaktionsprodukte erfolgt nach allgemein üblichen Methoden.

Zur Durchführung des erfindungsgemäßen Verfahrens (g) setzt man pro Mol an Phenylhydrazid der Formel (VIII) im allgemeinen 1.0 bis 5.0 Mol, vorzugsweise 1.0 bis 1.5 Mol an Phosgen und gegebenenfalls 1.0 bis 5.0 Mol, vorzugsweise 1.0 bis 3.0 Mol an Reaktionshilfsmittel ein.
Die Reaktionsdurchführung, Aufarbeitung und Isolierung der Reaktionsprodukte erfolgt nach allgemein üblichen Methoden (vgl. auch die Herstellungsbeispiele).

Als Verdünnungsmittel zur Durchführung des erfindungsgemäßen Verfahrens (h-$\alpha$) kommen inerte organische Lösungsmittel infrage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester oder Sulfoxide, wie Dimethylsulfoxid. Es ist auch möglich die als Reaktionspartner infrage kommenden Alkohole oder Thiole der Formel (IX) in einem entsprechenden Überschuß gleichzeitig als Verdünnungsmittel einzusetzen.

Das erfindungsgemäße Verfahre (h-$\alpha$) wird vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -amide, -alkoholate oder -carbonate wie beispielsweise Natriumhydrid, Natriumamid, Natriumhydroxid, Kaliumhydroxid, Natriummethylat, Natriumethylat, Kalium-t-butylat oder Kaliumcarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (h-$\alpha$) in einem größeren Bereich variiert werden. Im allgemeinen arbeitet man bei Temperaturen zwischen 0 °C und 150 °C, vorzugsweise bei Temperaturen zwischen 20 °C und 120 °C.

Zur Durchführung des erfindungsgemäßen Verfahrens (h-$\alpha$) setzt man pro Mol an N-Aryl-Stickstoffheterocyclus der Formel (Ik) im allgemeinen 1.0 bis 3.0 Mol, vorzugsweise 1.0 bis 1.5 Mol an Alkohol oder Thiol der Formel (IX) und 1.0 bis 3.0 Mol, vorzugsweise 1.0 bis 2.0 Mol an Reaktionshilfsmittel ein. Die Reaktionsdurchführung, Aufarbeitung und Isolierung der Reaktionsprodukte erfolgt nach allgemein üblichen Methoden (vgl. auch die Herstellungsbeispiele).

Zur Durchführung des erfindungsgemäßen Verfahrens (h-$\beta$) kommen als Verdünnungsmittel inerte organische Lösungsmittel infrage. Vorzugsweise verwendet man aliphatische, alicyclische oder aromatische, gegebenenfalls halogenierte Kohlenwasserstoffe, wie beispielsweise Benzin, Benzol, Toluol, Xylol, Pentan, Hexan, Heptan, Cyclohexan, Petrolether, Ligroin, Methylenchlorid, Chloroform, Tetrachlorkohlenstoff, Chlorbenzol oder Dichlorbenzol, Ether, wie Diethylether, Diisopropylether, Dioxan, Tetrahydrofuran oder Ethylenglykoldiethylether oder -dimethylether, Ketone, wie Aceton, Butanon, Methylisopropylketon oder Methylisobutylketon, Ester, wie Essigsäureethylester, Säuren, wie Essigsäure, Nitrile, wie Acetonitril oder Propionitril, Amide, wie Dimethylformamid, Dimethylacetamid, N-Methylpyrrolidon oder Hexamethylphosphorsäuretriamid. Verwendet man als Reaktionspartner Alkylierungsmittel der Formeln (VI), (VII) oder (XI) in flüssiger Form, so ist es auch möglich, diese in entsprechendem Überschuß gleichzeitig als Verdünnungsmittel einzusetzen.

Als Reaktionshilfsmittel zur Durchführung des erfindungsgemäßen Verfahrens (h-$\beta$) kommen alle üblicherweise verwendbaren anorganischen und organischen Basen in Frage. Vorzugsweise verwendet man Alkalimetallhydride, -hydroxide, -amide, -carbonate oder -hydrogencarbonate, wie beispielsweise Natriumhydrid, Natriumamid, Natriumhydroxid, Natriumcarbonat oder Natriumhydrogencarbonat, oder auch tertiäre Amine, wie beispielsweise Triethylamin, N,N-Dimethylanilin, Pyridin, 4-(N,N-Dimethylamino)-pyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (h-$\beta$) in einem größeren Bereich variiert werden. Im allgemeinen arbeitet man zwischen -20 °C und +150 °C, vorzugsweise zwischen 0 °C und +100 °C.

Zur Durchführung des erfindungsgemäßen Verfahrens (h-$\beta$) setzt man pro Mol an (Thio)Phenolderivat der Formel (X) im allgemeinen 1,0 bis 20,0 Mol, vorzugsweise jeweils 1,0 bis 15,0 Mol, an Alkylierungs- bzw. Acylierungsmittel der Formel (XI) und gegebenenfalls 1,0 bis 3,0 Mol, vorzugsweise 1,0 bis 2,0 Mol an Reaktionshilfsmittel, ein. Die Reaktionsdurchführung, Aufarbeitung und Isolierung der Reaktionsprodukte der Formel (Ih) erfolgt nach üblichen Methoden.

Als Verdünnungsmittel zur Durchführung des erfindungsgemäßen Verfahrens (i) kommen anorganische oder organische Lösungsmittel in Frage. Vorzugsweise verwendet man polare Lösungsmittel, insbesondere Alkohole, wie beispielsweise Methanol, Ethanol oder Propanol, oder deren Gemische mit Wasser.

Als Reaktionshilfsmittel zur Durchführung des erfindungsgemäßen Verfahrens (i) kommen alle üblicherweise für derartige Esterverseifungen und Decarboxylierungen verwendbaren Katalysatoren in Frage. Vorzugsweise verwendet man Basen, wie beispielsweise Natriumhydroxid, Natriumalkoholat oder Natriumcarbonat oder Säuren, wie beispielsweise Chlorwasserstoffsäure, Bromwasserstoffsäure oder Schwefelsäure.

Die Reaktionstemperaturen können bei der Durchführung des erfindungsgemäßen Verfahrens (i) in einem größeren Bereich variiert werden. Im allgemeinen arbeitet man bei Temperaturen zwischen 20 °C und 250 °C, vorzugsweise bei Temperaturen zwischen 50 °C und 150 °C.

Zur Durchführung des erfindungsgemäßen Verfahrens (i) setzt man pro Mol an 1-Arylpyrazolyl-4-carbonsäureester der Formel (XII) im allgemeinen 1.0 bis 15.0 Mol, vorzugsweise 1.0 bis 2.5 Mol an saurem oder basischem Reaktionshilfsmittel ein und erwärmt für mehrere Stunden auf die erforderliche Reaktionstemperatur.

Die Aufarbeitung und Isolierung der Reaktionsprodukte erfolgt nach allgemein üblichen Methoden.

In Abhängigkeit von der Reaktionstemperatur und der Reaktionsdauer ist es auch möglich, die intermediär auftretenden 1-Arylpyrazolyl-4-carbonsäuren zu isolieren und in einem getrennten Reaktionsschritt zu decarboxylieren.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe der Formel (I) mit besonders gutem Erfolg zur Bekämpfung von dikotylen Unkräutern in mono- und dikotylen Kulturen, wie beispielsweise Soja oder Weizen einsetzen. Auch die Zwischenprodukte der Formel (X) besitzen gute herbizide Wirksamkeit.

Darüberhinaus greifen die erfindungsgemäßen Wirkstoffe in den Metabolismus der Pflanzen ein und können deshalb als Wachstumsregulatoren eingesetzt werden.

Für die Wirkungsweise von Pflanzenwachstumsregulatoren gilt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage:

Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können bei der Anwendung als Herbizide als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (Amethydione) oder N-(2-Benzthiazolyl)-N,N′-dimethyl-harnstoff (Metabenzthiazuron) zur Unkrautbekämpfung in Getreide; 4-Amino-3-methyl-6-phenyl-1,2,4-triazin-5(4H)-on (Metamitron) zur Unkrautbekämpfung in Zuckerrüben und 4-Amino-6-(1,1-dimethylethyl)-3-methylthio-1,2,4-triazin-5(4H)-on (Metribuzin) zur Unkrautbe kämpfung in Sojabohnen, infrage. Auch Mischungen mit

2,4-Dichlorphenoxyessigsäure (2,4-D);
2,4-Dichlorphenoxypropionsäure (2,4-DP);
4-(2,4-Dichlorphenoxy)-buttersäure (2,4-DB);
(2-Methyl-4-chlorphenoxy)-essigsäure (MCPA);
(4-Chlor-2-methylphenoxy)-propionsäure (MCPP);
[(4-Amino-3,5-dichlor-6-fluor-2-pyridinyl)-oxy]-essigsäure bzw. deren 1-Methylheptylester (FLUROXYPYR);
2-[4-(2,4-Dichlorphenoxy)-phenoxy]-propionsäure deren Methyl-oder deren Ethylester (DICLOFOP-[METHYL]);
2-{4-[(6-Chlor-2-benzoxazolyl)-oxy]-phenoxy}-propansäure, deren Methyl- oder deren Ethylester (FENOXAPROP);
Methyl-5-(2,4-dichlorphenoxy)-2-nitrobenzoat (BIFENOX);
Chloressigsäure-N-(methoxymethyl)-2,6-diethylanilid (ALACHLOR);
2-Chlor-N-(2,6-dimethylphenyl)-N-[(1H)-pyrazol-1-yl-methyl]-acetamid (METAZACHLOR);
2-Ethyl-6-methyl-N-(1-methyl-2-methoxyethyl)-chloracetanilid (METOLACHLOR);
N-(1-Ethylpropyl)-3,4-dimethyl-2,6-dinitroanilin (PENDIMETHALIN);
2,6-Dinitro-4-trifluormethyl-N,N-dipropylanilin (TRIFLURALIN);
N,N-Dimethyl-N′-(3-chlor-4-methylphenyl)-harnstoff (CHLORTOLURON);
N,N-Dimethyl-N′-(4-isopropylphenyl)-harnstoff (ISOPROTURON);
2-[4,5-Dihydro-4-methyl-4-isopropyl-5-oxo-(1H)-imidazol-2-yl]-5-ethyl-pyridin-3-carbonsäure (IMAZETHAPYR);
Methyl-2-[4,5-dihydro-4-methyl-4-(1-methylethyl)-5-oxo-1H-imidazol-2-yl]-4(5)-methylbenzoat (IMAZAMETHABENZ);
2-[5-Methyl-5-(1-methylethyl)-4-oxo-2-imidazolin-2-yl]-3-chinolincarbonsäure (IMAZAQUIN);
3,5-Dibrom-4-hydroxy-benzonitril (BROMOXYNIL);
3,5-Diiod-4-hydroxybenzonitril (IOXYNIL);
N-Methyl-2-(1,3-benzthiazol-2-yloxy)-acetanilid (MEFENACET);

Ethyl-2-{[(4-chlor-6-methoxy-2-pyrimidinyl)-aminocarbonyl]-aminosulfonyl}-benzoat (CHLORIMURON);
2-Chlor-N-{[(4-methoxy-6-methyl-1,3,5-triazin-2-yl)-amino]-carbonyl}-benzolsulfonamid (CHLORSULFURON);
2-{[[((4-Methoxy-6-methyl-1,3,5-triazin-2-yl)-amino)-carbonyl]-amino]-sulfonyl}-benzoesäure oder deren Methylester (METSULFURON);
3-[[[[(4-Methoxy-6-methyl-1,3,5-triazin-2-yl)-amino]-carbonyl]-amino]-sulfonyl]-thiophen-2-carbonsäuremethylester (THIAMETHURON);
N,N-Diisopropyl-S-(2,3,3-trichlorallyl)-thiolcarbamat (TRIALLATE);
4-Ethylamino-2-t-butylamino-6-methylthio-s-triazin (TERBUTRYNE);
3-Isopropyl-2,1,3-benzothiadiazin-4-on-2,2-dioxid (BENTAZON);
4-Amino-6-t-butyl-3-ethylthio-1,2,4-triazin-5(4H)-on (ETHIOZIN);
2-[(2-Chlorphenyl)-methyl]-4,4-dimethylisoxazolidin-3-on [DIMETHAZONE);
exo-1-Methyl-4-(1-methylethyl)-2-(2-methylphenyl-methoxy)-7-oxabicyclo-(2,2,1)-heptan (CINMETHYLIN) und
0-(6-Chlor-3-phenyl-pyridazin-4-yl)-S-octyl-thiocarbonat (PYRIDATE) sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann bei der Anwendung als Herbizide in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die erfindungsgemäßen Wirkstoffe können bei der Anwendung als Wachstumregulatoren in den Formulierungen ebenfalls in Mischung mit anderen bekannten Wirkstoffen vorliegen, wie Fungizide, Insektizide, Akarizide und Herbizide sowie in Mischungen mit Düngemitteln und anderen Wachstumsregulatoren.

Die Wirkstoffe können dabei als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw.. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Die Aufwandmengen können bei der Anwendung als Wachstumsregulatoren ebenfalls in einem größeren Bereich variiert werden. Im allgemeinen verwendet man pro Hektar Bodenfläche 0,01 bis 50 kg, bevorzugt 0,05 bis 10 kg an Wirkstoff.

Für die Anwendungszeit gilt, daß die Anwendung der Wachstumsregulatoren in einem bevorzugten Zeitraum vorgenommen wird, dessen genaue Abgrenzung sich nach den klimatischen und vegetativen Gegebenheiten richtet.

Herstellungsbeispiele

Beispiel 1

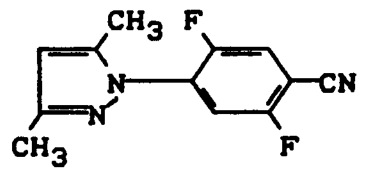

(Verfahren a)

21,1 g (0,125 Mol) 4-Cyano-2,5-difluorphenylhydrazin und 12,5 g (0,125 Mol) 2,4-Pentandion werden in 250 ml Ethanol 2 Stunden bei Raumtemperatur gerührt, anschließend für 15 Stunden auf 70 °C erhitzt und dann im Vakuum eingeengt. Der Rückstand wird mit Petrolether verrührt und abgesaugt.

Man erhält 28 g (96 % der Theorie) an 4-(3,5-Dimethyl-1-pyrazolyl)-2,5-difluorbenzonitril vom Schmelzpunkt 122 °C.

Beispiel 2

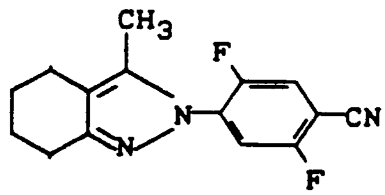

(Verfahren a)

Zu 6,76 g (0,04 Mol) 4-Cyano-2,5-difluorphenylhydrazin in 40 ml Eisessig gibt man 5,2 ml (0,04 Mol) 2-Acetylcyclohexanon (vgl. z.B. J. org. Chem. 34, 1425-1429 [1969]), rührt 2 Stunden bei Raumtemperatur, rührt dann die Reaktionsmischung in 250 ml Eiswasser, extrahiert mit Dichlormethan, trocknet über Natriumsulfat, engt im Vakuum ein und kristallisiert den Rückstand aus Dichlormethan/n-Hexan um.

Man erhält 3,21 g (23,5 % der Theorie) an 4-(5-Methyl-3,4-tetramethylen-1-pyrazolyl)-2,5-difluorbenzonitril vom Schmelzpunkt 120 °C.

Beispiel 3

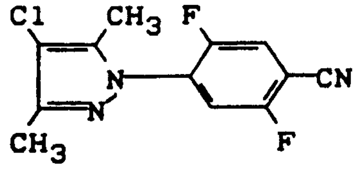

(Verfahren b)

Zu 2,3 g (0,01 Mol) 4-(3,5-Dimethyl-1-pyrazolyl)-2,5-Difluorbenzonitril in 50 ml Dichlormethan gibt man 1,68 g (=1 ml; 0,012 Mol) Sulfurylchlorid, rührt 15 Stunden bei 35 °C, läßt auf Raumtemperatur kommen, trocknet über Natriumsulfat, engt im Vakuum ein und reinigt den Rückstand durch Verrühren mit Petrolether.

Man erhält 2,0 g (75 % der Theorie) an 4-(3,5-Dimethyl-4-chlor-1-pyrazolyl)-2,5-difluorbenzonitril vom Schmelzpunkt 153 °C.

Beispiel 4

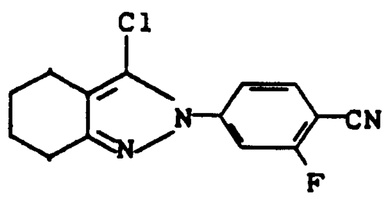

(Verfahren c)

Zu 15 g (0,068 Mol) 1-(4-Cyano-3-fluorphenyl)-3,4-tetramethylen-(1H,4H)-pyrazolin-5-on in 28,8 ml (0,2 Mol) Phosphoroxychlorid gibt man 1,78 g (0,0068 Mol) Triphenylphospin und erhitzt für 15 Stunden auf Rückflußtemperatur. Zur Aufarbeitung wird die abgekühlte Reaktionsmischung in 300 ml Eiswasser gegeben, 1 Stunde gerührt, ausgefallenes Produkt abgesaugt, mit Wasser gewaschen und getrocknet.

Man erhält 15,2 g (81 % der Theorie) an 1-(4-Cyano-3-fluorphenyl)-5-chlor-3,4-tetramethylenpyrazol vom Schmelzpunkt 84-86 °C.

Beispiel 5

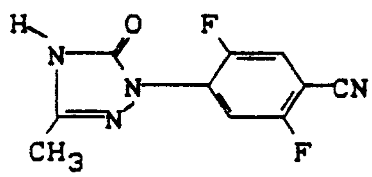

(Verfahren d)

8,45 g (0,05 Mol) 4-Cyano-2,5-difluorphenyl-hydrazin und 12,08 g (0,078 Mol) N-Ethoxycarbonylethanimidsäureethylester (vgl. z.B. Chem. Ber. 119, 2444-2457 [1986]) werden in 50 ml Xylol 8 Stunden auf Rückflußtemperatur erhitzt, danach abgekühlt auf Raumtemperatur, abgesaugt und aus Dichlormethan/Petrolether umkristallisiert.

Man erhält 5,91 g (50 % der Theorie) an 1-(4-Cyano-2,5-difluorphenyl)-3-methyl-4,5-dihydro-1,2,4-triazolin-5-on vom Schmelzpunkt 174 °C.

Beispiel 6

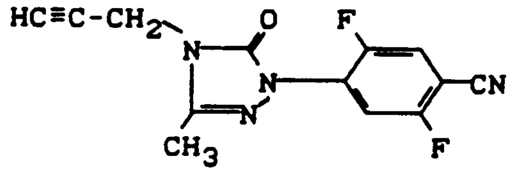

(Verfahren e)

2,36 g (0,01 Mol) 1-(4-Cyano-2,5-difluorphenyl)-2-methyl-4,5-dihydro-1,2,4-triazolin-5-on und 1,52 g (0,011 Mol) Kaliumcarbonat in 20 ml Acetonitril werden 2 Stunden auf Rückflußtemperatur erhitzt, anschließend auf 25 °C abgekühlt, mit einer 80 prozentigen Lösung von 1,31 g (0,011 Mol) Propargylbromid in Toluol versetzt und weitere 4 Stunden auf Rückflußtemperatur erhitzt. Zur Aufarbeitung wird im Vakuum eingeengt, der Rückstand in Dichlormethan aufgenommen, mit gesättigter wässriger Natriumhydrogencarbonatlösung und Wasser gewaschen, über Natriumsulfat getrocknet, im Vakuum eingeengt und umkristallisiert aus Dichlormethan/Petrolether.

Man erhält 2,16 g (79 % der Theorie) an 1-(4-Cyano-2,5-Difluorphenyl-3-methyl-4-propargyl-4,5-dihydro-1,2,4-triazolin-5-on vom Schmelzpunkt 127 °C.

Beispiel 7

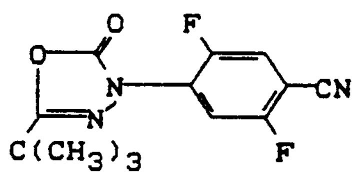

(Verfahren g)

In eine Lösung von 20,24 g (0,08 Mol) 1-(4-Cyano-2,5-difluorphenyl)-2-pivaloylhydrazin in 200 ml Toluol werden 11,9 g (0,12 Mol) Phosgen eingeleitet, danach tropfenweise unter Rühren 24,24 g (0,24 Mol) Triethylamin in 20 ml Toluol zugegeben und eine Stunde auf 100 °C erhitzt. Zur Aufarbeitung entfernt man überschüssiges Phosgen, filtriert ausgefallenes Triethylaminhydrochlorid ab, engt im Vakuum ein und kristallisiert den Rückstand aus Dichlormethan/Petrolether um.

Man erhält 14,33 g (64 % der Theorie) an 3-(4-Cyano-2,5-difluorphenyl)-5-t-butyl-1,3,4-oxadiazolin-2-on vom Schmelzpunkt 137 °C.

Beispiel 8

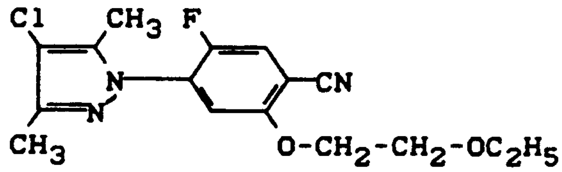

(Verfahren h-α)

Zu 2,7 (0,01 Mol) 4-(3,5-Dimethyl-4-chlor-1-pyrazolyl)-2,5-difluorbenzonitril in 50 ml Ethylenglykolmonomethylether gibt man bei 0 °C 0,8 g (0,025 Mol) 80prozentiges Natriumhydrid (in Paraffinöl), rührt 4 Stunden bei 0 °C und anschließend 15 Stunden bei Raumtemperatur. Zur Aufarbeitung gibt man unter Kühlung langsam 200 ml Wasser zu, rührt eine Stunde bei Raumtemperatur, filtriert ausgefallenen Feststoff ab, wäscht mit Wasser nach und trocknet.

Man erhält 3,2 g (95 % der Theorie) 4-(3,5-Dimethyl-4-chlor-1-pyrazolyl)-2-(2-ethoxyethoxy)-5-fluorbenzonitril vom Schmelzpunkt 86 °C.

Beispiel 9

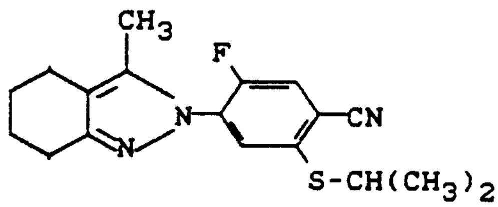

(Verfahren h-α)

Zu 3 g (0,011 Mol) 4-(5-Methyl-3,4-tetramethylen-1-pyrazolyl)-2,5-difluorbenzonitril in 30 ml absolutem Acetonitril gibt man nacheinander 1,02 ml (0,011 Mol) Isopropylmercaptan und 0,84 g (0,015 Mol) gepulvertes Kaliumhydroxid. Anschließend wird die Mischung so lange bei 40 °C gerührt, bis im Dünnschichtchromatogramm kein Ausgangsprodukt mehr nachweisbar ist, danach mit Dichlormethan versetzt und filtriert. Das Filtrat wird im Vakuum eingeengt, der Rückstand an Kieselgel (Laufmittel: Cyclohexan/Essigester 3:1) chromatographiert und aus n-Hexan umkristallisiert.

Man erhält 1,7 g (47 % der Theorie) an 4-(5-Methyl-3,4-tetramethylen-1-pyrazolyl)-5-fluor-2-isopropylthiobenzonitril vom Schmelzpunkt 105 °C.

Beispiel 10

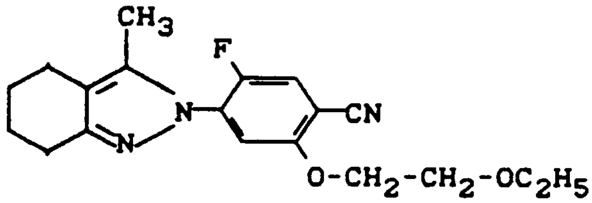

(Verfahren h-α)

Zu 0,51 g (0,017 Mol) Natriumhydrid in 30 ml N-Methylpyrrolidon gibt man bei Raumtemperatur tropfenweise unter Rühren 1,7 ml (0,017 Mol) 2-Ethoxyethanol, rührt weitere 15 Minuten bei Raumtemperatur, gibt dann 4,1 g (0,015 Mol) 4-(5-Methyl-3,4-tetramethylen-1-pyrazolyl)-2,5-difluorbenzonitril zu und rührt anschließend so lange bei 80 °C, bis im Dünnschichtchromatogramm kein Ausgangsprodukt mehr nachweisbar ist. Zur Aufarbeitung wird die abgekühlte Reaktionsmischung in 200 ml Eiswasser eingerührt, mit Toluol extrahiert, über Natriumsulfat getrocknet, im Vakuum eingeengt, an Kieselgel chromatographiert (Laufmittel: Cyclohexan/Essigester 2:1) und aus Diethylether/n-Hexan umkristallisiert.

Man erhält 3,51 g (68 % der Theorie) an 4-(5-Methyl-3,4-tetramethylen-1-pyrazolyl-2-(2-ethoxyethoxy)-5-fluorbenzonitril vom Schmelzpunkt 84 °C.

In entsprechender Weise und gemäß den allgemeinen Angaben zur Herstellung erhält man die folgenden N-Aryl-Stickstoffheterocyclen der allgemeinen Formel (I):

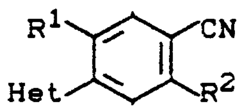

(I)

Tabelle 1

| Bsp. Nr. | $R^1$ | $R^2$ | Het | Schmelzpunkt/° C |
|---|---|---|---|---|
| 11 | F | $-O-CH_2-CH_2-OC_2H_5$ | 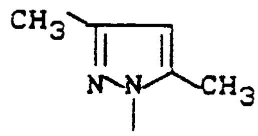 | 103 |
| 12 | H | $-S-CH_2-COOC_2H_5$ | 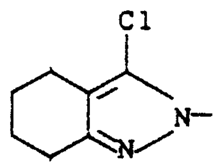 | 112-114 |
| 13 | H | $-O-CH_2-CH_2-OCH_3$ | 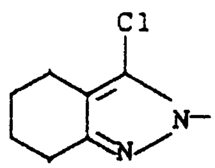 | 69-72 |
| 14 | H | $-O-CH_2-CH_2-OC_2H_5$ | 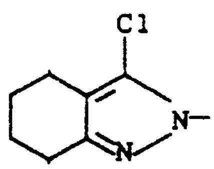 | 62-64 |
| 15 | H | $-O-CH\langle^{CH_2F}_{CH_2F}$ | 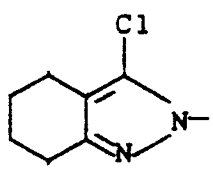 | |

Tabelle 1 - Fortsetzung

| Bsp. Nr. | $R^1$ | $R^2$ | Het | Schmelzpunkt/° C |
|---|---|---|---|---|
| 16 | F | F | 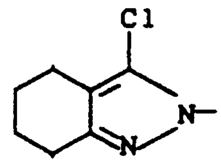 | 112-113 |
| 17 | F | $-S-CH_2-COOC_2H_5$ | 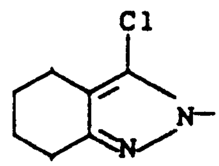 | 106-108 |
| 18 | F | $-O-CH_2-CH_2-OC_2H_5$ | 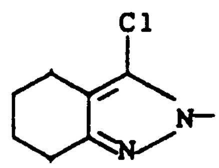 | 107-109 |
| 19 | F | F | 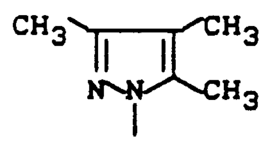 | 146-149 |
| 20 | F | $OCH_3$ | 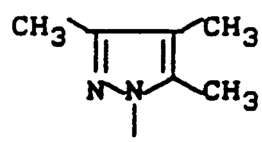 | 179-181 |
| 21 | F | $-S-CH_2-COOC_2H_5$ | 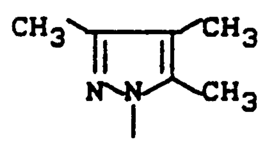 | 136-137 |
| 22 | F | $-O-CH_2-CH_2-OCH_3$ | 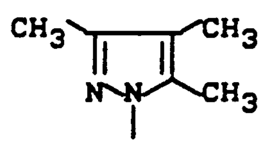 | 127-128 |
| 23 | F | $-O-CH\left\langle\begin{matrix}CH_2F\\CH_2F\end{matrix}\right.$ | 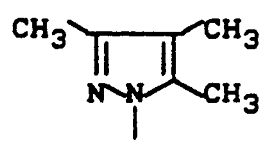 | 94-96 |

Tabelle 1 - Fortsetzung

| Bsp. Nr. | $R^1$ | $R^2$ | Het | Schmelzpunkt/°C |
|---|---|---|---|---|
| 24 | H | $-O-CH_2-CH=CH_2$ | 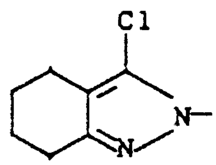 | 123-125 |
| 25 | H | F | 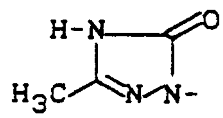 | 246-250 |
| 26 | H | F | 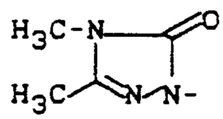 | 181-186 |
| 27 | F | $-O-CH_2-CH_2-O-CH_3$ | 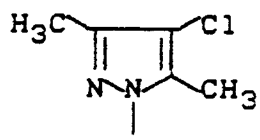 | 94 |
| 28 | F | $-O-CH_2-C{\equiv}CH$ | 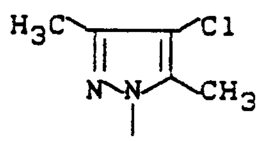 | 136 |
| 29 | F | $-O-CH_2-CH=CH_2$ | 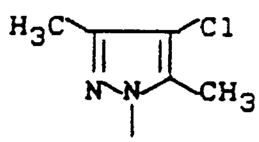 | 118 |
| 30 | F | $-O-CH(CH_3)_2$ | 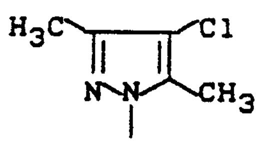 | 88 |

<u>Tabelle 1 - Fortsetzung</u>

| Bsp. Nr. | $R^1$ | $R^2$ | Het | Schmelzpunkt/° C |
|---|---|---|---|---|
| 31 | F | $-S-CH_2-COOC_2H_5$ | 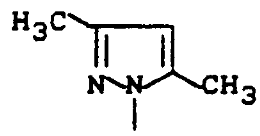 | 110 |
| 32 | F | $-O-CH(CH_2F)_2$ | 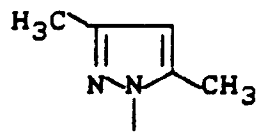 | 110 |
| 33 | F | $-O-CH-COOC_2H_5$<br>$CH_3$ | 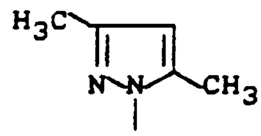 | 86 |
| 34 | F | $-O-CH(CH_2F)_2$ | 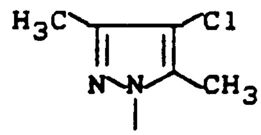 | 116 |
| 35 | F | $-O-CH_2CH_2-O-C_2H_5$ | 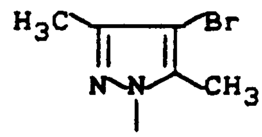 | 97 |
| 36 | F | $-S-CH_2-COOC_2H_5$ | 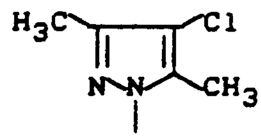 | 94 |
| 37 | F | $-O-CH_2CH_2-O-CH_3$ | 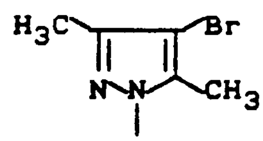 | 104 |

Tabelle 1 - Fortsetzung

| Bsp. Nr. | $R^1$ | $R^2$ | Het | Schmelzpunkt/° C |
|---|---|---|---|---|
| 38 | F | $-O-CH_2-CH=CH_2$ | 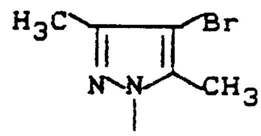 | 126 |
| 39 | F | $-O-CH(CH_2F)_2$ | 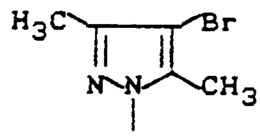 | 122 |
| 40 | F | $-S-CH_2-COOC_2H_5$ | 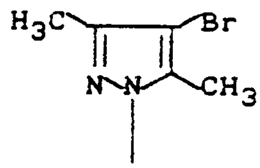 | 107 |
| 41 | F | $-O-CH_2-C≡CH$ | 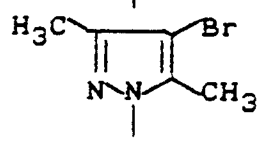 | 104 |
| 42 | F | $-O-CH(CH_3)_2$ | 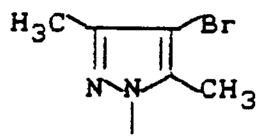 | 108 |
| 43 | F | $-S-CH_2-COOCH_3$ | 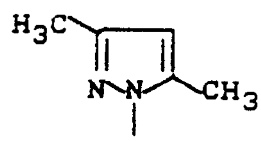 | 103 |
| 44 | F | $-S-CH_2-COOCH_3$ | 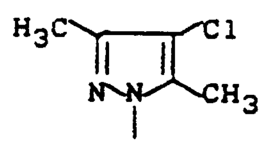 | 108 |

**Tabelle 1 - Fortsetzung**

| Bsp. Nr. | $R^1$ | $R^2$ | Het | Schmelzpunkt/°C |
|---|---|---|---|---|
| 45 | F | $-S-CH_2-COOCH(CH_3)_2$ | 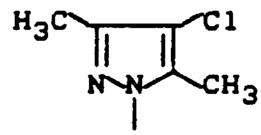 | 135 |
| 46 | F | $-S-CH_2-COO-$ 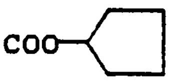 | 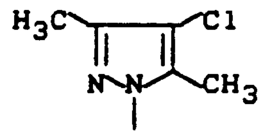 | 120 |
| 47 | F | $-O-CH_2CH_2OCH_3$ | 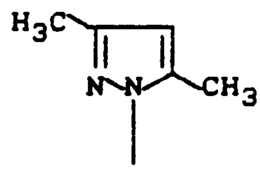 | 112 |
| 48 | F | $-O-CH_2-C{\equiv}CH$ | 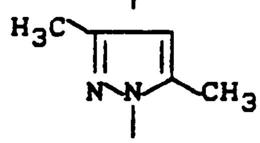 | 141 |
| 49 | F | $-O-CH_2CH{=}CH_2$ | 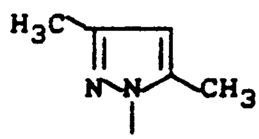 | 120 |
| 50 | F | $-O-C_3H_7$-iso | 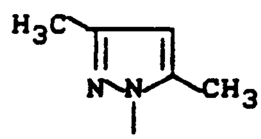 | 99 |

Herstellung der Ausgangsverbindungen

Beispiel II-1

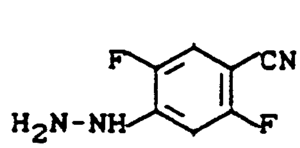

Zu 30 g (0,19 Mol) 2,4,5-Trifluorbenzonitril (vgl. z.B. EP-A 191 181) in 120 ml Ethanol gibt man 11 g (0,22 Mol) Hydrazinhydrat, erhitzt für 2 Stunden auf Rückflußtemperatur, kühlt ab auf Raumtemperatur, engt im Vakuum ein, verrührt den Rückstand mit 50 ml Wasser, saugt ausgefallenes Produkt ab und trocknet.

Man erhält 24 g (75 % der Theorie) an 4-Cyano-2,5-difluorphenylhydrazin vom Schmelzpunkt 158 °C.

Beispiel II-2

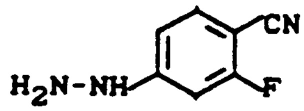

Zu 90 g (0,65 Mol) 2,4-Difluorbenzonitril (vgl. z.B. EP-A 122 693) in 300 ml Methanol gibt man tropfenweise unter Rühren 45 g (0,9 Mol) Hydrazinhydrat, erhitzt für 3 Stunden auf Rückflußtemperatur, engt dann im Vakuum ein, verrührt den Rückstand mit 300 ml Wasser, saugt ab und trocknet.

Man erhält 73 g (74 % der Theorie) an 4-Cyano-3-fluorphenylhydrazin vom Schmelzpunkt 136 °C.

Beispiel II-3

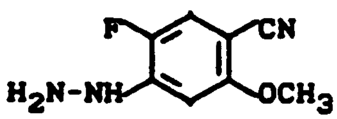

Zu 13 g (0,076 Mol) 4-Cyano-2,5-difluorphenylhydrazin in 100 ml Methanol gibt man 5 g (0,125 Mol) gepulvertes Natriumhydroxid, erhitzt dann für 6 Stunden auf Rückflußtemperatur, engt anschließend im Vakuum ein, gibt den Rückstand in 50 ml Wasser, neutralisiert durch tropfenweise Zugabe von Essigsäure, saugt ausgefallenen Feststoff ab und kristallisiert aus Toluol um.

Man erhält 9 g (65 % der Theorie) an 4-Cyano-2-fluor-5-methoxyphenylhydrazin vom Schmelzpunkt 155-156 °C.

Beispiel IV-1

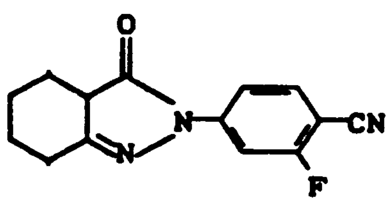

Zu 11 g (0,08 Mol) 4-Cyano-3-fluorphenylhydrazin in 80 ml Ethanol gibt man 11,3 ml (0,096 Mol) 2-Ethoxycarbonylcyclohexanon (vgl. z.B. J. chem. Soc. D 1970, 326-327), erhitzt für 8 Stunden auf Rückflußtemperatur, kühlt dann auf 60 °C, gibt 0,5 ml Schwefelsäure zu, rührt 15 Stunden bei 60 °C, gibt dann 500 ml Wasser zu, saugt ausgefallenes Produkt ab, wäscht mit Wasser nach und trocknet.

Man erhält 17,7 g (99 % der Theorie) an 1-(4-Cyano-3-fluorphenyl)-3,4-tetramethylen-(1H,4H)-pyrazolin-5-on vom Schmelzpunkt 218-220 °C.

Beispiel VIII-1

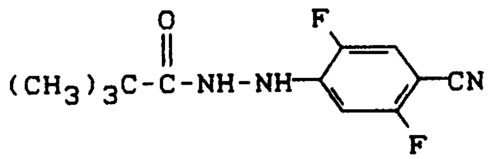

Zu 16,9 g (0,1 Mol) 4-Cyano-2,5-difluorphenylhydrazin und 12,4 g (0,105 Mol) Triethylamin in 50 ml Dichlormethan gibt man bei 0 °C unter Rühren und Eiskühlung tropfenweise 12,05 g (0,1 Mol) Pivaloylchlorid, rührt nach beendeter Zugabe 10 Stunden bei 20 °C, engt dann im Vakuum ein, verteilt den Rückstand zwischen Dichlormethan und Wasser, trocknet die organische Phase über Natriumsulfat und entfernt das Lösungsmittel im Vakuum.

Man erhält 24,85 g (98 % der Theorie) an 1-(4-Cyano-2,5-difluorphenyl)-2-pivaloylhydrazin vom Schmelzpunkt 167 °C.

Beispiel X-1:

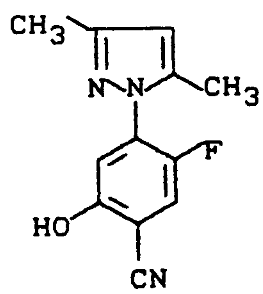

Schmelzpunkt: 116 °C

Beispiel X-2:

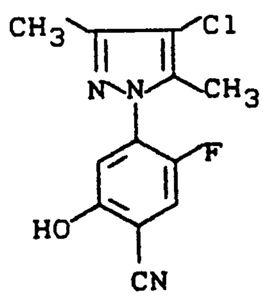

Schmelzpunkt: > 260 °C

Anwendungsbeispiele

In den folgenden Anwendungsbeispielen wurde die nachstehend aufgeführte Verbindung als Vergleichsubstanz eingesetzt:

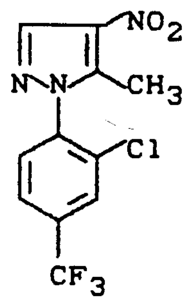

(A)

1-(2-Chlor-4-trifluormethylphenyl)-5-methyl-4-nitropyrazol
(bekannt aus EP-A 200 872/Beispiel 19)

Beispiel A

Pre-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß den Herstellungsbeispielen 10, 14, 16, 17, 18 und 23.

Beispiel B

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit ebenso wie in der Nutzpflanzenselektivität gegenüber dem Stand der Technik zeigen in diesem Test z.B. die Verbindungen gemäß folgender Herstellungsbeispiele: 9, 10, 12, 14, 16, 17, 18, 23 und 44.

Beispiel C

Entlaubung und Austrocknung der Blätter bei Baumwolle

Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
\+ leichtes Austrocknen der Blätter, geringer Blattfall
\+ + starkes Austrocknen der Blätter, starker Blattfall
\+ + + sehr starkes Austrocknen der Blätter, sehr starker Blattfall

Eine deutliche Überlegenheit im Vergleich zur unbehandelten Kontrolle zeigen in diesem Test beispielweise die Verbindungen gemäß folgender Herstellungsbeispiele: 13 und 16.

**Patentansprüche**

**1.** N-Aryl-Stickstoffheterocyclen der allgemeinen Formel (I),

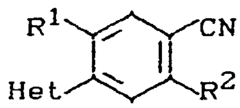

(I)

in welcher

Het für eine Heterocyclus der Formel

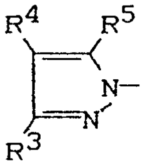

;

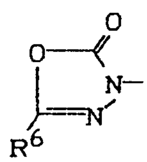

oder

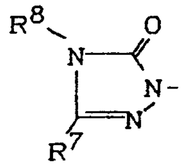

steht,

$R^1$ für Wasserstoff, Fluor, Chlor oder Brom steht und

$R^2$ für Fluor, Chlor oder Brom oder für einen Rest $-X-R^9$ steht, wobei

$R^3$ für Wasserstoff, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht und

$R^4$ für Wasserstoff, Fluor, Chlor, Brom, Iod, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht oder

$R^3$ und $R^4$ gemeinsam für einen zweifachverknüpften Alkandiylrest mit 2 bis 6 Kohlenstoffatomen stehen,

$R^5$ für Wasserstoff, Fluor, Chlor, Brom, Iod, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$R^6$ für Wasserstoff, für jeweils geradkettiges oder verzweigtes Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Halogenalkenyl mit 3 bis 6 Kohlenstoffatomen und 1 bis 5 gleichen oder verschiedenen Halogenatomen, Halogenalkinyl mit 3 bis 6 Kohlenstoffatomen und 1 bis 5 gleichen oder verschiedenen Halogenatomen oder Alkoxyalkyl mit jeweils 1 bis 4 Kohlenstoffatomen in den einzelnen geradkettigen oder verzweigten Alkylteilen steht oder für gegebenenfalls einfach oder mehrfach, gleich oder verschieden substituiertes Cycloalkyl mit 3 bis 7 Kohlenstoffatomen steht, wobei als Substituenten infrage kommen: Halogen, sowie jeweils geradkettiges oder verzweigtes Alkyl oder Alkoxy mit jeweils 1 bis 4 Kohlenstoffatomen,

$R^7$ für Wasserstoff, für jeweils geradkettiges oder verzweigtes Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Halogenalkenyl mit 3 bis 6 Kohlenstoffatomen und 1 bis 5 gleichen oder verschiedenen Halogenatomen, Halogenalkinyl mit 3 bis 6 Kohlenstoffatomen und 1 bis 5 gleichen oder verschiedenen Halogenatomen oder Alkoxyalkyl mit jeweils 1 bis 4 Kohlenstoffatomen in den einzelnen geradkettigen oder verzweigten Alkylteilen steht oder für gegebenenfalls einfach oder mehrfach, gleich oder verschieden substituiertes Cycloalkyl mit 3 bis 7 Kohlenstoffatomen steht, wobei als Substituenten infrage kommen: Halogen, sowie jeweils geradkettiges oder verzweigtes Alkyl oder Alkoxy mit jeweils 1 bis 4 Kohlenstoffatomen und

$R^8$ für Wasserstoff oder für jeweils geradkettiges oder verzeigtes Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Halogenalkenyl mit 3 bis 6 Kohlenstoffatomen und 1 bis 5 gleichen oder verschiedenen Halogenatomen oder Halogenalkinyl mit 3 bis 6 Kohlenstoffatomen und 1 bis 5 gleichen oder verschiedenen Halogenatomen steht oder

$R^7$ und $R^8$ gemeinsam für einen zweifach verknüpften Alkandiylrest mit 2 bis 6 Kohlenstoffatomen stehen

$R^9$ für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, für Cyanalkyl, Alkoxyalkyl, Alkylthioalkyl, Halogenalkoxyalkyl, Alkoxyalkoxyalkyl, (Bis-Alkoxy)alkyl, (Bis-Alkylthio)-alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen, für gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten jeweils infrage kommen: Halogen sowie jeweils geradkettiges oder verzweigtes Alkyl oder Alkoxy mit jeweils 1 bis 4 Kohlenstoffatomen, $R^9$ außerdem für jeweils gegebenenfalls durch Alkyl mit 1 bis 4 Kohlenstoffatomen substituiertes Oxetanylalkyl, Tetrahydrofuranylalkyl, Tetrahydrofuranylalkyloxycarbonylalkyl oder Tetrahydropyranylalkyl mit jeweils 1 bis 3 Kohlenstoffatomen in den jeweiligen Alkylteilen steht oder $R^9$ für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Arylsubstituenten infrage kommen:

Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen und

X für Sauerstoff oder Schwefel steht.

**2.** Verfahren zur Herstellung von N-Aryl-Stickstoffheterocyclen der Formel (I)

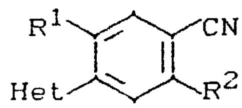

(I)

in welcher

Het für einen Heterocyclus der Formel

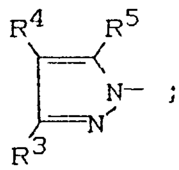 ; 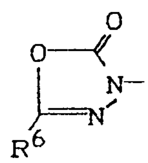 oder 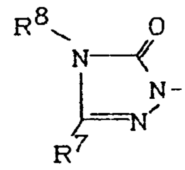

steht,

$R^1$, $R^2$, $R^3$, $R^4$, $R^5$, $R^6$, $R^7$ und $R^8$ die in Anspruch 1 angegebene Bedeutung haben,

dadurch gekennzeichnet, daß

(a) N-Aryl-Stickstoffheterocyclen der Formel (Ia),

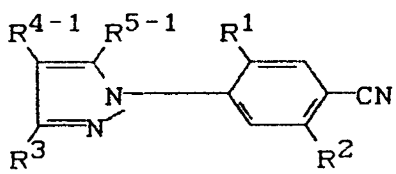

(Ia)

in welcher

$R^{4-1}$ für Wasserstoff, für geradkettiges oder verzweigtes Alkyl mit 1 bis 6 Kohlenstoffatomen und 1 bis 9 gleichen oder verschiedenen Halogenatomen steht,

$R^{5-1}$ für Wasserstoff, für geradkettiges oder verzeigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht und

$R^1$, $R^2$ und $R^3$ die oben angegebene Bedeutung haben, erhält,

wenn man 4-Cyanophenylhydrazine der Formel (II),

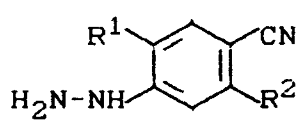

(II)

in welcher

$R^1$ und $R^2$ die oben angegebene Bedeutung haben,

mit 1,3-Diketonen der Formel (III),

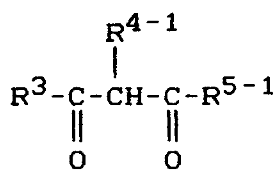

(III)

in welcher

$R^3$, $R^{4-1}$ und $R^{5-1}$ die oben angegebene Bedeutung haben,

oder mit Derivaten dieser Diketone, wie beispielsweise Enolethern, Enolestern, Ketalen, Enolether-Ketalen, Enaminen oder β-Halogenvinylketonen gegebenenfalls in Gegenwart eines Verdünnungsmittels und gegebenenfalls in Gegenwart eines Reaktionshilfsmittels umsetzt; oder daß man

(b) N-Aryl-Stickstoffheterocyclen der Formel (Ib),

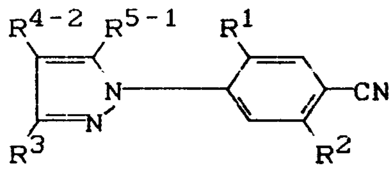

(Ib)

in welcher

$R^{4-2}$ für Halogen steht,

$R^{5-1}$ für Wasserstoff, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

$R^1$, $R^2$ und $R^3$ die oben angegebene Bedeutung haben,

erhält, wenn man N-Aryl-Stickstoffheterocyclen der Formel (Ij),

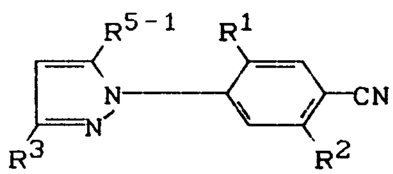

(Ij)

in welcher

$R^1$, $R^2$, $R^3$ und $R^{5-1}$ die oben angegebene Bedeutung haben,

mit einem Halogenierungsmittel gegebenenfalls in Gegenwart eines Verdünnungsmittels umsetzt; oder daß man

(c) N-Aryl-Stickstoffheterocyclen der Formel (Ic),

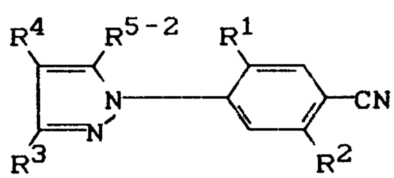

(Ic)

in welcher

$R^{5-2}$ für Halogen steht und

$R^1$, $R^2$, $R^3$ und $R^4$ die oben angegebene Bedeutung haben,

erhält, wenn man N-Aryl-pyrazolinone der Formel (IV),

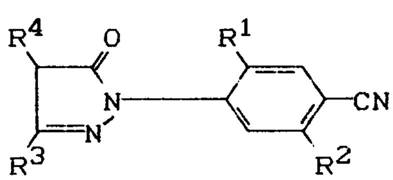

(IV)

in welcher
$R^1$, $R^2$, $R^3$ und $R^4$ die oben angegebene Bedeutung haben,
mit einem Halogenierungsmittel, gegebenenfalls in Gegenwart eines Verdünnungsmittels und gegebenenfalls in Gegenwart eines Reaktionshilfsmittels umsetzt; oder daß man
(d) N-Aryl-Stickstoffheterocyclen der Formel (Id),

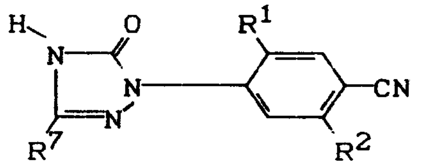
(Id)

in welcher
$R^1$, $R^2$ und $R^7$ die oben angegebene Bedeutung haben,
erhält, wenn man 4-Cyanophenylhydrazine der Formel (II),

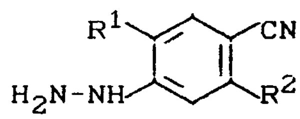
(II)

in welcher
$R^1$ und $R^2$ die oben angegebene Bedeutung haben,
mit Iminocarbonestern der Formel (V),

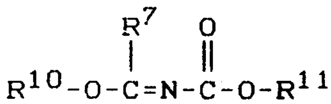
(V)

in welcher
$R^{10}$ und $R^{11}$ unabhängig voneinander jeweils für geradkettiges oder verzweigtes Alkyl mit 1 bis 4 Kohlenstoffatomen stehen und
$R^7$ die oben angegebene Bedeutung hat,
gegebenenfalls in Gegenwart eines Verdünnungsmittels umsetzt; oder daß man
(e) N-Aryl-Stickstoffheterocyclen der Formel (Ie),

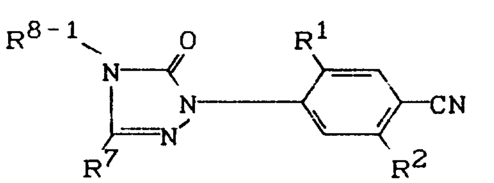
(Ie)

in welcher
$R^{8-1}$ für jeweils geradkettiges oder verzweigtes Alkyl mit 1 bis 6 Kohlenstoffatomen, Alkenyl mit 3 bis 6 Kohlenstoffatomen, Alkinyl mit 3 bis 6 Kohlenstoffatomen, Halogenalkyl mit 1 bis 4 Kohlenstoffatomen und 1 bis 9 gleichen oder verschiedenen Halogenatomen, Halogenalkenyl mit 3 bis 6 Kohlenstoffatomen und 1 bis 5 gleichen oder verschiedenen Halogenatomen oder Halogenalkinyl mit 3 bis 6 Kohlenstoffatomen und 1 bis 5 gleichen oder verschiedenen Halogenatomen steht und
$R^1$, $R^2$ und $R^7$ die oben angegebene Bedeutung haben,

erhält, wenn man N-Aryl-Stickstoffheterocyclen der Formel (Id),

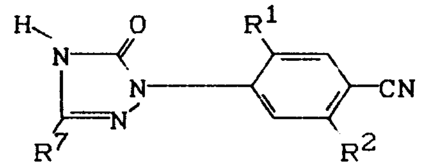

(Id)

in welcher

$R^1$, $R^2$ und $R^7$ die oben angegebene Bedeutung haben,

mit Alkylierungsmitteln der Formel (VI),

$R^{8-1}$-$E^1$ (VI)

in welcher

$R^{8-1}$ die oben angegebene Bedeutung hat und

$E^1$ für Halogen steht,

gegebenenfalls in Gegenwart eines Verdünnungsmittels und gegebenenfalls in Gegenwart eines Reaktionshilfsmittels umsetzt; oder daß man

(f) N-Aryl-Stickstoffheterocyclen der Formel (If),

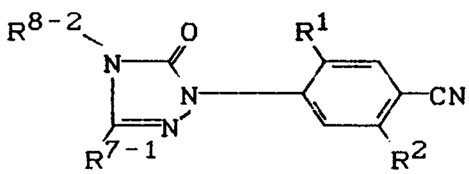

(If)

in welcher

$R^{7-1}$ und $R^{8-2}$ gemeinsam für einen zweifach verknüpften Alkandiylrest mit 2 bis 6 Kohlenstoffatomen stehen,

erhält, wenn man Amidrazone der Formel (VII),

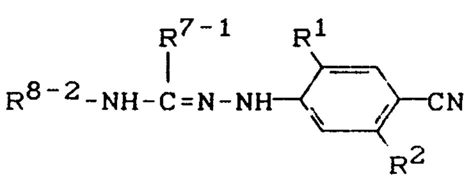

(VII)

in welcher

$R^{7-1}$ und $R^{8-2}$ die oben angegebene Bedeutung haben,

mit Phosgen gegebenenfalls in Gegenwart eines Verdünnungsmittels und gegebenenfalls in Gegenwart eines Reaktionshilfsmittels umsetzt; oder daß man

(g) N-Aryl-Stickstoffheterocyclen der Formel (Ig),

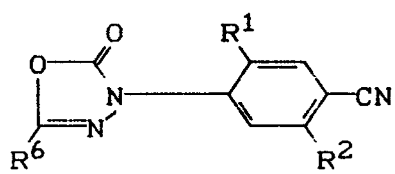

(Ig)

in welcher

$R^1$, $R^2$ und $R^6$ die oben angegebene Bedeutung haben,

erhält, wenn man Phenylhydrazide der Formel (VIII),

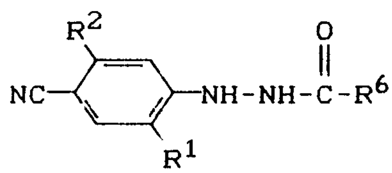

(VIII)

in welcher

$R^1$, $R^2$ und $R^6$ die oben angegebene Bedeutung haben,

mit Phosgen gegebenenfalls in Gegenwart eines Verdünnungsmittels und gegebenfalls in Gegenwart eines Reaktionshilfsmittels umsetzt; oder daß man

(h) N-Aryl-Stickstoffheterocyclen der Formel (Ih),

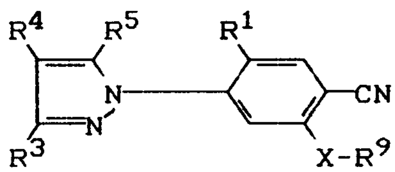

(Ih)

in welcher

$R^1$, $R^3$, $R^4$, $R^5$, $R^9$ und X die oben angegebene Bedeutung haben,

erhält, wenn man

(α) N-Aryl-Stickstoffheterocyclen der Formel (Ik),

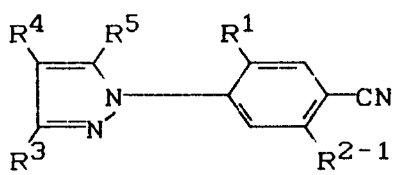

(Ik)

in welcher

$R^{2-1}$ für Halogen steht und

$R^1$, $R^3$, $R^4$ und $R^5$ die oben angegebene Bedeutung haben,

mit Alkoholen oder Thiolen der Formel (IX),

$R^9$-XH (IX)

in welcher

$R^9$ und X die oben angegebene Bedeutung haben,

gegebenenfalls in Gegenwart eines Verdünnungsmittels und gegebenenfalls in Gegenwart eines Reaktionshilfsmittels umsetzt oder daß man

($\beta$) (Thio)Phenolderivate der Formel (X),

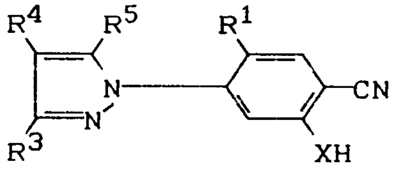

(X)

in welcher

$R^1$, $R^3$, $R^4$, $R^5$ und X die oben angegebene Bedeutung haben,

mit Alkylierungs- bzw. Acylierungsmitteln der Formel (XI),

$R^9$-$E^2$ (XI)

in welcher

$R^9$ die oben angegebene Bedeutung hat und

$E^2$ für eine elektronenanziehende Abgangsgruppe steht,

gegebenenfalls in Gegenwart eines Verdünnungsmittels und gegebenenfalls in Gegenwart eines Reaktionshilfsmittels umsetzt; oder daß man

(i) N-Aryl-Stickstoffheterocyclen der Formel (Ii),

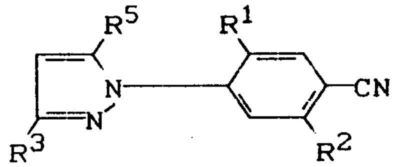

(Ii)

in welcher

$R^1$, $R^2$, $R^3$ und $R^5$ die oben angegebene Bedeutung haben,

erhält, wenn man 1-Arylpyrazolyl-4-carbonsäureester der Formel (XII),

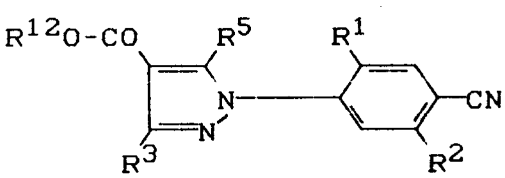

(XII)

in welcher

$R^{12}$ für geradkettiges oder verzweigtes Alkyl mit 1 bis 4 Kohlenstoffatomen steht und

$R^1$, $R^2$, $R^3$ und $R^5$ die oben angegebene Bedeutung haben,

in Gegenwart eines sauren oder basischen Katalysators und gegebenenfalls in Gegenwart eines Verdünnungsmittels verseift und anschließend thermisch decarboxyliert.

**3.** Herbizide und pflanzenwuchsregulierende Mittel, gekennzeichnet durch einen Gehalt an mindestens einem N-Aryl-Stickstoffheterocyclus der Formel (I) gemäß den Ansprüchen 1 und 2.

**4.** Verfahren zur Bekämpfung von Unkräutern, dadurch gekennzeichnet, daß man N-Aryl-Stickstoffheterocyclen der Formel (I) gemäß den Ansprüchen 1 und 2 auf die Unkräuter und/oder ihren Lebensraum einwirken läßt.

**5.** Verwendung von N-Aryl-Stickstoffheterocyclen der Formel (I) gemäß den Ansprüchen 1 und 2 zur Bekämpfung von Unkräutern und/oder als Pflanzenwachstumsregulatoren.

**6.** Verfahren zur Herstellung von herbiziden und/oder pflanzenwuchsregulierenden Mitteln, dadurch gekennzeichnet, daß man N-Aryl-Stickstoffheterocyclen der Formel (I) gemäß den Ansprüchen 1 und 2 mit Streckmitteln und/oder oberflächenaktiven Substanzen vermischt.

**7.** N-Aryl-pyrazolinone der Formel (IV),

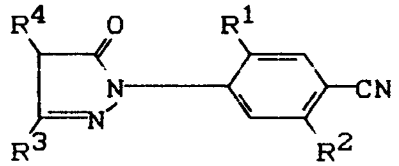

(IV)

in welcher

| | |
|---|---|
| $R^1$ | für Wasserstoff, Fluor, Chlor oder Brom steht und |
| $R^2$ | für Fluor, Chlor oder Brom oder für einen Rest -X-$R^9$ steht, wobei |
| X | für Sauerstoff oder Schwefel steht und |
| $R^9$ | für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, für Cyanalkyl, Alkoxyalkyl, Alkylthioalkyl, Halogenalkoxyalkyl, Alkoxyalkoxyalkyl, (Bis-Alkoxy)alkyl, (Bis-Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen, für gegebenenfalls einfach oder mehrfach, gleich oder verschieden substituiertes Cycloalkyl, Cycloalkyloxycarbonylalkyl oder Cycloalkylalkyl- mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten jeweils infrage kommen: Halogen sowie jeweils geradkettiges oder verzweigtes Alkyl oder Alkoxy mit jeweils 1 bis 4 Kohlenstoffatomen, $R^9$ außerdem für jeweils gegebenenfalls durch Alkyl mit 1 bis 4 Kohlenstoffatomen substituiertes Oxetanylalkyl, Tetrahydrofuranylalkyl, Tetrahydrofuranylalkyloxycarbonylalkyl oder Tetrahydropyranylalkyl mit jeweils 1 bis 3 Kohlenstoffatomen in den jeweiligen Alkylteilen steht oder $R^9$ für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Arylsubstituenten infrage kommen:<br>Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen, |
| $R^3$ | für Wasserstoff, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht und |
| $R^4$ | für Wasserstoff, Fluor, Chlor, Brom, Iod, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht oder |
| $R^3$ und $R^4$ | gemeinsam für einen zweifachverknüpften Alkandiylrest mit 2 bis 6 Kohlenstoffatomen stehen. |

**8.** (Thio)Phenolderivate der Formel (X),

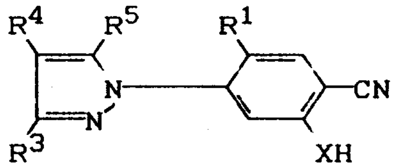

(X)

in welcher

$R^3$ für Wasserstoff, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht und

$R^4$ für Wasserstoff, Fluor, Chlor, Brom, Iod, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht oder

$R^3$ und $R^4$ gemeinsam für einen zweifachverknüpften Alkandiylrest mit 2 bis 6 Kohlenstoffatomen stehen,

$R^5$ für Wasserstoff, Fluor, Chlor, Brom, Iod, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht.

**9.** Verfahren zur Herstellung von N-Aryl-pyrazolinonen der Formel (IV)

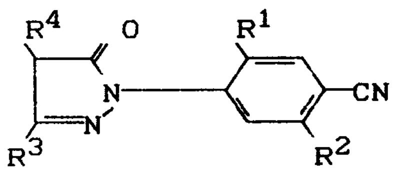

(IV)

in welcher

$R^1$ für Wasserstoff, Fluor, Chlor oder Brom steht und

$R^2$ für Fluor, Chlor oder Brom oder für einen Rest -X-$R^9$ steht,

X für Sauerstoff oder Schwefel steht,

$R^9$ für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, für Cyanalkyl, Alkoxyalkyl, Alkylthioalkyl, Halogenalkoxyalkyl, Alkoxyalkoxyalkyl, (Bis-Alkoxy)alkyl, (Bis-Alkylthio)-alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen, für gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten jeweils infrage kommen: Halogen sowie jeweils geradkettiges oder verzweigtes Alkyl oder Alkoxy mit jeweils 1 bis 4 Kohlenstoffatomen, $R^9$ außerdem für jeweils gegebenenfalls durch Alkyl mit 1 bis 4 Kohlenstoffatomen substituiertes Oxetanylalkyl, Tetrahydrofuranylalkyl, Tetrahydrofuranylalkyloxycarbonylalkyl oder Tetrahydropyranylalkyl mit jeweils 1 bis 3 Kohlenstoffatomen in den jeweiligen Alkylteilen steht oder $R^9$ für gegebenenfalls einfach oder mehrfach gleich

oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Arylsubstituenten infrage kommen:
Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen.

$R^3$ für Wasserstoff, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht und

$R^4$ für Wasserstoff, Fluor, Chlor, Brom, Iod, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht oder

$R^3$ und $R^4$ gemeinsam für einen zweifachverknüpften Alkandiylrest mit 2 bis 6 Kohlenstoffatomen stehen,

dadurch gekennzeichnet, daß man p-Ketoester der Formel (XV)

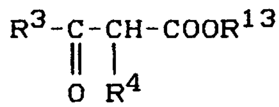

(XV)

in welcher

$R^{13}$ für Methyl oder Ethyl steht und

$R^3$ und $R^4$ die oben angegebene Bedeutung haben,

mit 4-Cyanophenylhydrazinen der Formel (II),

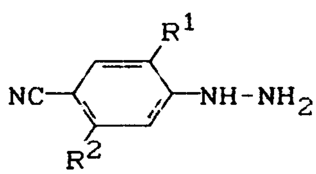

(II)

in welcher

$R^1$ und $R^2$ die oben angegebene Bedeutung haben,

gegebenenfalls in Gegenwart eines Verdünnungsmittels und gegebenenfalls in Gegenwart eines Reaktionshilfsmittels umsetzt.

**10.** Verfahren zur Herstellung von (Thio)Phenolderivaten der Formel (X)

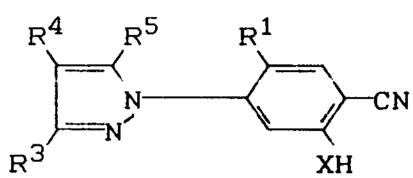

(X)

in welcher

$R^1$ für Wasserstoff, Fluor, Chlor oder Brom steht,

$R^3$ für Wasserstoff, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht und

$R^4$ für Wasserstoff, Fluor, Chlor, Brom, Iod, für geradkettiges oder verzweigtes Alkyl mit 1 bis 6 Kohlenstoffatomen oder für geradkettiges oder verzweigtes Halogenalkyl mit 1

bis 4 Kohlenstoffatomen und 1 bis 9 gleichen oder verschiedenen Halogenatomen steht oder

$R^3$ und $R^4$ gemeinsam für einen zweifachverknüpften Alkandiylrest mit 2 bis 6 Kohlenstoffatomen stehen,

$R^5$ für Wasserstoff, Fluor, Chlor, Brom, Iod, für geradkettiges oder verzweigtes Alkyl mit 1 bis 6 Kohlenstoffatomen oder für geradkettiges oder verzweigtes Halogenalkyl mit 1 bis 4 Kohlenstoffatomen und 1 bis 9 gleichen oder verschiedenen Halogenatomen steht,

dadurch gekennzeichnet, daß man N-Aryl-Stickstoffheterocyclen der Formel (Ik)

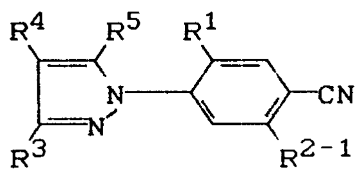

(Ik)

in welcher

$R^{2-1}$ für Halogen steht und

$R^1$, $R^3$, $R^4$ und $R^5$ die oben angegebene Bedeutung haben,

mit Natriumhydrogensulfid, gegebenenfalls in Gegenwart eines Verdünnungsmittels und gegebenenfalls in Gegenwart eines Reaktionshilfsmittels, gegebenenfalls in Gegenwart einer Stickstoff- oder Argonschutzgasatomosphäre umsetzt oder daß man N-Aryl-Stickstoffheterocyclen der Formel (Il),

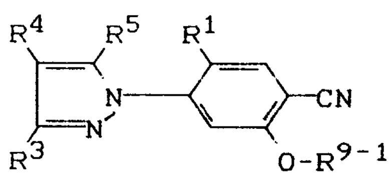

(Il)

in welcher

$R^1$, $R^3$, $R^4$ und $R^5$ die oben angegebene Bedeutung haben und

$R^{9-1}$ für Allyl oder für Benzyl steht,

mit Reduktionsmitteln in Gegenwart eines Hydrierkatalysators, gegebenenfalls in Gegenwart eines Verdünnungsmittels und gegebenenfalls in Gegenwart eines Raktionshilfsmittels umsetzt.

**11.** 4-Cyanophenylhydrazine der Formel (II)

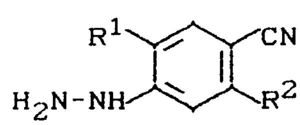

(II)

in welcher

$R^1$ für Wasserstoff, Fluor, Chlor oder Brom steht und

$R^2$ für Fluor, Chlor oder Brom oder für einen Rest $-X-R^9$ steht, wobei

$R^9$ für jeweils geradkettiges oder verzweigtes Alkyl mit 1 bis 8 Kohlenstoffatomen, Alkenyl mit 2 bis 8 Kohlenstoffatomen, Alkinyl mit 3 bis 8 Kohlenstoffatomen, Halogenalkyl mit 1 bis 8 Kohlenstoffatomen und 1 bis 17 gleichen oder verschiedenen Halogenatomen, Halogenalkenyl mit 2 bis 8 Kohlenstoffatomen und 1 bis 15 gleichen oder verschiedenen Halogenatomen, Halogenalkinyl mit 3 bis 8 Kohlenstoffatomen und 1 bis 13 gleichen oder verschiedenen Halogenatomen, für Cyanalkyl, Alkoxyalkyl, Alkylthioalkyl, Halogenalkoxyalkyl, Alkoxyalkoxyalkyl, (Bis-Alkoxy)alkyl, (Bis-Alkylthio)alkyl, Alkylcarbonylalkyl, Alkoxycarbonylalkyl oder Alkoxyalkoxycarbonylalkyl mit jeweils 1 bis 8 Kohlenstoffatomen in den einzelnen Alkylteilen und gegebenenfalls 1 bis 9 gleichen oder verschiedenen Halogenatomen für gegebenenfalls einfach oder mehrfach, gleich oder verschieden substituiertes Cycloalkyl, Cycloalkyloxycarbonylalkyl oder Cycloalkylalkyl mit jeweils 3 bis 7 Kohlenstoffatomen im Cycloalkylteil und gegebenenfalls 1 bis 4 Kohlenstoffatomen im geradkettigen oder verzweigten Alkylteil steht, wobei als Substituenten jeweils infrage kommen: Halogen sowie jeweils geradkettiges oder verzweigtes Alkyl oder Alkoxy mit jeweils 1 bis 4 Kohlenstoffatomen, $R^9$ außerdem für jeweils gegebenenfalls durch Alkyl mit 1 bis 4 Kohlenstoffatomen substituiertes Oxetanylalkyl, Tetrahydrofuranylalkyl, Tetrahydrofuranylalkyloxycarbonylalkyl oder Tetrahydropyranylalkyl mit jeweils 1 bis 3 Kohlenstoffatomen in den jeweiligen Alkylteilen steht oder $R^9$ für gegebenenfalls einfach oder mehrfach gleich oder verschieden substituiertes Aralkyl mit 6 bis 10 Kohlenstoffatomen im Arylteil und 1 bis 4 Kohlenstoffatomen im geradkettigen oder verzweigten Alkylteil steht, wobei als Arylsubstituenten infrage kommen:
Halogen, Cyano, Nitro, jeweils geradkettiges oder verzweigtes Alkyl, Alkoxy, Alkylthio oder Alkoxycarbonyl mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen und

X für Sauerstoff oder Schwefel steht.

**12.** Verfahren zur Herstellung von 4-Cyanophenylhydrazinen der Formel (II)

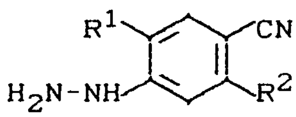

(II)

in welcher

$R^1$ und $R^2$ die in Anspruch 11 genannten Bedeutungen haben,

dadurch gekennzeichnet, daß man 4-Fluorbenzonitrile der Formel (XIII),

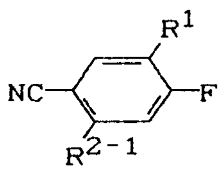

(XIII)

in welcher

$R^{2-1}$ für Fluor, Chlor oder Brom steht und

$R^1$ die in Anspruch 11 angegebene Bedeutung hat,

mit Hydrazinhydrat gegebenenfalls in Gegenwart eines Verdünnungsmittels umsetzt und die so erhältlichen 4-Cyano-phenylhydrazine der Formel (IIa),

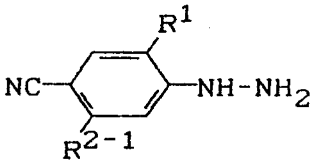

(IIa)

in welcher

$R^1$ und $R^{2-1}$ die oben angege bene Bedeutung haben,

gegebenenfalls in einer nachfolgenden 2. Stufe mit Alkoholen oder Thiolen der Formel (IX),

$R^9$-XH (IX)

in welcher
$R^9$ die in Anspruch 11 genannte Bedeutung hat und
X für Sauerstoff oder Schwefel steht,
gegebenenfalls in Gegenwart eines Verdünnungsmittels und gegebenenfalls in Gegenwart eines Reaktionshilfsmittels umsetzt oder daß man 4-Cyanophenylhydrazine der Formel (IIb),

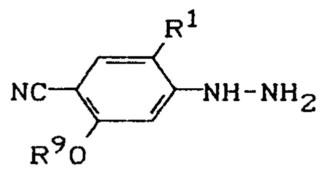
(IIb)

in welcher
$R^1$ und $R^9$ die oben angegebenen Bedeutungen haben
mit 4-Aminobenzonitrile der Formel (XIV),

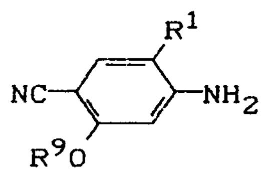
(XIV)

in welcher
$R^1$ und $R^9$ die oben angegebenen Bedeutungen haben,
zunächst mit Natriumnitrit in Gegenwart einer Säure diazotiert und anschließend mit einem Reduktionsmittel reduziert.

## Claims

**1.** N-Aryl-substituted nitrogen-containing heterocycles of the general formula (I)

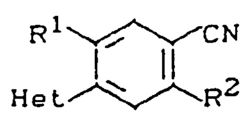
(I)

in which
Het represents a heterocycle of the formula

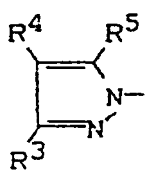 ; 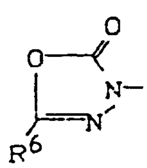 or 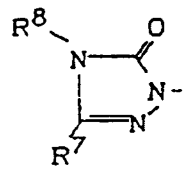 ,

$R^1$ represents hydrogen, fluorine, chlorine or bromine and
$R^2$ represents fluorine, chlorine or bromine, or represents a radical -X-$R^9$,
where
$R^3$ represents hydrogen, represents straight-chain or branched alkyl having 1 to 6 carbon

atoms or represents straight-chain or branched halogenoalkyl having 1 to 4 carbon atoms and 1 to 9 identical or different halogen atoms and

$R^4$ represents hydrogen, fluorine, chlorine, bromine, iodine, represents straight-chain or branched alkyl having 1 to 6 carbon atoms or represents straight-chain or branched halogenoalkyl having 1 to 4 carbon atoms and 1 to 9 identical or different halogen atoms, or

$R^3$ and $R^4$ together represent a double-linked alkanediyl radical having 2 to 6 carbon atoms,

$R^5$ represents hydrogen, fluorine, chlorine, bromine, iodine, represents straight-chain or branched alkyl having 1 to 6 carbon atoms or represents straight-chain or branched halogenoalkyl having 1 to 4 carbon atoms and 1 to 9 identical or different halogen atoms,

$R^6$ represents hydrogen, represents in each case straight-chain or branched alkyl having 1 to 6 carbon atoms, alkenyl having 3 to 6 carbon atoms, alkinyl having 3 to 6 carbon atoms, halogenoalkyl having 1 to 4 carbon atoms and 1 to 9 identical or different halogen atoms, halogenoalkenyl having 3 to 6 carbon atoms and 1 to 5 identical or different halogen atoms, halogenoalkinyl having 3 to 6 carbon atoms and 1 to 5 identical or different halogen atoms or alkoxyalkyl having in each case 1 to 4 carbon atoms in the individual straight-chain or branched alkyl moieties or represents cycloalkyl which has 3 to 7 carbon atoms and which is optionally monosubstituted or polysubstituted by identical or different substituents, suitable substituents being: halogen, and in each case straight-chain or branched alkyl or alkoxy, each having 1 to 4 carbon atoms,

$R^7$ represents hydrogen, represents in each case straight-chain or branched alkyl having 1 to 6 carbon atoms, alkenyl having 3 to 6 carbon atoms, alkinyl having 3 to 6 carbon atoms, halogenoalkyl having 1 to 4 carbon atoms and 1 to 9 identical or different halogen atoms, halogenoalkenyl having 3 to 6 carbon atoms and 1 to 5 identical or different halogen atoms, halogenoalkinyl having 3 to 6 carbon atoms and 1 to 5 identical or different halogen atoms or alkoxyalkyl having in each case 1 to 4 carbon atoms in the individual straight-chain or branched alkyl moieties or represents cycloalkyl which has 3 to 7 carbon atoms and which is optionally monosubstituted or polysubstituted by identical or different substituents, suitable substituents being: halogen, and in each case straight-chain or branched alkyl or alkoxy, having in each case 1 to 4 carbon atoms, and

$R^8$ represents hydrogen or represents in each case straight-chain or branched alkyl having 1 to 6 carbon atoms, alkenyl having 3 to 6 carbon atoms, alkinyl having 3 to 6 carbon atoms, halogenoalkyl having 1 to 4 carbon atoms and 1 to 9 identical or different halogen atoms, halogenoalkenyl having 3 to 6 carbon atoms and 1 to 5 identical or different halogen atoms or halogenoalkinyl having 3 to 6 carbon atoms and 1 to 5 identical or different halogen atoms, or

$R^7$ and $R^8$ together represent a double-linked alkanediyl radical having 2 to 6 carbon atoms,

$R^9$ represents in each case straight-chain or branched alkyl having 1 to 8 carbon atoms, alkenyl having 2 to 8 carbon atoms, alkinyl having 3 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 3 to 8 carbon atoms and 1 to 13 identical or different halogen atoms, represents cyanoalkyl, alkoxyalkyl, alkylthioalkyl, halogenoalkoxyalkyl, alkoxyalkoxyalkyl, (bis-alkoxy)alkyl, (bis-alkylthio)alkyl, alkylcarbonylalkyl, alkoxycarbonylalkyl or alkoxyalkoxycarbonylalkyl, each having 1 to 8 carbon atoms in the individual alkyl moieties and if appropriate 1 to 9 identical or different halogen atoms, represents cycloalkyl, cycloalkyloxycarbonylalkyl or cycloalkylalkyl, having in each case 3 to 7 carbon atoms in the cycloalkyl moiety and if appropriate 1 to 4 carbon atoms in the straight-chain or branched alkyl moiety and each of which is optionally monosubstituted or polysubstituted by identical or different substituents, suitable substituents in each case being: halogen and in each case straight-chain or branched alkyl or alkoxy, each having 1 to 4 carbon atoms, $R^9$ furthermore represents oxetanylalkyl, tetrahydrofuranylalkyl, tetrahydrofuranylalkyloxycarbonylalkyl or tetrahydropyranylalkyl, each of which has 1 to 3 carbon atoms in the respective alkyl moieties and each of which is optionally substituted by alkyl having 1 to 4 carbon

atoms or $R^9$ represents aralkyl which has 6 to 10 carbon atoms in the aryl moiety and 1 to 4 carbon atoms in the straight-chain or branched alkyl moiety and which is optionally monosubstituted or polysubstituted by identical or different substituents, suitable aryl substituents being:
halogen, cyano, nitro, in each case straight-chain or branched alkyl, alkoxy, alkylthio or alkoxycarbonyl, each having 1 to 4 carbon atoms, or in each case straight-chain or branched halogenoalkyl, halogenoalkoxy or halogenoalkylthio, each having 1 to 4 carbon atoms and 1 to 9 identical or different halogen atoms, and

X represents oxygen or sulphur.

**2.** Process for the preparation of N-aryl-substituted nitrogen-containing heterocycles of the formula (I)

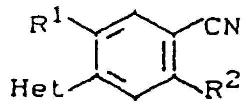

(I)

in which

Het represents a heterocycle of the formula

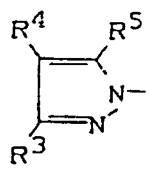 ; 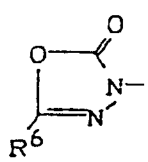 or 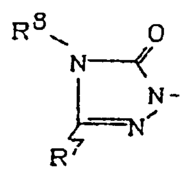 ,

$R^1$, $R^2$, $R^3$, $R^4$, $R^5$, $R^6$, $R^7$ and $R^8$ have the meaning given in Claim 1,

characterized in that

(a) N-aryl-substituted nitrogen-containing heterocycles of the formula (Ia)

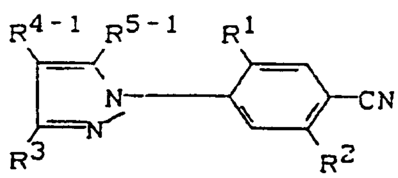

(Ia)

in which

$R^{4-1}$ represents hydrogen or straight-chain or branched alkyl having 1 to 6 carbon atoms and 1 to 9 identical or different halogen atoms,

$R^{5-1}$ represents hydrogen, straight-chain or branched alkyl having 1 to 6 carbon atoms or straight-chain or branched halogenoalkyl having 1 to 4 carbon atoms and 1 to 9 identical or different halogen atoms and

$R^1$, $R^2$ and $R^3$ have the abovementioned meaning,

are obtained when 4-cyanophenylhydrazines of the formula (II)

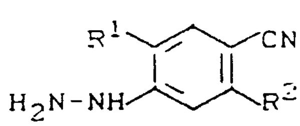

(II)

in which

$R^1$ and $R^2$ have the abovementioned meaning,

are reacted with 1,3-diketones of the formula (III)

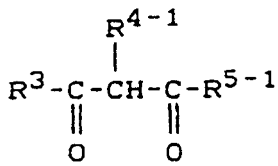

(III)

in which

$R^3$, $R^{4-1}$ and $R^{5-1}$ have the abovementioned meaning,

or with derivatives of these diketones, such as, for example, enol ethers, enol esters, ketals, enol ether ketals, enamines or β-halogenovinyl ketones, if appropriate in the presence of a diluent and if appropriate in the presence of a reaction auxiliary; or in that

(b) N-aryl substituted nitrogen-containing heterocycles of the formula (Ib)

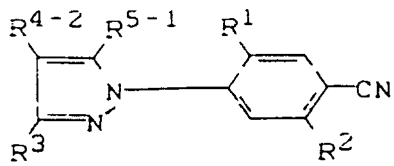

(Ib)

in which

$R^{4-2}$ represents halogen,

$R^{5-1}$ represents hydrogen, straight-chain or branched alkyl having 1 to 6 carbon atoms or straight-chain or branched halogenoalkyl having 1 to 4 carbon atoms and 1 to 9 identical or different halogen atoms and

$R^1$, $R^2$ and $R^3$ have the abovementioned meaning,

are obtained when N-aryl-substituted nitrogen-containing heterocycles of the formula (Ij)

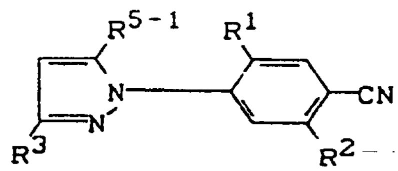

(Ij)

in which

$R^1$, $R^2$, $R^3$ and $R^{5-1}$ have the abovementioned meaning,

are reacted with a halogenating agent, if appropriate in the presence of a diluent; or in that

(c) N-aryl-substituted nitrogen-containing heterocycles of the formula (Ic)

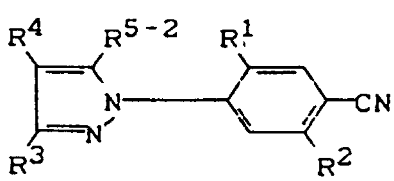

(Ic)

in which

$R^{5-2}$ represents halogen and

$R^1$, $R^2$, $R^3$ and $R^4$ have the abovementioned meaning,

are obtained when N-aryl-pyrazolinones of the formula (IV)

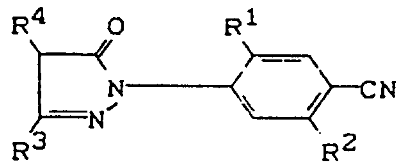 (IV)

in which

$R^1$, $R^2$, $R^3$ and $R^4$ have the abovementioned meaning,

are reacted with a halogenating agent, if appropriate in the presence of a diluent and if appropriate in the presence of a reaction auxiliary; or in that

(d) N-aryl-substituted nitrogen-containing heterocycles of the formula (Id)

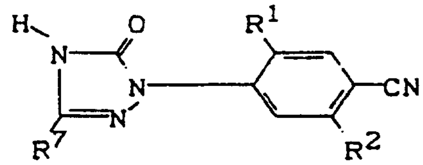 (Id)

in which

$R^1$, $R^2$ and $R^7$ have the abovementioned meaning,

are obtained when 4-cyanophenylhydrazines of the formula (II)

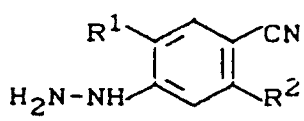 (II)

in which

$R^1$ and $R^2$ have the abovementioned meaning,

are reacted with iminocarboxylic acid esters of the formula (V)

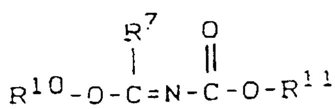 (V)

in which

$R^{10}$ and $R^{11}$ independently of one another each represent straight-chain or branched alkyl having 1 to 4 carbon atoms and

$R^7$ has the abovementioned meaning,

if appropriate in the presence of a diluent; or in that

(e) N-aryl-substituted nitrogen-containing heterocycles of the formula (Ie)

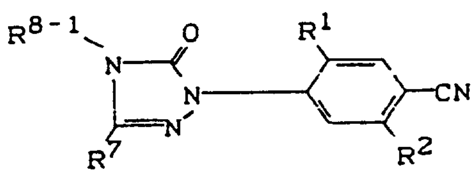 (Ie)

in which

$R^{8-1}$ represents in each case straight-chain or branched alkyl having 1 to 6 carbon atoms, alkenyl having 3 to 6 carbon atoms, alkinyl having 3 to 6 carbon atoms, halogenoalkyl having 1 to 4 carbon atoms and 1 to 9 identical or different

halogen atoms, halogenoalkenyl having 3 to 6 carbon atoms and 1 to 5 identical or different halogen atoms or halogenoalkinyl having 3 to 6 carbon atoms and 1 to 5 identical or different halogen atoms and

$R^1$, $R^2$ and $R^7$ have the abovementioned meaning,

are obtained when N-aryl-substituted nitrogen-containing heterocycles of the formula (Id)

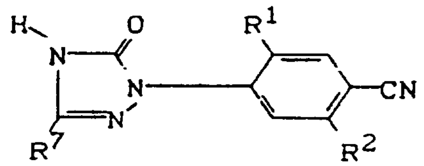

(Id)

in which

$R^1$, $R^2$ and $R^7$ have the abovementioned meaning,

are reacted with alkylating agents of the formula (VI)

$R^{8-1}$-$E^1$ (VI)

in which

$R^{8-1}$ has the abovementioned meaning and

$E^1$ represents halogen,

if appropriate in the presence of a diluent and if appropriate in the presence of a reaction auxiliary; or in that

(f) N-aryl-substituted nitrogen-containing heterocycles of the formula (If)

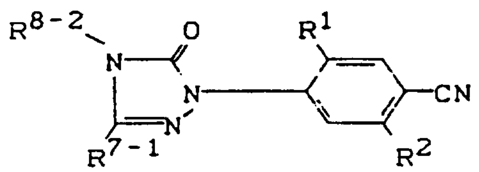

(If)

in which

$R^{7-1}$ and $R^{8-2}$ together represent a double-linked alkanediyl radical having 2 to 6 carbon atoms,

are obtained when amidrazones of the formula (VII)

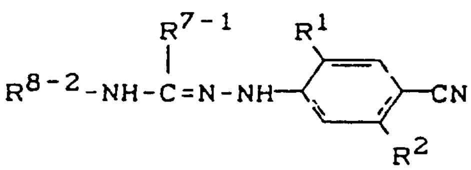

(VII)

in which

$R^{7-1}$ and $R^{8-2}$ have the abovementioned meaning,

are reacted with phosgene, if appropriate in the presence of a diluent and if appropriate in the presence of a reaction auxiliary; or in that

(g) N-aryl-substituted nitrogen-containing heterocycles of the formula (Ig)

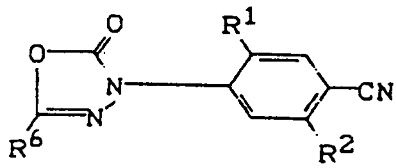

(Ig)

in which
$R^1$, $R^2$ and $R^6$ have the abovementioned meaning,
are obtained when phenylhydrazides of the formula (VIII)

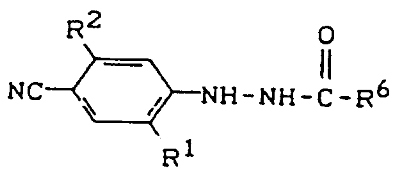

(VIII)

in which
$R^1$, $R^2$ and $R^6$ have the abovementioned meaning,
are reacted with phosgene, if appropriate in the presence of a diluent and if appropriate in the presence of a reaction auxiliary; or in that
(h) N-aryl-substituted nitrogen-containing heterocycles of the formula (Ih)

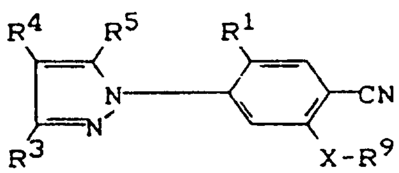

(Ih)

in which
$R^1$, $R^3$, $R^4$, $R^5$, $R^9$ and X have the abovementioned meaning,
are obtained when
($\alpha$) N-aryl-substituted nitrogen-containing heterocycles of the formula (Ik)

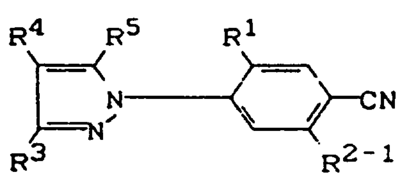

(Ik)

in which
$R^{2-1}$ represents halogen and
$R^1$, $R^3$, $R^4$ and $R^5$ have the abovementioned meaning,
are reacted with alcohols or thiols of the formula (IX)

$R^9$-XH (IX)

in which
$R^9$ and X have the abovementioned meaning,
if appropriate in the presence of a diluent and if appropriate in the presence of a reaction

auxiliary, or in that
($\beta$) (thio)phenol derivatives of the formula (X)

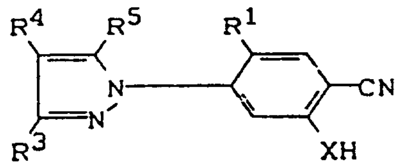
(X)

in which
$R^1$, $R^3$, $R^4$, $R^5$ and X have the abovementioned meaning,
are reacted with alkylating or acylating agents of the formula (XI)

$R^9$-$E^2$ (XI)

in which
$R^9$ has the abovementioned meaning and
$E^2$ represents an electron-withdrawing leaving group,
if appropriate in the presence of a diluent and if appropriate in the presence of a reaction auxiliary; or in that
(i) N-aryl-substituted nitrogen-containing heterocycles of the formula (Ii)

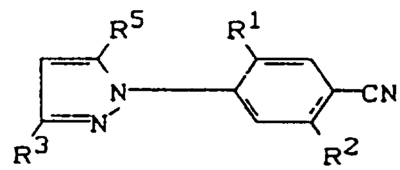
(Ii)

in which
$R^1$, $R^2$, $R^3$ and $R^5$ have the abovementioned meaning,
are obtained when 1-arylpyrazolyl-4-carboxylic acid esters of the formula (XII)

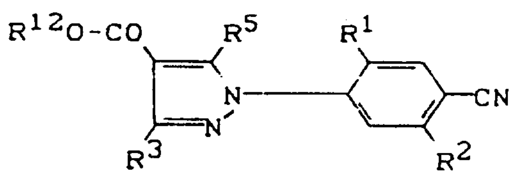
(XII)

in which
$R^{12}$ represents straight-chain or branched alkyl having 1 to 4 carbon atoms and
$R^1$, $R^2$, $R^3$ and $R^5$ have the abovementioned meaning,
are hydrolyzed in the presence of an acid or basic catalyst and, if appropriate, in the presence of a diluent, and the product is subsequently thermically decarboxylated.

**3.** Herbicidal and plant growth-regulating agents, characterized in that they contain at least one N-aryl-substituted nitrogen-containing heterocycle of the formula (I) according to Claims 1 and 2.

**4.** Method of combating weeds, characterized in that N-aryl-substituted nitrogen-containing heterocycles of the formula (I) according to Claims 1 and 2 are allowed to act on the weeds and/or their environment.

**5.** Use of N-aryl-substituted nitrogen-containing heterocycles of the formula (I) according to Claims 1 and 2 for combating weeds and/or as plant growth regulators.

6. Process for the preparation of herbicidal and/or plant growth-regulating agents, characterized in that N-aryl-substituted nitrogen-containing heterocycles of the formula (I) according to Claims 1 and 2 are mixed with extenders and/or surface-active substances.

7. N-Aryl-pyrazolinones of the formula (IV)

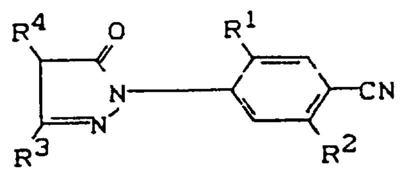

(IV)

in which

$R^1$ represents hydrogen, fluorine, chlorine or bromine and

$R^2$ represents fluorine, chlorine or bromine, or represents a radical $-X-R^9$,

where

X represents oxygen or sulphur and

$R^9$ represents in each case straight-chain or branched alkyl having 1 to 8 carbon atoms, alkenyl having 2 to 8 carbon atoms, alkinyl having 3 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 3 to 8 carbon atoms and 1 to 13 identical or different halogen atoms, represents cyanoalkyl, alkoxyalkyl, alkylthioalkyl, halogenoalkoxyalkyl, alkoxyalkoxyalkyl, (bis-alkoxy)alkyl, (bis-alkylthio)alkyl, alkylcarbonylalkyl, alkoxycarbonylalkyl or alkoxyalkoxycarbonylalkyl, each having 1 to 8 carbon atoms in the individual alkyl moieties and, if appropriate, 1 to 9 identical or different halogen atoms, represents cycloalkyl, cycloalkyloxycarbonylalkyl or cycloalkylalkyl, having in each case 3 to 7 carbon atoms in the cycloalkyl moiety and if appropriate 1 to 4 carbon atoms in the straight-chain or branched alkyl moiety and each of which is optionally monosubstituted or polysubstituted by identical or different substituents, suitable substituents in each case being: halogen and in each case straight-chain or branched alkyl or alkoxy, each having 1 to 4 carbon atoms, $R^9$ furthermore represents oxetanylalkyl, tetrahydrofuranylalkyl, tetrahydrofuranylalkyloxycarbonylalkyl or tetrahydropyranylalkyl, each of which has 1 to 3 carbon atoms in the respective alkyl moieties and each of which is optionally substituted by alkyl having 1 to 4 carbon atoms, or $R^9$ represents aralkyl which has 6 to 10 carbon atoms in the aryl moiety and 1 to 4 carbon atoms in the straight-chain or branched alkyl moiety and which is optionally monosubstituted or polysubstituted by identical or different substituents, suitable aryl substituents being:
halogen, cyano, nitro, in each case straight-chain or branched alkyl, alkoxy, alkylthio or alkoxycarbonyl, each having 1 to 4 carbon atoms, or in each case straight-chain or branched halogeno alkyl, halogenoalkoxy or halogenoalkylthio, each having 1 to 4 carbon atoms and 1 to 9 identical or different halogen atoms,

$R^3$ represents hydrogen, represents straight-chain or branched alkyl having 1 to 6 carbon atoms or represents straight-chain or branched halogenoalkyl having 1 to 4 carbon atoms and 1 to 9 identical or different halogen atoms and

$R^4$ represents hydrogen, fluorine, chlorine, bromine, iodine, represents straight-chain or branched alkyl having 1 to 6 carbon atoms or represents straight-chain or branched halogenoalkyl having 1 to 4 carbon atoms and 1 to 9 identical or different halogen atoms, or

$R^3$ and $R^4$ together represent a double-linked alkanediyl radical having 2 to 6 carbon atoms.

**8.** (Thio)phenol derivatives of the formula (X)

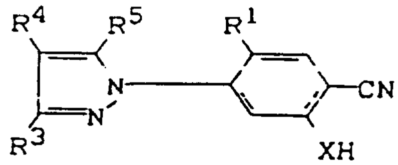

(X)

in which

$R^3$ represents hydrogen, represents straight-chain or branched alkyl having 1 to 6 carbon atoms or represents straight-chain or branched halogenoalkyl having 1 to 4 carbon atoms and 1 to 9 identical or different halogen atoms and

$R^4$ represents hydrogen, fluorine, chlorine, bromine, iodine, represents straight-chain or branched alkyl having 1 to 6 carbon atoms or represents straight-chain or branched halogenoalkyl having 1 to 4 carbon atoms and 1 to 9 identical or different halogen atoms, or

$R^3$ and $R^4$ together represent a double-linked alkanediyl radical having 2 to 6 carbon atoms,

$R^5$ represents hydrogen, fluorine, chlorine, bromine, iodine, represents straight-chain or branched alkyl having 1 to 6 carbon atoms or represents straight-chain or branched halogenoalkyl having 1 to 4 carbon atoms and 1 to 9 identical or different halogen atoms.

**9.** Process for the preparation of N-aryl-pyrazolinones of the formula (IV)

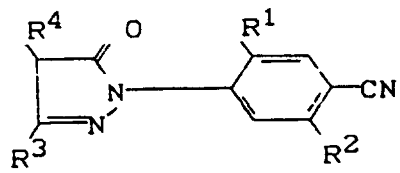

(IV)

in which

$R^1$ represents hydrogen, fluorine, chlorine or bromine and

$R^2$ represents fluorine, chlorine or bromine, or represents a radical -X-$R^9$,

X represents oxygen or sulphur,

$R^9$ represents in each case straight-chain or branched alkyl having 1 to 8 carbon atoms, alkenyl having 2 to 8 carbon atoms, alkinyl having 3 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 3 to 8 carbon atoms and 1 to 13 identical or different halogen atoms, represents cyanoalkyl, alkoxyalkyl, alkylthioalkyl, halogenoalkoxyalkyl, alkoxyalkoxyalkyl, (bis-alkoxy)alkyl, (bis-alkylthio)alkyl, alkylcarbonylalkyl, alkoxycarbonylalkyl or alkoxyalkoxycarbonylalkyl, each having 1 to 8 carbon atoms in the individual alkyl moieties and if appropriate 1 to 9 identical or different halogen atoms, represents cycloalkyl, cycloalkyloxycarbonylalkyl or cycloalkylalkyl, having in each case 3 to 7 carbon atoms in the cycloalkyl moiety and if appropriate 1 to 4 carbon atoms in the straight-chain or branched alkyl moiety and each of which is optionally monosubstituted or polysubstituted by identical or different substituents, suitable substituents in each case being: halogen and in each case straight-chain or branched alkyl or alkoxy, each having 1 to 4 carbon atoms, $R^9$ furthermore represents oxetanylalkyl, tetrahydrofuranylalkyl, tetrahydrofuranylalkyloxycarbonylalkyl or tetrahydropyranylalkyl, each of which has 1 to 3 carbon atoms in the respective alkyl moieties and each of which is optionally substituted by alkyl having 1 to 4 carbon atoms or $R^9$ represents aralkyl which has 6 to 10 carbon atoms in the aryl moiety and 1 to 4 carbon atoms in the straight-chain or branched alkyl moiety and which is optionally monosubstituted or polysubstituted by identical or different substituents,

suitable aryl substituents being:
halogen, cyano, nitro, in each case straight-chain or branched alkyl, alkoxy, alkylthio or alkoxycarbonyl, each having 1 to 4 carbon atoms, or in each case straight-chain or branched halogenoalkyl, halogenoalkoxy or halogenoalkylthio, each having 1 to 4 carbon atoms and 1 to 9 identical or different halogen atoms,

$R^3$ represents hydrogen, represents straight-chain or branched alkyl having 1 to 6 carbon atoms or represents straight-chain or branched halogenoalkyl having 1 to 4 carbon atoms and 1 to 9 identical or different halogen atoms and

$R^4$ represents hydrogen, fluorine, chlorine, bromine, iodine, represents straight-chain or branched alkyl having 1 to 6 carbon atoms or represents straight-chain or branched halogenoalkyl having 1 to 4 carbon atoms and 1 to 9 identical or different halogen atoms, or

$R^3$ and $R^4$ together represent a double-linked alkanediyl radical having 2 to 6 carbon atoms,

characterized in that p-keto esters of the formula (XV)

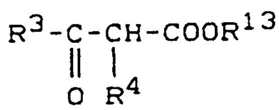

(XV)

in which

$R^{13}$ represents methyl or ethyl and

$R^3$ and $R^4$ have the abovementioned meaning,

are reacted with 4-cyanophenylhydrazines of the formula (II)

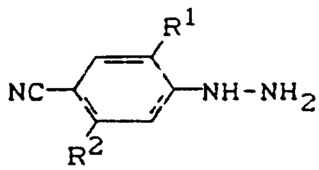

(II)

in which

$R^1$ and $R^2$ have the abovementioned meaning,

if appropriate in the presence of a diluent and if appropriate in the presence of a reaction auxiliary.

**10.** Process for the preparation of (thio)phenol derivatives of the formula (X)

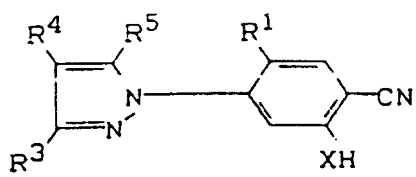

(X)

in which

$R^1$ represents hydrogen, fluorine, chlorine or bromine,

$R^3$ represents hydrogen, represents straight-chain or branched alkyl having 1 to 6 carbon atoms or represents straight-chain or branched halogenoalkyl having 1 to 4 carbon atoms and 1 to 9 identical or different halogen atoms and

$R^4$ represents hydrogen, fluorine, chlorine, bromine, iodine, represents straight-chain or branched alkyl having 1 to 6 carbon atoms or represents straight-chain or branched halogenoalkyl having 1 to 4 carbon atoms and 1 to 9 identical or different halogen atoms, or

$R^3$ and $R^4$ together represent a double-linked alkanediyl radical having 2 to 6 carbon atoms,

$R^5$ represents hydrogen, fluorine, chlorine, bromine, iodine, represents straight-chain or branched alkyl having 1 to 6 carbon atoms or represents straight-chain or branched halogenoalkyl having 1 to 4 carbon atoms and 1 to 9 identical or different halogen atoms,

characterized in that N-aryl-substituted nitrogen-containing heterocycles of the formula (Ik)

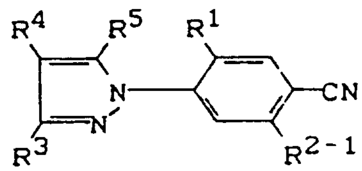

(Ik)

in which

$R^{2-1}$ represents halogen and

$R^1$, $R^3$, $R^4$ and $R^5$ have the abovementioned meaning

are reacted with sodium hydrogen sulphide, if appropriate in the presence of a diluent and if appropriate in the presence of a reaction auxiliary, if appropriate in the presence of a nitrogen or argon protective gas atmosphere or in that

N-aryl-substituted nitrogen-containing heterocycles of the formula (Il)

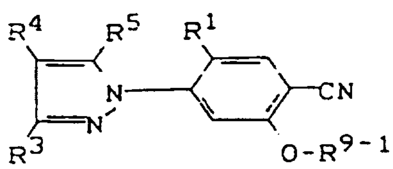

(Il)

in which

$R^1$, $R^3$, $R^4$ and $R^5$ have the abovementioned meaning, and

$R^{9-1}$ represents allyl or represents benzyl,

are reacted with reducing agents in the presence of a hydrogenation catalyst, if appropriate in the presence of a diluent and if appropriate in the presence of a reaction auxiliary.

**11.** 4-Cyanophenylhydrazines of the formula (II)

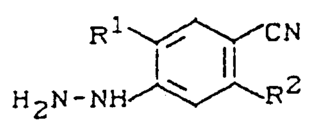

(II)

in which

$R^1$ represents hydrogen, fluorine, chlorine or bromine and

$R^2$ represents fluorine, chlorine or bromine, or a radical -X-$R^9$,

where

$R^9$ represents in each case straight-chain or branched alkyl having 1 to 8 carbon atoms, alkenyl having 2 to 8 carbon atoms, alkinyl having 3 to 8 carbon atoms, halogenoalkyl having 1 to 8 carbon atoms and 1 to 17 identical or different halogen atoms, halogenoalkenyl having 2 to 8 carbon atoms and 1 to 15 identical or different halogen atoms, halogenoalkinyl having 3 to 8 carbon atoms and 1 to 13 identical or different halogen atoms, represents cyanoalkyl, alkoxyalkyl, alkylthioalkyl, halogenoalkoxyalkyl, alkoxyalkoxyalkyl, (bis-alkoxy)alkyl, (bis-alkylthio)alkyl, alkylcarbonylalkyl, alkoxycarbonylalkyl or alkoxyalkoxycarbonylalkyl, each having 1 to 8 carbon atoms in the individual alkyl moieties and if appropriate 1 to 9 identical or different halogen atoms, represents cycloalkyl, cycloalkyloxycarbonylalkyl or cycloalkylalkyl, having in each case 3 to 7 carbon atoms in the cycloalkyl moiety and if appropriate 1 to 4 carbon atoms in the straight-chain or branched alkyl moiety and each of which is optionally monosubstituted or polysubstituted by identical or different substituents, suitable substituents

in each case being: halogen and in each case straight-chain or branched alkyl or alkoxy, each having 1 to 4 carbon atoms, $R^9$ furthermore represents oxetanylalkyl, tetrahydrofuranylalkyl, tetrahydrofuranylalkyloxycarbonylalkyl or tetrahydropyranylalkyl, each of which has 1 to 3 carbon atoms in the respective alkyl moieties and each of which is optionally substituted by alkyl having 1 to 4 carbon atoms or $R^9$ represents aralkyl which has 6 to 10 carbon atoms in the aryl moiety and 1 to 4 carbon atoms in the straight-chain or branched alkyl moiety and which is optionally monosubstituted or polysubstituted by identical or different substituents, suitable aryl substituents being:
halogen, cyano, nitro, in each case straight-chain or branched alkyl, alkoxy, alkylthio or alkoxycarbonyl, each having 1 to 4 carbon atoms, or in each case straight-chain or branched halogenoalkyl, halogenoalkoxy or halogenoalkylthio, each having 1 to 4 carbon atoms and 1 to 9 identical or different halogen atoms, and
X represents oxygen or sulphur.

**12.** Process for the preparation of 4-cyanophenylhydrazines of the formula (II)

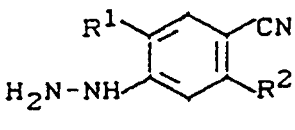
(II)

in which
$R^1$ and $R^2$ have the meanings given in Claim 11
characterized in that 4-fluorobenzonitriles of the formula (XIII)

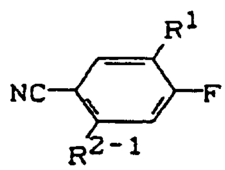
(XIII)

in which
$R^{2-1}$ represents fluorine, chlorine or bromine and
$R^1$ has the meaning given in Claim 11
are reacted with hydrazine hydrate, if appropriate in the presence of a diluent, and the resulting 4-cyano-phenylhydrazines of the formula (IIa)

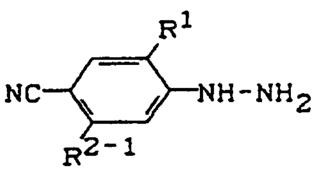
(IIa)

in which
$R^1$ and $R^{2-1}$ have the abovementioned meaning,
are, if appropriate, reacted in a following 2nd step with alcohols or thiols of the formula (IX)

$R^9$-XH (IX)

in which
$R^9$ has the meaning given in Claim 11 and
X represents oxygen or sulphur,
if appropriate in the presence of a diluent and if appropriate in the presence of a reaction auxiliary, or in that 4-cyanophenylhydrazines of the formula (IIb)

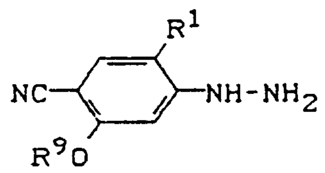
(IIb)

in which

$R^1$ and $R^9$ have the abovementioned meanings

are obtained when 4-aminobenzonitriles of the formula (XIV)

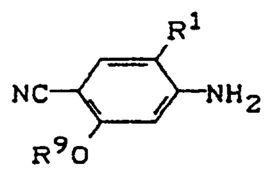
(XIV)

in which

$R^1$ and $R^9$ have the abovementioned meanings,

are initially diazotized with sodium nitrite in the presence of an acid and subsequently reduced with a reducing agent.

## Revendications

**1.** Composés hétérocycliques azotés N-arylés répondant à la formule générale (I)

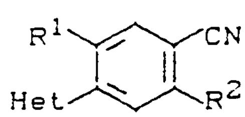
(I)

dans laquelle

Het représente un radical hétérocyclique de formule

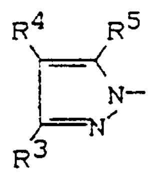
;
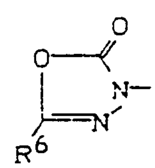
ou
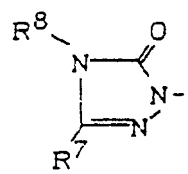

$R^1$ représente un atome d'hydrogène, un atome de fluor, un atome de chlore ou un atome de brome, et

$R^2$ représente un atome de fluor, un atome de chlore ou un atome de brome ou encore un radical -X-$R^9$.

où

$R^3$ représente un atome d'hydrogène, un groupe alkyle à chaîne droite ou ramifiée contenant de 1 à 6 atomes de carbone ou encore un groupe halogénalkyle à chaîne droite ou ramifiée contenant de 1 à 4 atomes de carbone et de 1 à 9 atomes d'halogène identiques ou différents, et

$R^4$ représente un atome d'hydrogène, un atome de fluor, un atome de chlore, un atome de brome, un atome d'iode, un groupe alkyle à chaîne droite ou ramifiée contenant de 1 à 6 atomes de carbone ou encore un groupe halogénalkyle à chaîne droite ou ramifiée contenant de 1 à 4 atomes de carbone et de 1 à 9 atomes d'halogène identiques ou

différents, ou

$R^3$ et $R^4$ représentent ensemble un radical alcanediyle deux fois lié contenant de 2 à 6 atomes de carbone,

$R^5$ représente un atome d'hydrogène, un atome de fluor, un atome de chlore, un atome de brome, un atome d'iode, un groupe alkyle à chaîne droite ou ramifiée contenant de 1 à 6 atomes de carbone ou encore un groupe halogénalkyle à chaîne droite ou ramifiée contenant de 1 à 4 atomes de carbone et de 1 à 9 atomes d'halogène identiques ou différents,

$R^6$ représente un atome d'hydrogène; un groupe alkyle contenant de 1 à 6 atomes de carbone, un groupe alcényle contenant de 3 à 6 atomes de carbone, un groupe alcynyle contenant de 3 à 6 atomes de carbone, un groupe halogénalkyle contenant de 1 à 4 atomes de carbone et de 1 à 9 atomes d'halogène identiques ou différents, un groupe halogénalcényle contenant de 3 à 6 atomes de carbone et de 1 à 5 atomes d'halogène identiques ou différents, un groupe halogénalcynyle contenant de 3 à 6 atomes de carbone et de 1 à 5 atomes d'halogène identiques ou différents ou encore un groupe alcoxyalkyle contenant chacun de 1 à 4 atomes de carbone dans les fractions alkyle individuelles à chaînes droites ou ramifiées, chacun de ces groupes étant à chaîne droite ou ramifiée; ou encore un groupe cycloalkyle contenant de 3 à 7 atomes de carbone éventuellement substitué une ou plusieurs fois de manière identique ou différente, dans lequel comme substituants, on envisage un atome d'halogène, ainsi qu'un groupe alkyle ou un groupe alcoxy contenant chacun de 1 à 4 atomes de carbone, chacun de ces groupes étant à chaîne droite ou ramifiée,

$R^7$ représente un atome d'hydrogène; un groupe alkyle contenant de 1 à 6 atomes de carbone, un groupe alcényle contenant de 3 à 6 atomes de carbone, un groupe alcynyle contenant de 3 à 6 atomes de carbone, un groupe halogénalkyle contenant de 1 à 4 atomes de carbone et de 1 à 9 atomes d'halogène identiques ou différents, un groupe halogénalcényle contenant de 3 à 6 atomes de carbone et de 1 à 5 atomes d'halogène identiques ou différents, un groupe halogénalcynyle contenant de 3 à 6 atomes de carbone et de 1 à 5 atomes d'halogène identiques ou différents ou encore un groupe alcoxyalkyle contenant chacun de 1 à 4 atomes de carbone dans les fractions alkyle individuelles à chaînes droites ou ramifiées, chacun de ces groupes étant à chaîne droite ou ramifiée; ou encore un groupe cycloalkyle contenant de 3 à 7 atomes de carbone éventuellement substitué une ou plusieurs fois de manière identique ou différente, dans lequel comme substituants, on envisage un atome d'halogène, ainsi qu'un groupe alkyle ou un groupe alcoxy contenant chacun de 1 à 4 atomes de carbone, chacun de ces groupes étant à chaîne droite ou ramifiée, et

$R^8$ représente un atome d'hydrogène ou un groupe alkyle contenant de 1 à 6 atomes de carbone, un groupe alcényle contenant de 3 à 6 atomes de carbone, un groupe alcynyle contenant de 3 à 6 atomes de carbone, un groupe halogénalkyle contenant de 1 à 4 atomes de carbone et de 1 à 9 atomes d'halogène identiques ou différents, un groupe halogénalcényle contenant de 3 à 6 atomes de carbone et de 1 à 5 atomes d'halogène identiques ou différents ou un groupe halogénalcynyle contenant de 3 à 6 atomes de carbone et de 1 à 5 atomes d'halogène identiques ou différents, chacun de ces groupes étant à chaîne droite ou ramifiée,

$R^7$ et $R^8$ représentent ensemble un radical alcanediyle deux fois lié contenant de 2 à 6 atomes de carbone,

$R^9$ représente un groupe alkyle contenant de 1 à 8 atomes de carbone, un groupe alcényle contenant de 2 à 8 atomes de carbone, un groupe alcynyle contenant de 3 à 8 atomes de carbone, un groupe halogénalkyle contenant de 1 à 8 atomes de carbone et de 1 à 17 atomes d'halogène identiques ou différents, un groupe halogénalcényle contenant de 2 à 8 atomes de carbone et de 1 à 15 atomes d'halogène identiques ou différents, un groupe halogénalcynyle contenant de 3 à 8 atomes de carbone et de 1 à 13 atomes d'halogène identiques ou différents, chacun de ces groupes étant à chaîne droite ou ramifiée; un groupe cyanalkyle, un groupe alcoxyalkyle, un groupe alkylthioalkyle, un groupe halogénalcoxyalkyle, un groupe alcoxyalcoxyalkyle, un groupe (bis-alcoxy)alkyle, un groupe (bis-alkylthio)alkyle, un groupe alkylcarbonylalkyle, un groupe alcoxycarbonylalkyle ou un groupe alcoxyalcoxycarbonylalkyle contenant chacun de 1 à 8 atomes de carbone dans les fractions alkyle individuelles et éventuellement de 1 à

9 atomes d'halogène identiques ou différents; un groupe cycloalkyle, un groupe cycloalkyloxycarbonylalkyle ou cycloalkylalkyle contenant chacun de 3 à 7 atomes de carbone dans la fraction cycloalkyle et éventuellement de 1 à 4 atomes de carbone dans la fraction alkyle à chaîne droite ou ramifiée, chacun de ces groupes étant éventuellement substitué une ou plusieurs fois de manière identique ou différente, dans lesquels comme substituants, on envisage chaque fois : un atome d'halogène; ainsi qu'un groupe alkyle ou un groupe alcoxy contenant chacun de 1 à 4 atomes de carbone, chacun de ces groupes étant à chaîne droite ou ramifiée; $R^9$ représente, en outre, un groupe oxétanylalkyle, un groupe tétrahydrofuranylalkyle, un groupe tétrahydrofuranylalkyloxycarbonylalkyle ou un groupe tétrahydropyranylalkyle contenant chacun de 1 à 3 atomes de carbone dans les fractions alkyle individuelles, chacun de ces groupes étant éventuellement substitué par un groupe alkyle contenant de 1 à 4 atomes de carbone; ou bien $R^9$ représente un groupe aralkyle contenant de 6 à 10 atomes de carbone dans la fraction aryle et de 1 à 4 atomes de carbone dans la fraction alkyle à chaîne droite ou ramifiée, ce groupe étant éventuellement substitué une ou plusieurs fois de manière identique ou différente, dans lequel, comme substituants du groupe aryle, on envisage : un atome d'halogène, un groupe cyano, un groupe nitro; un groupe alkyle, un groupe alcoxy, un groupe alkylthio ou un groupe alcoxycarbonyle, contenant chacun de 1 à 4 atomes de carbone, chacun de ces groupes étant à chaîne droite ou ramifiée; ou encore un groupe halogénalkyle, un groupe halogénalcoxy ou un groupe halogénalkylthio contenant chacun de 1 à 4 atomes de carbone et de 1 à 9 atomes d'halogène identiques ou différents, chacun de ces groupes étant à chaîne droite ou ramifiée, et

X représente un atome d'oxygène ou un atome de soufre.

**2.** Procédé pour la préparation de composés hétérocycliques azotés N-arylés de formule (I)

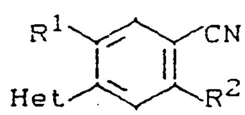

(I)

dans laquelle

Het représente un radical hétérocyclique de formules

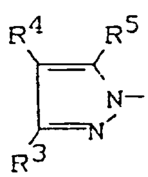

;

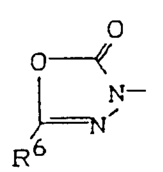

ou

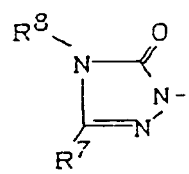

dans lesquelles

$R^1$, $R^2$, $R^3$, $R^4$, $R^5$, $R^6$, $R^7$ et $R^8$ ont la signification , indiquée à la revendication 1,

caractérisé en ce qu'on obtient

(a) des composés hétérocycliques azotés N-arylés de formule (Ia)

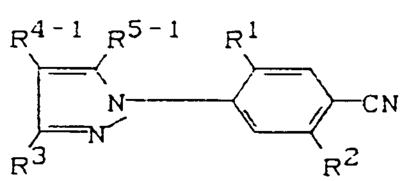

(Ia)

dans laquelle

$R^{4-1}$ représente un atome d'hydrogène; un groupe alkyle à chaîne droite ou ramifiée contenant de 1 à 6 atomes de carbone et de 1 à 9 atomes d'halogène identiques ou différents,

$R^{5-1}$ représente un atome d'hydrogène; un groupe alkyle à chaîne droite ou ramifiée contenant de 1 à 6 atomes de carbone ou encore un groupe halogénalkyle à chaîne droite ou ramifiée contenant de 1 à 4 atomes de carbone et de 1 à 9 atomes d'halogène identiques ou différents, et

$R^1$, $R^2$ et $R^3$ ont la signification indiquée ci-dessus,

lorsqu'on fait réagir des 4-cyanophénylhydrazines de formule (II),

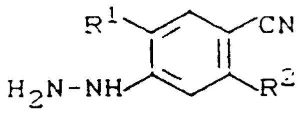

(II)

dans laquelle

$R^1$ et $R^2$ ont la signification indiquée ci-dessus,

avec des 1,3-dicétones de formule (III),

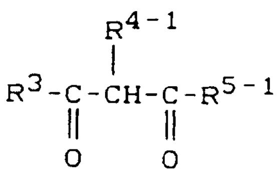

(III)

dans laquelle

$R^3$, $R^{4-1}$ et $R^{5-1}$ ont la signification indiquée ci-dessus,

ou bien avec des dérivés de ces dicétones, comme par exemple des éthers énoliques, des esters énoliques, des cétals, des éthers énoliques-cétals, des énamines ou des $\beta$-halogénovinylcétones, éventuellement en présence d'un diluant et éventuellement en présence d'un adjuvant réactionnel; ou bien en ce qu'on obtient

(b) des composés hétérocycliques azotés N-arylés de formule (Ib)

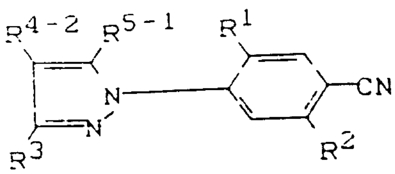

(Ib)

dans laquelle

$R^{4-2}$ représente un atome d'halogène,

$R^{5-1}$ représente un atome d'hydrogène; un groupe alkyle à chaîne droite ou ramifiée contenant de 1 à 6 atomes de carbone ou encore un groupe halogénalkyle à chaîne droite ou ramifiée contenant de 1 à 4 atomes de carbone et de 1 à 9 atomes d'halogène identiques ou différents,

$R^1$, $R^2$ et $R^3$ ont la signification indiquée ci-dessus,

lorsqu'on fait réagir des composés hétérocycliques azotés N-arylés de formule (Ij)

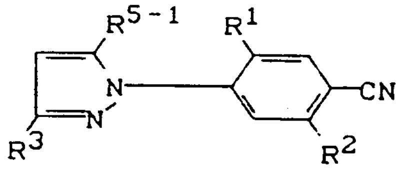 (Ij)

dans laquelle

$R^1$, $R^2$, $R^3$ et $R^{5-1}$ ont la signification indiquée ci-dessus,

avec un agent d'halogénation, éventuellement en présence d'un diluant;

ou bien en ce qu'on obtient

(c) des composés hétérocycliques azotés N-arylés de formule (Ic)

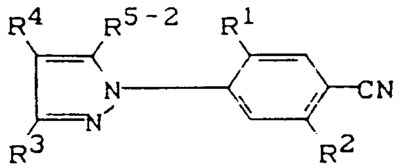 (Ic)

dans laquelle

$R^{5-2}$ représente un atome d'halogène, et

$R^1$, $R^2$, $R^3$ et $R^4$ ont la signification indiquée ci-dessus

lorsqu'on fait réagir des N-aryl-pyrazolinones de formule (IV)

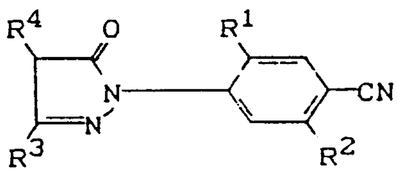 (IV)

dans laquelle

$R^1$, $R^2$, $R^3$ et $R^4$ ont la signification indiquée ci-dessus,

avec un agent d'halogénation, éventuellement en présence d'un diluant et éventuellement en présence d'un adjuvant réactionnel;

ou bien en ce qu'on obtient

(d) des composés hétérocycliques azotés N-arylés de formule (Id)

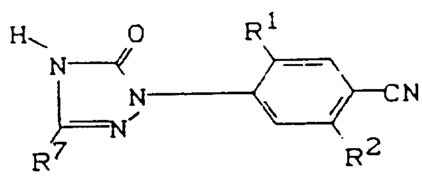 (Id)

dans laquelle

$R^1$, $R^2$ et $R^7$ ont la signification indiquée ci-dessus,

lorsqu'on fait réagir des 4-cyanophénylhydrazines de formule (II)

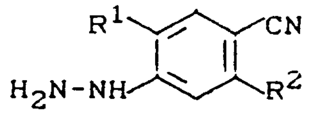

(II)

dans laquelle

$R^1$ et $R^2$ ont la signification indiquée ci-dessus,

avec des esters iminocarboxyliques de formule (V)

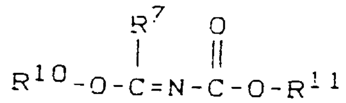

(V)

dans laquelle

$R^{10}$ et $R^{11}$ représentent, chacun indépendamment l'un de l'autre, un groupe alkyle à chaîne droite ou ramifiée contenant de 1 à 4 atomes de carbone, et

$R^7$ a la signification indiquée ci-dessus,

éventuellement en présence d'un diluant,

ou bien en ce qu'on obtient

(e) des composés hétérocycliques azotés N-arylés de formule (Ie)

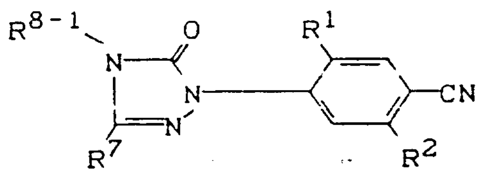

(Ie)

dans laquelle

$R^{8-1}$ représente un groupe alkyle contenant de 1 à 6 atomes de carbone, un groupe alcényle contenant de 3 à 6 atomes de carbone, un groupe alcynyle contenant de 3 à 6 atomes de carbone, un groupe halogénalkyle contenant de 1 à 4 atomes de carbone et de 1 à 9 atomes d'halogène identiques ou différents, un groupe halogénalcényle contenant de 3 à 6 atomes de carbone et de 1 à 5 atomes d'halogène identiques ou différents ou un groupe halogénalcynyle contenant de 3 à 6 atomes de carbone et de 1 à 5 atomes d'halogène identiques ou différents, chacun de ces groupes étant à chaîne droite ou ramifiée, et

$R^1$, $R^2$ et $R^7$ ont la signification indiquée ci-dessus,

lorsqu'on fait réagir des composés hétérocycliques azotés N-arylés de formule (Id)

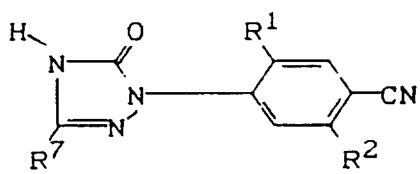

(Id)

dans laquelle

$R^1$, $R^2$ et $R^7$ ont la signification indiquée ci-dessus

avec des agents d'alkylation de formule (VI)

$R^{8-1}$-$E^1$ (VI)

dans laquelle

$R^{8-1}$ a la signification indiquée ci-dessus, et

$E^1$ représente un atome d'halogène,

éventuellement en présence d'un diluant et éventuellement en présence d'un adjuvant réactionnel, ou bien en ce qu'on obtient

(f) des composés hétérocycliques azotés N-arylés de formule (If)

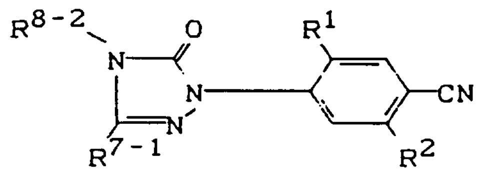

(If)

dans laquelle

$R^{7-1}$ et $R^{8-2}$ représentent ensemble un radical alcanediyle deux fois lié contenant de 2 à 6 atomes de carbone,

lorsqu'on fait réagir des amidrazones de formule (VII)

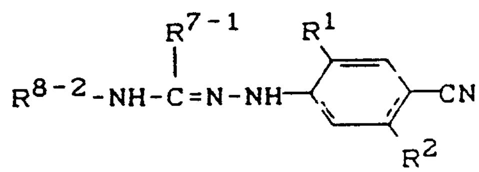

(VII)

dans laquelle

$R^{7-1}$ et $R^{8-2}$ ont la signification indiquée ci-dessus,

avec du phosgène, éventuellement en présence d'un diluant et éventuellement en présence d'un adjuvant réactionnel,

ou bien en ce qu'on obtient

(g) des composés hétérocycliques azotés N-arylés de formule (Ig)

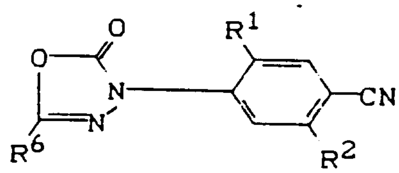

(Ig)

dans laquelle

$R^1$, $R^2$ et $R^6$ ont la signification indiquée ci-dessus,

lorsqu'on fait réagir des phénylhydrazides de formule (VIII)

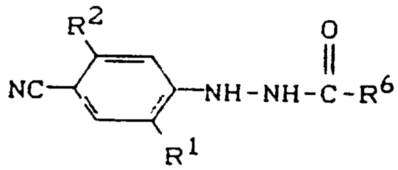

(VIII)

dans laquelle

$R^1$, $R^2$ et $R^6$ ont la signification indiquée ci-dessus,

avec du phosgène, éventuellement en présence d'un diluant et éventuellement en présence d'un adjuvant réactionnel,

ou bien en ce qu'on obtient

(h) des composés hétérocycliques azotés N-arylés de formule (Ih)

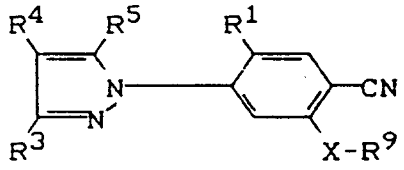

(Ih)

dans laquelle

$R^1$, $R^3$, $R^4$, $R^5$, $R^9$ et X ont la signification indiquée ci-dessus,

lorsqu'on fait réagir

($\alpha$) des composés hétérocycliques azotés N-arylés de formule (Ik)

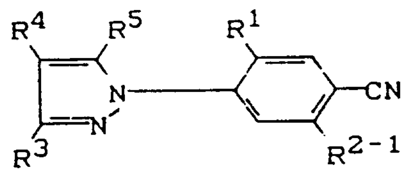

(Ik)

dans laquelle

$R^{2-1}$ représente un atome d'halogène,

$R^1$, $R^3$, $R^4$ et $R^5$ ont la signification indiquée ci-dessus,

avec des alcools ou des thiols de formule (IX)

$R^9$-XH (IX)

dans laquelle

$R^9$ et X ont la signification indiquée ci-dessus,

éventuellement en présence d'un diluant et éventuellement en présence d'un adjuvant réactionnel,

ou bien en ce qu'on fait réagir

($\beta$) des dérivés de (thio)phénols de formule (X),

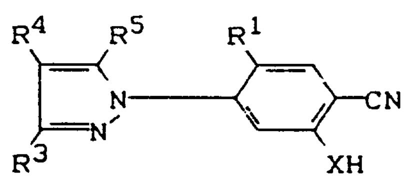

(X)

dans laquelle

$R^1$, $R^3$, $R^4$, $R^5$ et X ont la signification indiquée ci-dessus,

avec des agents d'alkylation ou d'acylation de formule (XI),

$R^9$-$E^2$ (XI)

dans laquelle

$R^9$ a la signification indiquée ci-dessus, et

$E^2$ représente un groupe qui se sépare attirant les électrons,

éventuellement en présence d'un diluant et éventuellement en présence d'un adjuvant réactionnel,

ou bien en ce qu'on obtient

(i) des composés hétérocycliques azotés N-arylés de formule (Ii)

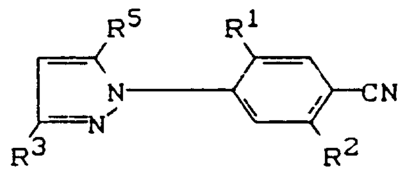

(Ii)

dans laquelle

$R^1$, $R^2$, $R^3$ et $R^5$ ont la signification indiquée ci-dessus,

lorsqu'on saponifie des esters 1-arylpyrazolyl-4-carboxyliques de formule (XII),

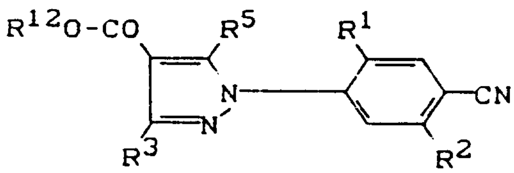

(XII)

dans laquelle

$R^{12}$ représente un groupe alkyle à chaîne droite ou ramifiée contenant de 1 à 4 atomes de carbone, et

$R^1$, $R^2$, $R^3$ et $R^5$ ont la signification indiquée ci-dessus,

en présence d'un catalyseur acide ou basique et éventuellement en présence d'un diluant et ensuite, on les soumet à une décarboxylation par voie thermique.

**3.** Herbicides et agents de régulation de la croissance des plantes, qui se caractérisent par une teneur en au moins un composé hétérocyclique azoté N-arylé de formule (I) selon les revendications 1 et 2.

**4.** Procédé pour lutter contre les mauvaises herbes, caractérisé en ce qu'on laisse agir des composés hétérocycliques azotés N-arylés de formule (I) selon les revendications 1 et 2 sur les mauvaises herbes et/ou sur leur biotope.

**5.** Utilisation de composés hétérocycliques azotés N-arylés de formule (I) selon les revendications 1 et 2 pour lutter contre les mauvaises herbes et/ou comme régulateurs de la croissance des plantes.

**6.** Procédé pour la préparation d'herbicides et/ou d'agents de régulation de la croissance des plantes, caractérisé en ce qu'on mélange des composés hétérocycliques azotés N-arylés de formule (I) selon les revendications 1 et 2 avec des diluants et/ou des substances tensioactives.

**7.** N-arylpyrazolinones de formule (IV),

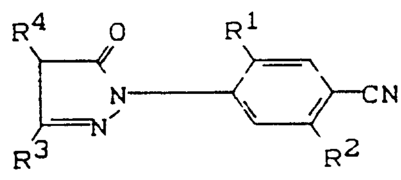

(IV)

dans laquelle

$R^1$ représente un atome d'hydrogène, un atome de fluor, un atome de chlore ou un atome de brome, et

$R^2$ représente un atome de fluor, un atome de chlore ou un atome de brome ou encore un radical -X-$R^9$,

où

X représente un atome d'oxygène ou un atome de soufre, et

$R^9$ représente un groupe alkyle contenant de 1 à 8 atomes de carbone, un groupe alcényle contenant de 2 à 8 atomes de carbone, un groupe alcynyle contenant de 3 à 8 atomes de carbone, un groupe halogénalkyle contenant de 1 à 8 atomes de carbone et de 1 à 17 atomes d'halogène identiques ou différents, un groupe halogénalcényle contenant de 2 à 8 atomes de carbone et de 1 à 15 atomes d'halogène identiques ou différents, un groupe halogénalcynyle contenant de 3 à 8 atomes de carbone et de 1 à 13 atomes d'halogène identiques ou différents, chacun de ces groupes étant à chaîne droite ou ramifiée; un groupe cyanalkyle, un groupe alcoxyalkyle, un groupe alkylthioalkyle, un groupe halogénalcoxyalkyle, un groupe alcoxyalcoxyalkyle, un groupe (bis-alcoxy)-alkyle, un groupe (bis-alkylthio)alkyle, un groupe alkylcarbonylalkyle, un groupe alcoxycarbonylalkyle ou un groupe alcoxyalcoxycarbonylalkyle contenant chacun de 1 à 8 atomes de carbone dans les fractions alkyle individuelles et éventuellement de 1 à 9 atomes d'halogène identiques ou différents; un groupe cycloalkyle, un groupe cycloalkyloxycarbonylalkyle ou cycloalkylalkyle contenant chacun de 3 à 7 atomes de carbone dans la fraction cycloalkyle et éventuellement de 1 à 4 atomes de carbone dans la fraction alkyle à chaîne droite ou ramifiée, chacun de ces groupes étant éventuellement substitué une ou plusieurs fois de manière identique ou différente, dans lesquels comme substituants, on envisage chaque fois : un atome d'halogène; ainsi qu'un groupe alkyle ou un groupe alcoxy contenant chacun de 1 à 4 atomes de carbone, chacun de ces groupes étant à chaîne droite ou ramifiée; $R^9$ représente, en outre, un groupe oxétanylalkyle, un groupe tétrahydrofuranylalkyle, un groupe tétrahydrofuranylal-kyloxycarbonylalkyle ou un groupe tétrahydropyranylalkyle contenant chacun de 1 à 3 atomes de carbone dans les fractions alkyle individuelles, chacun de ces groupes étant éventuellement substitué par un groupe alkyle contenant de 1 à 4 atomes de carbone; ou bien $R^9$ représente un groupe aralkyle contenant de 6 à 10 atomes de carbone dans la fraction aryle et de 1 à 4 atomes de carbone dans la fraction alkyle à chaîne droite ou ramifiée, ce groupe étant éventuellement substitué une ou plusieurs fois de manière identique ou différente, dans lequel, comme substituants du groupe aryle, on envisage : un atome d'halogène, un groupe cyano, un groupe nitro; un groupe alkyle, un groupe alcoxy, un groupe alkylthio ou un groupe alcoxycarbonyle, contenant chacun de 1 à 4 atomes de carbone, chacun de ces groupes étant à chaîne droite ou ramifiée; ou encore un groupe halogénalkyle, un groupe halogénalcoxy ou un groupe halogénalkylthio contenant chacun des 1 à 4 atomes de carbone et de 1 à 9 atomes d'halogène identiques ou différents, chacun de ces groupes étant à chaîne droite ou ramifiée, et

$R^3$ représente un atome d'hydrogène, un groupe alkyle à chaîne droite ou ramifiée contenant de 1 à 6 atomes de carbone ou encore un groupe halogénalkyle à chaîne droite ou ramifiée contenant de 1 à 4 atomes de carbone et de 1 à 9 atomes d'halogène identiques ou différents, et

$R^4$ représente un atome d'hydrogène, un atome de fluor, un atome de chlore, un atome de brome, un atome d'iode, un groupe alkyle à chaîne droite ou ramifiée contenant de 1 à 6 atomes de carbone ou encore un groupe halogénalkyle à chaîne droite ou ramifiée contenant de 1 à 4 atomes de carbone et de 1 à 9 atomes d'halogène identiques ou différents, ou

$R^3$ et $R^4$ représentent ensemble un radical alcanediyle deux fois lié contenant de 2 à 6 atomes de carbone.

**8.** Dérivés de (thio)phénols de formule (X),

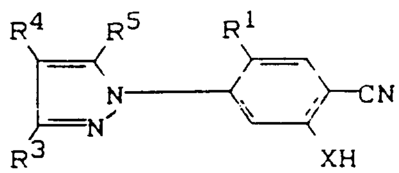

(X)

dans laquelle

| | |
|---|---|
| $R^3$ | représente un atome d'hydrogène, un groupe alkyle à chaîne droite ou ramifiée contenant de 1 à 6 atomes de carbone ou encore un groupe halogénalkyle à chaîne droite ou ramifiée contenant de 1 à 4 atomes de carbone et de 1 à 9 atomes d'halogène identiques ou différents, et |
| $R^4$ | représente un atome d'hydrogène, un atome de fluor, un atome de chlore, un atome de brome, un atome d'iode, un groupe alkyle à chaîne droite ou ramifiée contenant de 1 à 6 atomes de carbone ou encore un groupe halogénalkyle à chaîne droite ou ramifiée contenant de 1 à 4 atomes de carbone et de 1 à 9 atomes d'halogène identiques ou différents, ou |
| $R^3$ et $R^4$ | représentent ensemble un radical alcanediyle deux fois lié contenant de 2 à 6 atomes de carbone, |
| $R^5$ | représente un atome d'hydrogène, un atome de fluor, un atome de chlore, un atome de brome, un atome d'iode, un groupe alkyle à chaîne droite ou ramifiée contenant de 1 à 6 atomes de carbone ou encore un groupe halogénalkyle à chaîne droite ou ramifiée contenant de 1 à 4 atomes de carbone et de 1 à 9 atomes d'halogène identiques ou différents. |

**9.** Procédé pour la préparation de N-aryl-pyrazolinones de formule (IV)

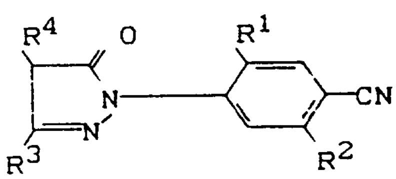

(IV)

dans laquelle

| | |
|---|---|
| $R^1$ | représente un atome d'hydrogène, un atome de fluor, un atome de chlore ou un atome de brome, et |
| $R^2$ | représente un atome de fluor, un atome de chlore ou un atome de brome ou encore un radical $-X-R^9$, |
| X | représente un atome d'oxygène ou un atome de soufre, |
| $R^9$ | représente un groupe alkyle contenant de 1 à 8 atomes de carbone, un groupe alcényle contenant de 2 à 8 atomes de carbone, un groupe alcynyle contenant de 3 à 8 atomes de carbone, un groupe halogénalkyle contenant de 1 à 8 atomes de carbone et de 1 à 17 atomes d'halogène identiques ou différents, un groupe halogénalcényle contenant de 2 à 8 atomes de carbone et de 1 à 15 atomes d'halogène identiques ou différents, un groupe halogénalcynyle contenant de 3 à 8 atomes de carbone et de 1 à 13 atomes d'halogène identiques ou différents, chacun de ces groupes étant à chaîne droite ou ramifiée; un groupe cyanalkyle, un groupe alcoxyalkyle, un groupe alkylthioalkyle, un groupe halogénalcoxyalkyle, un groupe alcoxyalcoxyalkyle, un groupe (bis-alcoxy)-alkyle, un groupe (bis-alkylthio)alkyle, un groupe alkylcarbonylalkyle, un groupe alcoxycarbonylalkyle ou un groupe alcoxyalcoxycarbonylalkyle contenant chacun de 1 à 8 atomes de carbone dans les fractions alkyle individuelles et éventuellement de 1 à 9 atomes d'halogène identiques ou différents; un groupe cycloalkyle, un groupe cycloalkyloxycarbonylalkyle ou cycloalkylalkyle contenant chacun de 3 à 7 atomes de carbone |

dans la fraction cycloalkyle et éventuellement de 1 à 4 atomes de carbone dans la fraction alkyle à chaîne droite ou ramifiée, chacun de ces groupes étant éventuellement substitué une ou plusieurs fois de manière identique ou différente, dans lesquels comme substituants, on envisage chaque fois : un atome d'halogène; ainsi qu'un groupe alkyle ou un groupe alcoxy contenant chacun de 1 à 4 atomes de carbone, chacun de ces groupes étant à chaîne droite ou ramifiée; $R^9$ représente, en outre, un groupe oxétanylalkyle, un groupe tétrahydrofuranylalkyle, un groupe tétrahydrofuranylalkyloxycarbonylalkyle ou un groupe tétrahydropyranylalkyle contenant chacun de 1 à 3 atomes de carbone dans les fractions alkyle individuelles, chacun de ces groupes étant éventuellement substitué par un groupe alkyle contenant de 1 à 4 atomes de carbone; ou bien $R^9$ représente un groupe aralkyle contenant de 6 à 10 atomes de carbone dans la fraction aryle et de 1 à 4 atomes de carbone dans la fraction alkyle à chaîne droite ou ramifiée, ce groupe étant éventuellement substitué une ou plusieurs fois de manière identique ou différente, dans lequel, comme substituants du groupe aryle, on envisage : un atome d'halogène, un groupe cyano, un groupe nitro; un groupe alkyle, un groupe alcoxy, un groupe alkylthio ou un groupe alcoxycarbonyle, contenant chacun de 1 à 4 atomes de carbone, chacun de ces groupes étant à chaîne droite ou ramifiée; ou encore un groupe halogénalkyle, un groupe halogénalcoxy ou un groupe halogénalkylthio contenant chacun de 1 à 4 atomes de carbone et de 1 à 9 atomes d'halogène identiques ou différents, chacun de ces groupes étant à chaîne droite ou ramifiée,

$R^3$ représente un atome d'hydrogène, un groupe alkyle à chaîne droite ou ramifiée contenant de 1 à 6 atomes de carbone ou encore un groupe halogénalkyle à chaîne droite ou ramifiée contenant de 1 à 4 atomes de carbone et de 1 à 9 atomes d'halogène identiques ou différents, et

$R^4$ représente un atome d'hydrogène, un atome de fluor, un atome de chlore, un atome de brome, un atome d'iode, un groupe alkyle à chaîne droite ou ramifiée contenant de 1 à 6 atomes de carbone ou encore un groupe halogénalkyle à chaîne droite ou ramifiée contenant de 1 à 4 atomes de carbone et de 1 à 9 atomes d'halogène identiques ou différents, ou

$R^3$ et $R^4$ représentent ensemble un radical alcanediyle deux fois lié contenant de 2 à 6 atomes de carbone,

caractérisé en ce qu'on fait réagir des esters p-cétoniques de formule (XV)

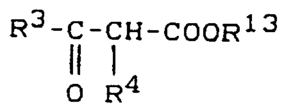

(XV)

dans laquelle

$R^{13}$ représente un groupe méthyle ou un groupe éthyle, et

$R^3$ et $R^4$ ont la signification indiquée ci-dessus,

avec des 4-cyanophénylhydrazines de formule (II)

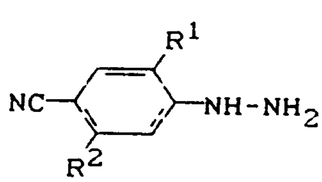

(II)

dans laquelle

$R^1$ et $R^2$ ont la signification indiquée ci-dessus,

éventuellement en présence d'un diluant et éventuellement en présence d'un adjuvant réactionnel.

**10.** Procédé pour la préparation de dérivés de (thio)phénols de formule (X),

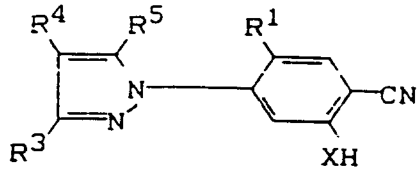

(X)

dans laquelle

- $R^1$ représente un atome d'hydrogène, un atome de fluor, un atome de chlore ou un atome de brome,
- $R^3$ représente un atome d'hydrogène, un groupe alkyle à chaîne droite ou ramifiée contenant de 1 à 6 atomes de carbone ou encore un groupe halogénalkyle à chaîne droite ou ramifiée contenant de 1 à 4 atomes de carbone et de 1 à 9 atomes d'halogène identiques ou différents, et
- $R^4$ représente un atome d'hydrogène, un atome de fluor, un atome de chlore, un atome de brome, un atome d'iode, un groupe alkyle à chaîne droite ou ramifiée contenant de 1 à 6 atomes de carbone ou encore un groupe halogénalkyle à chaîne droite ou ramifiée contenant de 1 à 4 atomes de carbone et de 1 à 9 atomes d'halogène identiques ou différents, ou
- $R^3$ et $R^4$ représentent ensemble un radical alcanediyle deux fois lié contenant de 2 à 6 atomes de carbone,
- $R^5$ représente un atome d'hydrogène, un atome de fluor, un atome de chlore, un atome de brome, un atome d'iode, un groupe alkyle à chaîne droite ou ramifiée contenant de 1 à 6 atomes de carbone ou encore un groupe halogénalkyle à chaîne droite ou ramifiée contenant de 1 à 4 atomes de carbone et de 1 à 9 atomes d'halogène identiques ou différents,

caractérisé en ce qu'on fait réagir des composés hétérocycliques azotés N-arylés de formule (Ik)

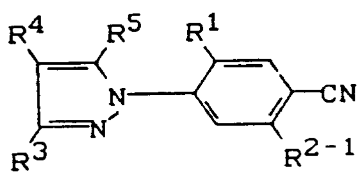

(Ik)

dans laquelle

- $R^{2-1}$ représente un atome d'halogène et
- $R^1$, $R^3$, $R^4$ et $R^5$ ont la signification indiquée ci-dessus,

avec de l'hydrogénosulfure de sodium, éventuellement en présence d'un diluant et éventuellement en présence d'un adjuvant réactionnel, éventuellement en présence d'une atmosphère de gaz protecteur d'azote ou d'argon,

ou bien en ce qu'on fait réagir des composés hétérocycliques azotés N-arylés de formule (Il)

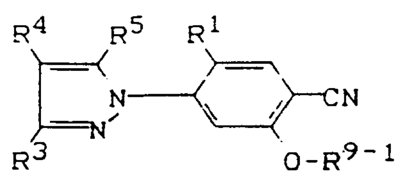

(Il)

dans laquelle

- $R^1$, $R^3$, $R^4$ et $R^5$ ont la signification indiquée ci-dessus et
- $R^{9-1}$ représente un groupe allyle ou un groupe benzyle,

avec des agents de réduction en présence d'un catalyseur d'hydrogénation, éventuellement en présence d'un diluant et éventuellement en présence d'un adjuvant réactionnel.

**11.** 4-cyanophénylhydrazines de formule (II)

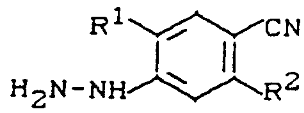

(II)

dans laquelle

$R^1$ représente un atome d'hydrogène, un atome de fluor, un atome de chlore ou un atome de brome, et

$R^2$ représente un atome de fluor, un atome de chlore ou un atome de brome ou encore un radical -X-$R^9$,

où

$R^9$ représente un groupe alkyle contenant de 1 à 8 atomes de carbone, un groupe alcényle contenant de 2 à 8 atomes de carbone, un groupe alcynyle contenant de 3 à 8 atomes de carbone, un groupe halogénalkyle contenant de 1 à 8 atomes de carbone et de 1 à 17 atomes d'halogène identiques ou différents, un groupe halogénalcényle contenant de 2 à 8 atomes de carbone et de 1 à 15 atomes d'halogène identiques ou différents, un groupe halogénalcynyle contenant de 3 à 8 atomes de carbone et de 1 à 13 atomes d'halogène identiques ou différents, chacun de ces groupes étant à chaîne droite ou ramifiée; un groupe cyanalkyle, un groupe alcoxyalkyle, un groupe alkylthioalkyle, un groupe halogénalcoxyalkyle, un groupe alcoxyalcoxyalkyle, un groupe (bis-alcoxy)alkyle, un groupe (bis-alkyl-thio)alkyle, un groupe alkylcarbonylalkyle, un groupe alcoxycarbonylalkyle ou un groupe alcoxyalcoxycarbonylalkyle contenant chacun de 1 à 8 atomes de carbone dans les fractions alkyle individuelles et éventuellement de 1 à 9 atomes d'halogène identiques ou différents; un groupe cycloalkyle, un groupe cycloalkyloxycarbonylalkyle ou cycloalkylalkyle contenant chacun de 3 à 7 atomes de carbone dans la fraction cycloalkyle et éventuellement de 1 à 4 atomes de carbone dans la fraction alkyle à chaîne droite ou ramifiée, chacun de ces groupes étant éventuellement substitué une ou plusieurs fois de manière identique ou différente, dans lesquels comme substituants, on envisage chaque fois : un atome d'halogène; ainsi qu'un groupe alkyle ou un groupe alcoxy contenant chacun de 1 à 4 atomes de carbone, chacun de ces groupes étant à chaîne droite ou ramifiée; $R^9$ représente, en outre, un groupe oxétanylalkyle, un groupe tétrahydrofuranylalkyle, un groupe tétrahydrofuranylalkyloxycarbonylalkyle ou un groupe tétrahydropyranylalkyle contenant chacun de 1 à 3 atomes de carbone dans les fractions alkyle individuelles, chacun de ces groupes étant éventuellement substitué par un groupe alkyle contenant de 1 à 4 atomes de carbone; ou bien $R^9$ représente un groupe aralkyle contenant de 6 à 10 atomes de carbone dans la fraction aryle et de 1 à 4 atomes de carbone dans la fraction alkyle à chaîne droite ou ramifiée, ce groupe étant éventuellement substitué une ou plusieurs fois de manière identique ou différente, dans lequel, comme substituants du groupe aryle, on envisage : un atome d'halogène, un groupe cyano, un groupe nitro; un groupe alkyle, un groupe alcoxy, un groupe alkylthio ou un groupe alcoxycarbonyle, contenant chacun de 1 à 4 atomes de carbone, chacun de ces groupes étant à chaîne droite ou ramifiée; ou encore un groupe halogénalkyle, un groupe halogénalcoxy ou un groupe halogénalkylthio contenant chacun de 1 à 4 atomes de carbone et de 1 à 9 atomes d'halogène identiques ou différents, chacun de ces groupes étant à chaîne droite ou ramifiée, et

X représente un atome d'oxygène ou un atome de soufre.

**12.** Procédé pour la préparation de 4-cyanophénylhydrazines de formule (II)

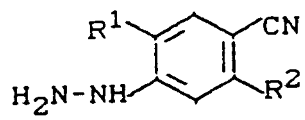

(II)

dans laquelle
$R^1$ et $R^2$ ont les significations mentionnées à la revendication 11,
caractérisé en ce qu'on fait réagir des 4-fluorobenzonitriles de formule (XIII)

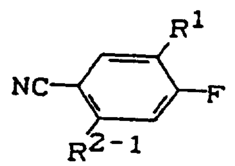

(XIII)

dans laquelle
$R^{2-1}$ représente un atome de fluor, un atome de chlore ou un atome de brome, et
$R^1$ a la significations indiquée à la revendication 11,
avec de l'hydrate d'hydrazine, éventuellement en présence d'un diluant et on fait éventuellement réagir les 4-cyanophénylhydrazines ainsi obtenues de formule (IIa)

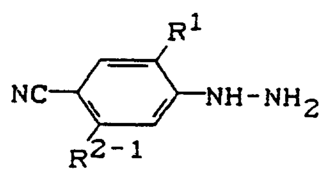

(IIa)

dans laquelle
$R^1$ et $R^{2-1}$ ont la signification indiquée ci-dessus,
dans une seconde étape avec des alcools ou des thiols de formule (IX),

$R^9$-XH (IX)

dans laquelle
$R^9$ a la signification mentionnée à la revendication 11, et
X représente un atome d'oxygène ou un atome de soufre,
éventuellement en présence d'un diluant et éventuellement en présence d'un adjuvant réactionnel,
ou bien en ce qu'on soumet des 4-cyanophénylhydrazines de formule (IIb)

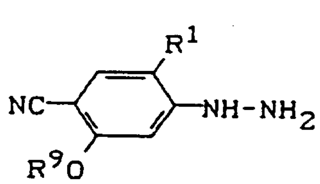

(IIb)

dans laquelle
$R^1$ et $R^9$ ont les significations indiquées ci-dessus
d'abord à une diazotation avec des 4-aminobenzonitriles de formule (XIV)

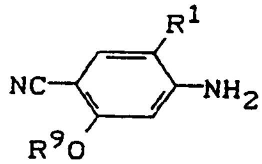

(XIV)

dans laquelle

$R^1$ et $R^9$ ont les significations indiquées ci-dessus,

avec du nitrite de sodium en présence d'un acide et ensuite à une réduction avec un agent de réduction.